(19) Europäisches Patentamt
European Patent Office
Office européen des brevets

(11) EP 4 168 068 B1

# (12) EUROPEAN PATENT SPECIFICATION

(45) Date of publication and mention of the grant of the patent:
**22.04.2026 Bulletin 2026/17**

(21) Application number: **21742977.8**

(22) Date of filing: **22.06.2021**

(51) International Patent Classification (IPC):
***A61M 5/31*** *(2006.01)* ***A61J 1/14*** *(2023.01)*
***C23C 16/00*** *(2006.01)* ***A61M 5/00*** *(2006.01)*
***A61M 5/28*** *(2006.01)* ***A61M 5/315*** *(2006.01)*
***A61M 5/32*** *(2006.01)* ***C23C 16/04*** *(2006.01)*
***C23C 16/40*** *(2006.01)* ***C23C 16/455*** *(2006.01)*
***C23C 16/505*** *(2006.01)* ***F03G 7/00*** *(2006.01)*

(52) Cooperative Patent Classification (CPC):
**A61M 5/3129; A61J 1/1468; A61M 5/001; A61M 5/002; A61M 5/008; A61M 5/28; A61M 5/284; A61M 5/31511; A61M 5/31513; A61M 5/31596; A61M 5/3202; C23C 16/045; C23C 16/401; C23C 16/403; C23C 16/45525;**
(Cont.)

(86) International application number:
**PCT/US2021/038548**

(87) International publication number:
**WO 2021/262764 (30.12.2021 Gazette 2021/52)**

## (54) ATOMIC LAYER DEPOSITION COATED PHARMACEUTICAL PACKAGING AND IMPROVED SYRINGES AND VIALS, E.G. FOR LYOPHILIZED/COLD-CHAIN DRUGS/VACCINES

MIT ATOMLAGENABSCHEIDUNG BESCHICHTETE PHARMAZEUTISCHE VERPACKUNG UND VERBESSERTE SPRITZEN UND PHIOLEN, Z. B. FÜR LYOPHILISIERTE/KALTKETTIGE ARZNEIMITTEL/IMPFSTOFFE

EMBALLAGE PHARMACEUTIQUE DÉPOSÉ PAR DÉPÔT DE COUCHE ATOMIQUE ET SERINGUES ET FLACONS AMÉLIORÉS, PAR EXEMPLE POUR DES MÉDICAMENTS/VACCINS LYOPHILISÉS/SOUMIS À LA CHAÎNE DU FROID

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:
**22.06.2020 US 202063042545 P**
**18.09.2020 US 202063080675 P**
**03.11.2020 US 202063109232 P**
**13.11.2020 US 202063113808 P**
**31.03.2021 US 202163168580 P**

(43) Date of publication of application:
**26.04.2023 Bulletin 2023/17**

(73) Proprietor: **SiO2 Medical Products, Inc.**
**Auburn, AL 36832 (US)**

(72) Inventors:
- **TAHA, Ahmad**
  **Auburn, Alabama 36832 (US)**
- **ABRAMS, Robert S.**
  **Auburn, Alabama 36832 (US)**
- **BREELAND, Adam**
  **Auburn, Alabama 36832 (US)**
- **FERRER, Javier**
  **Auburn, Alabama 36832 (US)**

(74) Representative: **Maiwald GmbH**
**Engineering**
**Elisenhof**
**Elisenstrasse 3**
**80335 München (DE)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(56) References cited:
**EP-A1- 3 437 743** **US-A1- 2014 251 859**
**US-A1- 2015 290 080** **US-A1- 2015 335 823**

(52) Cooperative Patent Classification (CPC): (Cont.)
**C23C 16/505; F03G 7/015;** A61M 2005/3104;
A61M 2005/312; A61M 2005/3123;
A61M 2005/3131; A61M 2205/0238

## Description

### FIELD OF THE INVENTION

The present disclosure relates to the technical field of barrier coated surfaces, for example interior surfaces of pharmaceutical packages or other vessels for storing or other contact with fluids. Examples of suitable fluids include foods, nutritional supplements, drugs, inhalation anaesthetics, diagnostic test materials, biologically active compounds, or body fluids, for example blood. The present disclosure also relates to a pharmaceutical package or other vessel and to a method for making a pharmaceutical package with a pH protective coating or layer between the contents and the barrier coating or layer. The present disclosure also relates more generally to medical articles, including articles other than packages or vessels, for example catheters.

The present disclosure also relates to improved methods for processing pharmaceutical packages or other vessels, for example multiple identical pharmaceutical packages or other vessels used for pharmaceutical preparation storage and delivery, venipuncture and other medical sample collection, and other purposes.

### BACKGROUND OF THE INVENTION

One important consideration in manufacturing pharmaceutical packages or other vessels for storing or other contact with fluids, for example vials and pre-filled syringes, is that the contents of the pharmaceutical package or other vessel desirably will have a substantial shelf life. During this shelf life, it is important to isolate the material filling the pharmaceutical package or other vessel from the vessel wall containing it, or from barrier layers or other functional layers applied to the pharmaceutical package or other vessel wall to avoid leaching material from the pharmaceutical package or other vessel wall, barrier layer, or other functional layers into the prefilled contents or vice versa.

The traditional glass pharmaceutical packages or other vessels are prone to breakage or degradation during manufacture, filling operations, shipping, and use, which means that glass particulates may enter the drug. The presence of glass particles has led to many FDA Warning Letters and to product recalls.

As a result, some companies have turned to plastic pharmaceutical packages or other vessels, which provide greater dimensional tolerance and less breakage than glass, but its use for primary pharmaceutical packaging remains limited due to its gas permeability: Plastic allows small molecule gases such as oxygen to permeate into (or out of) the article. In addition to oxygen, many plastic materials also allow moisture, i.e. water vapor, to permeate into (or out of) the article. The permeability of plastics to gases, such as oxygen and water vapor, is significantly greater than that of glass and, in many cases (as with oxygen-sensitive drugs such as epinephrine), plastics have been unacceptable for that reason.

The problem of gas permeability has been addressed by using specialty resins (for example Cyclic Olefin Polymer ("COP") or Cyclic Olefin Copolymer ("COC")) and by adding an oxygen barrier coating or layer to the plastic pharmaceutical package where it contacts fluid contents of the package. One such **oxygen** barrier layer is a very thin coating of $SiO_x$, as defined below, applied by plasma enhanced chemical vapor deposition. The COP and COC specialty resins have been utilized due to their water vapor barrier properties. This is because, in contrast to oxygen barrier properties which can be provided by the PECVD of a thin coating of SiOx, it is not known how to apply a suitable, e.g. thin yet effective and safe for use in a pharmaceutical package, water vapor barrier coating by PECVD. The inability to provide a suitable water vapor barrier coating by PECVD has necessitated the use of specialty resins such as COP and COC.

It has been shown that vessels made from specialty resins such as COP and COC may be provided with adequate properties for some applications. The properties include gas barrier properties that are protected from dissolution by the aqueous contents of the package, water vapor barrier properties (which are a property of the COP and COC resins), low levels of organic and inorganic extractables, and low levels of visible and subvisible particles (meeting the requirements of USP 789 - ophthalmic), among others. It would be desirable to be able to obtain similar or the same properties using commodity resins, which are much less expensive to produce. However, in contrast to the COP and COC specialty resins that have been successfully coated by PECVD processes to produce the desired attributes, commodity resins have a number of drawbacks.

Most notably, vessels produced from commodity resins allow for a significantly higher degree of water vapor transmission than the COP and COC specialty resins.

Additionally, in at least some instances, vessels produced from commodity resins have a significantly higher degree of surface roughness. Because coatings are deposited relatively quickly using a PECVD process, the surface roughness of the plastic surface on which the coatings are deposited may cause defects in the coatings, which render them unsuitable. Thus, the previously-described PECVD coating processes may not be suitable for obtaining a vessel made from a commodity resin and having the desired properties (including gas, e.g. oxygen and water vapor, barrier properties that are protected from dissolution by the aqueous contents of the package).

The storage of lyophilized or cold-chain drugs typically involves the use of very low temperatures and/or temperature changes such as where the drug primary package is brought up to room temperature before administration.

Glass vials and syringes are subject to cracking or breaking under such thermal stresses. Even conventional plastic vials and syringes may be subject to the loss of container closure integrity (CCI) under significant thermal stresses. Moreover, conventional plastic vials and syringes do not provide the barrier properties required by many lyophilized or cold-chain drugs.

Examples of a drug primary package may be known from US 2015/290080 A1, D2 US 2014/251859 A1, D3 US 2015/335823 A1 and D4 EP 3 437 743 A1.

## SUMMARY OF THE INVENTION

The invention is defined in the drug primary package of claim 1. Further aspects and preferred embodiments are defined in the dependent claims. Aspects, embodiments and examples of the present disclosure which do not fall under the scope of the appended claims do not form part of the invention and are merely provided for illustrative purposes.

An aspect of the invention is a vessel having a lumen defined at least in part by a wall, the wall having an interior surface facing the lumen, an outer surface, and a coating set on the interior surface comprising an optional tie coating or layer, a barrier coating or layer, such as a barrier coating comprising an oxygen barrier layer and/or a water vapor barrier layer, and a pH protective coating or layer, optionally in which one or more layers of the barrier coating consists essentially of a plurality of atomic monolayers of a pure element or compound such as may be applied by atomic layer deposition (ALD).

The tie coating or layer, if present, can comprise $SiO_xC_y$ or $Si(NH)_xC_y$. In either formulation, x is from about 0.5 to about 2.4 and y is from about 0.6 to about 3. The tie coating or layer has an interior surface facing the lumen and an outer surface facing the wall interior surface.

The barrier coating or layer can comprise $SiO_x$, wherein x is from 1.5 to 2.9. Alternatively or additionally, the barrier coating or layer can comprise one or more metals or metal oxides, such as $Al_2O_3$, or combinations thereof. The barrier layer can be from 2 to 1000 nm thick. It can have an interior surface facing the lumen and an outer surface facing the interior surface of the tie coating or layer. The barrier coating or layer optionally is effective to reduce the ingress of atmospheric gas into the lumen compared to a vessel without a barrier coating or layer. In some embodiments, the barrier coating or layer can comprise both one or more layers of $SiO_x$, wherein x is from 1.5 to 2.9, and one or more layers of of metal or metal oxide, such as $Al_2O_3$. The $SiO_x$ coating or layer may be effective to reduce the ingress of oxygen into the lumen compared to a vessel without a barrier coating or layer and the $Al_2O_3$ layer may be effective to reduce the ingress of water vapor (i.e. moisture) into the lumen compared to a vessel without a barrier coating or layer. The barrier coating or part of the barrier layer coating may comprise or consist of a plurality of monolayers of SiOx and/or a plurality of monolayers of aluminum oxide, each of which may be prepared by atomic layer deposition.

In any embodiment, the barrier coating may comprise at least one atomic monolayer of $Al_2O_3$, $Al_xTi_yO_z$, $HfO_2$, $In_2O_3$, MgO, $SiO_2$, $SrTiO_x$, $Ta_2O_5$, $TiO_2$, $Y_2O_3$, ZnO, ZnO:Al, $ZrO_2$, $La_2O_3$, $CeO_2$, AlN, TiAlCN, TiN, $TaN_x$, Ir, Pd, Pt, Si, Al, or Ru. In any embodiment, the barrier coating may comprise at least one atomic monolayer of $Al_2O_3$ or ZnO. In any embodiment, the barrier coating may comprise at least one atomic monolayer of $SiO_2$.

In any embodiment, the oxygen barrier coating or layer may be effective to reduce the ingress of oxygen into the lumen to less than 0.0005 cc/package/day at 25 °C, 60% relative humidity, and 0.21 bar, optionally less than 0.0004 cc/package/day at 25 °C, 60% relative humidity, and 0.21 bar, optionally less than 0.0003 cc/package/day at 25 °C, 60% relative humidity, and 0.21 bar, optionally less than 0.0002 cc/package/day at 25 °C, 60% relative humidity, and 0.21 bar, optionally less than 0.0001 cc/package/day at 25 °C, 60% relative humidity, and 0.21 bar.

In any embodiment, the water vapor barrier coating or layer may be effective to reduce the ingress of water vapor into the lumen to less than 0.05 mg/package/day at 60 °C and 40% relative humidity, optionally less than 0.04 mg/package/day at 60 °C and 40% relative humidity, optionally less than 0.03 mg/package/day at 60 °C and 40% relative humidity, optionally less than 0.02 mg/package/day at 60 °C and 40% relative humidity, optionally less than 0.01 mg/package/day at 60 °C and 40% relative humidity.

In any embodiment, one or more layers of the barrier coating may be supported on the interior surface of the wall. In some embodiments, a water vapor barrier coating or layer may be located between the interior surface of the wall and an oxygen barrier coating or layer, and the oxygen barrier coating or layer may be located between the water vapor barrier coating or layer and the lumen. In other embodiments, an oxygen barrier coating or layer may be located between the interior surface of the wall and a water vapor barrier coating or layer, and the water vapor barrier coating or layer may be located between the oxygen barrier coating or layer and the lumen.

The pH protective coating or layer can comprise or consist essentially of $SiO_xC_y$ or $Si(NH)_xC_y$, where x is from about 0.5 to about 2.4 and y is from about 0.6 to about 3. The pH protective coating or layer can have an interior surface facing the lumen and an outer surface facing the interior surface of the barrier coating or layer.

In the presence of a fluid composition contained in the lumen and having a pH between 5 and 9, the pH protective coating or layer or the combination of the pH protective coating or layer and the tie coating or layer may be effective to provide the package with a calculated shelf life of more than six months at a storage temperature of 4°C.

A vessel as previously described is contemplated in any embodiment, in which at least a portion of the wall of the vessel comprises, consists essentially of, or consists of a thermoplastic material, such as a cyclic olefin polymer (e.g. COP or COC) or a lower-cost commodity resin, such as PET, PETG, polypropylene, a polyamide, polystyrene, polycarbonate, TRITAN™ (a product of Eastman Chemical Company), a thermoplastic olefinic polymer, or the like. In some embodiments, the vessel, vessel wall, or at least a portion of the vessel wall may comprise or be made of a cyclic block co-polymer (CBC). Cyclic block copolymers are fully hydrogenated polymers based on styrene and conjugated dienes via anionic polymerization. Examples of cyclic block co-polymers include, for example, those in the VIVION™ family, such as VIVION™ 0510 or VIVION™ 0510HF or VIVION™ 1325, manufactured by USI Corporation (Taiwan). Cyclic block copolymers are lower cost materials relative to COP and COC resins, due at least in part to lower cost raw materials (styrene, butadiene, hydrogen, and cyclohexane solvent) and lower cost catalysts used in the polymerization and finishing processes.

A vessel as previously described is contemplated in any embodiment, comprising a syringe barrel, a vial, or a blister package. In some embodiments, the lumen may have a volume of 10 mL or less, optionally a volume of 5 mL or less, optionally a volume of 2 mL or less.

A vessel as previously described is contemplated in any embodiment, in which the barrier coating or layer, or at least a portion of the barrier cotaing or layer, is applied by ALD and is from 1 to 50 nm thick, alternatively from 1 to 20 nm thick, alternatively from 2 to 15 nm thick, alternatively from 2 to 10 nm thick, alternatively from 3 to 9 nm thick, alternatively from 4 to 8 nm thick, alternatively from 5 to 7 nm thick. In some embodiments, the water vapor barrier coating or layer may be between 1 and 15 nm thick, alternatively between 2 and 12 nm thick, alternatively between 3 and 10 nm thick, alternatively between 4 and 8 nm thick, alternatively between 5 and 7 nm thick. In some embodiments, the oxygen barrier coating or layer may be between 1 and 15 nm thick, alternatively between 2 and 12 nm thick, alternatively between 3 and 10 nm thick, alternatively between 4 and 8 nm thick, alternatively between 5 and 7 nm thick.

A vessel as previously described is contemplated in any embodiment, in which the pH protective coating or layer comprises $SiO_xC_y$.

A vessel as previously described is contemplated in any embodiment, in which the pH protective coating or layer is applied by PECVD of a precursor feed comprising an acyclic siloxane, a monocyclic siloxane, a polycyclic siloxane, a polysilsesquioxane, a monocyclic silazane, a polycyclic silazane, a polysilsesquiazane, a silatrane, a silquasilatrane, a silproatrane, an azasilatrane, an azasilquasiatrane, an azasilproatrane, or a combination of any two or more of these precursors.

A vessel as previously described is contemplated in any embodiment, in which the pH protective coating or layer is applied by PECVD of a precursor feed comprising octamethylcyclotetrasiloxane (OMCTS).

A vessel as previously described is contemplated in any embodiment, in which the pH protective coating or layer as applied is between 10 and 1000 nm thick.

A vessel as previously described is contemplated in any embodiment, in which the rate of erosion of the pH protective coating or layer, if directly contacted by a fluid composition having a pH of 8, is less than 20% of the rate of erosion of the barrier coating or layer, if directly contacted by the same fluid composition under the same conditions.

A vessel as previously described is contemplated in any embodiment, in which the pH protective coating or layer is at least coextensive with the barrier coating or layer.

A vessel as previously described is contemplated in any embodiment, in which the fluid composition removes the pH protective coating or layer at a rate of 1 nm or less of pH protective coating or layer thickness per 44 hours of contact with the fluid composition.

A vessel as previously described is contemplated in any embodiment, further comprising a lubricity coating or layer applied between the pH protective coating or layer and the lumen. The lubricity coating or layer may comprise or consist essentially of of SiOxCy, in which x is from about 0.5 to about 2.4 and y is from about 0.6 to about 3. In some embodiments, the lubricity coating or layer may be applied by PECVD, e.g. PECVD of a linear siloxane, a monocyclic siloxane, a polycyclic siloxane, a polysilsesquioxane, or any combination thereof, optionally by PECVD of a monocyclic siloxane, optionally by PECVD of octamethylcyclotetrasiloxane (OMCTS).

A vessel as previously described is contemplated in any embodiment, in which the vessel is a syringe barrel and the lubricity coating or layer provides (i) a lower plunger sliding force, (ii) a lower plunger breakout force, or (iii) both (i) and (ii), when compared against the same syringe barrel but lacking the lubricity coating or layer. For instance, a vessel as previously described is contemplated in any embodiment, in which the vessel is a syringe barrel and the lubricity coating or layer provides (i) a plunger sliding force, (ii) a plunger breakout force, or (iii) both (i) and (ii), that is reduced at least 45 percent, optionally at least 60 percent, relative to the same syringe barrel but lacking the lubricity coating or layer.

In some embodiments, the lubricity coating or layer may be located between the pH protective coating or layer and the lumen.

A vessel as previously described is contemplated in any embodiment, in which an FTIR absorbance spectrum of the pH protective coating or layer has a ratio greater than 0.75, optionally greater than 0.8, optionally greater than 0.85, optionally greater than 0.9, between:

- the maximum amplitude of the Si-O-Si symmetrical stretch peak between about 1000 and 1040 cm-1, and
- the maximum amplitude of the Si-O-Si assymmetric stretch peak between about 1060 and about 1100 cm-1.

A vessel as previously described is contemplated in any embodiment, in which an FTIR absorbance spectrum of the lubricity coating or layer has a ratio of at most 0.75 between:

- the maximum amplitude of the Si-O-Si symmetrical stretch peak between about 1000 and 1040 cm-1, and
- the maximum amplitude of the Si-O-Si assymmetric stretch peak between about 1060 and about 1100 cm-1.

A vessel as previously described is contemplated in any embodiment, in which the silicon dissolution rate by a 50 mM potassium phosphate buffer diluted in water for injection, adjusted to pH 8 with concentrated nitric acid, and containing 0.2 wt. % polysorbate-80 surfactant from the vessel is less than 170 ppb/day.

A vessel as previously described is contemplated in any embodiment, in which the total silicon content of the pH protective coating or layer and barrier coating or layer, upon dissolution into 0.1 N potassium hydroxide aqueous solution at 40°C from the vessel, is less than 66 ppm.

A vessel as previously described is contemplated in any embodiment, in which the calculated shelf life (total Si / Si dissolution rate) is more than 2 years.

A vessel as previously described is contemplated in any embodiment, wherein the pH protective coating or layer shows an O-Parameter measured with attenuated total reflection (ATR) of less than 0.4, measured as:

$$\text{O-Parameter} = \frac{\text{Intensity at 1253 cm-1}}{\text{Maximum intensity in the range 1000 to 1100 cm-1.}}$$

A vessel as previously described is contemplated in any embodiment, wherein the pH protective coating or layer shows an N-Parameter measured with attenuated total reflection (ATR) of less than 0.7, measured as:

$$\text{N-Parameter} = \frac{\text{Intensity at 840 cm-1}}{\text{Intensity at 799 cm-1.}}$$

A vessel as previously described is contemplated in any embodiment, in which the tie coating or layer is applied by PECVD of a precursor feed comprising octamethylcyclotetrasiloxane (OMCTS), tetramethyldisiloxane (TMDSO), or hexamethyldisiloxane (HMDSO).

A vessel as previously described is contemplated in any embodiment, in which the tie coating or layer, if present, is on average between 5 and 200 nm thick. In some embodiments, the tie coating or layer may be between 1 and 15 nm thick, alternatively between 2 and 12 nm thick, alternatively between 3 and 10 nm thick, alternatively between 4 and 8 nm thick, alternatively between 5 and 7 nm thick.

A vessel as previously described is contemplated in any embodiment, in which the tie coating or layer is at least coextensive with the barrier coating or layer.

A vessel as previously described is contemplated in any embodiment, in which the tie coating or layer is applied by atomic layer deposition (ALD).

A vessel as previously described is contemplated in any embodiment, in which the barrier coating or layer is 1 to 50 nm thick, alternatively from 1 to 20 nm thick, alternatively from 2 to 15 nm thick, alternatively from 2 to 10 nm thick, alternatively from 3 to 9 nm thick, alternatively from 4 to 8 nm thick, alternatively from 5 to 7 nm thick.

A vessel as previously described is contemplated in any embodiment, in which the barrier coating or layer is applied by atomic layer deposition (ALD).

An aspect of the present invention is a container comprising a vessel having a lumen defined at least in part by a wall, the wall having an interior surface facing the lumen and an outer surface, and in which the wall consists predominantly of a commodity resin. The vessel is provided with a water vapor barrier coating or layer, the water vapor barrier coating or layer being effective to reduce the ingress of water vapor into the lumen, such that the container has a water vapor transmission rate that is at least equivalent to the water vapor transmission rate of an identical vessel made from COP resin and lacking the water vapor barrier coating or layer, optionally a water vapor transmission rate that is lower than the water vapor transmission rate of an identical vessel made from COP resin and lacking the water vapor barrier coating or layer, optionally at least 5% lower, optionally at least 10% lower, optionally at least 20% lower, optionally at least 30% lower, optionally at least 40% lower, optionally at least 50% lower, optionally at least 60% lower, optionally at least 70% lower, optionally at least 80% lower, optionally at least 90% lower.

A container as previously described is contemplated in any embodiment, in which without the water vapor barrier coating or layer, the vessel has a water vapor transmission rate that is at least double, optionally at least three times, optionally at least four times, optionally at least five times the water vapor transmission rate of the vessel made from COP resin and lacking the water vapor barrier coating or layer.

An aspect of the present invention is a container comprising a vessel having a lumen defined at least in part by a wall, the wall having an interior surface facing the lumen and an outer surface, and in which the wall consists predominantly of a commodity resin. The vessel is provided with a water vapor barrier coating or layer, the water vapor barrier coating or layer being effective to reduce the ingress of water vapor into the lumen, compared to a vessel without a water vapor barrier coating or layer, such that the container has a water vapor transmission rate less than 0.05 mg/container/day at 60 °C and 40% relative humidity, optionally less than 0.04 mg/container/day at 60 °C and 40% relative humidity, optionally less than 0.03 mg/container/day at 60 °C and 40% relative humidity, optionally less than 0.02 mg/container/day at 60 °C and 40% relative humidity, optionally less than 0.01 mg/container/day at 60 °C and 40% relative humidity.

A container as previously described is contemplated in any embodiment, in which without the water vapor barrier or coating, the vessel has a water vapor transmission rate greater than 1.0 g/container/day, optionally greater than 2.0 g/container/day, optionally greater than 3.0 g/container/day.

A container as previously described is contemplated in any embodiment, in which the commodity resin comprises such as PET, PETG, polypropylene, a polyamide, polystyrene, polycarbonate, TRITAN™ (a product of Eastman Chemical Company), a thermoplastic olefinic polymer, a cyclic block co-polymer (CBC), or the like. In some embodiments, the vessel, vessel wall, or at least a portion of the vessel wall may comprise or be made of a cyclic block co-polymer (CBC). Cyclic block copolymers are fully hydrogenated polymers based on styrene and conjugated dienes via anionic polymerization. Examples of cyclic block co-polymers include, for example, those in the VIVION™ family, such as VIVION™ 0510 or VIVION™ 0510HF or VIVION™ 1325, manufactured by USI Corporation (Taiwan).

An aspect of the present invention is a container comprising a vessel having a lumen defined at least in part by a wall, the wall having an interior surface facing the lumen and an outer surface, and in which the wall consists predominantly of a COP resin. The vessel is provided with a water vapor barrier coating or layer, the water vapor barrier coating or layer being effective to reduce the ingress of water vapor into the lumen, such that the container has a water vapor transmission rate that is lower than the water vapor transmission rate of an identical vessel made from COP resin and lacking the water vapor barrier coating or layer, optionally at least 5% lower, optionally at least 10% lower, optionally at least 20% lower, optionally at least 30% lower, optionally at least 40% lower, optionally at least 50% lower, optionally at least 60% lower, optionally at least 70% lower, optionally at least 80% lower, optionally at least 90% lower.

An aspect of the present invention is a container comprising a vessel having a lumen defined at least in part by a wall, the wall having an interior surface facing the lumen and an outer surface, and in which the wall consists predominantly of a COC resin. The vessel is provided with a water vapor barrier coating or layer, the water vapor barrier coating or layer being effective to reduce the ingress of water vapor into the lumen, such that the container has a water vapor transmission rate that is lower than the water vapor transmission rate of an identical vessel made from COC resin and lacking the water vapor barrier coating or layer, optionally at least 5% lower, optionally at least 10% lower, optionally at least 20% lower, optionally at least 30% lower, optionally at least 40% lower, optionally at least 50% lower, optionally at least 60% lower, optionally at least 70% lower, optionally at least 80% lower, optionally at least 90% lower.

In some embodiments, the container may have a lumen volume of 10 mL or less, optionally a volume of 5 mL or less, optionally a volume of 2 mL or less. In some embodiments, the container may be a syringe or vial.

In some embodiments, the water vapor barrier coating or layer may comprises or consist essentially of a plurality of atomic monolayers, optionally wherein the water vapor barrier coating or layer is applied by atomic layer deposition, optionally by plasma-assisted atomic layer deposition.

In some embodiments, the water vapor barrier coating or layer comprises or consists essentially of a metal oxide coating, e.g. aluminum oxide.

In some embodiments, the water vapor barrier coating or layer may be supported by the interior surface of the vessel wall such that the water vapor barrier coating or layer has an inner surface facing the lumen and an outer surface facing the wall interior surface. In other embodiments, the water vapor barrier coating or layer may be supported by the outer surface of the vessel wall, such that the water vapor barrier coating or layer has an inner surface facing the wall outer surface. In other embodiments, the water vapor barrier coating or layer may be applied during the molding of the vessel, such that it is sandwiched between thermoplastic layers ofhe vessel wall and thus has an inner surface facing the wall interior surface and an outer surface facing the wall outer surface.

In some embodiments, the water vapor barrier coating or layer may be between 1 and 50 nm thick, alternatively between 5 and 50 nm thick, alternatively between 10 and 50 nm thick, alternatively between 1 and 40 nm thick, alternatively between 5 and 40 nm thick, alternatively between 10 and 40 nm thick, alternatively between 1 and 30 nm thick, alternatively between 5 and 30 nm thick, alternatively between 10 and 30 nm thick.

A container as previously described is contemplated in any embodiment, in which the vessel is further provided with an oxygen barrier coating or layer, the oxygen barrier coating or layer being effective to reduce the ingress of

atmospheric gas into the lumen compared to a vessel without an oxygen barrier coating or layer.

In some embodiments, the oxygen barrier coating or layer may comprise or consist essentially of a plurality of atomic monolayers, optionally wherein the oxygen barrier coating or layer is applied by atomic layer deposition, optionally plasma-assisted atomic layer deposition. In other embodiments, the oxygen barrier coating or layer may be applied by PECVD.

In some embodiments, the oxygen barrier coating or layer may comprise SiOx, wherein x is from 1.5 to 2.9, optionally $SiO_2$.

In some embodiments, the oxygen barrier coating or layer may be supported by the interior surface of the vessel wall, such that the oxygen barrier coating or layer has an inner surface facing the lumen and an outer surface facing the wall interior surface. In some embodiments, the water vapor barrier coating or layer may be positioned between the oxygen barrier coating or layer and the wall interior surface.

In some embodiments, the vessel may further comprise a pH protective coating or layer and/or a lubricity coating or layer such as is described herein.

An aspect of the present invention is a vessel comprising a lumen defined at least in part by a wall, the wall consisting predominantly of a commodity resin and having an interior surface facing the lumen and an outer surface. The wall is provided with both (a) an oxygen barrier coating or layer, the oxygen barrier coating or layer being effective to reduce the ingress of atmospheric gas into the lumen compared to a vessel without an oxygen barrier coating or layer and (b) a water vapor barrier coating or layer, the water vapor barrier coating or layer being effective to reduce the ingress of water vapor into the lumen. In some embodiments, the wall may also be provided with a pH protective coating or layer, the pH protective coating or layer being effective to increase the calculated shelf life of the vessel.

An aspect of the present invention is a vessel comprising a lumen defined at least in part by a wall, the wall consisting predominantly of a COP or COC resin and having an interior surface facing the lumen and an outer surface. The wall is provided with both (a) an oxygen barrier coating or layer, the oxygen barrier coating or layer being effective to reduce the ingress of atmospheric gas into the lumen compared to a vessel without an oxygen barrier coating or layer and (b) a water vapor barrier coating or layer, the water vapor barrier coating or layer being effective to reduce the ingress of water vapor into the lumen. In some embodiments, the wall may also be provided with a pH protective coating or layer, the pH protective coating or layer being effective to increase the calculated shelf life of the vessel.

Another aspect of the present invention is a vessel having an oxygen barrier coating or layer applied by atomic layer deposition and which represents an improvement over an SiOx oxygen barrier applied by PECVD in that a relatively thin coating or layer may provide barrier properties well above those obtained by a PECVD-applied oxygen barrier at the same thickness. An aspect of the present invention is a vessel comprising a lumen defined at least in part by a wall, the wall having an interior surface facing the lumen and an outer surface; and a coating set on the interior or outer surface, the coating set comprising an oxygen barrier coating or layer in which the oxygen barrier coating or layer has a thickness between 1 nm and 15 nm, optionally a thickness between 1 nm and 10 nm; and is effective to provide the vessel wall with an oxygen transmission rate constant is less than 0.0003 d-1; optionally less than 0.0002 d-1; optionally less than 0.0001 d-1.

In some embodiments, the vessel may have an oxygen transmission rate constant that is less than the oxygen transmission rate constant of an otherwise equivalent vessel in which an oxygen barrier coating or layer having substantially the same composition and thickness is applied by PECVD, optionally at least 10% less, optionally at least 20% less, optionally at least 30% less, optionally at least 40% less, optionally at least 50% less, optionally at least 60% less, optionally at least 70% less, optionally at least 80% less, optionally at least 90% less.

An aspect of the present invention is a method of preparing a vessel with suitable barrier properties for storing a liquid drug formulation over a period of time, by providing a vessel comprising a lumen defined at least in part by a wall, the wall consisting predominantly of either a COP or COC resin or a commodity resin and having an interior surface facing the lumen and an outer surface, and applying a water vapor barrier coating by atomic layer deposition, the water vapor barrier coating being effective to reduce the ingress of water vapor into the lumen.

A method as previously described is contemplated in any embodiment, in which the water vapor barrier coating comprises a metal oxide coating, optionally aluminum oxide, optionally an aluminum oxdie coating deposited using a trimethylaluminum precursor.

A method as previously described is contemplated in any embodiment, in which the water vapor barrier coating is applied by plasma assisted atomic layer deposition. A method as previously described is contemplated in any embodiment, in which the wall is maintained at a temperature less than 100 °C, and optionally less than 80°C, during deposition of the coating.

A method as previously described is contemplated in any embodiment, in which the outer surface of the wall is masked during the deposition, such that the coating is deposited only on the interior surface of the wall. A method as previously described is contemplated in any embodiment, in which the interior surface of the wall is masked during the deposition, such that the coating is deposited only on the outer surface of the wall.

A method as previously described is contemplated in any embodiment, in which a plurality of vessels are coated substantially evenly, the method further comprising providing at least 20 vessels, optionally at least 50 vessels, optionally

at least 100 vessels, optionally at least 150 vessels, optionally at least 200 vessels, optionally at least 500 vessels, optionally at least 800 vessels, optionally at least 1000 vessels in a reactor, optionally a PICOSUN™ P-1000B PRO; and providing substantially uniform flows of precursor gases to each of the vessels under conditions sufficient to cause layers of the water vapor barrier coating to build-up substantially uniformly, optionally with at least 95% uniformity, optionally with at least 96% uniformity, optionally with at least 97% uniformity, across the plurality of vessels.

A method as previously described is contemplated in any embodiment, in which an oxygen barrier coating is also applied to the vessel wall. In some embodiments, the oxygen barrier coating is applied by atomic layer deposition, optionally plasma-assisted atomic layer deposition. In some embodiments, the oxygen barrier coating comprises SiOx wherein x is from 1.5 to 2.9. In some embodiments, the SiOx barrier coating or layer may be deposited using a silicon-containing precursor selected from the group consisting of: aminosilanes; alkyl-aminosilanes; 1,2-bis(diisopropylamino) disilane; diisopropylaminosilane; tris(dimethylamino)silane; bis(ethyl-methyl-amino)silane; and combinations thereof. In some embodiments, the oxygen barrier coating may be applied in the same reactor as the water vapor barrier coating.

An aspect of the present invention is a method of preparing a vessel with suitable barrier properties for storing a liquid drug formulation over a period of time, by providing a vessel comprising a lumen defined at least in part by a wall, the wall consisting predominantly of either a COP or COC resin or a commodity resin and having an interior surface facing the lumen and an outer surface, and applying an oxygen barrier coating by atomic layer deposition, the oxygen barrier coating being effective to reduce the ingress of oxygen into the lumen.

A method as previously described is contemplated in any embodiment, in which an oxygen barrier coating comprises SiOx wherein x is from 1.5 to 2.9, optionally wherein x is 2. In some embodiments, the SiOx barrier coating or layer may be deposited using a silicon-containing precursor selected from the group consisting of: aminosilanes; alkyl-aminosilanes; 1,2-bis(diisopropylamino)disilane; diisopropylaminosilane; tris(dimethylamino)silane; bis(ethylmethyl-amino)silane; and combinations thereof.

A method as previously described is contemplated in any embodiment, in which the the oxygen barrier coating is applied by plasma assisted atomic layer deposition. A method as previously described is contemplated in any embodiment, in which the wall is maintained at a temperature less than 100 °C, and optionally less than 80°C, during deposition of the coating.

A method as previously described is contemplated in any embodiment, in which the outer surface of the wall is masked during the deposition, such that the coating is deposited only on the interior surface of the wall. A method as previously described is contemplated in any embodiment, in which the interior surface of the wall is masked during the deposition, such that the coating is deposited only on the outer surface of the wall.

A method as previously described is contemplated in any embodiment, in which a plurality of vessels are coated substantially evenly, the method further comprising providing at least 20 vessels, optionally at least 50 vessels, optionally at least 100 vessels, optionally at least 150 vessels, optionally at least 200 vessels, optionally at least 500 vessels, optionally at least 800 vessels, optionally at least 1000 vessels in a reactor, optionally a PICOSUN™ P-1000B PRO; and providing substantially uniform flows of precursor gases to each of the vessels under conditions sufficient to cause layers of the oxygen barrier coating to build-up substantially uniformly, optionally with at least 95% uniformity, optionally with at least 96% uniformity, optionally with at least 97% uniformity, across the plurality of vessels.

A method as previously described is contemplated in any embodiment, in which a water vapor barrier coating is also applied to the vessel wall.

An aspect of the present invention is a thermoplastic vial comprising a lumen defined at least in part by a side wall and a bottom wall, the side wall having an interior surface facing the lumen and an outer surface; the bottom wall having an upper surface facing the lumen and a lower surface; and a gas barrier coating supported by at least one of the interior surface and the outer surface of the wall, at least a portion of the gas barrier coating consisting essentially of a plurality of atomic monolayers of a pure element or compound. The thermoplastic vial may further comprise a stopper seated in the opening (the combination of which may also be referred to as a package). Another aspect of the present invention is a drug primary package comprising the thermoplastic vial described above, a stopper, and a liquid formulation of a drug stored within the lumen of the vial. In some embodiments, the drug may comprise a cold-chain drug, optionally a DNA-based or mRNA-based vaccine.

A thermoplastic vial or drug primary package as previously described is contemplated in any embodiment, in which the lower surface of the thermoplastic vial is flat or substantially flat, for instance in which the lower surface of the thermoplastic vial produces an ink blot that covers at least 50% of a surface area corresponding to the footprint of the vial, optionally at least 60%, optionally at least 70%, optionally at least 75%, optionally at least 80%, optionally at least 85%, optionally at least 90%.

A thermoplastic vial or drug primary package as previously described is contemplated in any embodiment, in which the vial is configured so that during lyophilization, the vial has a heat transfer (Kv x $10^4$) of at least 3.3 cal/s/$cm^2$/°C, alternatively at least 3.4 cal/s/$cm^2$/°C, alternatively at least 3.5 cal/s/$cm^2$/°C.

In some embodiments, moreover, a plurality of drug primary packages or thermoplastic vials may, during lyophilization, have heat transfers with a standard deviation less than 0.15 cal/s/$cm^2$/°C, alternatively less than 0.12

cal/s/cm$^2$/°C, alternatively less than 0.10 cal/s/cm$^2$/°C, alternatively less than 0.08 cal/s/cm$^2$/°C, for instance in which the standard deviation is calculated across a sample of at least 20 units, optionally at least 50 units, optionally at least 100 units, optionally at least 200 units, optionally at least 300 units.

A thermoplastic vial or drug primary package as previously described is contemplated in any embodiment, in which the package is configured to maintain container closure integrity for at least 3 months, optionally for at least 6 months, optionally for at least 9 months, optionally for at least 12 months, when stored at -80 °C.

A thermoplastic vial or drug primary package as previously described is contemplated in any embodiment, in which the package has an oxygen transmission rate constant less than 0.005 d$^{-1}$, optionally less than 0.004 d$^{-1}$, optionally less than 0.003 d$^{-1}$, optionally less than 0.002 d$^{-1}$, optionally less than 0.001 d$^{-1}$, optionally less than 0.0005 d$^{-1}$ after storage at -80 °C for at least 3 months, optionally for at least 6 months, optionally for at least 9 months, optionally for at least 12 months.

A thermoplastic vial or drug primary package as previously described is contemplated in any embodiment, in which the gas barrier coating is supported by the interior surface of the wall.

A thermoplastic vial or drug primary package as previously described is contemplated in any embodiment, in which the vial further includes a pH protective coating between the lumen and the gas barrier coating, the pH protective coating being effective to increase the calculated shelf life of the vessel.

In some embodiments, at least a lumen-facing surface of the pH protective coating may comprises a surface energy that is customized to the fluid drug product stored in the lumen.

In some embodiments, for instance, at least a lumen-facing surface of the pH protective coating may be hydrophilic, e.g. having a water contact angle between 25° and 60°, alternatively between 25° and 50°, alternatively between 30° and 60°, alternatively between 30° and 50°, alternatively between 40° and 60°, alternatively between 40° and 50°. In other embodiments, at least a lumen-facing surface of the pH protective coating may be hydrophobic, e.g. having a water contact angle between 70° and 105°, alternatively between 75° and 105°, alternatively between 80° and 105°, alternatively between 85° and 105°, alternatively between 90° and 105°, alternatively between 95° and 105°. In yet other embodiments, at least a lumen-facing surface of the pH protective coating may have a water contact angle between 50° and 80°, alternatively between 55° and 75°, alternatively between 60° and 70°.

In some embodiments, for instance, at least a lumen-facing surface of the pH protective coating may have a surface free energy, measured using the Kitazaki-Hata Method, between 20 mJ/m$^2$ and 50 mJ/m$^2$, alternatively between 25 mJ/m$^2$ and 50 mJ/m$^2$, alternatively between 20 mJ/m$^2$ and 45 mJ/m$^2$, alternatively between 25 mJ/m$^2$ and 45 mJ/m$^2$, alternatively between 20 mJ/m$^2$ and 40 mJ/m$^2$, alternatively between 25 mJ/m$^2$ and 40 mJ/m$^2$. In other embodiments, at least a lumen-facing surface of the pH protective coating may have a surface free energy, measured using the Kitazaki-Hata Method, between 60 mJ/m$^2$ and 100 mJ/m$^2$, alternatively between 60 mJ/m$^2$ and 90 mJ/m$^2$, alternatively between 65 mJ/m$^2$ and 100 mJ/m$^2$, alternatively between 65 mJ/m$^2$ and 90 mJ/m$^2$, alternatively between 70 mJ/m$^2$ and 100 mJ/m$^2$, alternatively between 70 mJ/m$^2$ and 90 mJ/m$^2$.

A thermoplastic vial or drug primary package as previously described is contemplated in any embodiment, in which the thermoplastic vial having the gas barrier coating contains less than 50 particles/mL of 2 $\mu$m in size or greater, optionally less than 40 particles/mL of 2 $\mu$m in size or greater, optionally less than 30 particles/mL of 2 $\mu$m in size or greater, optionally less than 25 particles/mL of 2 $\mu$m in size or greater, optionally less than 20 particles/mL of 2 $\mu$m in size or greater, optionally less than 15 particles/mL of 2 $\mu$m in size or greater, optionally less than 12 particles/mL of 2 $\mu$m in size or greater, optionally less than 10 particles/mL of 2 $\mu$m in size or greater.

A thermoplastic vial or drug primary package as previously described is contemplated in any embodiment, in which the gas barrier coating comprises an oxygen barrier coating or layer, the oxygen barrier coating or layer being effective to reduce the ingress of oxygen into the lumen to less than 0.0005 cc/package/day at 25 °C, 60% relative humidity and 0.21 bar, optionally less than 0.0004 cc/package/day at 25 °C, 60% relative humidity and 0.21 bar, optionally less than 0.0003 cc/package/day at 25 °C, 60% relative humidity and 0.21 bar, optionally less than 0.0002 cc/package/day at 25 °C, 60% relative humidity and 0.21 bar, optionally less than 0.0001 cc/package/day at 25 °C, 60% relative humidity and 0.21 bar.

A thermoplastic vial or drug primary package as previously described is contemplated in any embodiment, in which the gas barrier coating comprises an oxygen barrier coating or layer, the oxygen barrier coating or layer being effective to provide the package or vial with an oxygen transmission rate constant less than 0.0010 d$^{-1}$; optionally less than 0.0008 d$^{-1}$; optionally less than 0.0006 d$^{-1}$; optionally less than 0.0004 d$^{-1}$; optionally less than 0.0003 d$^{-1}$; optionally less than 0.0002 d$^{-1}$; optionally less than 0.0001 d$^{-1}$.

A thermoplastic vial or drug primary package as previously described is contemplated in any embodiment, in which the gas barrier coating comprises an oxygen barrier coating or layer, wherein the oxygen barrier coating or layer consists essentially of a plurality of atomic monolayers, optionally wherein the oxygen barrier coating or layer is deposited by atomic layer deposition, optionally by plasma-assisted atomic layer deposition.

A thermoplastic vial or drug primary package as previously described is contemplated in any embodiment, in which the oxygen barrier coating or layer comprises or consists essentially of a metal oxide, optionally $Al_2O_3$. A

thermoplastic vial or drug primary package as previously described is contemplated in any embodiment, in which oxygen barrier coating or layer comprises or consists essentially of $SiO_x$, wherein x is from 1.5 to 2.9, optionally wherein x is 2.

A thermoplastic vial or drug primary package as previously described is contemplated in any embodiment, in which the gas barrier coating comprises a water vapor barrier coating or layer, the water vapor barrier coating or layer being effective to reduce the ingress of water vapor into the lumen to less than 0.05 mg/package/day at 60 °C and 40% relative humidity, optionally less than 0.04 mg/package/day at 60 °C and 40% relative humidity, optionally less than 0.03 mg/package/day at 60 °C and 40% relative humidity, optionally less than 0.02 mg/package/day at 60 °C and 40% relative humidity, optionally less than 0.01 mg/package/day at 60 °C and 40% relative humidity.

A thermoplastic vial or drug primary package as previously described is contemplated in any embodiment, in which in which the gas barrier coating comprises a water vapor barrier coating or layer and in which the water vapor barrier coating or layer consists essentially of a plurality of atomic monolayers, optionally wherein the water vapor barrier coating or layer is deposited by atomic layer deposition, optionally by plasma-assisted atomic layer deposition.

A thermoplastic vial or drug primary package as previously described is contemplated in any embodiment, in which the water vapor barrier coating or layer comprises or consists essentially of a metal oxide, optionally $Al_2O_3$. A thermoplastic vial or drug primary package as previously described is contemplated in any embodiment, in which the water vapor barrier coating or layer comprises or consists essentially of $SiO_x$, wherein x is from 1.5 to 2.9, optionally wherein x is 2.

A thermoplastic vial or drug primary package as previously described is contemplated in any embodiment, further comprising a nitrogen gas in a headspace of the lumen, and in which the gas barrier coating comprises a nitrogen barrier coating or layer, the nitrogen barrier coating or layer being effective to reduce egress of the nitrogen gas out of the lumen to less than 0.0002 cc/package/day at 25 °C, 60% relative humidity and 0.21 bar, optionally less than 0.00015 cc/package/-day at 25 °C, 60% relative humidity and 0.21 bar, optionally less than 0.0001 cc/package/day at 25 °C, 60% relative humidity and 0.21 bar, optionally less than 0.00005 cc/package/day at 25 °C, 60% relative humidity and 0.21 bar, optionally less than 0.00002 cc/package/day at 25 °C, 60% relative humidity and 0.21 bar, optionally less than 0.00001 cc/package/-day at 25 °C, 60% relative humidity and 0.21 bar.

A thermoplastic vial or drug primary package as previously described is contemplated in any embodiment, in which the gas barrier coating comprises a nitrogen barrier coating or layer, the nitrogen barrier coating or layer being effective to provide the package or vial with a nitrogen transmission rate constant (NTR) less than 0.0003 $d^{-1}$; optionally less than 0.0002 $d^{-1}$; optionally less than 0.0001 $d^{-1}$, optionally less than 0.00008 $d^{-1}$; optionally less than 0.00006 $d^{-1}$; optionally less than 0.00004 $d^{-1}$, optionally less than 0.00003 $d^{-1}$; optionally less than 0.00002 $d^{-1}$; optionally less than 0.00001 $d^{-1}$.

A thermoplastic vial or drug primary package as previously described is contemplated in any embodiment, in which the gas barrier coating comprises a nitrogen barrier coating or layer, and in which the nitrogen barrier coating or layer consists essentially of a plurality of atomic monolayers, optionally wherein the nitrogen barrier coating or layer is deposited by atomic layer deposition, optionally by plasma-assisted atomic layer deposition.

A thermoplastic vial or drug primary package as previously described is contemplated in any embodiment, in which the nitrogen barrier coating or layer comprises or consists essentially of a metal oxide, optionally $Al_2O_3$. A thermoplastic vial or drug primary package as previously described is contemplated in any embodiment, in which the nitrogen barrier coating or layer comprises or consists essentially of $SiO_x$, wherein x is from 1.5 to 2.9, optionally wherein x is 2.

A thermoplastic vial or drug primary package as previously described is contemplated in any embodiment, further comprising carbon monoxide in the lumen, and in which the gas barrier coating comprises a carbon monoxide barrier coating or layer, the carbon monoxide barrier coating or layer being effective to reduce egress of carbon monoxide out of the lumen to less than 0.0002 cc/package/day at 25 °C, 60% relative humidity and 0.21 bar, optionally less than 0.00015 cc/package/day at 25 °C, 60% relative humidity and 0.21 bar, optionally less than 0.0001 cc/package/day at 25 °C, 60% relative humidity and 0.21 bar, optionally less than 0.00005 cc/package/day at 25 °C, 60% relative humidity and 0.21 bar, optionally less than 0.00002 cc/package/day at 25 °C, 60% relative humidity and 0.21 bar, optionally less than 0.00001 cc/package/day at 25 °C, 60% relative humidity and 0.21 bar.

A thermoplastic vial or drug primary package as previously described is contemplated in any embodiment, in which the gas barrier coating comprises a carbon monoxide barrier coating or layer, the carbon monoxide barrier coating or layer being effective to provide the package or vial with a carbon monoxide transmission rate (COTR) less than 0.0003 $d^{-1}$; optionally less than 0.0002 $d^{-1}$; optionally less than 0.0001 $d^{-1}$, optionally less than 0.00008 $d^{-1}$; optionally less than 0.00006 $d^{-1}$; optionally less than 0.00004 $d^{-1}$, optionally less than 0.00003 $d^{-1}$; optionally less than 0.00002 $d^{-1}$; optionally less than 0.00001 $d^{-1}$.

A thermoplastic vial or drug primary package as previously described is contemplated in any embodiment, in which the gas barrier coating comprises a carbon monoxide barrier coating or layer, and in which the carbon monoxide barrier coating or layer consists essentially of a plurality of atomic monolayers, optionally wherein the carbon monoxide barrier coating or layer is deposited by atomic layer deposition, optionally by plasma-assisted atomic layer deposition.

A thermoplastic vial or drug primary package as previously described is contemplated in any embodiment, in which the carbon monoxide barrier coating or layer comprises or consists essentially of a metal oxide, optionally $Al_2O_3$. A thermoplastic vial or drug primary package as previously described is contemplated in any embodiment, in which the carbon monoxide barrier coating or layer comprises or consists essentially of $SiO_x$, wherein x is from 1.5 to 2.9, optionally wherein x is 2.

A thermoplastic vial or drug primary package as previously described is contemplated in any embodiment, further comprising carbon dioxide in the lumen, and in which the gas barrier coating comprises a carbon dioxide barrier coating or layer, the carbon dioxide barrier coating or layer being effective to reduce egress of carbon dioxide out of the lumen to less than 0.005 cc/package/day at 25 °C, 60% relative humidity and 0.21 bar, optionally less than 0.004 cc/package/day at 25 °C, 60% relative humidity and 0.21 bar, optionally less than 0.003 cc/package/day at 25 °C, 60% relative humidity and 0.21 bar, optionally less than 0.002 cc/package/day at 25 °C, 60% relative humidity and 0.21 bar, optionally less than 0.001 cc/package/day at 25 °C, 60% relative humidity and 0.21 bar, optionally less than 0.0008 cc/package/day at 25 °C, 60% relative humidity and 0.21 bar, optionally less than 0.0005 cc/package/day at 25 °C, 60% relative humidity and 0.21 bar.

A thermoplastic vial or drug primary package as previously described is contemplated in any embodiment, in which the gas barrier coating comprises a carbon dioxide barrier coating or layer, the carbon dioxide barrier coating or layer being effective to provide the package or vial with a carbon dioxide transmission rate (CO2TR) less than 0.005 d-1; optionally less than 0.004 d-1; optionally less than 0.002 d-1; optionally less than 0.001 d-1; optionally less than 0.0008 d-1, optionally less than 0.0006 d-1; optionally less than 0.0005 d-1; optionally less than 0.0004 d-1, optionally less than 0.0003 d-1; optionally less than 0.0002 d-1; optionally less than 0.0001 d-1.

A thermoplastic vial or drug primary package as previously described is contemplated in any embodiment, in which the gas barrier coating comprises a carbon dioxide barrier coating or layer, wherein the carbon dioxide barrier coating or layer consists essentially of a plurality of atomic monolayers, optionally wherein the carbon dioxide barrier coating or layer is deposited by atomic layer deposition, optionally by plasma-assisted atomic layer deposition.

A thermoplastic vial or drug primary package as previously described is contemplated in any embodiment, in which the carbon dioxide barrier coating or layer comprises or consists essentially of a metal oxide, optionally $Al_2O_3$. A thermoplastic vial or drug primary package as previously described is contemplated in any embodiment, in which the carbon dioxide barrier coating or layer comprises or consists essentially of $SiO_x$, wherein x is from 1.5 to 2.9, optionally wherein x is 2.

A thermoplastic vial or drug primary package as previously described is contemplated in any embodiment, in which the gas barrier coating comprises an ethylene oxide barrier coating or layer. A thermoplastic vial or drug primary package as previously described is contemplated in any embodiment, in which the drug primary package is terminally sterilized, optionally using ethylene oxide.

A thermoplastic vial or drug primary package as previously described is contemplated in any embodiment, in which the vial consists predominantly of a thermoplastic material selected from the following: PET, PETG, polypropylene, a polyamide, polystyrene, polycarbonate, TRITAN™, a cyclic block copolymer (CBC) resin, a thermoplastic olefinic polymer, COP, COC, or any combination thereof.

A thermoplastic vial or drug primary package, or a plurality of thermoplastic vials or drug primary packages, as previously described is contemplated in any embodiment, in which the package(s) or vial(s) is configured to maintain container closure integrity (CCI) when cycled between -20 °C and 10 °C, optionally when cycled between -20 °C and 20 °C, optionally when cycled between -20 °C and 30 °C, optionally when cycled between -20 °C and 40 °C, optionally when cycled between -40 °C and 10 °C, optionally when cycled between -40 °C and 20 °C, optionally when cycled between -40 °C and 30 °C, optionally when cycled between -40 °C and 40 °C, optionally when cycled between -70 °C and 10 °C, optionally when cycled between -70 °C and 20 °C, optionally when cycled between -70 °C and 30 °C, optionally when cycled between - 70 °C and 40 °C. In some embodiments, the package(s) or vial(s) may be subjected to at least three cycles, optionally in which the package(s) or vial(s) is subjected to three cycles. During each cycle the package(s) or vial(s) may be held both at the lower temperature for 24 hours or more and at the upper temperature for 24 hours or more; optionally in which during each cycle the package(s) or vial(s) is held both at the lower temperature for about 24 hours and at the upper temperature for about 24 hours.

A thermoplastic vial or drug primary package, or a plurality of thermoplastic vials or drug primary packages, as previously described is contemplated in any embodiment, in which the fill volume of the vial(s) is within at least 20% of the nominal volume of the vial, optionally in which the fill volume of the vial is within at least 10% of the nominal volume of the vial, optionally in which the fill volume of the vial is within at least 5% of the nominal volume of the vial. In some embodiments, the vial(s) may have a nominal volume of either 10 mL or 2 mL, optionally where the vial(s) has a nominal volume of 10 mL, optionally where the vial(s) has a nominal volume of 2 mL.

A thermoplastic vial or drug primary package as previously described is contemplated in any embodiment, in which the plurality of vials or packages comprises at least 50 previously untested packages, optionally in which the plurality of packages consists of a sample of 50 previously untested packages, optionally in which the plurality of packages comprises at least 100 previously untested packages, optionally in which the plurality of packages consists of a sample of

100 previously untested packages, optionally in which the plurality of packages comprises at least 500 previously untested packages, optionally in which the plurality of packages consists of a sample of 500 previously untested packages, optionally in which the plurality of packages comprises at least 1000 previously untested packages, optionally in which the plurality of packages consists of a sample of 1000 previously untested packages.

An aspect of the present invention is a thermoplastic syringe barrel comprising a lumen defined at least in part by a side wall, the side wall having an interior surface facing the lumen and an outer surface; a front dispensing opening and a rear opening; and a gas barrier coating supported by at least one of the interior surface and the outer surface of the side wall, at least a portion of the gas barrier coating consisting essentially of a plurality of atomic monolayers of a pure element or compound. Another aspect of the present invention is a syringe comprising the thermoplastic syringe barrel described above and a plunger seated in the rear opening. Another aspect of the present invention is a drug primary package comprising the thermoplastic syringe barrel described above, a liquid formulation of a drug within the lumen; and a plunger seated in the syringe barrel and having a front face facing the liquid formulation. In some embodiments, the liquid formulation of a drug may optionally comprise a cold-chain drug, optionally a DNA-based or mRNA-based vaccine.

A thermoplastic syringe barrel, syringe, or drug primary package as previously described is contemplated in any embodiment, in which the front dispensing opening comprises a staked needle or a luer lock.

A thermoplastic syringe barrel, syringe, or drug primary package as previously described is contemplated in any embodiment, in which the lumen has a nominal fill volume between 0.25 and 10 mL, optionally between 0.5 and 5 mL.

A thermoplastic syringe barrel, syringe, or drug primary package as previously described is contemplated in any embodiment, in which the syringe barrel is configured such that when filled with Milli-Q water and subjected to any one or more of inversion for 2 hours at 50 rpm, incubation for two weeks at 4 °C, and five cycles of freeze thawing between 20 °C and -40 °C, the contents of the syringe has less than 500,000 particles sized 300 nm or higher, alternatively less than 400,000 particles sized 300 nm or higher, alternatively less than 300,000 particles sized 300 nm or higher per resonant mass measurement.

In some embodiments, for instance the syringe barrel or syringe is configured such that when filled with Milli-Q water and inverted for two hours at 50 rpm, the contents of the syringe has less than 500 particles sized 2 μm or higher, alternatively less than 400 particles sized 2 μm or higher, alternatively less than 300 particles sized 2 μm or higher, alternatively less than 200 particles sized 2 μm or higher per FlowCAM® microflow digital imaging, light obscuration testing, or both. In some embodiments, for instance, the syringe barrel or syringe is configured such that when filled with Milli-Q water and incubated for two weeks at 4 °C, the contents of the syringe has less than 2,000 particles sized 2 μm or higher, alternatively less than 1,000 particles sized 2 μm or higher, alternatively less than 900 particles sized 2 μm or higher, alternatively less than 800 particles sized 2 μm or higher, alternatively less than 700 particles sized 2 μm or higher, alternatively less than 600 particles sized 2 μm or higher, alternatively less than 500 particles sized 2 μm or higher per FlowCAM® microflow digital imaging, light obscuration testing, or both. In some embodiments, for instance, the syringe barrel is configured such that when filled with Milli-Q water and subjected to five cycles of freeze thawing between 20 °C and -40 °C, the contents of the syringe has less than 20,000 particles sized 2 μm or higher, alternatively less than 10,000 particles sized 2 μm or higher, alternatively less than 5,000 particles sized 2 μm or higher, alternatively less than 2,000 particles sized 2 μm or higher, alternatively less than 1,000 particles sized 2 μm or higher, alternatively less than 500 particles sized 2 μm or higher, alternatively less than 300 particles sized 2 μm or higher per FlowCAM® microflow digital imaging, light obscuration testing, or both.

A syringe barrel, syringe, or drug primary package as previously described is contemplated in any embodiment, in which the liquid formulation of drug comprises less than 50 particles having a size of more than 10 μm after the vessel has been rotated at 40°C for five minutes, two weeks or four weeks after three freeze-thaw cycles from +5°C to -20°C with 1°C per minute, or after storage of the vessel at 5°C, 25°C and 60% relative humidity or 40°C and 75% relative humidity for three months.

A thermoplastic syringe barrel, syringe, or drug primary package as previously described is contemplated in any embodiment, in which the liquid formulation of drug comprises less than 5 particles having a size of more than 25 μm after the vessel has been rotated at 40°C for five minutes, two weeks or four weeks, or after three freeze-thaw cycles from +5°C to -20°C with 1°C per minute, or after storage of the vessel at 5°C, 25°C/60% relative humidity or 40°C/75% relative humidity for three months.

A thermoplastic syringe barrel, syringe, or drug primary package as previously described is contemplated in any embodiment, which is free of silicone oil or baked-on silicone on the syringe barrel and plunger.

A thermoplastic syringe barrel, syringe, or drug primary package as previously described is contemplated in any embodiment, in which a lubricity coating or layer as described herein is supported by the interior surface of the wall.

A thermoplastic syringe barrel, syringe, or drug primary package as previously described is contemplated in any embodiment, in which the gas barrier coating is supported by the interior surface of the wall.

A thermoplastic syringe barrel, syringe, or drug primary package as previously described is contemplated in any embodiment, comprising a pH protective coating as described herein between the lumen and the gas barrier coating, the pH protective coating being effective to increase the calculated shelf life of the vessel.

In some embodiments, at least a lumen-facing surface of the pH protective coating may comprises a surface energy that is customized to the fluid drug product stored in the lumen.

In some embodiments, for instance, at least a lumen-facing surface of the pH protective coating may be hydrophilic, e.g. having a water contact angle between 25° and 60°, alternatively between 25° and 50°, alternatively between 30° and 60°, alternatively between 30° and 50°, alternatively between 40° and 60°, alternatively between 40° and 50°. In other embodiments, at least a lumen-facing surface of the pH protective coating may be hydrophobic, e.g. having a water contact angle between 70° and 105°, alternatively between 75° and 105°, alternatively between 80° and 105°, alternatively between 85° and 105°, alternatively between 90° and 105°, alternatively between 95° and 105°. In yet other embodiments, at least a lumen-facing surface of the pH protective coating may have a water contact angle between 50° and 80°, alternatively between 55° and 75°, alternatively between 60° and 70°.

In some embodiments, for instance, at least a lumen-facing surface of the pH protective coating may have a surface free energy, measured using the Kitazaki-Hata Method, between 20 $mJ/m^2$ and 50 $mJ/m^2$, alternatively between 25 $mJ/m^2$ and 50 $mJ/m^2$, alternatively between 20 $mJ/m^2$ and 45 $mJ/m^2$, alternatively between 25 $mJ/m^2$ and 45 $mJ/m^2$, alternatively between 20 $mJ/m^2$ and 40 $mJ/m^2$, alternatively between 25 $mJ/m^2$ and 40 $mJ/m^2$. In other embodiments, at least a lumen-facing surface of the pH protective coating may have a surface free energy, measured using the Kitazaki-Hata Method, between 60 $mJ/m^2$ and 100 $mJ/m^2$, alternatively between 60 $mJ/m^2$ and 90 $mJ/m^2$, alternatively between 65 $mJ/m^2$ and 100 $mJ/m^2$, alternatively between 65 $mJ/m^2$ and 90 $mJ/m^2$, alternatively between 70 $mJ/m^2$ and 100 $mJ/m^2$, alternatively between 70 $mJ/m^2$ and 90 $mJ/m^2$.

A plurality of thermoplastic syringe barrels, syringes, or drug primary packages as previously described is contemplated in any embodiment, in which the syringe barrels have consistent inner diameters with a standard deviation less than 0.03 mm, optionally less than 0.02 mm, optionally less than 0.01 mm, optionally less than 0.008 mm, optionally less than 0.006 mm, optionally less than 0.005 mm, optionally less than 0.004 mm. A plurality of thermoplastic syringe barrels, syringes, or drug primary packages as previously described is contemplated in any embodiment, in which the syringe barrels have consistent needle hub outer diameters, with a standard deviation less than 0.15 mm, optionally less than 0.10 mm, optionally less than 0.08 mm, optionally less than 0.05 mm, optionally less than 0.02 mm, optionally less than 0.008 mm, optionally less than 0.005 mm. A plurality of thermoplastic syringe barrels, syringes, or drug primary packages as previously described is contemplated in any embodiment, in which the syringe barrels have consistent lengths, with a standard deviation less than 0.06 mm, optionally less than 0.05 mm, optionally less than 0.04 mm, optionally less than 0.03 mm, optionally less than 0.02 mm, optionally less than 0.01 mm. A plurality of thermoplastic syringe barrels, syringes, or drug primary packages as previously described is contemplated in any embodiment, in which the syringe barrels have consistent weights, with a standard deviation less than 0.025 g, optionally less than 0.020 g, optionally less than 0.015 g, optionally less than 0.010 g, optionally less than 0.0075 g, optionally less than 0.005 g. A plurality of thermoplastic syringe barrels, syringes, or drug primary packages as previously described is contemplated in any embodiment, in which the standard deviation is calculated across a sample of at least 20 units, optionally at least 50 units, optionally at least 100 units, optionally at least 200 units, optionally at least 300 units.

A thermoplastic syringe barrel, syringe, or drug primary package as previously described is contemplated in any embodiment, in which the gas barrier coating comprises an oxygen barrier coating or layer, the oxygen barrier coating or layer being effective to reduce the ingress of oxygen into the lumen to less than 0.0005 cc/package/day at 25 °C, 60% relative humidity and 0.21 bar, optionally less than 0.0004 cc/package/day at 25 °C, 60% relative humidity and 0.21 bar, optionally less than 0.0003 cc/package/day at 25 °C, 60% relative humidity and 0.21 bar, optionally less than 0.0002 cc/package/day at 25 °C, 60% relative humidity and 0.21 bar, optionally less than 0.0001 cc/package/day at 25 °C, 60% relative humidity and 0.21 bar.

A thermoplastic syringe barrel, syringe, or drug primary package as previously described is contemplated in any embodiment, in which the gas barrier coating comprises an oxygen barrier coating or layer, the oxygen barrier coating or layer being effective to provide the syringe barrel, syringe, or drug primary package with an oxygen transmission rate constant less than 0.0010 $d^{-1}$; optionally less than 0.0008 $d^{-1}$; optionally less than 0.0006 $d^{-1}$; optionally less than 0.0004 $d^{-1}$; optionally less than 0.0003 $d^{-1}$; optionally less than 0.0002 $d^{-1}$; optionally less than 0.0001 $d^{-1}$.

A thermoplastic syringe barrel, syringe, or drug primary package as previously described is contemplated in any embodiment, in which the gas barrier coating comprises an oxygen barrier coating or layer, wherein the oxygen barrier coating or layer consists essentially of a plurality of atomic monolayers, optionally wherein the oxygen barrier coating or layer is deposited by atomic layer deposition, optionally by plasma-assisted atomic layer deposition.

A thermoplastic syringe barrel, syringe, or drug primary package as previously described is contemplated in any embodiment, in which the oxygen barrier coating or layer comprises or consists essentially of a metal oxide, optionally $Al_2O_3$. A thermoplastic syringe barrel, syringe, or drug primary package as previously described is contemplated in any embodiment, in which oxygen barrier coating or layer comprises or consists essentially of $SiO_x$, wherein x is from 1.5 to 2.9, optionally wherein x is 2.

A thermoplastic syringe barrel, syringe, or drug primary package as previously described is contemplated in any embodiment, in which the gas barrier coating comprises a water vapor barrier coating or layer, the water vapor barrier

coating or layer being effective to reduce the ingress of water vapor into the lumen to less than 0.05 mg/package/day at 60 °C and 40% relative humidity, optionally less than 0.04 mg/package/day at 60 °C and 40% relative humidity, optionally less than 0.03 mg/package/day at 60 °C and 40% relative humidity, optionally less than 0.02 mg/package/day at 60 °C and 40% relative humidity, optionally less than 0.01 mg/package/day at 60 °C and 40% relative humidity.

A thermoplastic syringe barrel, syringe, or drug primary package as previously described is contemplated in any embodiment, in which in which the gas barrier coating comprises a water vapor barrier coating or layer and in which the water vapor barrier coating or layer consists essentially of a plurality of atomic monolayers, optionally wherein the water vapor barrier coating or layer is deposited by atomic layer deposition, optionally by plasma-assisted atomic layer deposition.

A thermoplastic syringe barrel, syringe, or drug primary package as previously described is contemplated in any embodiment, in which the water vapor barrier coating or layer comprises or consists essentially of a metal oxide, optionally $Al_2O_3$. A thermoplastic syringe barrel, syringe, or drug primary package as previously described is contemplated in any embodiment, in which the water vapor barrier coating or layer comprises or consists essentially of $SiO_x$, wherein x is from 1.5 to 2.9, optionally wherein x is 2.

A thermoplastic syringe barrel, syringe, or drug primary package as previously described is contemplated in any embodiment, further comprising a nitrogen gas in a headspace of the lumen, and in which the gas barrier coating comprises a nitrogen barrier coating or layer, the nitrogen barrier coating or layer being effective to reduce egress of the nitrogen gas out of the lumen to less than 0.0002 cc/package/day at 25 °C, 60% relative humidity and 0.21 bar, optionally less than 0.00015 cc/package/day at 25 °C, 60% relative humidity and 0.21 bar, optionally less than 0.0001 cc/package/-day at 25 °C, 60% relative humidity and 0.21 bar, optionally less than 0.00005 cc/package/day at 25 °C, 60% relative humidity and 0.21 bar, optionally less than 0.00002 cc/package/day at 25 °C, 60% relative humidity and 0.21 bar, optionally less than 0.00001 cc/package/day at 25 °C, 60% relative humidity and 0.21 bar.

A thermoplastic syringe barrel, syringe, or drug primary package as previously described is contemplated in any embodiment, in which the gas barrier coating comprises a nitrogen barrier coating or layer, the nitrogen barrier coating or layer being effective to provide the syringe barrel, syringe, or drug primary package with a nitrogen transmission rate constant (NTR) less than 0.0003 $d^{-1}$; optionally less than 0.0002 $d^{-1}$; optionally less than 0.0001 $d^{-1}$, optionally less than 0.00008 $d^{-1}$; optionally less than 0.00006 $d^{-1}$; optionally less than 0.00004 $d^{-1}$, optionally less than 0.00003 $d^{-1}$; optionally less than 0.00002 $d^{-1}$; optionally less than 0.00001 $d^{-1}$.

A thermoplastic syringe barrel, syringe, or drug primary package as previously described is contemplated in any embodiment, in which the gas barrier coating comprises a nitrogen barrier coating or layer, and in which the nitrogen barrier coating or layer consists essentially of a plurality of atomic monolayers, optionally wherein the nitrogen barrier coating or layer is deposited by atomic layer deposition, optionally by plasma-assisted atomic layer deposition.

A thermoplastic syringe barrel, syringe, or drug primary package as previously described is contemplated in any embodiment, in which the nitrogen barrier coating or layer comprises or consists essentially of a metal oxide, optionally $Al_2O_3$. A thermoplastic syringe barrel, syringe, or drug primary package as previously described is contemplated in any embodiment, in which the nitrogen barrier coating or layer comprises or consists essentially of $SiO_x$, wherein x is from 1.5 to 2.9, optionally wherein x is 2.

A thermoplastic syringe barrel, syringe, or drug primary package as previously described is contemplated in any embodiment, further comprising carbon monoxide in the lumen, and in which the gas barrier coating comprises a carbon monoxide barrier coating or layer, the carbon monoxide barrier coating or layer being effective to reduce egress of carbon monoxide out of the lumen to less than 0.0002 cc/package/day at 25 °C, 60% relative humidity and 0.21 bar, optionally less than 0.00015 cc/package/day at 25 °C, 60% relative humidity and 0.21 bar, optionally less than 0.0001 cc/package/day at 25 °C, 60% relative humidity and 0.21 bar, optionally less than 0.00005 cc/package/day at 25 °C, 60% relative humidity and 0.21 bar, optionally less than 0.00002 cc/package/day at 25 °C, 60% relative humidity and 0.21 bar, optionally less than 0.00001 cc/package/day at 25 °C, 60% relative humidity and 0.21 bar.

A thermoplastic syringe barrel, syringe, or drug primary package as previously described is contemplated in any embodiment, in which the gas barrier coating comprises a carbon monoxide barrier coating or layer, the carbon monoxide barrier coating or layer being effective to provide the syringe barrel, syringe, or drug primary package with a carbon monoxide transmission rate (COTR) less than 0.0003 $d^{-1}$; optionally less than 0.0002 $d^{-1}$; optionally less than 0.0001 $d^{-1}$, optionally less than 0.00008 $d^{-1}$; optionally less than 0.00006 $d^{-1}$; optionally less than 0.00004 $d^{-1}$, optionally less than 0.00003 $d^{-1}$; optionally less than 0.00002 $d^{-1}$; optionally less than 0.00001 $d^{-1}$.

A thermoplastic syringe barrel, syringe, or drug primary package as previously described is contemplated in any embodiment, in which the gas barrier coating comprises a carbon monoxide barrier coating or layer, and in which the carbon monoxide barrier coating or layer consists essentially of a plurality of atomic monolayers, optionally wherein the carbon monoxide barrier coating or layer is deposited by atomic layer deposition, optionally by plasma-assisted atomic layer deposition.

A thermoplastic syringe barrel, syringe, or drug primary package as previously described is contemplated in any embodiment, in which the carbon monoxide barrier coating or layer comprises or consists essentially of a metal oxide,

optionally $Al_2O_3$. A thermoplastic syringe barrel, syringe, or drug primary package as previously described is contemplated in any embodiment, in which the carbon monoxide barrier coating or layer comprises or consists essentially of $SiO_x$, wherein x is from 1.5 to 2.9, optionally wherein x is 2.

A thermoplastic syringe barrel, syringe, or drug primary package as previously described is contemplated in any embodiment, further comprising carbon dioxide in the lumen, and in which the gas barrier coating comprises a carbon dioxide barrier coating or layer, the carbon dioxide barrier coating or layer being effective to reduce egress of carbon dioxide out of the lumen to less than 0.005 cc/package/day at 25 °C, 60% relative humidity and 0.21 bar, optionally less than 0.004 cc/package/day at 25 °C, 60% relative humidity and 0.21 bar, optionally less than 0.003 cc/package/day at 25 °C, 60% relative humidity and 0.21 bar, optionally less than 0.002 cc/package/day at 25 °C, 60% relative humidity and 0.21 bar, optionally less than 0.001 cc/package/day at 25 °C, 60% relative humidity and 0.21 bar, optionally less than 0.0008 cc/package/day at 25 °C, 60% relative humidity and 0.21 bar, optionally less than 0.0005 cc/package/day at 25 °C, 60% relative humidity and 0.21 bar.

A thermoplastic syringe barrel, syringe, or drug primary package as previously described is contemplated in any embodiment, in which the gas barrier coating comprises a carbon dioxide barrier coating or layer, the carbon dioxide barrier coating or layer being effective to provide the syringe barrel, syringe, or drug primary package with a carbon dioxide transmission rate (CO2TR) less than 0.005 d-1; optionally less than 0.004 d-1; optionally less than 0.002 d-1; optionally less than 0.001 d-1; optionally less than 0.0008 d-1, optionally less than 0.0006 d-1; optionally less than 0.0005 d-1; optionally less than 0.0004 d-1, optionally less than 0.0003 d-1; optionally less than 0.0002 d-1; optionally less than 0.0001 d-1.

A thermoplastic syringe barrel, syringe, or drug primary package as previously described is contemplated in any embodiment, in which the gas barrier coating comprises a carbon dioxide barrier coating or layer, wherein the carbon dioxide barrier coating or layer consists essentially of a plurality of atomic monolayers, optionally wherein the carbon dioxide barrier coating or layer is deposited by atomic layer deposition, optionally by plasma-assisted atomic layer deposition.

A thermoplastic syringe barrel, syringe, or drug primary package as previously described is contemplated in any embodiment, in which the carbon dioxide barrier coating or layer comprises or consists essentially of a metal oxide, optionally $Al_2O_3$. A thermoplastic syringe barrel, syringe, or drug primary package as previously described is contemplated in any embodiment, in which the carbon dioxide barrier coating or layer comprises or consists essentially of $SiO_x$, wherein x is from 1.5 to 2.9, optionally wherein x is 2.

A thermoplastic syringe barrel, syringe, or drug primary package as previously described is contemplated in any embodiment, in which the gas barrier coating comprises an ethylene oxide barrier coating or layer. A thermoplastic syringe barrel, syringe, or drug primary package as previously described is contemplated in any embodiment, in which the drug primary package is terminally sterilized, optionally using ethylene oxide.

A thermoplastic syringe barrel, syringe, or drug primary package as previously described is contemplated in any embodiment, in which the syringe barrel consists predominantly of a thermoplastic material selected from the following: PET, PETG, polypropylene, a polyamide, polystyrene, polycarbonate, TRITAN™, a cyclic block copolymer (CBC) resin, a thermoplastic olefinic polymer, COP, COC, or any combination thereof.

A thermoplastic syringe barrel, syringe, or drug primary package as previously described is contemplated in any embodiment, further comprising a rigid needle shield.

A thermoplastic syringe barrel, syringe, or drug primary package, or a plurality of thermoplastic syringe barrels, syringes, or drug primary packages, as previously described is contemplated in any embodiment, in which the package(s) or syringe(s) is configured to maintain container closure integrity (CCI) when cycled between -20 °C and 10 °C, optionally when cycled between -20 °C and 20 °C, optionally when cycled between -20 °C and 30 °C, optionally when cycled between -20 °C and 40 °C, optionally when cycled between -40 °C and 10 °C, optionally when cycled between -40 °C and 20 °C, optionally when cycled between -40 °C and 30 °C, optionally when cycled between -40 °C and 40 °C, optionally when cycled between -70 °C and 10 °C, optionally when cycled between -70 °C and 20 °C, optionally when cycled between -70 °C and 30 °C, optionally when cycled between -70 °C and 40 °C. In some embodiments, the package(s) or syringe(s) may be subjected to at least three cycles, optionally in which the package(s) or syringe(s) is subjected to three cycles. During each cycle the package(s) or syringe(s) may be held both at the lower temperature for 24 hours or more and at the upper temperature for 24 hours or more; optionally in which during each cycle the package(s) or syringe(s) is held both at the lower temperature for about 24 hours and at the upper temperature for about 24 hours.

A thermoplastic syringe barrel, syringe, or drug primary package, or a plurality of thermoplastic syringe barrels, syringes, or drug primary packages, as previously described is contemplated in any embodiment, in which the fill volume of the syringe(s) is within at least 20% of the nominal volume of the syringe, optionally in which the fill volume of the syringe is within at least 10% of the nominal volume of the syringe, optionally in which the fill volume of the syringe is within at least 5% of the nominal volume of the syringe. In some embodiments, the syringe(s) may have a nominal fill volume between 0.25 and 10 mL, optionally between 0.5 and 5 mL, optionally between 0.5 and 1 mL, optionally 0.5 mL, optionally 1 mL, optionally 2.25 mL.

A plurality of thermoplastic syringe barrels, syringes, or drug primary packages, as previously described is contemplated in any embodiment, in which the plurality of drug primary packages, syringes, or syringe barrels comprises at least 50 previously untested packages, syringes, or syringe barrels, optionally in which the plurality of drug primary packages, syringes, or syringe barrels consists of a sample of 50 previously untested packages, syringes, or syringe barrels, optionally in which the plurality of drug primary packages, syringes, or syringe barrels comprises at least 100 previously untested packages, syringes, or syringe barrels, optionally in which the plurality of drug primary packages, syringes, or syringe barrels consists of a sample of 100 previously untested packages, syringes, or syringe barrels, optionally in which the plurality of drug primary packages, syringes, or syringe barrels comprises at least 500 previously untested packages, syringes, or syringe barrels, optionally in which the plurality of drug primary packages, syringes, or syringe barrels consists of a sample of 500 previously untested packages, syringes, or syringe barrels, optionally in which the plurality of drug primary packages, syringes, or syringe barrels comprises at least 1000 previously untested packages, syringes, or syringe barrels, optionally in which the plurality of drug primary packages, syringes, or syringe barrels consists of a sample of 1000 previously untested packages, syringes, or syringe barrels.

A syringe or drug primary package as previously described is contemplated in any embodiment, in which the plunger comprises a gasket attached to a distal end of the plunger, optionally in which the gasket comprises an elastic material. In some embodiments, the gasket may have a film, optionally a fluoropolymer film, residing on at least a circumferential outer surface portion. In some embodiments, the gasket may have one or more channels on at least a circumferential outer surface portion. In some embodiments, at least one, and optionally each, of the one or more channels is non-continuous and comprises a non-channel interrupting portion. In some embodiments, the gasket may comprise a plurality of channels on a circumferential outer surface portion, each of plurality of channels being approximately parallel with and axially spaced from one another. In some embodiments, the non-channel interrupting portion of each of the plurality of channels is not aligned with the non-channel interrupting portion of one or more adjacent channels.

A syringe or drug primary package as previously described is contemplated in any embodiment, in which the plunger and attached gasket has a break loose force between 4 and 20 Newtons (N). A syringe or drug primary package as previously described is contemplated in any embodiment, in which the plunger and attached gasket has a glide force between 4 and 20 Newtons (N).

A syringe or drug primary package as previously described is contemplated in any embodiment, in which the syringe barrel and gasket are respectively sized to provide spacing between a smallest syringe barrel inner diameter and a largest gasket outer diameter, when assembled, deviating from the nominal spacing by no more than: $\pm$ 100 microns, $\pm$ 50 microns, $\pm$ 35 microns, $\pm$ 25 microns, $\pm$ 20 microns, $\pm$ 15 microns, $\pm$ 10 microns, $\pm$ 5 microns or $\pm$ 2 microns.

A syringe or drug primary package as previously described is contemplated in any embodiment, in which in which the package or syringe is configured such that the plunger does not move axially when the package or syringe is cycled between -20 °C and 10 °C, optionally when cycled between -20 °C and 20 °C, optionally when cycled between -20 °C and 30 °C, optionally when cycled between -20 °C and 40 °C, optionally when cycled between -40 °C and 10 °C, optionally when cycled between -40 °C and 20 °C, optionally when cycled between -40 °C and 30 °C, optionally when cycled between -40 °C and 40 °C, optionally when cycled between - 70 °C and 10 °C, optionally when cycled between -70 °C and 20 °C, optionally when cycled between -70 °C and 30 °C, optionally when cycled between -70 °C and 40 °C.

A syringe or drug primary package as previously described is contemplated in any embodiment, which includes a plunger rod and a backstop element that together prevent axially rearward movement of the plunger.

A syringe or drug primary package as previously described is contemplated in any embodiment, in which the plunger rod and backstop element together prevent axially rearward movement of the plunger when the package or filled syringe is subjected to a temperature at or below -20 °C, optionally a temperature at or below -30 °C, optionally a temperature at or below - 40 °C, optionally a temperature at or below -50 °C, optionally a temperature at or below -60 °C, optionally a temperature at or below -70 °C. In some embodiments, the plunger rod and backstop element may prevent axially rearward movement of the plunger when the package or filled syringe is cycled between -20 °C and 10 °C, optionally when cycled between -20 °C and 20 °C, optionally when cycled between -20 °C and 30 °C, optionally when cycled between -20 °C and 40 °C, optionally when cycled between -40 °C and 10 °C, optionally when cycled between -40 °C and 20 °C, optionally when cycled between -40 °C and 30 °C, optionally when cycled between - 40 °C and 40 °C, optionally when cycled between -70 °C and 10 °C, optionally when cycled between -70 °C and 20 °C, optionally when cycled between -70 °C and 30 °C, optionally when cycled between -70 °C and 40 °C.

A syringe or drug primary package as previously described is contemplated in any embodiment, in which the backstop element is attached to the syringe barrel and extends over top of the rear opening. A syringe or drug primary package as previously described is contemplated in any embodiment, in which the backstop element comprises an extended finger flange.

A syringe or drug primary package as previously described is contemplated in any embodiment, in which the backstop engagement feature is a radial projection, optionally a radially-projecting continuous ring or a radially-projecting discontinuous ring. In some embodiments, the backstop engagement feature may be wedge-shaped.

A syringe or drug primary package as previously described is contemplated in any embodiment, in which the

backstop element comprises an aperture, the aperture being aligned with the rear opening of the syringe barrel. In some embodiments, the aperture may be defined by an interior wall, optionally one in which at least a portion of the interior wall is angled inward moving toward the rear opening of the syringe barrel.

A syringe or drug primary package as previously described is contemplated in any embodiment, in which, once the plunger rod has been inserted into the syringe barrel to its stop position, a rearward force on the plunger rod causes the backstop engagement feature to abut against a contact surface of the backstop element, thereby preventing further rearward movement of the plunger rod; optionally wherein the contact surface of the backstop element comprises the lower edge of the interior wall of the aperture.

A syringe or drug primary package as previously described is contemplated in any embodiment, in which the backstop engagement feature is positioned adjacent the contact surface of the backstop when the plunger is in its stop position within the syringe barrel; optionally in which the two are within about 1.5 mm, optionally within about 1.0 mm, optionally within about 0.75 mm, optionally within about 0.5 mm, optionally within about 0.25 mm.

A syringe or drug primary package as previously described is contemplated in any embodiment, in which the position of the backstop engagement feature on the plunger rod is coordinated with the plunger insertion depth in the syringe barrel that corresponds to a fill volume of a filled and fully assembled drug primary package.

A syringe or drug primary package as previously described is contemplated in any embodiment, in which the backstop element comprises a locking collet, a threaded housing, and a twist lock thumb nut and in which the plunger rod does not comprise a backstop engagement feature.

In some embodiments, the backstop element comprises an aperture, the aperture is aligned with the rear opening of the syringe barrel, and at least part of the aperture is defined by a flexible locking collet. The flexible locking collet may be configured to be compressed such that an interior surface of the locking collet presses against a portion of a plunger rod that extends within the aperture. And a lower portion of the twist lock thumb nut may be configured to interface with the upper portion of the locking collet to compress the locking collet. A threaded housing, e.g. a housing having a threaded interior wall, may at least partially surrounds the locking collet and may be configured to engage with a threaded portion of the twist lock thumb nut. The threaded housing may be engaged with a portion of the backstop element to secure the threaded housing in place, e.g. by a snap-on connection.

In some embodiments, an upper portion of the locking collet may be drafted such that the upper portion of the locking collet has an increased diameter moving downward. In some embodiments, the locking collet may be divided into a plurality of sections by circumferential gaps. In some embodiments, the lower wall portion of the twist lock thumb nut may be drafted such that the aperture defined by the lower wall portion of the thumb nut has an increased diameter moving downward.

A syringe or drug primary package as previously described is contemplated in any embodiment, in which the backstop element comprises a locking block cavity and a locking block that is slidable within the locking block cavity.

In some embodiments, the backstop element may comprise a central aperture that is aligned with the rear opening of the syringe barrel and the locking block cavity may be transverse to the central aperture. The locking block may comprise an aperture having a larger cross-section portion and a smaller cross-section portion, in which the effective diameter of the larger cross-section portion is greater than the diameter of the backstop engagement feature(s) on the plunger rod and the effective diameter of the smaller cross-section portion is less than the diameter of the backstop engagement feature(s) on the plunger rod. Sliding the locking block into the locked position may bring the smaller cross-section portion of the aperture into alignment with the rear opening of the syringe barrel; and sliding the locking block into the unlocked position may bring the larger cross-section portion of the aperture into alignment with the rear opening of the syringe barrel. When the locking block is in a locked position, a rearward force on the plunger rod causes a backstop engagement feature of the plunger rod to abut against a lower contact surface of the locking block, thereby preventing further rearward movement of the plunger rod.

In some embodiments, an interior wall that at least partially defines the smaller cross-section portion may have a radius of curvature that substantially corresponds with that of the plunger rod.

In some embodiments, the larger cross-section portion and the smaller cross-section portion are separated by one or more ribs, optionally by a pair of opposing ribs located on the side walls. Each of the one or more ribs may comprise an angled or curved surface facing the larger cross-section portion of the aperture, and the angled or curved surface may be configured to facilitate movement of the rib surface over the plunger rod when the locking block is moved from an unlocked position to a locked position. Each of the one or more ribs may comprise an angled or curved surface facing the smaller cross-section portion of the aperture, and the angled or curved surface may be configured to facilitate movement of the rib surface over the plunger rod when the locking block is moved from a locked position to an unlocked position.

In some embodiments, the locking block may comprise a first end and a second end, the locking block being configured so that (i) a user can slide the locking block into an unlocked position by pressing on the first end, and (ii) a user can slide the locking block into a locked position by pressing on the second end. The first end may comprise a marking to identify that pressing the first end brings the locking block into the unlocked position and/or the second end may comprise a marking to identify that pressing the second end brings the locking block into the locked position.

In some embodiments, the plunger rod may have one or more backstop engagement features, optionally two or more backstop engagement features. In some embodiments, one of the one or more backstop engagements feature is positioned adjacent the lower contact surface of the locking block when the plunger is in its stop position within the syringe barrel; optionally with the two being within about 1.5 mm, optionally within about 1.0 mm, optionally within about 0.75 mm, optionally within about 0.5 mm, optionally within about 0.25 mm.

In some embodiments, wherein when the locking block is in a locked position, a forward force on the plunger rod may cause a second one of the one or more backstop engagement features to abut against an upper contact surface of the locking block, thereby preventing further forward movement of the plunger rod. In some embodiments, the second one of the one or more backstop engagements feature is positioned adjacent the upper contact surface of the locking block when the plunger is in its stop position within the syringe barrel; optionally with the two being within about 1.5 mm, optionally within about 1.0 mm, optionally within about 0.75 mm, optionally within about 0.5 mm, optionally within about 0.25 mm.

In some embodiments, the plunger rod may instead not comprise any backstop engagement features, and the smaller cross-section portion of the aperture may instead be configured to create an interference fit with the plunger rod.

In some embodiments, the backstop element may further comprise (i) one or more retention elements that require a threshold force to be applied to slide the locking block out of the locked position; (ii) one or more retention elements that require a threshold force to be applied to slide the locking block out of the unlocked position; or (iii) both (i) and (ii). For example, at least one of an interior surface defining the locking block cavity and an exterior surface of the locking block may comprise one or more retention ribs and the other of the interior surface defining the locking block cavity and the exterior surface of the locking block may comprise one or more indents, and wherein at least one of the one or more indents is configured to receive at least one of the one or more retention ribs when the locking block is in the locked position. Similarly, at least one of an interior surface defining the locking block cavity and an exterior surface of the locking block may comprise one or more retention ribs and the other of the interior surface defining the locking block cavity and the exterior surface of the locking block may comprise one or more indents, and wherein at least one of the one or more indents is configured to receive at least one of the one or more retention ribs when the locking block is in the unlocked position.

A syringe or drug primary package as previously described is contemplated in any embodiment, in which the backstop element is configured to prevent movement of the plunger in both axial rearward and axial forward directions.

A syringe or drug primary package as previously described is contemplated in any embodiment, in which the backstop element is configured so that a user can place the package or syringe in a locked configuration, in which the plunger rod is prevented from moving within the syringe barrel; and an unlocked configuration, in which the plunger rod moves within the syringe barrel. In some embodiments, for example, the backstop element may be configured so that a user moves between the locked configuration and the unlocked configuration by rotating a rotatable component of the backstop element, optionally a twist lock thumb nut. In other embodiments, for example, the backstop element may be configured so that a user moves between the locked configuration and the unlocked configuration by pushing a movable component of the backstop element, optionally a locking block, in a direction transverse to longitudinal axis of the syringe barrel.

A syringe or drug primary package as previously described is contemplated in any embodiment, in which the backstop element is configured so that, when in an unlocked configuration, the plunger rod moves within the syringe barrel with no resistance or substantially no resistance from the backstop element. In some embodiments, for instance, when in an unlocked configuration, the plunger sliding force may be the same or substantially the same as the plunger sliding force of the same package or syringe but without the backstop element; optionally in which the plunger sliding force is within 10%, optionally within 5%, optionally within 3%, optionally within 1% of the plunger sliding force of the same package or syringe but without the backstop element. In some embodiments, for instance, when in an unlocked configuration, the plunger breakout force may be the same or substantially the same as the plunger breakout force of the same package or syringe but without the backstop element; optionally in which the plunger breakout force is within 10%, optionally within 5%, optionally within 3%, optionally within 1% of the plunger breakout force of the same package or syringe but without the backstop element.

An aspect of the present invention is an evacuated blood tube comprising a lumen defined at least in part by a thermoplastic side wall, the thermoplastic side wall having an interior surface facing the lumen and an outer surface; a gas barrier coating supported by at least one of the interior surface and the outer surface of the side wall, at least a portion of the gas barrier coating consisting essentially of a plurality of atomic monolayers of a pure element or compound; a top defining an opening; and a stopper seated within the opening and sealing the lumen.

An evacuated blood tube as previously described is contemplated in any embodiment, in which the gas barrier coating comprises an oxygen barrier coating or layer, the oxygen barrier coating or layer being effective to reduce the ingress of oxygen into the lumen to less than 0.0005 cc/package/day at 25 °C, 60% relative humidity and 0.21 bar, optionally less than 0.0004 cc/package/day at 25 °C, 60% relative humidity and 0.21 bar, optionally less than 0.0003 cc/package/day at 25 °C, 60% relative humidity and 0.21 bar, optionally less than 0.0002 cc/package/day at 25 °C, 60% relative humidity and 0.21 bar, optionally less than 0.0001 cc/package/day at 25 °C, 60% relative humidity and 0.21 bar.

An evacuated blood tube as previously described is contemplated in any embodiment, in which the gas barrier

coating comprises an oxygen barrier coating or layer, the oxygen barrier coating or layer being effective to provide the evacuated blood tube with an oxygen transmission rate constant less than 0.0010 $d^{-1}$; optionally less than 0.0008 $d^{-1}$; optionally less than 0.0006 $d^{-1}$; optionally less than 0.0004 $d^{-1}$; optionally less than 0.0003 $d^{-1}$; optionally less than 0.0002 $d^{-1}$; optionally less than 0.0001 $d^{-1}$.

An evacuated blood tube as previously described is contemplated in any embodiment, in which the gas barrier coating comprises an oxygen barrier coating or layer, wherein the oxygen barrier coating or layer consists essentially of a plurality of atomic monolayers, optionally wherein the oxygen barrier coating or layer is deposited by atomic layer deposition, optionally by plasma-assisted atomic layer deposition.

An evacuated blood tube as previously described is contemplated in any embodiment, in which the oxygen barrier coating or layer comprises or consists essentially of a metal oxide, optionally $Al_2O_3$. An evacuated blood tube as previously described is contemplated in any embodiment, in which oxygen barrier coating or layer comprises or consists essentially of $SiO_x$, wherein x is from 1.5 to 2.9, optionally wherein x is 2.

An evacuated blood tube as previously described is contemplated in any embodiment, in which the gas barrier coating comprises a water vapor barrier coating or layer, the water vapor barrier coating or layer being effective to reduce the ingress of water vapor into the lumen to less than 0.05 mg/package/day at 60 °C and 40% relative humidity, optionally less than 0.04 mg/package/day at 60 °C and 40% relative humidity, optionally less than 0.03 mg/package/day at 60 °C and 40% relative humidity, optionally less than 0.02 mg/package/day at 60 °C and 40% relative humidity, optionally less than 0.01 mg/package/day at 60 °C and 40% relative humidity.

An evacuated blood tube as previously described is contemplated in any embodiment, in which in which the gas barrier coating comprises a water vapor barrier coating or layer and in which the water vapor barrier coating or layer consists essentially of a plurality of atomic monolayers, optionally wherein the water vapor barrier coating or layer is deposited by atomic layer deposition, optionally by plasma-assisted atomic layer deposition.

An evacuated blood tube as previously described is contemplated in any embodiment, in which the water vapor barrier coating or layer comprises or consists essentially of a metal oxide, optionally $Al_2O_3$. An evacuated blood tube as previously described is contemplated in any embodiment, in which the water vapor barrier coating or layer comprises or consists essentially of $SiO_x$, wherein x is from 1.5 to 2.9, optionally wherein x is 2.

An evacuated blood tube as previously described is contemplated in any embodiment, further comprising a nitrogen gas in a headspace of the lumen, and in which the gas barrier coating comprises a nitrogen barrier coating or layer, the nitrogen barrier coating or layer being effective to reduce egress of the nitrogen gas out of the lumen to less than 0.0002 cc/package/day at 25 °C, 60% relative humidity and 0.21 bar, optionally less than 0.00015 cc/package/day at 25 °C, 60% relative humidity and 0.21 bar, optionally less than 0.0001 cc/package/day at 25 °C, 60% relative humidity and 0.21 bar, optionally less than 0.00005 cc/package/day at 25 °C, 60% relative humidity and 0.21 bar, optionally less than 0.00002 cc/package/day at 25 °C, 60% relative humidity and 0.21 bar, optionally less than 0.00001 cc/package/day at 25 °C, 60% relative humidity and 0.21 bar.

An evacuated blood tube as previously described is contemplated in any embodiment, in which the gas barrier coating comprises a nitrogen barrier coating or layer, the nitrogen barrier coating or layer being effective to provide the evacuated blood tube with a nitrogen transmission rate constant (NTR) less than 0.0003 $d^{-1}$; optionally less than 0.0002 $d^{-1}$; optionally less than 0.0001 $d^{-1}$, optionally less than 0.00008 $d^{-1}$; optionally less than 0.00006 $d^{-1}$; optionally less than 0.00004 $d^{-1}$, optionally less than 0.00003 $d^{-1}$; optionally less than 0.00002 $d^{-1}$; optionally less than 0.00001 $d^{-1}$.

An evacuated blood tube as previously described is contemplated in any embodiment, in which the gas barrier coating comprises a nitrogen barrier coating or layer, and in which the nitrogen barrier coating or layer consists essentially of a plurality of atomic monolayers, optionally wherein the nitrogen barrier coating or layer is deposited by atomic layer deposition, optionally by plasma-assisted atomic layer deposition.

An evacuated blood tube as previously described is contemplated in any embodiment, in which the nitrogen barrier coating or layer comprises or consists essentially of a metal oxide, optionally $Al_2O_3$. An evacuated blood tube as previously described is contemplated in any embodiment, in which the nitrogen barrier coating or layer comprises or consists essentially of $SiO_x$, wherein x is from 1.5 to 2.9, optionally wherein x is 2.

An evacuated blood tube as previously described is contemplated in any embodiment, further comprising carbon dioxide in the lumen, and in which the gas barrier coating comprises a carbon dioxide barrier coating or layer, the carbon dioxide barrier coating or layer being effective to reduce egress of carbon dioxide out of the lumen to less than 0.005 cc/package/day at 25 °C, 60% relative humidity and 0.21 bar, optionally less than 0.004 cc/package/day at 25 °C, 60% relative humidity and 0.21 bar, optionally less than 0.003 cc/package/day at 25 °C, 60% relative humidity and 0.21 bar, optionally less than 0.002 cc/package/day at 25 °C, 60% relative humidity and 0.21 bar, optionally less than 0.001 cc/package/day at 25 °C, 60% relative humidity and 0.21 bar, optionally less than 0.0008 cc/package/day at 25 °C, 60% relative humidity and 0.21 bar, optionally less than 0.0005 cc/package/day at 25 °C, 60% relative humidity and 0.21 bar.

An evacuated blood tube as previously described is contemplated in any embodiment, in which the gas barrier coating comprises a carbon dioxide barrier coating or layer, the carbon dioxide barrier coating or layer being effective to provide the evacuated blood tube with a carbon dioxide transmission rate (CO2TR) less than 0.005 d-1; optionally less

than 0.004 d-1; optionally less than 0.002 d-1; optionally less than 0.001 d-1; optionally less than 0.0008 d-1, optionally less than 0.0006 d-1; optionally less than 0.0005 d-1; optionally less than 0.0004 d-1, optionally less than 0.0003 d-1; optionally less than 0.0002 d-1; optionally less than 0.0001 d-1.

An evacuated blood tube as previously described is contemplated in any embodiment, in which the gas barrier coating comprises a carbon dioxide barrier coating or layer, wherein the carbon dioxide barrier coating or layer consists essentially of a plurality of atomic monolayers, optionally wherein the carbon dioxide barrier coating or layer is deposited by atomic layer deposition, optionally by plasma-assisted atomic layer deposition.

An evacuated blood tube as previously described is contemplated in any embodiment, in which the carbon dioxide barrier coating or layer comprises or consists essentially of a metal oxide, optionally $Al_2O_3$. An evacuated blood tube as previously described is contemplated in any embodiment, in which the carbon dioxide barrier coating or layer comprises or consists essentially of $SiO_x$, wherein x is from 1.5 to 2.9, optionally wherein x is 2.

An evacuated blood tube as previously described is contemplated in any embodiment, in which the gas barrier coating is effective to maintain a vacuum level within the lumen, relative to ambient pressure at sea level, sufficient to draw blood from a patient's vein into the lumen for at least 28 months, optionally at least 30 months, optionally at least 32 months, optionally at least 34 months, optionally at least 36 months.

An evacuated blood tube as previously described is contemplated in any embodiment, in which the gas barrier coating is effective to extend the shelf life of the evacuated blood tube to at least 28 months, optionally at least 30 months, optionally at least 32 months, optionally at least 34 months, optionally at least 36 months, the shelf life defined by the amount of time after evacuation the tube maintains a draw volume capacity of at least 90% of the draw volume capacity of a newly evacuated vessel of the same kind.

An evacuated blood tube as previously described is contemplated in any embodiment, further comprising a blood preservative within the lumen, and in which the gas barrier coating is effective to reduce the amount of solvent loss of the blood preservative over the shelf life of the blood tube.

An evacuated blood tube as previously described is contemplated in any embodiment, in which the gas barrier coating is supported by the interior surface of the wall, and optionally further comprising a pH protective coating between the lumen and the gas barrier coating.

A vessel, container, vial, syringe, or drug primary package, as previously described is contemplated in any embodiment, in which the fluid within the lumen comprises a member selected from the group consisting of:

## BIOLOGIC DRUGS

abatacept; abciximab; abobotulinumtoxinA; adalimumab; adalimumab-adaz; adalimumab-adbm; adalimumab-afzb; adalimumab-atto; adalimumab-bwwd; ado-trastuzumab emtansine; aflibercept; agalsidase beta; albiglutide; albumin chromated CR-51 serum; aldesleukin; alefacept; alemtuzumab; alglucosidase alfa; alirocumab; alteplase; anakinra; aprotinin; asfotas alfa; asparaginase; asparaginase Erwinia chrysanthemi; atezolizumab; avelumab; basiliximab; becaplermin; belatacept; belimumab; benralizumab; beractant; bevacizumab; bevacizumab-awwb; bevacizumab-bvzr; bezlotoxumab; blinatumomab; brentuximab vedotin; brodalumab; brolucizumab-dbll; burosumab-twza; calaspargase pegol-mknl; calfactant; canakinumab; caplacizumab-yhdp; capromab pendetide; cemiplimab-rwlc; cenegermin-bkbj; cerliponase alfa; certolizumab pegol; cetuximab; choriogonadotropin alfa; chorionic gonadotropin; chymopapain; collagenase; collagenase clostridium histolyticum; corticorelin ovine triflutate; crizanlizumab-tmca; daclizumab; daratumumab; daratumumab and hyaluronidase-fihj; darbepoetin alpha; denileukin diftitox; denosumab; desirudin; dinutuximab; dornase alfa; drotrecogin alfa; dulaglutide; dupilumab; durvalumab; ecallantide; eculizumab; efalizumab; elapegademase-lvlr; elosulfase alfa; elotuzumab; emapalumab-lzsg; emicizumab-kxwh; enfortumab vedotin-ejfv; epoetin alfa; epoetin alfa-epbx; erenumab-aooe; etanercept; etanercept-szzs; etanercept-ykro; evolocumab; fam-trastuzumab deruxtecan-nxki; fibrinolysin and desoxyribonuclease combined [bovine], with chloramphenicol; filgrastim; filgrastim-aafi; filgrastim-sndz; follitropin alfa; follitropin beta; fremanezumab-vfrm; galcanezumab-gnlm; galsulfase; gemtuzumab ozogamicin; glucarpidase; golimumab; guselkumab; hyaluronidase; hyaluronidase human; ibalizumab-uiyk; ibritumomab tiuxetan; idarucizumab; idursulfase; imiglucerase; incobotulinumtoxinA; inebilizumab-cdon; infliximab; infliximab-abda; infliximab-axxq; infliximab-dyyb; infliximab-qbtx; inotuzumab ozogamicin; insulin aspart; insulin aspart protamine and insulin aspart; insulin degludec; insulin degludec and insulin aspart; insulin degludec and liraglutide; insulin detemir; insulin glargine; insulin glargine and lixisenatide; insulin glulisine; insulin human; insulin isophane human; insulin isophane human and insulin human; insulin lispro; insulin lispro protamine and insulin lispro; insulin lispro-aabc; interferon alfa-2a; interferon alfa-2b; interferon alfacon-1; interferon alfa-n3 (human leukocyte derived); interferon beta-1a; interferon beta-1b; interferon gamma-1b; ipilimumab; isatuximab-irfc; ixekizumab; lanadelumab-flyo; laronidase; lixisenatide; luspatercept-aamt; mecasermin; mecasermin rinfabate; menotropins; mepolizumab; methoxy polyethylene glycol-epoetin beta; metreleptin; mogamulizumab-kpkc; moxetumomab pasudotox-tdfk; muromanab-CD3; natalizumab; necitumumab; nivolumab; nofetumomab; obiltoxaximab; obinutuzumab; ocrelizumab; ocriplasmin; ofatumumab; olaratumab; omalizumab; onabotulinumtoxinA; oprelvekin; palifermin; palivizumab; pancrelipase; panitumumab; parathyroid hormone;

pegademase bovine; pegaspargase; pegfilgrastim; pegfilgrastim-apgf; pegfilgrastim-bmez; pegfilgrastim-cbqv; pegfilgrastim-jmdb; peginterferon alfa-2a; peginterferon alfa-2a and ribavirin; peginterferon alfa-2b; peginterferon alfa-2b and ribavirin; peginterferon beta-1a; pegloticase; pegvaliase-pqpz; pegvisomant; pembrolizumab; pertuzumab; polatuzumab vedotin-piiq; poractant alfa; prabotulinumtoxinA-xvfs; radiolabeled albumin technetium Tc-99m albumin colloid kit; ramucirumab; ranibizumab; rasburicase; ravulizumab-cwvz; raxibacumab; reslizumab; reteplase; rilonacept; rimabotulinumtoxinB; risankizumab-rzaa; rituximab; rituximab and hyaluronidase human; rituximab-abbs; rituximab-pvvr; romiplostim; romosozumab-aqqg; sacituzumab govitecan-hziy; sacrosidase; sargramostim; sarilumab; sebelipase alfa; secukinumab; siltuximab; somatropin; tagraxofusp-erzs; taliglucerase alfa; tbo-filgrastim; technetium 99m tc fanolesomab; tenecteplase; teprotumumab-trbw; tesamorelin acetate; thyrotropin alfa; tildrakizumab-asmn; tocilizumab; tositumomab and iodine I-131 tositumomab; trastuzumab; trastuzumab and hyaluronidase-oysk; trastuzumab-anns; trastuzumab-dkst; trastuzumab-dttb; trastuzumab-pkrb; trastuzumab-qyyp; urofollitropin; urokinase; ustekinumab; vedolizumab; velaglucerase alfa; vestronidase alfa-vjbk; Ziv-Aflibercept; Amjevita (adalimumab-atto); Dupixent (dupilumab); Fulphila (pegfilgrastim-jmdb); Ilaris (canakinumab); Ixifi (infliximab-qbtx); Lyumjev (insulin lispro-aabc); Nyvepria (pegfilgrastim-apgf); Ogivri (trastuzumab-dkst); Semglee (insulin glargine); Uplizna (inebilizumab-cdon); A.P.L. (chorionic gonadotropin); Abrilada (adalimumab-afzb); Accretropin (somatropin); Actemra (tocilizumab); Acthrel (corticorelin ovine triflutate); Actimmune (interferon gamma-1b); Activase (alteplase); Adagen (pegademase bovine); Adakveo (crizanlizumab-tmca); Adcetris (brentuximab vedotin); Adlyxin (lixisenatide); Admelog (insulin lispro); Afrezza (insulin human); Aimovig (erenumab-aooe); Ajovy (fremanezumab-vfrm); Aldurazyme (laronidase); Alferon N Injection (interferon alfa-n3 (human leukocyte derived)); Amevive (alefacept); Amphadase (hyaluronidase); Anthim (obiltoxaximab); Apidra (insulin glulisine); Aranesp (darbepoetin alpha); Arcalyst (rilonacept); Arzerra (ofatumumab); Asparlas (calaspargase pegol-mknl); Avastin (bevacizumab); Avonex (interferon beta-1a); Avsola (infliximab-axxq); Basaglar (insulin glargine); Bavencio (avelumab); Benlysta (belimumab); Beovu (brolucizumab-dbll); Besponsa (inotuzumab ozogamicin); Betaseron (interferon beta-1b); Bexxar (tositumomab and iodine I-131 tositumomab); Blincyto (blinatumomab); Botox (onabotulinumtoxinA); Botox Cosmetic (onabotulinumtoxinA); Bravelle (urofollitropin); Brineura (cerliponase alfa); Cablivi (caplacizumab-yhdp); Campath (alemtuzumab); Cathflo Activase (alteplase); Cerezyme (imiglucerase); Chorionic Gonadotropin (chorionic gonadotropin); Chromalbin (albumin chromated CR-51 serum); Chymodiactin (chymopapain); Cimzia (certolizumab pegol); Cinqair (reslizumab); Cosentyx (secukinumab); Cotazym (pancrelipase); Creon (pancrelipase); Crysvita (burosumab-twza); Curosurf (poractant alfa); Cyltezo (adalimumab-adbm); Cyramza (ramucirumab); Darzalex (daratumumab); Darzalex Faspro (daratumumab and hyaluronidase-fihj); Draximage MAA (kit for the preparation of technetium Tc-99m albumin aggregated); Dysport (abobotulinumtoxinA); Egrifta (tesamorelin acetate); Egrifta SV (tesamorelin acetate); Elaprase (idursulfase); Elase-chloromycetin (fibrinolysin and desoxyribonuclease combined [bovine], with chloramphenicol); Elelyso (taliglucerase alfa); Elitek (rasburicase); Elspar (asparaginase); Elzonris (tagraxofusp-erzs); Emgality (galcanezumab-gnlm); Empliciti (elotuzumab); Enbrel (etanercept); Enbrel Mini (etanercept); Enhertu (fam-trastuzumab deruxetecan-nxki); Entyvio (vedolizumab); Epogen/Procrit (epoetin alfa); Erbitux (cetuximab); Erelzi (etanercept-szzs); Erelzi Sensoready (etanercept-szzs); Erwinaze (asparaginase Erwinia chrysanthemi); Eticovo (etanercept-ykro); Evenity (romosozumab-aqqg); Extavia (interferon beta-1b); Eylea (aflibercept); Fabrazyme (agalsidase beta); Fasenra (benralizumab); Fiasp (insulin aspart); Follistim (follitropin beta); Follistim AQ (follitropin beta); Follistim AQ Cartridge (follitropin beta); Gamifant (emapalumab-lzsg); Gazyva (obinutuzumab); Genotropin (somatropin); Gonal-f (follitropin alfa); Gonal-f RFF (follitropin alfa); Gonal-f RFF RediJect (follitropin alfa); Granix (tbo-filgrastim); Hadlima (adalimumab-bwwd); Hemlibra (emicizumab-kxwh); Herceptin (trastuzumab); Herceptin Hylecta (trastuzumab and hyaluronidase-oysk); Herzuma (trastuzumab-pkrb); Humalog (insulin lispro); Humalog Mix 50/50 (insulin lispro protamine and insulin lispro); Humalog Mix 75/25 (insulin lispro protamine and insulin lispro); Humatrope (somatropin); Humegon (menotropins); Humira (adalimumab); Humulin 70/30 (insulin isophane human and insulin human); Humulin N (insulin isophane human); Humulin R U-100 (insulin human); Humulin R U-500 (insulin human); Hydase (hyaluronidase); Hylenex recombinant (hyaluronidase human); Hyrimoz (adalimumab-adaz); Ilumya (tildrakizumab-asmn); Imfinzi (durvalumab); Increlex (mecasermin); Infasurf (calfactant); Infergen (interferon alfacon-1); Inflectra (infliximab-dyyb); Intron A (interferon alfa-2b); Iplex (mecasermin rinfabate); Iprivask (desirudin); Jeanatope (kit for iodinated I-125 albumin); Jetrea (ocriplasmin); Jeuveau (prabotulinumtoxinA-xvfs); Kadcyla (ado-trastuzumab emtansine); Kalbitor (ecallantide); Kanjinti (trastuzumab-anns); Kanuma (sebelipase alfa); Kepivance (palifermin); Kevzara (sarilumab); Keytruda (pembrolizumab); Kineret (anakinra); Kinlytic (urokinase); Krystexxa (pegloticase); Lantus (insulin glargine); Lartruvo (olaratumab); Lemtrada (alemtuzumab); Leukine (sargramostim); Levemir (insulin detemir); Libtayo (cemiplimab-rwlc); Lucentis (ranibizumab); Lumizyme (alglucosidase alfa); Lumoxiti (moxetumomab pasudotox-tdfk); Macrotec (kit for the preparation of technetium Tc-99m albumin aggregated); Megatope (kit for iodinated 1-131 albumin); Menopur (menotropins); Mepsevii (vestronidase alfa-vjbk); Microlite (radiolabeled albumin technetium Tc-99m albumin colloid kit); Mircera (methoxy polyethylene glycol-epoetin beta); Mvasi (bevacizumab-awwb); Myalept (metreleptin); Mylotarg (gemtuzumab ozogamicin); Myobloc (rimabotulinumtoxinB); Myozyme (alglucosidase alfa); Myxredlin (insulin human); N/A (raxibacumab); Naglazyme (galsulfase); Natpara (parathyroid hormone); Neulasta (pegfilgrastim); Neulasta Onpro (pegfilgrastim); Neumega (oprelvekin); Neupogen (filgrastim); NeutroSpec (technetium 99m tc fanolesomab); Nivestym (filgrastim-aafi);

Norditropin (somatropin); Novarel (chorionic gonadotropin); Novolin 70/30 (insulin isophane human and insulin human); Novolin N (insulin isophane human); Novolin R (insulin human); Novolog (insulin aspart); Novolog Mix 50/50 (insulin aspart protamine and insulin aspart); Novolog Mix 70/30 (insulin aspart protamine and insulin aspart); Nplate (romiplostim); Nucala (mepolizumab); Nulojix (belatacept); Nutropin (somatropin); Nutropin AQ (somatropin); Ocrevus (ocrelizumab); Omnitrope (somatropin); Oncaspar (pegaspargase); Ontak (denileukin diftitox); Ontruzant (trastuzumab-dttb); Opdivo (nivolumab); Orencia (abatacept); Orthoclone OKT3 (muromanab-CD3); Ovidrel (choriogonadotropin alfa); Oxervate (cenegermin-bkbj); Padcev (enfortumab vedotin-ejfv); Palynziq (pegvaliase-pqpz); Pancreaze (pancrelipase); Pegasys (peginterferon alfa-2a); Pegasys Copegus Combination Pack (peginterferon alfa-2a and ribavirin); Pegintron (peginterferon alfa-2b); PegIntron/ Rebetol Combo Pack (peginterferon alfa-2b and ribavirin); Pergonal (menotropins); Perjeta (pertuzumab); Pertzye (pancrelipase); Plegridy (peginterferon beta-1a); Polivy (polatuzumab vedotin-piiq); Portrazza (necitumumab); Poteligeo (mogamulizumab-kpkc); Praluent (alirocumab); Praxbind (idarucizumab); Pregnyl (chorionic gonadotropin); Procrit (epoetin alfa); Proleukin (aldesleukin); Prolia (denosumab); ProstaScint (capromab pendetide); Pulmolite (kit for the preparation of technetium Tc-99m albumin aggregated); Pulmotech MAA (kit for the preparation of technetium Tc-99m albumin aggregated); Pulmozyme (dornase alfa); Raptiva (efalizumab); Rebif (interferon beta-1a); Reblozyl (luspatercept-aamt); Regranex (becaplermin); Remicade (infliximab); Renflexis (infliximab-abda); Reopro (abciximab); Repatha (evolocumab); Repronex (menotropins); Retacrit (epoetin alfa-epbx); Retavase (reteplase); Revcovi (elapegademase-lvlr); Rituxan (rituximab); Rituxan Hycela (rituximab and hyaluronidase human); Roferon-A (interferon alfa-2a); Ruxience (rituximab-pvvr); Ryzodeg 70/30 (insulin degludec and insulin aspart); Saizen (somatropin); Santyl (collagenase); Sarclisa (isatuximab-irfc); Serostim (somatropin); Siliq (brodalumab); Simponi (golimumab); Simponi Aria (golimumab); Simulect (basiliximab); Skyrizi (risankizumab-rzaa); Soliqua 100/33 (insulin glargine and lixisenatide); Soliris (eculizumab); Somavert (pegvisomant); Stelara (ustekinumab); Strensiq (asfotas alfa); Sucraid (sacrosidase); Survanta (beractant); Sylvant (siltuximab); Synagis (palivizumab); Takhzyro (lanadelumab-flyo); Taltz (ixekizumab); Tanzeum (albiglutide); Tecentriq (atezolizumab); Tepezza (teprotumumab-trbw); Thyrogen (thyrotropin alfa); TNKase (tenecteplase); Toujeo (insulin glargine); Trasylol (aprotinin); Trazimera (trastuzumab-qyyp); Tremfya (guselkumab); Tresiba (insulin degludec); Trodelvy (sacituzumab govitecan-hziy); Trogarzo (ibalizumab-uiyk); Trulicity (dulaglutide); Truxima (rituximab-abbs); Tysabri (natalizumab); Udenyca (pegfilgrastim-cbqv); Ultomiris (ravulizumab-cwvz); Unituxin (dinutuximab); Vectibix (panitumumab); Verluma (nofetumomab); Vimizim (elosulfase alfa); Viokace (pancrelipase); Vitrase (hyaluronidase); Voraxaze (glucarpidase); VPRIV (velaglucerase alfa); Xeomin (incobotulinumtoxinA); Xgeva (denosumab); Xiaflex (collagenase clostridium histolyticum); Xigris (drotrecogin alfa); Xolair (omalizumab); Xultophy 100/3.6 (insulin degludec and liraglutide); Yervoy (ipilimumab); Zaltrap (Ziv-Aflibercept); Zarxio (filgrastim-sndz); Zenapax (daclizumab); Zenpep (pancrelipase); Zevalin (ibritumomab tiuxetan); Ziextenzo (pegfilgrastim-bmez); Zinbryta (daclizumab); Zinplava (bezlotoxumab); Zirabev (bevacizumab-bvzr); Zomacton (somatropin); Zorbtive/Serostim (somatropin);

## INHALATION ANESTHETICS

Aliflurane; Chloroform; Cyclopropane; Desflurane (Suprane); Diethyl Ether; Enflurane (Ethrane); Ethyl Chloride; Ethylene; Halothane (Fluothane); Isoflurane (Forane, Isoflo); Isopropenyl vinyl ether; Methoxyflurane; methoxyflurane; Methoxypropane; Nitrous Oxide; Roflurane; Sevoflurane (Sevorane, Ultane, Sevoflo); Teflurane; Trichloroethylene; Vinyl Ether; Xenon

## INJECTABLE DRUGS

Ablavar (Gadofosveset Trisodium Injection); Abarelix Depot; Abobotulinumtoxin A Injection (Dysport); ABT-263; ABT-869; ABX-EFG; Accretropin (Somatropin Injection); Acetadote (Acetylcysteine Injection); Acetazolamide Injection (Acetazolamide Injection); Acetylcysteine Injection (Acetadote); Actemra (Tocilizumab Injection); Acthrel (Corticorelin Ovine Triflutate for Injection); Actummune; Activase; Acyclovir for Injection (Zovirax Injection); Adacel; Adalimumab; Adenoscan (Adenosine Injection); Adenosine Injection (Adenoscan); Adrenaclick; AdreView (lobenguane I 123 Injection for Intravenous Use); Afluria; Ak-Fluor (Fluorescein Injection); Aldurazyme (Laronidase); Alglucerase Injection (Ceredase); Alkeran Injection (Melphalan Hcl Injection); Allopurinol Sodium for Injection (Aloprim); Aloprim (Allopurinol Sodium for Injection); Alprostadil; Alsuma (Sumatriptan Injection); ALTU-238; Amino Acid Injections; Aminosyn; Apidra; Apremilast; Alprostadil Dual Chamber System for Injection (Caverject Impulse); AMG 009; AMG 076; AMG 102; AMG 108; AMG 114; AMG 162; AMG 220; AMG 221; AMG 222; AMG 223; AMG 317; AMG 379; AMG 386; AMG 403; AMG 477; AMG 479; AMG 517; AMG 531; AMG 557; AMG 623; AMG 655; AMG 706; AMG 714; AMG 745; AMG 785; AMG 811; AMG 827; AMG 837; AMG 853; AMG 951; Amiodarone HCl Injection (Amiodarone HCl Injection); Amobarbital Sodium Injection (Amytal Sodium); Amytal Sodium (Amobarbital Sodium Injection); Anakinra; Anti-Abeta; Anti-Beta7; Anti-Beta20; Anti-CD4; Anti-CD20; Anti-CD40; Anti-IFNalpha; Anti-IL13; Anti-OX40L; Anti-oxLDS; Anti-NGF; Anti-NRP1; Arixtra; Amphadase (Hyaluronidase Inj); Ammonul (Sodium Phenylacetate and Sodium Benzoate Injection); Anaprox; Anzemet Injection

(Dolasetron Mesylate Injection); Apidra (Insulin Glulisine [rDNA origin] Inj); Apomab; Aranesp (darbepoetin alfa); Argatroban (Argatroban Injection); Arginine Hydrochloride Injection (R-Gene 10); Aristocort; Aristospan; Arsenic Trioxide Injection (Trisenox); Articane HCl and Epinephrine Injection (Septocaine); Arzerra (Ofatumumab Injection); Asclera (Polidocanol Injection); Ataluren; Ataluren-DMD; Atenolol Inj (Tenormin I.V. Injection); Atracurium Besylate Injection (Atracurium Besylate Injection); Avastin; Azactam Injection (Aztreonam Injection); Azithromycin (Zithromax Injection); Aztreonam Injection (Azactam Injection); Baclofen Injection (Lioresal Intrathecal); Bacteriostatic Water (Bacteriostatic Water for Injection); Baclofen Injection (Lioresal Intrathecal); Bal in Oil Ampules (Dimercarprol Injection); BayHepB; BayTet; Benadryl; Bendamustine Hydrochloride Injection (Treanda); Benztropine Mesylate Injection (Cogentin); Betamethasone Injectable Suspension (Celestone Soluspan); Bexxar; Bicillin C-R 900/300 (Penicillin G Benzathine and Penicillin G Procaine Injection); Blenoxane (Bleomycin Sulfate Injection); Bleomycin Sulfate Injection (Blenoxane); Boniva Injection (Ibandronate Sodium Injection); Botox Cosmetic (OnabotulinumtoxinA for Injection); BR3-FC; Bravelle (Urofollitropin Injection); Bretylium (Bretylium Tosylate Injection ); Brevital Sodium (Methohexital Sodium for Injection); Brethine; Briobacept; BTT-1023; Bupivacaine HCl; Byetta; Ca-DTPA (Pentetate Calcium Trisodium Inj); Cabazitaxel Injection (Jevtana); Caffeine Alkaloid (Caffeine and Sodium Benzoate Injection); Calcijex Injection (Calcitrol); Calcitrol (Calcijex Injection); Calcium Chloride (Calcium Chloride Injection 10%); Calcium Disodium Versenate (Edetate Calcium Disodium Injection); Campath (Altemtuzumab); Camptosar Injection (Irinotecan Hydrochloride); Canakinumab Injection (Ilaris); Capastat Sulfate (Capreomycin for Injection); Capreomycin for Injection (Capastat Sulfate); Cardiolite (Prep kit for Technetium Tc99 Sestamibi for Injection); Carticel; Cathflo; Cefazolin and Dextrose for Injection (Cefazolin Injection); Cefepime Hydrochloride; Cefotaxime; Ceftriaxone; Cerezyme; Carnitor Injection; Caverject; Celestone Soluspan; Celsior; Cerebyx (Fosphenytoin Sodium Injection); Ceredase (Alglucerase Injection); Ceretec (Technetium Tc99m Exametazime Injection); Certolizumab; CF-101; Chloramphenicol Sodium Succinate (Chloramphenicol Sodium Succinate Injection); Chloramphenicol Sodium Succinate Injection (Chloramphenicol Sodium Succinate); Cholestagel (Colesevelam HCL); Choriogonadotropin Alfa Injection (Ovidrel); Cimzia; Cisplatin (Cisplatin Injection); Clolar (Clofarabine Injection); Clomiphine Citrate; Clonidine Injection (Duraclon); Cogentin (Benztropine Mesylate Injection); Colistimethate Injection (Coly-Mycin M); Coly-Mycin M (Colistimethate Injection); Compath; Conivaptan Hcl Injection (Vaprisol); Conjugated Estrogens for Injection (Premarin Injection); Copaxone; Corticorelin Ovine Triflutate for Injection (Acthrel); Corvert (Ibutilide Fumarate Injection); Cubicin (Daptomycin Injection); CF-101; Cyanokit (Hydroxocobalamin for Injection); Cytarabine Liposome Injection (DepoCyt); Cyanocobalamin; Cytovene (ganciclovir); D.H.E. 45; Dacetuzumab; Dacogen (Decitabine Injection); Dalteparin; Dantrium IV (Dantrolene Sodium for Injection); Dantrolene Sodium for Injection (Dantrium IV); Daptomycin Injection (Cubicin); Darbepoietin Alfa; DDAVP Injection (Desmopressin Acetate Injection); Decavax; Decitabine Injection (Dacogen); Dehydrated Alcohol (Dehydrated Alcohol Injection); Denosumab Injection (Prolia); Delatestryl; Delestrogen; Delteparin Sodium; Depacon (Valproate Sodium Injection); Depo Medrol (Methylprednisolone Acetate Injectable Suspension); DepoCyt (Cytarabine Liposome Injection); DepoDur (Morphine Sulfate XR Liposome Injection); Desmopressin Acetate Injection (DDAVP Injection); Depo-Estradiol; Depo-Provera 104mg/ml; Depo-Provera 150mg/ml; Depo-Testosterone; Dexrazoxane for Injection, Intravenous Infusion Only (Totect); Dextrose / Electrolytes; Dextrose and Sodium Chloride Inj (Dextrose 5% in 0.9% Sodium Chloride); Dextrose; Diazepam Injection (Diazepam Injection); Digoxin Injection (Lanoxin Injection); Dilaudid- HP (Hydromorphone Hydrochloride Injection); Dimercarprol Injection (Bal in Oil Ampules); Diphenhydramine Injection (Benadryl Injection); Dipyridamole Injection (Dipyridamole Injection); DMOAD; Docetaxel for Injection (Taxotere); Dolasetron Mesylate Injection (Anzemet Injection); Doribax (Doripenem for Injection); Doripenem for Injection (Doribax); Doxercalciferol Injection (Hectorol Injection); Doxil (Doxorubicin Hcl Liposome Injection); Doxorubicin Hcl Liposome Injection (Doxil); Duraclon (Clonidine Injection); Duramorph (Morphine Injection); Dysport (Abobotulinumtoxin A Injection); Ecallantide Injection (Kalbitor); EC-Naprosyn (naproxen); Edetate Calcium Disodium Injection (Calcium Disodium Versenate); Edex (Alprostadil for Injection); Engerix; Edrophonium Injection (Enlon); Eliglustat Tartate; Eloxatin (Oxaliplatin Injection); Emend Injection (Fosaprepitant Dimeglumine Injection); Enalaprilat Injection (Enalaprilat Injection); Enlon (Edrophonium Injection); Enoxaparin Sodium Injection (Lovenox); Eovist (Gadoxetate Disodium Injection); Enbrel (etanercept); Enoxaparin; Epicel; Epinepherine; Epipen; Epipen Jr.; Epratuzumab; Erbitux; Ertapenem Injection (Invanz); Erythropoieten; Essential Amino Acid Injection (Nephramine); Estradiol Cypionate; Estradiol Valerate; Etanercept; Exenatide Injection (Byetta); Evlotra; Fabrazyme (Adalsidase beta); Famotidine Injection; FDG (Fludeoxyglucose F 18 Injection); Feraheme (Ferumoxytol Injection); Feridex I.V. (Ferumoxides Injectable Solution); Fertinex; Ferumoxides Injectable Solution (Feridex I.V.); Ferumoxytol Injection (Feraheme); Flagyl Injection (Metronidazole Injection); Fluarix; Fludara (Fludarabine Phosphate); Fludeoxyglucose F 18 Injection (FDG); Fluorescein Injection (Ak-Fluor); Follistim AQ Cartridge (Follitropin Beta Injection); Follitropin Alfa Injection (Gonal-f RFF); Follitropin Beta Injection (Follistim AQ Cartridge); Folotyn (Pralatrexate Solution for Intravenous Injection); Fondaparinux; Forteo (Teriparatide (rDNA origin) Injection); Fostamatinib; Fosaprepitant Dimeglumine Injection (Emend Injection); Foscarnet Sodium Injection (Foscavir); Foscavir (Foscarnet Sodium Injection); Fosphenytoin Sodium Injection (Cerebyx); Fospropofol Disodium Injection (Lusedra); Fragmin; Fuzeon (enfuvirtide); GA101; Gadobenate Dimeglumine Injection (Multihance); Gadofosveset Trisodium Injection (Ablavar); Gadoteridol Injection Solution (ProHance); Gadoversetamide Injection (OptiMARK); Gadoxetate Disodium Injection (Eovist); Ganirelix (Ganirelix Acetate Injection); Gardasil; GC1008; GDFD; Gemtuzumab Ozogamicin for Injection (Mylotarg); Genotropin; Gentamicin Injection; GENZ-112638; Golimumab Injection (Simponi Injection); Gonal-f RFF (Follitropin Alfa Injection); Granisetron Hydrochloride (Kytril Injection); Gentamicin Sulfate; Glatiramer Acetate; Glucagen; Glucagon; HAE1; Haldol (Haloperidol Injection); Havrix; Hectorol Injection (Doxercalciferol Injection); Hedgehog Pathway Inhibitor; Heparin; Herceptin; hG-CSF; Humalog; Human Growth Hormone; Humatrope; HuMax; Humegon; Humira; Humulin; Ibandronate Sodium Injection (Boniva Injection); Ibuprofen Lysine Injection (NeoProfen); Ibutilide Fumarate Injection (Corvert); Idamycin PFS (Idarubicin Hydrochloride Injection); Idarubicin Hydrochloride Injection (Idamycin PFS); Ilaris (Canakinumab Injection); Imipenem and Cilastatin for Injection (Primaxin I.V.); Imitrex; Incobotulinumtoxin A for Injection (Xeomin); Increlex (Mecasermin [rDNA origin] Injection); Indocin IV (Indomethacin Inj); Indomethacin Inj (Indocin IV); Infanrix; Innohep; Insulin; Insulin Aspart [rDNA origin] Inj (NovoLog); Insulin Glargine [rDNA origin] Injection (Lantus); Insulin Glulisine [rDNA origin] Inj (Apidra); Interferon alfa-2b, Recombinant for Injection (Intron A); Intron A (Interferon alfa-2b, Recombinant for Injection); Invanz (Ertapenem Injection); Invega Sustenna (Paliperidone Palmitate Extended-Release Injectable Suspension); Invirase (saquinavir mesylate); Iobenguane I 123 Injection for Intravenous Use (AdreView); Iopromide Injection (Ultravist); Ioversol Injection (Optiray Injection); Iplex (Mecasermin Rinfabate [rDNA origin] Injection); Iprivask; Irinotecan Hydrochloride (Camptosar Injection); Iron Sucrose Injection (Venofer); Istodax (Romidepsin for Injection); Itraconazole Injection (Sporanox Injection); Jevtana (Cabazitaxel Injection); Jonexa; Kalbitor (Ecallantide Injection); KCL in D5NS (Potassium Chloride in 5% Dextrose and Sodium Chloride Injection); KCL in D5W; KCL in NS; Kenalog 10 Injection (Triamcinolone Acetonide Injectable Suspension); Kepivance (Palifermin); Keppra Injection (Levetiracetam); Keratinocyte; KFG; Kinase Inhibitor; Kineret (Anakinra); Kinlytic (Urokinase Injection); Kinrix; Klonopin (clonazepam); Kytril Injection (Granisetron Hydrochloride); lacosamide Tablet and Injection (Vimpat); Lactated Ringer's; Lanoxin Injection (Digoxin Injection); Lansoprazole for Injection (Prevacid I.V.); Lantus; Leucovorin Calcium (Leucovorin Calcium Injection); Lente (L); Leptin; Levemir; Leukine Sargramostim; Leuprolide Acetate; Levothyroxine; Levetiracetam (Keppra Injection); Lovenox; Levocarnitine Injection (Carnitor Injection); Lexiscan (Regadenoson Injection); Lioresal Intrathecal (Baclofen Injection); Liraglutide [rDNA] Injection (Victoza); Lovenox (Enoxaparin Sodium Injection); Lucentis (Ranibizumab Injection); Lumizyme; Lupron (Leuprolide Acetate Injection); Lusedra (Fospropofol Disodium Injection); Maci; Magnesium Sulfate (Magnesium Sulfate Injection); Mannitol Injection (Mannitol IV); Marcaine (Bupivacaine Hydrochloride and Epinephrine Injection); Maxipime (Cefepime Hydrochloride for Injection); MDP Multidose Kit of Technetium Injection (Technetium Tc99m Medronate Injection); Mecasermin [rDNA origin] Injection (Increlex); Mecasermin Rinfabate [rDNA origin] Injection (Iplex); Melphalan Hcl Injection (Alkeran Injection); Methotrexate; Menactra; Menopur (Menotropins Injection); Menotropins for Injection (Repronex); Methohexital Sodium for Injection (Brevital Sodium); Methyldopate Hydrochloride Injection, Solution (Methyldopate Hcl); Methylene Blue (Methylene Blue Injection); Methylprednisolone Acetate Injectable Suspension (Depo Medrol); MetMab; Metoclopramide Injection (Reglan Injection); Metrodin (Urofollitropin for Injection); Metronidazole Injection (Flagyl Injection); Miacalcin; Midazolam (Midazolam Injection); Mimpara (Cinacalet); Minocin Injection (Minocycline Inj); Minocycline Inj (Minocin Injection); Mipomersen; Mitoxantrone for Injection Concentrate (Novantrone); Morphine Injection (Duramorph); Morphine Sulfate XR Liposome Injection (DepoDur); Morrhuate Sodium (Morrhuate Sodium Injection); Motesanib; Mozobil (Plerixafor Injection); Multihance (Gadobenate Dimeglumine Injection); Multiple Electrolytes and Dextrose Injection; Multiple Electrolytes Injection; Mylotarg (Gemtuzumab Ozogamicin for Injection); Myozyme (Alglucosidase alfa); Nafcillin Injection (Nafcillin Sodium); Nafcillin Sodium (Nafcillin Injection); Naltrexone XR Inj (Vivitrol); Naprosyn (naproxen); NeoProfen (Ibuprofen Lysine Injection); Nandrol Decanoate; Neostigmine Methylsulfate (Neostigmine Methylsulfate Injection); NEO-GAA; NeoTect (Technetium Tc 99m Depreotide Injection); Nephramine (Essential Amino Acid Injection); Neulasta (pegfilgrastim); Neupogen (Filgrastim); Novolin; Novolog; NeoRecormon; Neutrexin (Trimetrexate Glucuronate Inj); NPH (N); Nexterone (Amiodarone HCl Injection); Norditropin (Somatropin Injection); Normal Saline (Sodium Chloride Injection); Novantrone (Mitoxantrone for Injection Concentrate); Novolin 70/30 Innolet (70% NPH, Human Insulin Isophane Suspension and 30% Regular, Human Insulin Injection); NovoLog (Insulin Aspart [rDNA origin] Inj); Nplate (romiplostim); Nutropin (Somatropin (rDNA origin) for Inj); Nutropin AQ; Nutropin Depot (Somatropin (rDNA origin) for Inj); Octreotide Acetate Injection (Sandostatin LAR); Ocrelizumab; Ofatumumab Injection (Arzerra); Olanzapine Extended Release Injectable Suspension (Zyprexa Relprevv); Omnitarg; Omnitrope (Somatropin [ rDNA origin] Injection); Ondansetron Hydrochloride Injection (Zofran Injection); OptiMARK (Gadoversetamide Injection); Optiray Injection (Ioversol Injection); Orencia; Osmitrol Injection in Aviva (Mannitol Injection in Aviva Plastic Vessel); Osmitrol Injection in Viaflex (Mannitol Injection in Viaflex Plastic Vessel); Osteoprotegrin; Ovidrel (Choriogonadotropin Alfa Injection); Oxacillin (Oxacillin for Injection); Oxaliplatin Injection (Eloxatin); Oxytocin Injection (Pitocin); Paliperidone Palmitate Extended- Release Injectable Suspension (Invega Sustenna); Pamidronate Disodium Injection (Pamidronate Disodium Injection); Panitumumab Injection for Intravenous Use (Vectibix); Papaverine Hydrochloride Injection (Papaverine Injection); Papaverine Injection (Papaverine Hydrochloride Injection); Parathyroid Hormone; Paricalcitol Injection Fliptop Vial (Zemplar Injection); PARP Inhibitor; Pediarix; PEGIntron; Peginterferon; Pegfilgrastim; Penicillin G Benzathine and Penicillin G Procaine; Pentetate Calcium Trisodium Inj (Ca-DTPA); Pentetate Zinc Trisodium Injection (Zn- DTPA); Pepcid Injection (Famotidine Injection); Pergonal; Pertuzumab; Phentolamine Mesylate (Phentolamine Mesylate for Injection); Physostigmine Salicylate (Physostigmine Salicylate (injection)); Physostigmine Salicylate (injection) (Physostigmine Salicylate); Piperacillin and Tazobactam Injection (Zosyn); Pitocin (Oxytocin Injection); Plasma-Lyte 148 (Multiple Electrolytes Inj); Plasma-Lyte 56 and Dextrose (Multiple Electrolytes and Dextrose Injection in Viaflex Plastic Vessel); PlasmaLyte; Plerixafor Injection (Mozobil); Polidocanol Injection (Asclera); Potassium Chloride; Pralatrexate Solution for Intravenous Injection (Folotyn); Pramlintide Acetate Injection (Symlin); Premarin Injection (Conjugated Estrogens for Injection); Prep kit for Technetium Tc99 Sestamibi for Injection (Cardiolite); Prevacid I.V. (Lansoprazole for Injection); Primaxin I.V. (Imipenem and Cilastatin for Injection); Prochymal; Procrit; Progesterone; ProHance (Gadoteridol Injection Solution); Prolia (Denosumab Injection); Promethazine HCl Injection (Promethazine Hydrochloride Injection); Propranolol Hydrochloride Injection (Propranolol Hydrochloride Injection); Quinidine Gluconate Injection (Quinidine Injection); Quinidine Injection (Quinidine Gluconate Injection); R- Gene 10 (Arginine Hydrochloride Injection); Ranibizumab Injection (Lucentis); Ranitidine Hydrochloride Injection (Zantac Injection); Raptiva; Reclast (Zoledronic Acid Injection); Recombivarix HB; Regadenoson Injection (Lexiscan); Reglan Injection (Metoclopramide Injection); Remicade; Renagel; Renvela (Sevelamer Carbonate); Repronex (Menotropins for Injection); Retrovir IV (Zidovudine Injection); rhApo2L/TRAIL; Ringer's and 5% Dextrose Injection (Ringers in Dextrose); Ringer's Injection (Ringers Injection); Rituxan; Rituximab; Rocephin (ceftriaxone); Rocuronium Bromide Injection (Zemuron); Roferon-A (interferon alfa-2a); Romazicon (flumazenil); Romidepsin for Injection (Istodax); Saizen (Somatropin Injection); Sandostatin LAR (Octreotide Acetate Injection); Sclerostin Ab; Sensipar (cinacalcet); Sensorcaine (Bupivacaine HCl Injections); Septocaine (Articane HCl and Epinephrine Injection); Serostim LQ (Somatropin (rDNA origin) Injection); Simponi Injection (Golimumab Injection); Sodium Acetate (Sodium Acetate Injection); Sodium Bicarbonate (Sodium Bicarbonate 5% Injection); Sodium Lactate (Sodium Lactate Injection in AVIVA); Sodium Phenylacetate and Sodium Benzoate Injection (Ammonul); Somatropin (rDNA origin) for Inj (Nutropin); Sporanox Injection (Itraconazole Injection); Stelara Injection (Ustekinumab); Stemgen; Sufenta (Sufentanil Citrate Injection); Sufentanil Citrate Injection (Sufenta ); Sumavel; Sumatriptan Injection (Alsuma); Symlin; Symlin Pen; Systemic Hedgehog Antagonist; Synvisc-One (Hylan G-F 20 Single Intra-articular Injection); Tarceva; Taxotere (Docetaxel for Injection); Technetium Tc 99m; Telavancin for Injection (Vibativ); Temsirolimus Injection (Torisel); Tenormin I.V. Injection (Atenolol Inj); Teriparatide (rDNA origin) Injection (Forteo); Testosterone Cypionate; Testosterone Enanthate; Testosterone Propionate; Tev-Tropin (Somatropin, rDNA Origin, for Injection); tgAAC94; Thallous Chloride; Theophylline; Thiotepa (Thiotepa Injection); Thymoglobulin (Anti- Thymocyte Globulin (Rabbit); Thyrogen (Thyrotropin Alfa for Injection); Ticarcillin Disodium and Clavulanate Potassium Galaxy (Timentin Injection); Tigan Injection (Trimethobenzamide Hydrochloride Injectable); Timentin Injection (Ticarcillin Disodium and Clavulanate Potassium Galaxy); TNKase; Tobramycin Injection (Tobramycin Injection); Tocilizumab Injection (Actemra); Torisel (Temsirolimus Injection); Totect (Dexrazoxane for Injection, Intravenous Infusion Only ); Trastuzumab-DM1; Travasol (Amino Acids (Injection)); Treanda (Bendamustine Hydrochloride Injection); Trelstar (Triptorelin Pamoate for Injectable Suspension); Triamcinolone Acetonide; Triamcinolone Diacetate; Triamcinolone Hexacetonide Injectable Suspension (Aristospan Injection 20 mg); Triesence (Triamcinolone Acetonide Injectable Suspension); Trimethobenzamide Hydrochloride Injectable (Tigan Injection); Trimetrexate Glucuronate Inj (Neutrexin); Triptorelin Pamoate for Injectable Suspension (Trelstar); Twinject; Trivaris (Triamcinolone Acetonide Injectable Suspension); Trisenox (Arsenic Trioxide Injection); Twinrix; Typhoid Vi; Ultravist (Iopromide Injection); Urofollitropin for Injection (Metrodin); Urokinase Injection (Kinlytic); Ustekinumab (Stelara Injection); Ultralente (U); Valium (diazepam); Valproate Sodium Injection (Depacon); Valtropin (Somatropin Injection); Vancomycin Hydrochloride (Vancomycin Hydrochloride Injection); Vancomycin Hydrochloride Injection (Vancomycin Hydrochloride); Vaprisol (Conivaptan Hcl Injection); VAQTA; Vasovist (Gadofosveset Trisodium Injection for Intravenous Use); Vectibix (Panitumumab Injection for Intravenous Use); Venofer (Iron Sucrose Injection); Verteporfin Inj (Visudyne); Vibativ (Telavancin for Injection); Victoza (Liraglutide [rDNA] Injection); Vimpat (lacosamide Tablet and Injection); Vinblastine Sulfate (Vinblastine Sulfate Injection); Vincasar PFS (Vincristine Sulfate Injection); Victoza; Vincristine Sulfate (Vincristine Sulfate Injection); Visudyne (Verteporfin Inj); Vitamin B-12; Vivitrol (Naltrexone XR Inj); Voluven (Hydroxyethyl Starch in Sodium Chloride Injection); Xeloda; Xenical (orlistat); Xeomin (Incobotulinumtoxin A for Injection); Xolair; Zantac Injection (Ranitidine Hydrochloride Injection); Zemplar Injection (Paricalcitol Injection Fliptop Vial); Zemuron (Rocuronium Bromide Injection); Zenapax (daclizumab); Zevalin; Zidovudine Injection (Retrovir IV); Zithromax Injection (Azithromycin); Zn-DTPA (Pentetate Zinc Trisodium Injection); Zofran Injection (Ondansetron Hydrochloride Injection); Zingo; Zoledronic Acid for Inj (Zometa); Zoledronic Acid Injection (Reclast); Zometa (Zoledronic Acid for Inj); Zosyn (Piperacillin and Tazobactam Injection); Zyprexa Relprevv (Olanzapine Extended Release Injectable Suspension)

**LIQUID DRUGS (NON-INJECTABLE)**

Abilify; AccuNeb (Albuterol Sulfate Inhalation Solution); Actidose Aqua (Activated Charcoal Suspension); Activated Charcoal Suspension (Actidose Aqua); Advair; Agenerase Oral Solution (Amprenavir Oral Solution); Akten (Lidocaine Hydrochloride Ophthalmic Gel); Alamast (Pemirolast Potassium Ophthalmic Solution); Albumin (Human) 5% Solution (Buminate 5%); Albuterol Sulfate Inhalation Solution; Alinia; Alocril; Alphagan; Alrex; Alvesco; Amprenavir Oral Solution; Analpram-HC; Arformoterol Tartrate Inhalation Solution (Brovana); Aristospan Injection 20 mg (Triamcinolone

Hexacetonide Injectable Suspension); Asacol; Asmanex; Astepro; Astepro (Azelastine Hydrochloride Nasal Spray); Atrovent Nasal Spray (Ipratropium Bromide Nasal Spray); Atrovent Nasal Spray .06; Augmentin ES-600; Azasite (Azithromycin Ophthalmic Solution); Azelaic Acid (Finacea Gel); Azelastine Hydrochloride Nasal Spray (Astepro); Azelex (Azelaic Acid Cream); Azopt (Brinzolamide Ophthalmic Suspension); Bacteriostatic Saline; Balanced Salt; Bepotastine; Bactroban Nasal; Bactroban; Beclovent; Benzac W; Betimol; Betoptic S; Bepreve; Bimatoprost Ophthalmic Solution; Bleph 10 (Sulfacetamide Sodium Ophthalmic Solution 10%); Brinzolamide Ophthalmic Suspension (Azopt); Bromfenac Ophthalmic Solution (Xibrom); Bromhist; Brovana (Arformoterol Tartrate Inhalation Solution); Budesonide Inhalation Suspension (Pulmicort Respules); Cambia (Diclofenac Potassium for Oral Solution); Capex; Carac; Carboxine-PSE; Carnitor; Cayston (Aztreonam for Inhalation Solution); Cellcept; Centany; Cerumenex; Ciloxan Ophthalmic Solution (Ciprofloxacin HCL Ophthalmic Solution); Ciprodex; Ciprofloxacin HCL Ophthalmic Solution (Ciloxan Ophthalmic Solution); Clemastine Fumarate Syrup (Clemastine Fumarate Syrup); CoLyte (PEG Electrolytes Solution); Combiven; Comtan; Condylox; Cordran; Cortisporin Ophthalmic Suspension; Cortisporin Otic Suspension; Cromolyn Sodium Inhalation Solution (Intal Nebulizer Solution); Cromolyn Sodium Ophthalmic Solution (Opticrom); Crystalline Amino Acid Solution with Electrolytes (Aminosyn Electrolytes); Cutivate; Cuvposa (Glycopyrrolate Oral Solution); Cyanocobalamin (CaloMist Nasal Spray); Cyclosporine Oral Solution (Gengraf Oral Solution); Cyclogyl; Cysview (Hexaminolevulinate Hydrochloride Intravesical Solution); DermOtic Oil (Fluocinolone Acetonide Oil Ear Drops); Desmopressin Acetate Nasal Spray; DDAVP; Derma-Smoothe/FS; Dexamethasone Intensol; Dianeal Low Calcium; Dianeal PD; Diclofenac Potassium for Oral Solution (Cambia); Didanosine Pediatric Powder for Oral Solution (Videx); Differin; Dilantin 125 (Phenytoin Oral Suspension); Ditropan; Dorzolamide Hydrochloride Ophthalmic Solution (Trusopt); Dorzolamide Hydrochloride-Timolol Maleate Ophthalmic Solution (Cosopt); Dovonex Scalp (Calcipotriene Solution); Doxycycline Calcium Oral Suspension (Vibramycin Oral); Efudex; Elaprase (Idursulfase Solution); Elestat (Epinastine HCl Ophthalmic Solution); Elocon; Epinastine HCl Ophthalmic Solution (Elestat); Epivir HBV; Epogen (Epoetin alfa); Erythromycin Topical Solution 1.5% (Staticin); Ethiodol (Ethiodized Oil); Ethosuximide Oral Solution (Zarontin Oral Solution); Eurax; Extraneal (Icodextrin Peritoneal Dialysis Solution); Felbatol; Feridex I.V. (Ferumoxides Injectable Solution); Flovent; Floxin Otic (Ofloxacin Otic Solution); Flo- Pred (Prednisolone Acetate Oral Suspension); Fluoroplex; Flunisolide Nasal Solution (Flunisolide Nasal Spray .025%); Fluorometholone Ophthalmic Suspension (FML); Flurbiprofen Sodium Ophthalmic Solution (Ocufen); FML; Foradil; Formoterol Fumarate Inhalation Solution (Perforomist); Fosamax; Furadantin (Nitrofurantoin Oral Suspension); Furoxone; Gammagard Liquid (Immune Globulin Intravenous (Human) 10%); Gantrisin (Acetyl Sulfisoxazole Pediatric Suspension); Gatifloxacin Ophthalmic Solution (Zymar); Gengraf Oral Solution (Cyclosporine Oral Solution); Glycopyrrolate Oral Solution (Cuvposa); Halcinonide Topical Solution (Halog Solution); Halog Solution (Halcinonide Topical Solution); HEP-LOCK U/P (Preservative-Free Heparin Lock Flush Solution); Heparin Lock Flush Solution (Hepflush 10); Hexaminolevulinate Hydrochloride Intravesical Solution (Cysview); Hydrocodone Bitartrate and Acetaminophen Oral Solution (Lortab Elixir); Hydroquinone 3% Topical Solution (Melquin-3 Topical Solution); IAP Antagonist; Isopto; Ipratropium Bromide Nasal Spray (Atrovent Nasal Spray); Itraconazole Oral Solution (Sporanox Oral Solution); Ketorolac Tromethamine Ophthalmic Solution (Acular LS); Kaletra; Lanoxin; Lexiva; Leuprolide Acetate for Depot Suspension (Lupron Depot 11.25 mg); Levobetaxolol Hydrochloride Ophthalmic Suspension (Betaxon); Levocarnitine Tablets, Oral Solution, Sugar-Free (Carnitor); Levofloxacin Ophthalmic Solution 0.5% (Quixin); Lidocaine HCl Sterile Solution (Xylocaine MPF Sterile Solution); Lok Pak (Heparin Lock Flush Solution); Lorazepam Intensol; Lortab Elixir (Hydrocodone Bitartrate and Acetaminophen Oral Solution); Lotemax (Loteprednol Etabonate Ophthalmic Suspension); Loteprednol Etabonate Ophthalmic Suspension (Alrex); Low Calcium Peritoneal Dialysis Solutions (Dianeal Low Calcium); Lumigan (Bimatoprost Ophthalmic Solution 0.03% for Glaucoma); Lupron Depot 11.25 mg (Leuprolide Acetate for Depot Suspension); Megestrol Acetate Oral Suspension (Megestrol Acetate Oral Suspension); MEK Inhibitor; Mepron; Mesnex; Mestinon; Mesalamine Rectal Suspension Enema (Rowasa); Melquin-3 Topical Solution (Hydroquinone 3% Topical Solution); MetMab; Methyldopate Hcl (Methyldopate Hydrochloride Injection, Solution); Methylin Oral Solution (Methylphenidate HCl Oral Solution 5 mg/5 mL and 10 mg/5 mL); Methylprednisolone Acetate Injectable Suspension (Depo Medrol); Methylphenidate HCl Oral Solution 5 mg/5 mL and 10 mg/5 mL (Methylin Oral Solution); Methylprednisolone sodium succinate (Solu Medrol); Metipranolol Ophthalmic Solution (Optipranolol); Migranal; Miochol-E (Acetylcholine Chloride Intraocular Solution); Micro-K for Liquid Suspension (Potassium Chloride Extended Release Formulation for Liquid Suspension); Minocin (Minocycline Hydrochloride Oral Suspension); Nasacort; Neomycin and Polymyxin B Sulfates and Hydrocortisone; Nepafenac Ophthalmic Suspension (Nevanac); Nevanac (Nepafenac Ophthalmic Suspension); Nitrofurantoin Oral Suspension (Furadantin); Noxafil (Posaconazole Oral Suspension); Nystatin (oral) (Nystatin Oral Suspension); Nystatin Oral Suspension (Nystatin (oral)); Ocufen (Flurbiprofen Sodium Ophthalmic Solution); Ofloxacin Ophthalmic Solution (Ofloxacin Ophthalmic Solution); Ofloxacin Otic Solution (Floxin Otic); Olopatadine Hydrochloride Ophthalmic Solution (Pataday); Opticrom (Cromolyn Sodium Ophthalmic Solution); Optipranolol (Metipranolol Ophthalmic Solution); Patanol; Pediapred; PerioGard; Phenytoin Oral Suspension (Dilantin 125); Phisohex; Posaconazole Oral Suspension (Noxafil); Potassium Chloride Extended Release Formulation for Liquid Suspension (Micro-K for Liquid Suspension); Pataday (Olopatadine Hydrochloride Ophthalmic Solution); Patanase Nasal Spray (Olopatadine Hydrochloride Nasal Spray); PEG Electrolytes Solution (CoLyte); Pemirolast Potassium Ophthalmic

Solution (Alamast); Penlac (Ciclopirox Topical Solution); PENNSAID (Diclofenac Sodium Topical Solution); Perforomist (Formoterol Fumarate Inhalation Solution); Peritoneal Dialysis Solution; Phenylephrine Hydrochloride Ophthalmic Solution (Neo-Synephrine); Phospholine Iodide (Echothiophate Iodide for Ophthalmic Solution); Podofilox (Podofilox Topical Solution); Pred Forte (Prednisolone Acetate Ophthalmic Suspension); Pralatrexate Solution for Intravenous Injection (Folotyn); Pred Mild; Prednisone Intensol; Prednisolone Acetate Ophthalmic Suspension (Pred Forte); Prevacid; PrismaSol Solution (Sterile Hemofiltration Hemodiafiltration Solution); ProAir; Proglycem; ProHance (Gadoteridol Injection Solution); Proparacaine Hydrochloride Ophthalmic Solution (Alcaine); Propine; Pulmicort; Pulmozyme; Quixin (Levofloxacin Ophthalmic Solution 0.5%); QVAR; Rapamune; Rebetol; Relacon-HC; Rotarix (Rotavirus Vaccine, Live, Oral Suspension); Rotavirus Vaccine, Live, Oral Suspension (Rotarix); Rowasa (Mesalamine Rectal Suspension Enema); Sabril (Vigabatrin Oral Solution); Sacrosidase Oral Solution (Sucraid); Sandimmune; Sepra; Serevent Diskus; Solu Cortef (Hydrocortisone Sodium Succinate); Solu Medrol (Methylprednisolone sodium succinate); Spiriva; Sporanox Oral Solution (Itraconazole Oral Solution); Staticin (Erythromycin Topical Solution 1.5%); Stalevo; Starlix; Sterile Hemofiltration Hemodiafiltration Solution (PrismaSol Solution); Stimate; Sucralfate (Carafate Suspension); Sulfacetamide Sodium Ophthalmic Solution 10% (Bleph 10); Synarel Nasal Solution (Nafarelin Acetate Nasal Solution for Endometriosis); Taclonex Scalp (Calcipotriene and Betamethasone Dipropionate Topical Suspension); Tamiflu; Tobi; TobraDex; Tobradex ST (Tobramycin / Dexamethasone Ophthalmic Suspension 0.3%/0.05%); Tobramycin / Dexamethasone Ophthalmic Suspension 0.3%/0.05% (Tobradex ST); Timolol; Timoptic; Travatan Z; Treprostinil Inhalation Solution (Tyvaso); Trusopt (Dorzolamide Hydrochloride Ophthalmic Solution); Tyvaso (Treprostinil Inhalation Solution); Ventolin; Vfend; Vibramycin Oral (Doxycycline Calcium Oral Suspension); Videx (Didanosine Pediatric Powder for Oral Solution); Vigabatrin Oral Solution (Sabril); Viokase; Viracept; Viramune; Vitamin K1 (Fluid Colloidal Solution of Vitamin K1); Voltaren Ophthalmic (Diclofenac Sodium Ophthalmic Solution); Zarontin Oral Solution (Ethosuximide Oral Solution); Ziagen; Zyvox; Zymar (Gatifloxacin Ophthalmic Solution); Zymaxid (Gatifloxacin Ophthalmic Solution)

**DRUG CLASSES**

5-alpha-reductase inhibitors; 5-aminosalicylates; 5HT3 receptor antagonists; adamantane antivirals; adrenal cortical steroids; adrenal corticosteroid inhibitors; adrenergic bronchodilators; agents for hypertensive emergencies; agents for pulmonary hypertension; aldosterone receptor antagonists; alkylating agents; alpha-adrenoreceptor antagonists; alpha-glucosidase inhibitors; alternative medicines; amebicides; aminoglycosides; aminopenicillins; aminosalicylates; amylin analogs; Analgesic Combinations; Analgesics; androgens and anabolic steroids; angiotensin converting enzyme inhibitors; angiotensin II inhibitors; anorectal preparations; anorexiants; antacids; anthelmintics; anti-angiogenic ophthalmic agents; anti-CTLA-4 monoclonal antibodies; anti-infectives; antiadrenergic agents, centrally acting; antiadrenergic agents, peripherally acting; antiandrogens; antianginal agents; antiarrhythmic agents; antiasthmatic combinations; antibiotics/antineoplastics; anticholinergic antiemetics; anticholinergic antiparkinson agents; anticholinergic bronchodilators; anticholinergic chronotropic agents; anticholinergics/antispasmodics; anticoagulants; anticonvulsants; antidepressants; antidiabetic agents; antidiabetic combinations; antidiarrheals; antidiuretic hormones; antidotes; antiemetic/antivertigo agents; antifungals; antigonadotropic agents; antigout agents; antihistamines; antihyperlipidemic agents; antihyperlipidemic combinations; antihypertensive combinations; antihyperuricemic agents; antimalarial agents; antimalarial combinations; antimalarial quinolines; antimetabolites; antimigraine agents; antineoplastic detoxifying agents; antineoplastic interferons; antineoplastic monoclonal antibodies; antineoplastics; antiparkinson agents; antiplatelet agents; antipseudomonal penicillins; antipsoriatics; antipsychotics; antirheumatics; antiseptic and germicides; antithyroid agents; antitoxins and antivenins; antituberculosis agents; antituberculosis combinations; antitussives; antiviral agents; antiviral combinations; antiviral interferons; anxiolytics, sedatives, and hypnotics; aromatase inhibitors; atypical antipsychotics; azole antifungals; bacterial vaccines; barbiturate anticonvulsants; barbiturates; BCR-ABL tyrosine kinase inhibitors; benzodiazepine anticonvulsants; benzodiazepines; beta-adrenergic blocking agents; beta-lactamase inhibitors; bile acid sequestrants; biologicals; bisphosphonates; bone resorption inhibitors; bronchodilator combinations; bronchodilators; calcitonin; calcium channel blocking agents; carbamate anticonvulsants; carbapenems; carbonic anhydrase inhibitor anticonvulsants; carbonic anhydrase inhibitors; cardiac stressing agents; cardioselective beta blockers; cardiovascular agents; catecholamines; CD20 monoclonal antibodies; CD33 monoclonal antibodies; CD52 monoclonal antibodies; central nervous system agents; cephalosporins; cerumenolytics; chelating agents; chemokine receptor antagonist; chloride channel activators; cholesterol absorption inhibitors; cholinergic agonists; cholinergic muscle stimulants; cholinesterase inhibitors; CNS stimulants; coagulation modifiers; colony stimulating factors; contraceptives; corticotropin; coumarins and indandiones; cox-2 inhibitors; decongestants; dermatological agents; diagnostic radiopharmaceuticals; dibenzazepine anticonvulsants; digestive enzymes; dipeptidyl peptidase 4 inhibitors; diuretics; dopaminergic antiparkinsonism agents; drugs used in alcohol dependence; echinocandins; EGFR inhibitors; estrogen receptor antagonists; estrogens; expectorants; factor Xa inhibitors; fatty acid derivative anticonvulsants; fibric acid derivatives; first generation cephalosporins; fourth generation cephalosporins; functional bowel disorder agents; gallstone solubilizing agents; gamma-aminobutyric acid analogs; gamma-aminobutyric acid reuptake inhibitors; gamma-aminobutyric acid transaminase inhibitors; gastrointestinal agents; general anesthetics; genitourinary tract agents; GI stimulants; glucocorticoids; glucose elevating agents; glycopeptide antibiotics; glycoprotein platelet inhibitors; glycylcyclines; gonadotropin releasing hormones; gonadotropin-releasing hormone antagonists; gonadotropins; group I antiarrhythmics; group II antiarrhythmics; group III antiarrhythmics; group IV antiarrhythmics; group V antiarrhythmics; growth hormone receptor blockers; growth hormones; H. pylori eradication agents; H2 antagonists; hematopoietic stem cell mobilizer; heparin antagonists; heparins; HER2 inhibitors; herbal products; histone deacetylase inhibitors; hormone replacement therapy; hormones; hormones/antineoplastics; hydantoin anticonvulsants; illicit (street) drugs; immune globulins; immunologic agents; immunosuppressive agents; impotence agents; in vivo diagnostic biologicals; incretin mimetics; inhaled anti-infectives; inhaled corticosteroids; inotropic agents; insulin; insulin-like growth factor; integrase strand transfer inhibitor; interferons; intravenous nutritional products; iodinated contrast media; ionic iodinated contrast media; iron products; ketolides; laxatives; leprostatics; leukotriene modifiers; lincomycin derivatives; lipoglycopeptides; local injectable anesthetics; loop diuretics; lung surfactants; lymphatic staining agents; lysosomal enzymes; macrolide derivatives; macrolides; magnetic resonance imaging contrast media; mast cell stabilizers; medical gas; meglitinides; metabolic agents; methylxanthines; mineralocorticoids; minerals and electrolytes; miscellaneous agents; miscellaneous analgesics; miscellaneous antibiotics; miscellaneous anticonvulsants; miscellaneous antidepressants; miscellaneous antidiabetic agents; miscellaneous antiemetics; miscellaneous antifungals; miscellaneous antihyperlipidemic agents; miscellaneous antimalarials; miscellaneous antineoplastics; miscellaneous antiparkinson agents; miscellaneous antipsychotic agents; miscellaneous antituberculosis agents; miscellaneous antivirals; miscellaneous anxiolytics, sedatives and hypnotics; miscellaneous biologicals; miscellaneous bone resorption inhibitors; miscellaneous cardiovascular agents; miscellaneous central nervous system agents; miscellaneous coagulation modifiers; miscellaneous diuretics; miscellaneous genitourinary tract agents; miscellaneous GI agents; miscellaneous hormones; miscellaneous metabolic agents; miscellaneous ophthalmic agents; miscellaneous otic agents; miscellaneous respiratory agents; miscellaneous sex hormones; miscellaneous topical agents; miscellaneous uncategorized agents; miscellaneous vaginal agents; mitotic inhibitors; monoamine oxidase inhibitors; monoclonal antibodies; mouth and throat products; mTOR inhibitors; mTOR kinase inhibitors; mucolytics; multikinase inhibitors; muscle relaxants; mydriatics; narcotic analgesic combinations; narcotic analgesics; nasal anti-infectives; nasal antihistamines and decongestants; nasal lubricants and irrigations; nasal preparations; nasal steroids; natural penicillins; neuraminidase inhibitors; neuromuscular blocking agents; next generation cephalosporins; nicotinic acid derivatives; nitrates; NNRTIs; non- cardioselective beta blockers; non-iodinated contrast media; non-ionic iodinated contrast media; non-sulfonylureas; nonsteroidal anti-inflammatory agents; norepinephrine reuptake inhibitors; norepinephrine-dopamine reuptake inhibitors; nucleoside reverse transcriptase inhibitors (NRTIs); nutraceutical products; nutritional products; ophthalmic anesthetics; ophthalmic anti-infectives; ophthalmic anti-inflammatory agents; ophthalmic antihistamines and decongestants; ophthalmic diagnostic agents; ophthalmic glaucoma agents; ophthalmic lubricants and irrigations; ophthalmic preparations; ophthalmic steroids; ophthalmic steroids with anti-infectives; ophthalmic surgical agents; oral nutritional supplements; otic anesthetics; otic anti- infectives; otic preparations; otic steroids; otic steroids with anti-infectives; oxazolidinedione anticonvulsants; parathyroid hormone and analogs; penicillinase resistant penicillins; penicillins; peripheral opioid receptor antagonists; peripheral vasodilators; peripherally acting antiobesity agents; phenothiazine antiemetics; phenothiazine antipsychotics; phenylpiperazine antidepressants; plasma expanders; platelet aggregation inhibitors; platelet-stimulating agents; polyenes; potassium-sparing diuretics; probiotics; progesterone receptor modulators; progestins; prolactin inhibitors; prostaglandin D2 antagonists; protease inhibitors; proton pump inhibitors; psoralens; psychotherapeutic agents; psychotherapeutic combinations; purine nucleosides; pyrrolidine anticonvulsants; quinolones; radiocontrast agents; radiologic adjuncts; radiologic agents; radiologic conjugating agents; radiopharmaceuticals; RANK ligand inhibitors; recombinant human erythropoietins; renin inhibitors; respiratory agents; respiratory inhalant products; rifamycin derivatives; salicylates; sclerosing agents; second generation cephalosporins; selective estrogen receptor modulators; selective serotonin reuptake inhibitors; serotonin-norepinephrine reuptake inhibitors; serotoninergic neuroenteric modulators; sex hormone combinations; sex hormones; skeletal muscle relaxant combinations; skeletal muscle relaxants; smoking cessation agents; somatostatin and somatostatin analogs; spermicides; statins; sterile irrigating solutions; streptomyces derivatives; succinimide anticonvulsants; sulfonamides; sulfonylureas; synthetic ovulation stimulants; tetracyclic antidepressants; tetracyclines; therapeutic radiopharmaceuticals; thiazide diuretics; thiazolidinediones; thioxanthenes; third generation cephalosporins; thrombin inhibitors; thrombolytics; thyroid drugs; tocolytic agents; topical acne agents; topical agents; topical anesthetics; topical anti-infectives; topical antibiotics; topical antifungals; topical antihistamines; topical antipsoriatics; topical antivirals; topical astringents; topical debriding agents; topical depigmenting agents; topical emollients; topical keratolytics; topical steroids; topical steroids with anti-infectives; toxoids; triazine anticonvulsants; tricyclic antidepressants; trifunctional monoclonal antibodies; tumor necrosis factor (TNF) inhibitors; tyrosine kinase inhibitors; ultrasound contrast media; upper respiratory combinations; urea anticonvulsants; urinary anti-infectives; urinary antispasmodics; urinary pH modifiers; uterotonic agents; vaccine; vaccine combinations; vaginal anti-infectives; vaginal preparations; vasodilators; vasopressin antagonists; vasopressors; VEGF/VEGFR inhibitors; viral vaccines; viscosupplementation agents; vitamin and mineral combinations; vitamins; protein-based vaccines; DNA-based vaccines; mRNA-based vaccines;

## DIAGNOSTIC TESTS

17-Hydroxyprogesterone; ACE (Angiotensin I converting enzyme); Acetaminophen; Acid phosphatase; ACTH; Activated clotting time; Activated protein C resistance; Adrenocorticotropic hormone (ACTH); Alanine aminotransferase (ALT); Albumin; Aldolase; Aldosterone; Alkaline phosphatase; Alkaline phosphatase (ALP); Alpha1- antitrypsin; Alpha-fetoprotein; Alpha-fetoprotien; Ammonia levels; Amylase; ANA (antinuclear antbodies); ANA (antinuclear antibodies); Angiotensin-converting enzyme (ACE); Anion gap; Anticardiolipin antibody; Anticardiolipin antivbodies (ACA); Anti-centromere antibody; Antidiuretic hormone; Anti-DNA; Anti-Dnase-B; Anti-Gliadin antibody; Anti-glomerular basement membrane antibody; Anti-HBc (Hepatitis B core antibodies; Anti-HBs (Hepatitis B surface antibody; Antiphospholipid antibody; Anti-RNA polymerase; Anti-Smith (Sm) antibodies; Anti-Smooth Muscle antibody; Antistreptolysin O (ASO); Antithrombin III; Anti-Xa activity; Anti-Xa assay; Apolipoproteins; Arsenic; Aspartate aminotransferase (AST); B12; Basophil; Beta-2-Microglobulin; Beta-hydroxybutyrate; B-HCG; Bilirubin; Bilirubin, direct; Bilirubin, indirect; Bilirubin, total; Bleeding time; Blood gases (arterial); Blood urea nitrogen (BUN); BUN; BUN (blood urea nitrogen); CA 125; CA 15-3; CA 19-9; Calcitonin; Calcium; Calcium (ionized); Carbon monoxide (CO); Carcinoembryonic antigen (CEA); CBC; CEA; CEA (carcinoembryonic antigen); Ceruloplasmin; CH50Chloride; Cholesterol; Cholesterol, HDL; Clot lysis time; Clot retraction time; CMP; CO2; Cold agglutinins; Complement C3; Copper; Corticotrophin releasing hormone (CRH) stimulation test; Cortisol; Cortrosyn stimulation test; C-peptide; CPK (Total); CPK-MB; C-reactive protein; Creatinine; Creatinine kinase (CK); Cryoglobulins; DAT (Direct antiglobulin test); D-Dimer; Dexamethasone suppression test; DHEA-S; Dilute Russell viper venom; Elliptocytes; Eosinophil; Erythrocyte sedimentation rate (ESR); Estradiol; Estriol; Ethanol; Ethylene glycol; Euglobulin lysis; Factor V Leiden; Factor VIII inhibitor; Factor VIII level; Ferritin; Fibrin split products; Fibrinogen; Folate; Folate (serum; Fractional excretion of sodium (FENA); FSH (follicle stimulating factor); FTA-ABS; Gamma glutamyl transferase (GGT); Gastrin; GGTP (Gamma glutamyl transferase); Glucose; Growth hormone; Haptoglobin; HBeAg (Hepatitis Be antigen); HBs-Ag (Hepatitis B surface antigen); Helicobacter pylori; Hematocrit; Hematocrit (HCT); Hemoglobin; Hemoglobin A1C; Hemoglobin electrophoresis; Hepatitis A antibodies; Hepatitis C antibodies; IAT (Indirect antiglobulin test); Immunofixation (IFE); Iron; Lactate dehydrogenase (LDH); Lactic acid (lactate); LDH; LH (Leutinizing hormone; Lipase; Lupus anticoagulant; Lymphocyte; Magnesium; MCH (mean corpuscular hemoglobin; MCHC (mean corpuscular hemoglobin concentration); MCV (mean corpuscular volume); Methylmalonate; Monocyte; MPV (mean platelet volume); Myoglobin; Neutrophil; Parathyroid hormone (PTH); Phosphorus; Platelets (plt); Potassium; Prealbumin; Prolactin; Prostate specific antigen (PSA); Protein C; Protein S; PSA (prostate specific antigen); PT (Prothrombin time); PTT (Partial thromboplastin time); RDW (red cell distribution width); Renin; Rennin; Reticulocyte count; reticulocytes; Rheumatoid factor (RF); Sed Rate; Serum glutamic-pyruvic transaminase (SGPT; Serum protein electrophoresis (SPEP); Sodium; T3-resin uptake (T3RU); T4, Free; Thrombin time; Thyroid stimulating hormone (TSH); Thyroxine (T4); Total iron binding capacity (TIBC); Total protein; Transferrin; Transferrin saturation; Triglyceride (TG); Troponin; Uric acid; Vitamin B12; White blood cells (WBC); Widal test.

## BRIEF DESCRIPTION OF THE DRAWINGS

FIG. 1 is a schematic sectional view of a vessel according to any embodiment of the invention.

FIG. 2 is an enlarged detail view of a portion of the vessel wall and coatings of FIG. 1.

FIG. 3 is a schematic view of a pharmaceutical package in the form of a syringe barrel as the vessel of FIGS. 1 and 2, containing a fluid and closed with a closure in the form of a plunger.

FIG. 4 is a schematic view of a pharmaceutical package in the form of a vial as the vessel of FIGS. 1 and 2 containing a fluid and closed with a closure.

FIG. 5 is a schematic view of a pharmaceutical package in the form of a blister package as the vessel of FIGS. 1 and 2 containing a fluid and closed with a closure in the form of a coated sheet defining an additional vessel wall.

FIG. 6 is a plot of silicon dissolution versus exposure time at pH 6 for a glass container versus a plastic container having an $SiO_x$ barrier layer coated in the inside wall.

FIG. 7 is a plot of silicon dissolution versus exposure time at pH 7 for a glass container versus a plastic container having an $SiO_x$ barrier layer coated in the inside wall.

FIG. 8 is a plot of silicon dissolution versus exposure time at pH 8 for a glass container versus a plastic container having an $SiO_x$ barrier layer coated in the inside wall.

FIG. 9 is a plot of the $SiO_x$ coating thickness necessary initially to leave a 30 nm residual coating thickness when stored with solutions at different nominal pH values from 3 to 9.

FIG. 10 shows the silicon dissolution rates at pH 8 and 40°C of various PECVD coatings.

FIG. 11 is a plot of the ratio of Si-O-Si symmetric/asymmetric stretching mode versus energy input per unit mass (W/FM or KJ/kg) of a PECVD coating using as the reactive precursor gases OMCTS and oxygen.

FIG. 12 is a plot of silicon shelf life (days) versus energy input per unit mass (W/FM or KJ/kg) of a PECVD coating using as the reactive precursor gases OMCTS and oxygen.

FIG. 13 is a Fourier Transform Infrared Spectrophotometer (FTIR) absorbance spectrum of a PECVD coating.

FIG. 14 is a Fourier Transform Infrared Spectrophotometer (FTIR) absorbance spectrum of a PECVD coating.

FIG. 15 is a Fourier Transform Infrared Spectrophotometer (FTIR) absorbance spectrum of a PECVD coating.

FIG. 16 is a Fourier Transform Infrared Spectrophotometer (FTIR) absorbance spectrum of a PECVD coating.

FIG. 17 is a Fourier Transform Infrared Spectrophotometer (FTIR) absorbance spectrum of a PECVD coating, originally presented as FIG. 5 of U.S. Pat. No.8,067,070, annotated to show the calculation of the O-Parameter referred to in that patent.

FIG. 18 is a schematic view of a syringe with a trilayer coating according to FIGS. 1, 2, and 3, showing a cylindrical region and specific points where data was taken.

FIG. 19 is a Trimetric map of the overall trilayer coating thickness versus position in the cylindrical region of a syringe illustrated by FIGS. 18, 1, 2, and 3.

FIG. 20 is a photomicrograhic sectional view showing the substrate and coatings of the trilayer coating at position 2 shown in FIG. 18.

FIG. 21 is another Trimetric map of the overall trilayer coating thickness versus position in the cylindrical region of a syringe illustrated by FIGS. 18, 1, 2, and 3.

FIG. 22 is a plot of coating thickness, representing the same coating as FIG. 21, at Positions 1, 2, 3, and 4 shown in FIG. 18.

FIG. 23 is a schematic illustration of a syringe, showing points on its surface where measurements were made in a working example.

FIG. 24 is a photograph showing the benefit of the present trilayer coating in preventing pinholes after attack by an alkaline reagent, as discussed in the working examples.

FIG. 24A is an enlarged detail view of the indicated portion of FIG. 24.

FIG. 25 is a view of an embodiment of a coated surface as described herein.

FIG. 26 is a schematic showing an example of a process for the atomic layer deposition of an aluminum oxide coating consisting of a plurality of aluminum oxide monolayers.

FIG. 27 is an illustration of various coatings applied by atomic layer deposition.

FIG. 28 is a graph showing the results of water vapor transmission rate testing.

FIG. 29 is a graph showing the results of oxygen transmission rate testing.

FIG. 30A is a side view, taken in cross-section, showing an embodiment of a vial as described herein.

FIG. 30B is a side view, taken in cross-section, showing an embodiment of a vial as described herein that includes a stopper and a crimp.

FIG. 31 is a comparative view showing the results of an ink-blot test for a standard vial and the embodiment shown in FIG. 30.

FIG. 32 is a graph showing the variance in outside diameter of embodiments of vials described herein and conventional glass vials.

FIG. 33A is a graph showing a sample lyophilization cycle.

FIG. 33B is an illustration showing the positions of vials selected for testing from within a 240 count tray.

FIG. 34 shows the results of container closure integrity (CCI) testing of embodiments of vials described herein.

FIG. 35 shows the results of oxygen transmission rate testing of embodiments of vials described herein after being subjected to extreme cryogenic conditions.

FIG. 36 is a comparison between a hydrophobic and a hydrophilic protective layer.

FIG. 37 is a comparison between a hydrophobic and a hydrophilic protective layer.

FIG. 38 is a graph showing the results of testing of hydrophobic and hydrophilic protective layers using the Kitazaki-Hata Method.

FIG. 39 is a graph showing the results of light obscuration (LO) testing of embodiments of vials described herein.

FIG. 40 is a graph showing the results of comparative micro flow imaging (MFI) testing of embodiments of vials described herein and conventional commercial products.

FIG. 41 is a graph showing the variance in inside diameter of embodiments of syringe barrels described herein and conventional glass syringe barrels.

FIG. 42 is a graph showing the variance in inside diameter of embodiments of syringe barrels described herein and conventional glass syringe barrels.

FIG. 43 is a graph showing the variance in needle hub outside diameter of embodiments of syringe barrels described herein and conventional glass syringe barrels.

FIG. 44 is a graph showing the variance in overall length of embodiments of syringe barrels described herein and conventional glass syringe barrels.

FIG. 45 is a graph showing the variance in overall length of embodiments of syringe barrels described herein and conventional glass syringe barrels.

FIG. 46 is a graph showing the variance in flange outside diameter of embodiments of syringe barrels described herein and conventional glass syringe barrels.

FIG. 47 is a graph showing the variance in weights of embodiments of syringe barrels described herein and conventional glass syringe barrels.

FIG. 48 is a graph showing the results of resonant mass measurement (RMM) testing of embodiments of syringe barrels described herein.

FIG. 49 is a graph showing the results of FlowCAM® microflow digital imaging of embodiments of syringe barrels described herein.

FIG. 50 is a graph showing the results of light obscuration (LO) testing of embodiments of syringe barrels described herein.

FIG. 51 is a graph showing the results of ethylene oxide (EO) barrier testing of embodiments of syringe barrels described herein.

FIG. 52 is a side elevation view, in cross-section, of an embodiment of a 1 mL staked needle syringe as described herein.

FIG. 53 is a side elevation view, in cross-section, of an embodiment of a 0.5 mL staked needle syringe as described herein.

FIG. 54 is a graph showing the results of testing of embodiments of syringes described herein for break-loose force and glide force.

FIG. 55 is a cross-sectional view showing a relationship between the inner diameter (ID) of a syringe barrel and the outer diameter (OD) of an embodiment of a lubricious gasket as described herein.

FIG. 56 is a schematic sectional view taken along section lines 3A-3A of Figure 55.

FIG. 57 is a fragmentary detail view of the structure of Figure 56.

FIG. 58 is a top view of an embodiment of a lubricious gasket described herein showing an approximate geometric distribution of first and second non-continuous channels.

FIG. 59 is a top view of an embodiment of a lubricious gasket described herein showing an approximate geometric distribution of a first, second, and third non-continuous channel.

FIG. 60 shows a fragmentary detail view of an embodiment of a non-continuous channel of an embodiment of a lubricious gasked described herein.

FIG. 61 shows an example of a cold storage life cycle for a vial.

FIG. 62 shows an example freeze-thaw cycle that was used to test embodiments of vials as described herein.

FIG. 63 shows the results of testing for defects of embodiments of vials described herein after being subjected to the freeze-thaw cycle of FIG. 62.

FIG. 64 shows a side elevation view, partly in cross-section, of a syringe assembly having an embodiment of a plunger anti-backout feature as described herein.

FIG. 65 is a perspective view of a syringe assembly having an embodiment of a plunger anti-backout feature described herein.

FIG. 66 is a perspective view of an embodiment of the plunger rod for the syringe assembly shown in FIG. 65.

FIG. 67 is a side detail view, in cross-section, showing an interaction between an embodiment of the plunger rod and an embodiment of the backstop element for the syringe assembly shown in FIG. 65.

FIG. 68 is a perspective view of a syringe assembly having an embodiment of a plunger anti-backout feature described herein.

FIG. 69 is a perspective view of an embodiment of the plunger rod for the syringe assembly shown in FIG. 68.

FIG. 70 is a perspective view of an embodiment of the backstop element for the syringe assembly shown in FIG. 68.

FIG. 71 is a side elevation view, in cross-section, of the backstop element shown in FIG. 70.

FIG. 72 is a top plan view of the backstop element shown in FIG. 70.

FIG. 73 is a perspective view of an embodiment of a threaded housing for the syringe assembly shown in FIG. 68.

FIG. 74A is a perspective view of an embodiment of a twist lock thumb nut for the syringe assembly shown in FIG. 68.

FIG. 74B is a side elevation view, in cross-section, of the twist lock thumb nut shown in FIG 74A.

FIG. 75 is a side detail view, in cross-section, showing an interaction between an embodiment of backstop element, a plunger rod, a threaded housing, and a twist lock thumb nut for the syringe assembly shown in FIG. 68.

FIG. 76 is a perspective view of a syringe assembly having an embodiment of a plunger anti-backout feature described herein.

FIG. 77 is a side elevation view of the syringe assembly shown in FIG. 76.

FIG. 78 is a perspective view of an embodiment of a plunger rod for the syringe assembly shown in FIG. 76.

FIG. 79 is a perspective view of an embodiment of the backstop element for the syringe assembly shown in FIG. 76.

FIG. 80 is a side elevation view, in cross-section, of the backstop element shown in FIG. 79.

FIG. 81 is a perspective view of an embodiment of a locking bar for the syringe assembly shown in FIG. 76.

FIG. 82 is a side detail view, in cross-section, showing an interaction between an embodiment of a backstop element, a plunger rod, and a locking bar for the syringe assembly shown in FIG. 76 when in a locked position.

FIG. 83 is a top plan view, in cross-section, showing an interaction between an embodiment of a backstop element, a plunger rod, and a locking bar for the syringe assembly shown in FIG. 76 when in a locked position.

FIG. 84 is a side detail view, in cross-section, showing an interaction between an embodiment of a backstop element, a plunger rod, and a locking bar for the syringe assembly shown in FIG. 76 when in an unlocked position.

FIG. 85 is a top plan view, in cross-section, showing an interaction between an embodiment of a backstop element, a plunger rod, and a locking bar for the syringe assembly shown in FIG. 76 when in an unlocked position.

FIG. 86 is a graph showing the water vapour transmission rates (WVTR) of embodiments of 10 mL vials prepared in accordance with embodiments of the present disclosure.

FIG. 87 is a graph showing the oxygen transmission rates (OTR) of embodiments of 10 mL vials prepared in accordance with embodiments of the present disclosure.

FIG. 88 is a graph showing the water vapour transmission rates (WVTR) of embodiments of 10 mL vials prepared in accordance with embodiments of the present disclosure.

FIG. 89 is a graph showing the oxygen transmission rates (OTR) of embodiments of 10 mL vials prepared in accordance with embodiments of the present disclosure.

FIG. 90 is a graph showing the oxygen transmission rates (OTR) of various syringes prepared in accordance with embodiments of the present disclosure.

FIG. 91 is a graph showing the oxygen transmission rates (OTR) of embodiments of 9mL blood tubes prepared in accordance with embodiments of the present disclosure.

FIG. 92 is a graph showing the water vapour transmission rates (WVTR) of embodiments of 9mL blood tubes prepared in accordance with embodiments of the present disclosure.

FIG. 93 is a perspective view showing an embodiment of a blood tube as described herein

The following reference characters are used in the drawing figures:

| | |
|---|---|
| 210 | Pharmaceutical package |
| 212 | Lumen |
| 214 | Wall |
| 216 | Outer surface |
| 218 | Fluid |
| 220 | Interior surface (of 288) |
| 222 | Outer surface (of 288) |
| 224 | Interior surface (of 286) |
| 226 | Outer surface (of 286) |
| 228 | Vial |
| 230 | Blister package |
| 250 | Syringe barrel |
| 252 | Syringe |
| 254 | Inner or interior surface (of 250) |
| 256 | Back end (of 250) |
| 258 | Plunger (of 252) (relatively sliding part) |
| 259 | Lubricant |
| 260 | Front end (of 250) |
| 262 | Closure |
| 264 | Inner or interior surface (of 262) |
| 268 | Vessel |
| 270 | Closure |
| 272 | Interior facing surface |
| 274 | Lumen |
| 276 | Wall-contacting surface |
| 278 | Inner or interior surface (of 280) |
| 280 | Vessel wall |
| 281 | Lubricity coating or layer |
| 282 | Stopper |
| 283 | Primer coating or layer |
| 284 | Shield |
| 285 | Vessel coating or layer set |
| 286 | pH protective coating or layer |
| 287 | Deposit of lubricant |
| 288 | Barrier layer |
| 289 | Tie coating or layer |
| 290 | Apparatus for coating, for example, 250 |
| 292 | Inner or interior surface (of 294) |

(continued)

| | |
|---|---|
| 294 | Restricted opening (of 250) |
| 296 | Processing vessel |
| 298 | Outer surface (of 250) |
| 300 | Moisture (water vapor) barrier layer |
| 301 | Oxygen barrier layer |
| 400 | Vial |
| 401 | Bottom Wall |
| 402 | Side Wall |
| 403 | Transition Region (Side Wall to Bottom Wall) |
| 404 | Shoulder |
| 405 | Neck |
| 405a | Neck flange |
| 406 | Opening |
| 407 | Lower surface (of bottom wall) |
| 408 | tray |
| 409 | Plot of COP vial outer diameters |
| 410 | Plot of glass vial outer diameters |
| 411 | Rubber stopper/plug |
| 412 | Metal crimp/cap |
| 500 | Syringe |
| 501 | Syringe barrel |
| 502 | Syringe barrel inner diameter |
| 503 | Syringe barrel outer diameter |
| 504 | Syringe barrel length |
| 505 | Syringe flange outer diameter |
| 506 | Needle hub |
| 507 | Luer hub |
| 508 | Flange |
| 509 | Plunger |
| 510 | Plunger rod |
| 511 | Rigid needle shield |
| 520 | Plunger anti-backout feature |
| 521 | Finger flange |
| 522 | Central aperture |
| 523 | Backstop engagement feature |
| 524 | Backstop element |
| 525 | Outer surface (of backstop engagement feature) |
| 526 | Interior Wall (of backstop element) |
| 527 | Contact surface (of backstop element) |
| 528 | Locking collet |

(continued)

| | |
|---|---|
| 529 | Threaded Housing |
| 530 | Twist lock thumb nut |
| 531 | Interior surface (of locking collet) |
| 532 | Drafted portion (of locking collet) |
| 533 | Central aperture (of threaded housing) |
| 534 | Interior wall (of threaded housing) |
| 535 | Male anti-rotation element |
| 536 | Female anti-rotation element |
| 537 | Central aperture (of thumb nut) |
| 538 | Wall (of thumb nut) |
| 539 | Drafted portion (of thumb nut) |
| 540 | Gripping portion (of thumb nut) |
| 541 | Locking block cavity |
| 542 | Locking block |
| 543 | Aperture (of locking block) |
| 544 | Larger portion of aperture |
| 545 | Smaller portion of aperture |
| 546 | Rib |
| 547 | Upper contact surface |
| 548 | First end surface (of locking block) |
| 549 | Second end surface (of locking block) |
| 550 | Retention ribs |
| 551 | Indents |

In the context of the present invention, the following definitions and abbreviations are used:
ALD is atomic layer deposition and includes both thermally-assisted atomic layer deposition and plasma enhanced atomic layer deposition, which might also be referred to as PEALD.

Commodity resins are plastics that are inexpensive, easy to process, and can be produced at high volumes. Commodity resins are distinguished from specialty resins and engineering resins, such as the previously disclosed COP and COC, by lower cost and higher production volume, among other things. Commodity resins include, for example, ABS, acrylics, polyethylene and HDPE, PVC, PET, PETG, polypropylene, polyamides, polystyrene, polycarbonate, TRITAN™ (a product of Eastman Chemical Company), thermoplastic olefinic polymers, and the like. Though not widely available or used, for purposes of the present disclosure, CBC resins can also be considered commodity resins.

RF is radio frequency.

The term "at least" in the context of the present invention means "equal or more" than the integer following the term. The word "comprising" does not exclude other elements or steps, and the indefinite article "a" or "an" does not exclude a plurality unless indicated otherwise. Whenever a parameter range is indicated, it is intended to disclose the parameter values given as limits of the range and all values of the parameter falling within said range.

"First" and "second" or similar references to, for example, deposits of lubricant, processing stations or processing devices refer to the minimum number of deposits, processing stations or devices that are present, but do not necessarily represent the order or total number of deposits, processing stations and devices or require additional deposits, processing stations and devices beyond the stated number. These terms do not limit the number of processing stations or the particular processing carried out at the respective stations. For example, a "first" deposit in the context of this specification can be either the only deposit or any one of plural deposits, without limitation. In other words, recitation of a "first" deposit allows but does not require an embodiment that also has a second or further deposit.

For purposes of the present invention, an "organosilicon precursor" is a compound having at least one of the linkages:

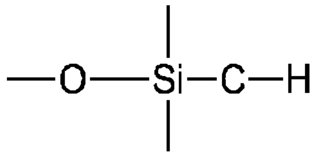

or

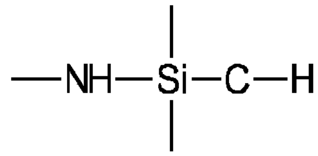

which is a tetravalent silicon atom connected to an oxygen or nitrogen atom and an organic carbon atom (an organic carbon atom being a carbon atom bonded to at least one hydrogen atom). A volatile organosilicon precursor, defined as such a precursor that can be supplied as a vapor in a PECVD apparatus, is an optional organosilicon precursor. Optionally, the organosilicon precursor is selected from the group consisting of a linear siloxane, a monocyclic siloxane, a polycyclic siloxane, a polysilsesquioxane, an alkyl trimethoxysilane, a linear silazane, a monocyclic silazane, a polycyclic silazane, a polysilsesquiazane, and a combination of any two or more of these precursors.

The feed amounts of PECVD precursors, gaseous reactant or process gases, and carrier gas are sometimes expressed in "standard volumes" in the specification and claims. The standard volume of a charge or other fixed amount of gas is the volume the fixed amount of the gas would occupy at a standard temperature and pressure (without regard to the actual temperature and pressure of delivery). Standard volumes can be measured using different units of volume, and still be within the scope of the present disclosure. For example, the same fixed amount of gas could be expressed as the number of standard cubic centimeters, the number of standard cubic meters, or the number of standard cubic feet. Standard volumes can also be defined using different standard temperatures and pressures, and still be within the scope of the present disclosure. For example, the standard temperature might be 0°C and the standard pressure might be 760 Torr (as is conventional), or the standard temperature might be 20°C and the standard pressure might be 1 Torr. But whatever standard is used in a given case, when comparing relative amounts of two or more different gases without specifying particular parameters, the same units of volume, standard temperature, and standard pressure are to be used relative to each gas, unless otherwise indicated.

The corresponding feed rates of PECVD precursors, gaseous reactant or process gases, and carrier gas are expressed in standard volumes per unit of time in the specification. For example, in the working examples the flow rates are expressed as standard cubic centimeters per minute, abbreviated as sccm. As with the other parameters, other units of time can be used, such as seconds or hours, but consistent parameters are to be used when comparing the flow rates of two or more gases, unless otherwise indicated.

A "vessel" in the context of the present invention can be any type of vessel with at least one opening and a wall defining an inner or interior surface. The substrate can be the wall of a vessel having a lumen. Though the disclosure is not necessarily limited to pharmaceutical packages or other vessels of a particular volume, pharmaceutical packages or other vessels are contemplated in which the lumen has a void volume of from 0.5 to 50 mL, optionally from 1 to 10 mL, optionally from 0.5 to 5 mL, optionally from 1 to 3 mL. The substrate surface can be part or all of the inner or interior surface of a vessel having at least one opening and an inner or interior surface. Some examples of a pharmaceutical package include, but are not limited to, a vial, a plastic-coated vial, a syringe, a plastic coated syringe, a blister pack, an ampoule, a plastic coated ampoule, a cartridge, a bottle, a plastic coated bottle, a pouch, a pump, a sprayer, a stopper, a needle, a plunger, a cap, a stent, a catheter or an implant.

The term "at least" in the context of the present invention means "equal or more" than the integer following the term. Thus, a vessel in the context of the present invention has one or more openings. One or two openings, like the openings of a sample tube (one opening) or a syringe barrel (two openings) are preferred. If the vessel has two openings, they can be of same or different size. If there is more than one opening, one opening can be used for the gas inlet for a PECVD coating method according to the present invention, while the other openings are either capped or open. A vessel according to the present invention can be a sample tube, for example for collecting or storing biological fluids like blood or urine, a syringe (or a part thereof, for example a syringe barrel) for storing or delivering a biologically active compound or composition, for example a medicament or pharmaceutical composition, a vial for storing biological materials or biologically active compounds or compositions, a pipe, for example a catheter for transporting biological materials or biologically active compounds or compositions, or a cuvette for holding fluids, for example for holding biological materials or biologically active compounds or compositions.

A vessel can be of any shape, a vessel having a substantially cylindrical wall adjacent to at least one of its open ends being preferred. Generally, the interior wall of the vessel is cylindrically shaped, like, for example in a sample tube or a syringe barrel. Sample tubes and syringes or their parts (for example syringe barrels) are contemplated.

A "hydrophobic layer" in the context of the present invention means that the coating or layer lowers the wetting tension of a surface coated with the coating or layer, compared to the corresponding uncoated surface. Hydrophobicity is thus a function of both the uncoated substrate and the coating or layer. The same applies with appropriate alterations for other contexts wherein the term "hydrophobic" is used. The term "hydrophilic" means the opposite, i.e. that the wetting tension is increased compared to reference sample. The present hydrophobic layers are primarily defined by their hydrophobicity and the process conditions providing hydrophobicity

These values of w, x, y, and z are applicable to the empirical composition $Si_wO_xC_yH_z$ throughout this specification. The values of w, x, y, and z used throughout this specification should be understood as ratios or an empirical formula (for example for a coating or layer), rather than as a limit on the number or type of atoms in a molecule. For example, octamethylcyclotetrasiloxane, which has the molecular composition $Si_4O_4C_8H_{24}$, can be described by the following empirical formula, arrived at by dividing each of w, x, y, and z in the molecular formula by 4, the largest common factor: $Si_1O_1C_2H_6$. The values of w, x, y, and z are also not limited to integers. For example, (acyclic) octamethyltrisiloxane, molecular composition $Si_3O_2C_8H_{24}$,is reducible to $Si_1O_{0.67}C_{2.67}H_8$. Also, although $SiO_xC_yH_z$ is described as equivalent to $SiO_xC_y$, it is not necessary to show the presence of hydrogen in any proportion to show the presence of $SiO_xC_y$.

"Wetting tension" is a specific measure for the hydrophobicity or hydrophilicity of a surface. An optional wetting tension measurement method in the context of the present invention is ASTM D 2578 or a modification of the method described in ASTM D 2578. This method uses standard wetting tension solutions (called dyne solutions) to determine the solution that comes nearest to wetting a plastic film surface for exactly two seconds. This is the film's wetting tension. The procedure utilized is varied herein from ASTM D 2578 in that the substrates are not flat plastic films, but are tubes made according to the Protocol for Forming PET Tube and (except for controls) coated according to the Protocol for coating Tube Interior with Hydrophobic Coating or Layer (see Example 9 of EP2251671 A2).

The atomic ratio can be determined by XPS. Taking into account the H atoms, which are not measured by XPS, the coating or layer may thus in one aspect have the formula $Si_wO_xC_yH_z$ (or its equivalent $SiO_xC_y$), for example where w is 1, x is from about 0.5 to about 2.4, y is from about 0.6 to about 3, and z is from about 2 to about 9. Typically, such coating or layer would hence contain 36% to 41% carbon normalized to 100% carbon plus oxygen plus silicon.

The term "syringe" is broadly defined to include cartridges, injection "pens," and other types of barrels or reservoirs adapted to be assembled with one or more other components to provide a functional syringe. "Syringe" is also broadly defined to include related articles such as auto-injectors, which provide a mechanism for dispensing the contents.

A coating or layer or treatment is defined as "hydrophobic" if it lowers the wetting tension of a surface, compared to the corresponding uncoated or untreated surface. Hydrophobicity is thus a function of both the untreated substrate and the treatment.

"Drug product" refers to a composition, typically a fluid, containing a pharmacologically active substance (also referred to as an active pharmaceutical ingredient or API) and optionally one or more excipients. A reduction in the rate and/or amount of degradation of a drug product includes a reduction in the rate and/or amount of degradation of the pharmaceuticaly active substance as well as a reduction in the rate and/or amount of degradation of the one or more of excipients. For instance, a reduction in the rate and/or amount of degradation of a drug product may include either a reduction solely in the rate and/or amount of degradation of the pharmaceutically active substance or a reduction solely in the rate and/or amount of degradation of the one or more excipients. A reduction in the rate and/or amount of degradation of a drug product may also include both a reduction in the rate and/or amount of degradation of the pharmaceutically active substance and a reduction in the rate and/or amount of degradation of the one or more excipients.

"Excipient" refers to any pharmacologically inactive substance that, when combined with a pharmacologically active substance, provides a benefit to the drug product. These benefits may include, for instance, (a) enhancing solubility of the active substance, (b) enhancing process and/or shelf life stability of the active substance, (c) controlling pH and tonicity of the composition, (d) maintaining a preferred stable conformation for active proteins or vaccines, including exposure of the functional epitopes, (e) preventing aggregation or degradation of the active substance, (f) enhancing the pharmacological effect of the active substance or increasing the ability of an antigen to stimulate the immune system, e.g., an adjuvant, and (g) one or more of several other functions including but not limited to bulking agents, antioxidants, colorants, and preservatives. Due to the complexity and fragility of biologic drugs, excipients are of particular importance for biological drug products, e.g. to increase product stability, maintain tonicity, and/or facilitate drug delivery.

Common excipients include buffering agents (pH modifiers) such as acetate, citrate, citric acid, sodium citrate, tartrate, histidine, glutamate, phosphate, tris(hydroxymethyl)aminomethane ("Tris"), glycine, bicarbonate, succinate, sulfate, and nitrate; tonicity modifiers such as mannitol, sorbitol, lactose, dextrose, trehalose, sucrose, sodium chloride, potassium chloride, glycerol, and glycerine; bulking agents such as arginine, aspartic acid, glutamic acid, lysine, proline, glycine, histidine, methionine, alanine, gelatin, PVP, PLGA, PEG, dextran, cyclodextrin and derivatives, starch derivatives, HSA, and BSA; surfactants (wetting and/or solubilizing agents) such as polysorbates (e.g. polysorbate 20 and polysorbate 80), poloxamers (e.g. Pluronic F68 and F127), Triton X-100, Brij 30, Brij 35, and sodiuim lauryl sulfate; antioxidant preservatives such as histamine, cysteine, methionine, ascorbic acid, glutathione, vitamin E, vitamin A, propyl gallate, retinyl palmitate, selenium, and poly(ethylenimine); antimicrobial preservatives such as benzyl alcohol, metacresol, phenol, 2-phenoxyethanol, and parabens (e.g. methyl paraben and propyl paraben); chelating and/or complexing agents (preservatives) such as edetate disodium, diethylenetriamine pentaacetic acid (DTPA), citric acid, hexaphosphate, thioglycolic acid, and zinc; adjuvants; and colorants. In particular, sodium chloride, polysorbate (e.g. polysorbate 20 or polysorbate 80), sucrose, and mannitol are present as excipients in many drug products.

The word "comprising" does not exclude other elements or steps.

The indefinite article "a" or "an" does not exclude a plurality.

## DETAILED DESCRIPTION

The present invention will now be described more fully, with reference to the accompanying drawings, in which several embodiments are shown. This invention can, however, be embodied in many different forms and should not be construed as limited to the embodiments set forth here. Rather, these embodiments are examples of the invention, which has the full scope indicated by the language of the claims. Like numbers refer to like or corresponding elements throughout. The following disclosure relates to all embodiments unless specifically limited to a certain embodiment.

Embodiments of the present disclosure are directed to the coating of vessels made, at least in part, from one or more specialty resins or one or more commodity resins to achieve coated vessels that are suitable for containing, for instance, an injectable solution. This may be achieved using a combination of ALD and PECVD coating processes to apply a variety of layers that serve as an oxygen barrier, optionally a water vapor transmission (or moisture) barrier, and a pH protective layer. By using ALD in place of PECVD, coating defects may be minimized. In contrast to PECVD deposited coatings and layers, because ALD is a relatively slow and extremely precise deposition process, films deposited by ALD do not contain the same degree of defects as films deposited by PECVD due to the surface roughness of some specialty and commodity resins.

Without being bound by theory, it is believed that once a sufficient coating or layer has been deposited by ALD, coatings or layers that are subsequently applied by PECVD do not contain the same defects as a PECVD coating applied directly to the surface of a commodity resin. It is also believed that defects in a subsequently applied PECVD coating or layer may have less impact on the performance attributes of the coated vessel than defects in a PECVD layer applied directly to the surface of a commodity resin, since the defects do not span all the way to the vessel wall itself, but rather only to the ALD-deposited coating or layer.

Embodiments of the present disclosure are directed to drug primary packages such as vials and syringes, and to thermoplastic vials and syringes configured for such uses and having a variety of benefits. The vials and syringes may be provided with a gas barrier coating, e.g. by ALD, that serves as an oxygen barrier, a water vapor barrier, a nitrogen barrier, a carbon monoxide barrier, a carbon dioxide barrier, an ethylene oxide barrier, or any combination thereof. The vials and syringes may also be prepared and configured to provide other benefits, such as low particles, improved thermal exchange (vials), superior closure integrity including at very low temperatures and/or when subjected to freeze-thaw cycles, a drug-interfacing interior surface having a customizable surface energy, lubricity (syringes) without silicone oil or baked-on silicone, and enhanced dimensional consistency.

Embodiments of the present disclosure are specifically directed to vials and syringes that are specifically configured and suitable for the storage of lyophilized or cold-chain drug products, such as DNA-based and mRNA-based vaccines. In particular, embodiments of the vials and syringes are configured to maintain container closure integrity throughout the life cycle of a lyophilized or cold-chain drug product. In some embodiments, for example, the vials and syringes may be produced with a degree of dimensional consistency beyond that seen in the art, allowing for tight tolerances with stoppers, plungers, rigid needle shields, etc. Moreover, in some embodiments, the vials or syringes may comprise one or more features designed to maintain CCI at low temperatures, including for instance a CCI-enhancing plunger gasket and/or a plunger anti-backout feature, as disclosed in detail herein. Further, embodiments of the vials and syringes may be provided with one or more barrier coatings or layers, which may be configured and/or customized to provide a suitable gas barrier for a particular lyophilized or cold-chain drug, e.g. a DNA-based or mRNA-based vaccine.

Embodiments of the present disclosure are directed to blood tubes. The blood tubes may be provided with a gas barrier coating, e.g. by ALD, that serves as an oxygen barrier, a water vapor barrier, a nitrogen barrier, a carbon dioxide barrier, or any combination thereof. Due to the provision of an enhanced barrier against environmental gases, the shelf life of an evacuated blood tube may for the first time be extended to 36 months or more. Moreover, the inclusion of a water vapor barrier coating or layer may prevent the loss of solvent from a preservative agent contained within the blood tube, improving the shelf life of the preservative as well.

### Vessels and Coating Sets

An aspect of the invention, illustrated most broadly by Figure 1 and the detail view of Fig. 2, is a vessel 210 including a wall 214 enclosing a lumen 212 and a vessel coating or layer set 285 on at least a portion of the wall 214 facing the lumen 212. The vessel may be more specifically a vial, a syringe, a blister pack, an ampoule, a cartridge, a bottle, a pouch, a pump, a sprayer, a stopper, a needle, a plunger, a cap, a stent, a catheter or an implant, or any other type of container or conduit for a fluid. Figs. 1 through 5 show a vessel having at least a single opening, and should be understood to include a vessel having two or more openings, such as a syringe, or a vessel having no openings, such as a pouch, blister pack, or ampoule.

An embodiment of the vessel coating or layer set 285 is at least one tie coating or layer 289, at least one barrier coating or layer 288, and at least one pH protective coating or layer 286, illustrated in Figs. 1, 2. This embodiment of the vessel coating or layer set is sometimes known as a "trilayer coating" in which the barrier coating or layer 288 of $SiO_x$ is

protected against contents having a pH otherwise high enough to remove it by being sandwiched between the pH protective coating or layer 286 and the tie coating or layer 289, each an organic layer of $SiO_xC_y$ as defined in this specification. A specific example of this trilayer coating is provided in this specification. The contemplated thicknesses of the respective layers in nm (preferred ranges in parentheses) are given in the Trilayer Thickness Table.

| Trilayer Thickness Table | | |
|---|---|---|
| **Adhesion** | **Barrier** | **Protection** |
| 5-100 (5-20) if by PECVD<br>1-20 (2-15) if by ALD | 20-200 (20-30) if by PECVD<br>1-20 (2-15) if by ALD | 10-500 (100-200) |

Several particular coordinating coating sets 285, 285a, and 285b for a vessel 210 and closure of Fig. #1 are shown in the Table of Coating Sets:

| Table of Coating Sets | | | |
|---|---|---|---|
| **Set** | **Vessel wall (285)** | **Closure sliding surface (285a)** | **Closure facing surface (285b)** |
| 1 | • pH protective (286)<br>• barrier (288)<br>• tie (289)<br>• syringe barrel wall (214) | • Lubricity (281) e.g. Parylene.<br>• Sliding surface of closure, e.g. plunger tip | • Barrier (288) - e.g. Parylene<br>• Facing surface of closure, e.g. plunger tip. |
| 2 | • Lubricant deposit (287)<br>• SiOx primer (283)<br>• pH protective (286)<br>• barrier (288)<br>• tie (289)<br>• syringe barrel wall (214) | • No coating set 285a<br>• Sliding surface of closure, e.g. plunger tip | • pH protective (286)<br>• barrier (288)<br>• Facing surface of closure |
| 3 | • pH protective (286)<br>• barrier (288)<br>• syringe barrel wall (214) | • Lubricity (281) e.g. Parylene.<br>• Sliding surface of closure, e.g. plunger tip | • Barrier (288) - e.g. Parylene<br>• Facing surface of closure, e.g. plunger tip. |
| 4 | • SiOx primer (283)<br>• pH protective (286)<br>• barrier (288)<br>• syringe barrel wall (214) | • Lubricity (281) e.g. SiOxCy<br>• Sliding surface of closure, e.g. plunger tip | • pH protective (286)<br>• barrier (288)<br>• Facing surface of closure |
| 5 | • pH protective (286)<br>• barrier (288)<br>• Vial wall (214) | • Lubricant deposit (287)<br>• Sliding surface of closure (e.g. septum) | • Lubricant deposit (287)<br>• Facing surface of closure (e.g. septum) |
| 6 | • pH protective (286)<br>• barrier (288)<br>• tie (289)<br>• Vial wall (214) | • Lubricant deposit (287)<br>• Sliding surface of closure (e.g. septum) | • Lubricant deposit (287)<br>• Facing surface of closure (e.g. septum) |
| 7 | • Lubricity (281) e.g. SiOxCy<br>• pH protective (286)<br>• barrier (288)<br>• tie (289)<br>• Vial wall (214) | • Barrier (288) - e.g. Parylene | • Barrier (288) - e.g. Parylene |
| 8 | • Lubricity (281) e.g. SiOxCy<br>• pH protective (286)<br>• barrier (288)<br>• Vial or syringe wall (214) | • Barrier (288) - e.g. Parylene | • Barrier (288) - e.g. Parylene |
| 9 | • pH protective (286) | • Lubricant deposit (287) | • Lubricant deposit (287) |

(continued)

| Table of Coating Sets | | | |
|---|---|---|---|
| Set | Vessel wall (285) | Closure sliding surface (285a) | Closure facing surface (285b) |
| | • gas barrier (301)<br>• moisture barrier (300)<br>• tie (289)<br>• Vial or syringe wall (214) | • Sliding surface of closure (e.g. septum) | • Facing surface of closure (e.g. septum) |
| 10 | • Lubricity (281) e.g. SiOxCy<br>• pH protective (286)<br>• gas barrier (301)<br>• tie (289)<br>• moisture barrier (300)<br>• Vial or syringe wall (214) | • Barrier (288) - e.g. Parylene | • Barrier (288) - e.g. Parylene |

Sets 1-4 and 7-8 and 10 in the Table of Coating Sets are among the useful alternatives for a syringe. The syringe barrel wall coatings (left column) of Set 1 are one example of the previously described trilayer coating, and Set 7 is a modification of the trilayer coating in which a PECVD or ALD lubricant coating or layer is the top layer of the set. Set 8 is an embodiment in which the tie coating or layer is rendered unnecessary by the use of ALD to apply the barrier coating or layer 288. Set 10 is an embodiment in which the barrier coating or layer 288 comprises both a moisture barrier layer and a gas barrier layer, and in which those two barrier layers are not adjacent one another.

The Set 1 trilayer coating set 285, illustrated in Fig. #2, is applied to a COP syringe barrel in one embodiment.

The Set 1 trilayer coating set 285 includes as a first layer an adhesion or tie coating or layer 289 that improves adhesion of the barrier coating or layer to the COP substrate. The adhesion or tie coating or layer 289 is also believed to relieve stress on the barrier coating or layer 288, making the barrier layer less subject to damage from thermal expansion or contraction or mechanical shock. The adhesion or tie coating or layer 289 is also believed to decouple defects between the barrier coating or layer 288 and the COP substrate. This is believed to occur because any pinholes or other defects that may be formed when the adhesion or tie coating or layer 289 is applied tend not to be continued when the barrier coating or layer 288 is applied, so the pinholes or other defects in one coating do not line up with defects in the other. The adhesion or tie coating or layer 289 has some efficacy as a barrier layer, so even a defect providing a leakage path extending through the barrier coating or layer 289 is blocked by the adhesion or tie coating or layer 289.

The Set 1 trilayer coating set 285 includes as a second layer a barrier coating or layer 288 that provides a barrier to oxygen that has permeated the COP barrel wall and optionally a barrier to moisture that may permeate a plastic barrel wall. The barrier coating or layer 288 also is a barrier to extraction of the composition of the barrel wall 214 by the contents of the lumen 214.

The Set 1 trilayer coating set 285 includes as a third layer a pH protective coating or layer 286 that provides protection of the underlying barrier coating or layer 288 against contents of the syringe having a pH from 4 to 8, including where a surfactant is present. For a prefilled syringe that is in contact with the contents of the syringe from the time it is manufactured to the time it is used, the pH protective coating or layer 286 prevents or inhibits attack of the barrier coating or layer 288 sufficiently to maintain an effective oxygen and/or moisture barrier over the intended shelf life of the prefilled syringe.

Sets 5 and 6 and 9 are useful for a vial, for instance. The lubricant deposit as the coating set 285b represents a siliconized septum in which the entire surface is coated with a lubricant to aid insertion into a vial neck, so the facing surface of the closure is coated although the coating is not needed there.

The vessel wall coating set 285 represented by Set 6 is another trilayer coating set, again illustrated in Fig. 2, applied to a COP vial in one embodiment. The trilayer coating has the same layers and provides the same performance as the syringe trilayer coating of Set 1 described above.

## Tie Coating or Layer

The tie coating or layer 289 has at least two functions. One function of the tie coating or layer 289 is to improve adhesion of a barrier coating or layer 288 to a substrate, in particular a thermoplastic substrate, although a tie layer can be used to improve adhesion to a glass substrate or to another coating or layer. For example, a tie coating or layer, also referred to as an adhesion layer or coating can be applied to the substrate and the barrier layer can be applied to the adhesion layer to improve adhesion of the barrier layer or coating to the substrate.

Another function of the tie coating or layer 289 has been discovered: a tie coating or layer 289 applied under a

barrier coating or layer 288 can improve the function of a pH protective coating or layer 286 applied over the barrier coating or layer 288.

The tie coating or layer 289 can be composed of, comprise, or consist essentially of $SiO_xC_y$, in which x is between 0.5 and 2.4 and y is between 0.6 and 3. Alternatively, the atomic ratio can be expressed as the formula $Si_wO_xC_y$, The atomic ratios of Si, O, and C in the tie coating or layer 289 are, as several options:

- Si 100 : O 50-150 : C 90-200 (i.e. w = 1, x = 0.5 to 1.5, y = 0.9 to 2);
- Si 100 : O 70-130 : C 90-200 (i.e. w = 1, x = 0.7 to 1.3, y = 0.9 to 2)
- Si 100 : O 80-120 : C 90-150 (i.e. w = 1, x = 0.8 to 1.2, y = 0.9 to 1.5)
- Si 100 : O 90-120 : C 90-140 (i.e. w = 1, x = 0.9 to 1.2, y = 0.9 to 1.4), or
- Si 100 : O 92-107 : C 116-133 (i.e. w = 1, x = 0.92 to 1.07, y = 1.16 to 1.33)

The atomic ratio can be determined by XPS. Taking into account the H atoms, which are not measured by XPS, the tie coating or layer 289 may thus in one aspect have the formula $Si_wO_xC_yH_z$ (or its equivalent $SiO_xC_y$), for example where w is 1, x is from about 0.5 to about 2.4, y is from about 0.6 to about 3, and z is from about 2 to about 9. Typically, tie coating or layer 289 would hence contain 36% to 41% carbon normalized to 100% carbon plus oxygen plus silicon.

Optionally, the tie coating or layer can be similar or identical in composition with the pH protective coating or layer 286 described elsewhere in this specification, although this is not a requirement.

The tie coating or layer 289 is contemplated in any embodiment generally to be from 5 nm to 100 nm thick, preferably from 5 to 20 nm thick, particularly if applied by chemical vapor deposition. These thicknesses are not critical. Commonly but not necessarily, the tie coating or layer 289 will be relatively thin, since its function is to change the surface properties of the substrate.

In some embodiments, the tie coating or layer 289 may be omitted. Where, for instance, the barrier coating or layer 288 is applied by ALD, the adhesion-improving properties of the tie coating or layer may be unnecessary.

In other embodiments, a thin tie coating or layer 289 may be applied by ALD prior to application of a barrier coating or layer 288 by ALD. In addition to the above-described $SiO_xC_y$, the tie coating or layer 289 applied by ALD may be any material that is effective to improve adhesion between the subsequently applied barrier coating or layer 288 and the vessel wall 214 or any coating already applied thereon. Such materials include metals and metal oxides such as: Al2O3, TiO2, ZrO2, HfO2, Ta2O5, Nb2, O5, Y2O3, MgO, CeO2, La2,O3, SrTiO3, BaTiO3, BixTiyOz, In2O3, In2O3:Sn, In2O3:F, In2O3:Zr, SnO2, SnO2:Sb, ZnO, ZnO:Al, Ga2O3, NiO, CoOx, YBa2Cu3O7-x, LaCoO3, LaNiO3, Si, Ge, Cu, Mo, Ta, and W. In some embodiments, zinc oxide (ZnO) or aluminum oxide ($Al_2O_3$) may be applied by ALD as a tie coating or layer 289. Due to its adhesion to polymeric films, zinc oxide (ZnO) in particular may serve as a high-quality tie coating or layer 289.

Where a tie coating or layer 289 is applied by ALD, the thickness of the tie coating or layer may be, for example, from 1 to 50 nm thick, alternatively from 1 to 20 nm thick, alternatively from 2 to 15 nm thick, alternatively from 2 to 10 nm thick, alternatively from 3 to 9 nm thick, alternatively from 4 to 8 nm thick, alternatively from 5 to 7 nm thick.

In some embodiments, the barrier coating or layer 288 may be split between an oxygen barrier layer 301 and a moisture barrier layer 300, which may or may not be applied as adjacent coatings. In some embodiments, therefore, the tie coating or layer 289 may be applied (either by PECVD or ALD) between the vessel wall 214 and a barrier coating 288 that includes both an oxygen barrier layer 301 and a moisture barrier layer 300. In other emdbodiments, however, the tie coating or layer 289 may be applied (either by PECVD or ALD) between an oxygen barrier layer 301 and a moisture barrier layer 300. For example, a moisture barrier layer 300 may be applied, e.g. by ALD, to the vessel wall 214, after which the tie coating or layer 289 may be applied, after which the oxygen barrier layer 301 may be applied. Such a coating is shown, for example, in Figure 25.

In one example, for instance, a moisture barrier layer (e.g. of $Al_2O_3$) is applied to the vessel wall by ALD. Then, a tie coating or layer 289 is applied by PECVD, an oxygen barrier layer of $SiO_x$ is applied by PECVD, and a pH protective coating or layer 286 is applied by PECVD. In another example, a moisture barrier layer is applied to the vessel wall by ALD, then a tie coating or layer 289 is applied by PECVD, an oxygen barrier layer of SiOx is applied by ALD, and a pH protective coating or layer 286 is applied by PECVD. In another example, a moisture barrier layer is applied to the vessel wall by ALD, then a tie coating or layer 289 is applied by ALD, an oxygen barrier layer of SiOx is applied by ALD, and a pH protective coating or layer 286 is applied by PECVD. In another example, a moisture barrier layer is applied to the vessel wall by ALD, then a tie coating or layer 289 is applied by ALD, an oxygen barrier layer of SiOx is applied by PECVD, and a pH protective coating or layer 286 is applied by PECVD.

In other embodiments, multiple tie coating or layers 289 may be applied. For instance, a first tie coating or layer 289 may be applied by ALD, followed by a first barrier layer such as a moisture barrier (e.g. Al2O3), followed by a second tie

coating or layer, followed by a second barrier layer such as an oxygen barrier (e.g. SiOx), followed by a pH protective coating or layer 286.

In yet other examples, a moisture barrier layer (e.g. of $Al_2O_3$) is applied to the vessel wall by ALD. Then, an oxygen barrier layer of $SiO_x$ is applied by ALD or PECVD and a pH protective coating or layer 286 is applied by PECVD.

**Barrier Layer**

A barrier coating or layer 288 optionally can be deposited by atomic layer deposition (ALD), plasma enhanced chemical vapor deposition (PECVD) or other chemical vapor deposition processes on the vessel of a pharmaceutical package, in particular a thermoplastic package, to prevent oxygen, carbon dioxide, or other gases from entering the vessel and/or to prevent leaching of the pharmaceutical material into or through the package wall.

The barrier coating or layer may comprise an SiOx coating or layer, optionally applied by PECVD as indicated in U.S. Pat. No. 7,985,188, or applied by ALD as described herein. The barrier layer optionally is characterized as an "$SiO_x$" coating, and contains silicon, oxygen, and optionally other elements, in which x, the ratio of oxygen to silicon atoms, is from about 1.5 to about 2.9, or 1.5 to about 2.6, or about 2. These alternative definitions of x apply to any use of the term $SiO_x$ in this specification. The barrier coating or layer is applied, for example to the interior of a pharmaceutical package or other vessel, for example a sample collection tube, a syringe barrel, a vial, or another type of vessel.

In some embodiments, the barrier coating 288 may comprise or consists essentially of $SiO_x$, wherein x is from 1.5 to 2.9, from 2 to 1000 nm thick, the barrier coating 288 of $SiO_x$ having an interior surface 220 facing the lumen 212 and an outer surface 222 facing the wall 214 article surface 254, the barrier coating 288 being effective to reduce the ingress of atmospheric gas into the lumen 212 compared to an uncoated vessel 250. One suitable barrier composition is one where x is 2.3, for example. For example, the barrier coating or layer such as 288 of any embodiment can be applied at a thickness of at least 2 nm, or at least 4 nm, or at least 7 nm, or at least 10 nm, or at least 20 nm, or at least 30 nm, or at least 40 nm, or at least 50 nm, or at least 100 nm, or at least 150 nm, or at least 200 nm, or at least 300 nm, or at least 400 nm, or at least 500 nm, or at least 600 nm, or at least 700 nm, or at least 800 nm, or at least 900 nm. The barrier coating or layer can be up to 1000 nm, or at most 900 nm, or at most 800 nm, or at most 700 nm, or at most 600 nm, or at most 500 nm, or at most 400 nm, or at most 300 nm, or at most 200 nm, or at most 100 nm, or at most 90 nm, or at most 80 nm, or at most 70 nm, or at most 60 nm, or at most 50 nm, or at most 40 nm, or at most 30 nm, or at most 20 nm, or at most 10 nm, or at most 5 nm thick. Ranges of 20-200 nm, optionally 20-30 nm, are particularly contemplated where the barrier coating or layer is applied by PECVD. Specific thickness ranges composed of any one of the minimum thicknesses expressed above, plus any equal or greater one of the maximum thicknesses expressed above, are also expressly contemplated.

Where the barrier coating or layer is applied by ALD, the thickness of the barrier coating or layer may be, for example, from 1 to 50 nm thick, alternatively from 1 to 20 nm thick, alternatively from 2 to 15 nm thick, alternatively from 2 to 10 nm thick, alternatively from 3 to 9 nm thick, alternatively from 4 to 8 nm thick, alternatively from 5 to 7 nm thick.

The thickness of the $SiO_x$ or other barrier coating or layer can be measured, for example, by transmission electron microscopy (TEM), and its composition can be measured by X-ray photoelectron spectroscopy (XPS). The primer coating or layer described herein can be applied to a variety of pharmaceutical packages or other vessels made from plastic or glass, for example to plastic tubes, vials, and syringes.

A barrier coating or layer 288 of $SiO_x$, in which x is between 1.5 and 2.9, is applied by atomic layer deposition (ALD) or plasma enhanced chemical vapor deposition (PECVD) directly or indirectly to the thermoplastic wall 214 (for example a tie coating or layer 289 can be interposed between them) so that in the filled pharmaceutical package or other vessel 210 the barrier coating or layer 288 is located between the inner or interior surface 220 of the thermoplastic wall 214 and the fluid 218.

The barrier coating or layer 288 of $SiO_x$ is supported by the thermoplastic wall 214. The barrier coating or layer 288 as described elsewhere in this specification, or in U.S. Patent No. 7,985,188, can be used in any embodiment.

Certain barrier coatings or layers 288 such as $SiO_x$ as defined here have been found to have the characteristic of being subject to being measurably diminished in barrier improvement factor in less than six months as a result of attack by certain relatively high pH contents of the coated vessel as described elsewhere in this specification, particularly where the barrier coating or layer directly contacts the contents. This issue can be addressed using a pH protective coating or layer as discussed in this specification.

The barrier coating or layer 288 of $SiO_x$ also can function as a primer coating or layer 283, as discussed elsewhere in this specification.

In some embodiments, the barrier coating or layer 288 may be applied by atomic layer deposition (ALD). Although ALD is a more time-consuming process than PECVD, it can be used to produce a barrier coating, e.g. an $SiO_x$ barrier coating as described above (optionally $SiO_2$), having higher density and less defects than a similar barrier coating, e.g. an $SiO_x$ barrier coating, produced by PECVD. As a result, the barrier coating or layer 288 applied by ALD may have a reduced thickness when compared to the barrier coating or layer applied by PECVD. It is also contemplated that barrier coating or layer 288 applied by ALD may have improved gas (e.g. oxygen) barrier properties when compared to a barrier coating or

layer of the same composition applied by PECVD, even when applied at a reduced thickness.

In some embodiments, the barrier coating or layer 288 may comprise one or more layers in addition to the SiOx layer described above. For instance, regardless of whether the SiOx layer is applied by ALD or PECVD, in some embodiments, one or more additional barrier layers may also be applied.

In some embodiments, it may be desirable to apply an additional moisture, i.e. water vapor, barrier layer in addition to the SiOx layer, which may function primarily as an oxygen barrier. For instance, while some plastic materials that may make up the vessel wall may themselves have adequate moisture barrier properties for some applications, other plastic materials may require that one or more moisture barrier coatings or layers be applied. Or plastic materials that may have adequate moisture barrier properties for some applications may be improved, e.g. to be equivalent or substantially equivalent to glass, for applications in which better water vapor barrier properties are particularly desirable. In some embodiments, the moisture barrier coating or layer may be applied by ALD as described herein.

In some embodiments, for instance, the barrier coating or layer 288 may comprise both (i) one or more SiOx (e.g. $SiO_2$) oxygen barrier layer(s) applied by ALD and (ii) one or more moisture barrier layer(s), e.g. $Al_2O_3$ applied by ALD. In other embodiments, for instance, the barrier coating or layer 288 may comprise both (i) one or more SiOx oxygen barrier layer(s) applied by PECVD and (ii) one or more moisture barrier layer(s), e.g. $Al_2O_3$, applied by ALD. The oxygen barrier layer and the moisture barrier layer may be applied sequentially, such that they are adjacent to one another, or they may be separated by one or more additional coatings or layers (e.g. a tie coating or layer as described above). When applied sequentially, the SiOx (e.g. $SiO_2$) oxygen barrier layer may be applied first and the moisture barrier layer may be applied second, or vice versa. In some embodiments, particularly where both are applied by ALD, the barrier coating or layer 288 may comprise a plurality of alternating layers of SiOx (e.g. $SiO_2$) and $Al_2O_3$. For instance, in some embodiments, the barrier coating or layer 288 may comprise at least two layers of $SiO_2$, alternatively at least three layers of $SiO_2$, alternatively at least four layers of $SiO_2$, and/or at least two layers of $Al_2O_3$, alternatively at least three layers of $Al_2O_3$, alternatively at least four layers of $Al_2O_3$,

In some embodiments, it has presently been found that it may be desirable to have a $SiO_2$ layer applied to the polymeric vessel wall (e.g. prior to an $Al_2O_3$ layer), as without being bound by theory, it is believed that the chemical interactions of the specific polymeric material from which the vessel wall is made and the $SiO_2$ atomic layers may produce a stronger barrier coating.

In some embodiments, the water vapor barrier coating may be a metal oxide, such as aluminum oxide, applied by ALD. The water vapor barrier coating may be applied to the interior surface of the vessel wall, to the outer surface of the vessel wall, or to both. In some embodiments, the water vapor barrier coating may even be applied as an intermediate step during the preparation of the vessel wall, with the result being that the water vapor barrier layer is sandwiched between portions of polymer that make up the vessel wall. In such an embodiment, it can be said that the water vapor barrier layer is positioned between the interior surface of the vessel wall and the outer surface of the vessel wall.

In alternative embodients, the barrier coating or layer 288 may comprise or consist essentially of any of any material that provides the vessel with adequate oxygen and/or moisture barrier properties. Such materials may include metals and metal oxides that can be deposited by ALD, such as: Al2O3, TiO2, ZrO2, HfO2, Ta2O5, Nb2, O5, Y2O3, MgO, CeO2, La2,O3, SrTiO3, BaTiO3, BixTiyOz, In2O3, In2O3:Sn, In2O3:F, In2O3:Zr, SnO2, SnO2:Sb, ZnO, ZnO:Al, Ga2O3, NiO, CoOx, YBa2Cu3O7-x, LaCoO3, LaNiO3, Si, Ge, Cu, Mo, Ta, and W.

When applied in combination, the one or more SiO2 layers and the one or more Al2O3 layers may each contribute to the oxygen barrier properties of the coating and/or the water vapour barrier properties of the coating.

In some embodiments, for example, the vessel coated with an oxygen barrier coating or layer may have an oxygen transmission rate that is equivalent to or lower than that previously obtained using PECVD-deposited SiOx coatings.

In some embodiments, for example, a vial such as a 10 mL thermoplastic vial may be coated with a barrier coating or layer as described herein and the vial (with its associated stopper) may have an OTR constant less than 0.00030 $d^{-1}$, alternatively less than 0.00025 $d^{-1}$, alternatively less than 0.00020 $d^{-1}$, alternatively less than 0.00015 $d^{-1}$, alternatively less than 0.00010 $d^{-1}$, e.g. as determined using the Oxygen Transmission Rate Protocol described herein. In some embodiments, the thermoplastic vial may comprise a polycarbonate vessel wall. In other embodiments, the thermoplastic vial may comprise a vessel wall made from a cyclic block copolymer (CBC) resin as described herein. In other embodiments, the thermoplastic vial may comprise a vessel wall made from a COP or COC resin.

In some embodiments, for example, a syringe such as a 0.3 mL syringe, a 0.5 mL syringe, or a 1 mL syringe, may be coated with a barrier coating or layer as described herein and the syringe may have an OTR constant less than 0.005 d-1, alternatively less than 0.004 d-1, alternatively less than 0.003 d-1, alternatively less than 0.002 d-1, alternatively less than 0.001 d-1, alternatively less than 0.00050 d-1, alternatively less than 0.00045 d-1, alternatively less than 0.00040 d-1, alternatively less than 0.00035 d-1, alternatively less than 0.00030 d-1, e.g. as determined using the Oxygen Transmission Rate Protocol described herein. In some embodiments, the thermoplastic vial may comprise a polycarbonate vessel wall. In other embodiments, the thermoplastic vial may comprise a vessel wall made from a cyclic block copolymer (CBC) resin as described herein. In other embodiments, the thermoplastic vial may comprise a vessel wall made from a COP or COC

resin.

In some embodiments, for example, a blood tube such as a 9 mL blood tube may be coated with a barrier coating or layer as described herein and the blood tube may have an OTR constant less than 0.00050 d-1, alternatively less than 0.00040 d-1, alternatively less than 0.00030 d-1, alternatively less than 0.00030 d-1, alternatively less than 0.00015 d-1, e.g. as determined using the Oxygen Transmission Rate Protocol described herein. In some embodiments, the thermoplastic vial may comprise a polycarbonate vessel wall. In other embodiments, the thermoplastic vial may comprise a vessel wall made from a cyclic block copolymer (CBC) resin as described herein. In other embodiments, the thermoplastic vial may comprise a vessel wall made from a COP or COC resin.

**pH Protective Coating or Layer**

Barrier layers or coatings of $SiO_x$ are eroded or dissolved by some fluids, for example aqueous compositions having a pH above about 5. Since coatings applied by chemical vapor deposition can be very thin - tens to hundreds of nanometers thick - even a relatively slow rate of erosion can remove or reduce the effectiveness of the barrier layer in less time than the desired shelf life of a product package. This is particularly a problem for fluid pharmaceutical compositions, since many of them have a pH of roughly 7, or more broadly in the range of 5 to 9, similar to the pH of blood and other human or animal fluids. The higher the pH of the pharmaceutical preparation, the more quickly it erodes or dissolves the $SiO_x$ coating. Optionally, this problem can be addressed by protecting the barrier coating or layer 288, or other pH sensitive material, with a pH protective coating or layer 286.

Optionally, the pH protective coating or layer 286 can be composed of, comprise, or consist essentially of $Si_wO_xC_yH_z$ (or its equivalent $SiO_xC_y$) or $Si_wN_xC_yH_z$ or its equivalent $Si(NH)_xC_y$), each as defined previously. The atomic ratio of Si : O : C or Si : N : C can be determined by XPS (X-ray photoelectron spectroscopy). Taking into account the H atoms, the pH protective coating or layer may thus in one aspect have the formula $Si_wO_xC_yH_z$, or its equivalent $SiO_xC_y$, for example where w is 1, x is from about 0.5 to about 2.4, y is from about to about 3, and z is from about 2 to about 9.

Typically, expressed as the formula $Si_wO_xC_y$, the atomic ratios of Si, O, and C are, as several options:

- Si 100 : O 50-150 : C 90-200 (i.e. w = 1, x = 0.5 to 1.5, y = 0.9 to 2);
- Si 100 : O 70-130 : C 90-200 (i.e. w = 1, x = 0.7 to 1.3, y = 0.9 to 2)
- Si 100 : O 80-120 : C 90-150 (i.e. w = 1, x = 0.8 to 1.2, y = 0.9 to 1.5)
- Si 100 : O 90-120 : C 90-140 (i.e. w = 1, x = 0.9 to 1.2, y = 0.9 to 1.4)
- Si 100 : O 92-107 : C 116-133 (i.e. w = 1, x = 0.92 to 1.07, y = 1.16 to 1.33), or
- Si 100 : O 80-130 : C 90-150.

Alternatively, the pH protective coating or layer can have atomic concentrations normalized to 100% carbon, oxygen, and silicon, as determined by X-ray photoelectron spectroscopy (XPS) of less than 50% carbon and more than 25% silicon. Alternatively, the atomic concentrations are from 25 to 45% carbon, 25 to 65% silicon, and 10 to 35% oxygen.

Alternatively, the atomic concentrations are from 30 to 40% carbon, 32 to 52% silicon, and 20 to 27% oxygen. Alternatively, the atomic concentrations are from 33 to 37% carbon, 37 to 47% silicon, and 22 to 26% oxygen.

The thickness of the pH protective coating or layer can be, for example: from 10 nm to 1000 nm; alternatively from 10 nm to 1000 nm; alternatively from 10 nm to 900 nm; alternatively from 10 nm to 800 nm; alternatively from 10 nm to 700 nm; alternatively from 10 nm to 600 nm; alternatively from 10 nm to 500 nm; alternatively from 10 nm to 400 nm; alternatively from 10 nm to 300 nm; alternatively from 10 nm to 200 nm; alternatively from 10 nm to 100 nm; alternatively from 10 nm to 50 nm; alternatively from 20 nm to 1000 nm; alternatively from 50 nm to 1000 nm; alternatively from 10 nm to 1000 nm; alternatively from 50 nm to 800 nm; alternatively from 100 nm to 700 nm; or alternatively from 300 to 600 nm.

Optionally, the atomic concentration of carbon in the protective layer, normalized to 100% of carbon, oxygen, and silicon, as determined by X-ray photoelectron spectroscopy (XPS), can be greater than the atomic concentration of carbon in the atomic formula for the organosilicon precursor. For example, embodiments are contemplated in which the atomic concentration of carbon increases by from 1 to 80 atomic percent, alternatively from 10 to 70 atomic percent, alternatively from 20 to 60 atomic percent, alternatively from 30 to 50 atomic percent, alternatively from 35 to 45 atomic percent, alternatively from 37 to 41 atomic percent.

Optionally, the atomic ratio of carbon to oxygen in the pH protective coating or layer can be increased in comparison to the organosilicon precursor, and/or the atomic ratio of oxygen to silicon can be decreased in comparison to the organosilicon precursor.

Optionally, the pH protective coating or layer can have an atomic concentration of silicon, normalized to 100% of carbon, oxygen, and silicon, as determined by X-ray photoelectron spectroscopy (XPS), less than the atomic concentration of silicon in the atomic formula for the feed gas. For example, embodiments are contemplated in which the atomic concentration of silicon decreases by from 1 to 80 atomic percent, alternatively by from 10 to 70 atomic percent, alternatively by from 20 to 60 atomic percent, alternatively by from 30 to 55 atomic percent, alternatively by from 40 to 50 atomic percent, alternatively by from 42 to 46 atomic percent.

As another option, a pH protective coating or layer is contemplated in any embodiment that can be characterized by a sum formula wherein the atomic ratio C : O can be increased and/or the atomic ratio Si : O can be decreased in comparison to the sum formula of the organosilicon precursor.

The pH protective coating or layer 286 commonly is located between the barrier coating or layer 288 and the fluid 218 in the finished article. The pH protective coating or layer 286 is supported by the thermoplastic wall 214.

The pH protective coating or layer 286 optionally is effective to keep the barrier coating or layer 288 at least substantially undissolved as a result of attack by the fluid 218 for a period of at least six months.

The pH protective coating or layer can have a density between 1.25 and 1.65 g/cm$^3$, alternatively between 1.35 and 1.55 g/cm$^3$, alternatively between 1.4 and 1.5 g/cm$^3$, alternatively between 1.4 and 1.5 g/cm$^3$, alternatively between 1.44 and 1.48 g/cm$^3$, as determined by X-ray reflectivity (XRR). Optionally, the organosilicon compound can be octamethylcyclotetrasiloxane and the pH protective coating or layer can have a density which can be higher than the density of a pH protective coating or layer made from HMDSO as the organosilicon compound under the same PECVD reaction conditions.

**The** pH protective coating or layer optionally can prevent or reduce the precipitation of a compound or component of a composition in contact with the pH protective coating or layer, in particular can prevent or reduce insulin precipitation or blood clotting, in comparison to the uncoated surface and/or to a barrier coated surface using HMDSO as precursor.

The pH protective coating or layer optionally can have an RMS surface roughness value (measured by AFM) of from about 5 to about 9, optionally from about 6 to about 8, optionally from about 6.4 to about 7.8. The $R_a$ surface roughness value of the pH protective coating or layer, measured by AFM, can be from about 4 to about 6, optionally from about 4.6 to about 5.8. The $R_{max}$ surface roughness value of the pH protective coating or layer, measured by AFM, can be from about 70 to about 160, optionally from about 84 to about 142, optionally from about 90 to about 130.

The interior surface of the pH protective optionally can have a contact angle (with distilled water) of from 90°to 110°, optionally from 80°to 120°, optionally from 70°to 130°, as measured by Goniometer Angle measurement of a water droplet on the pH protective surface, per ASTM D7334 - 08 "Standard Practice for Surface Wettability of Coatings, Substrates and Pigments by Advancing Contact Angle Measurement."

The passivation layer or pH protective coating or layer 286 optionally shows an O-Parameter measured with attenuated total reflection (ATR) of less than 0.4, measured as:

$$\text{O-Parameter} = \frac{\text{Intensity at 1253 cm-1}}{\text{Maximum intensity in the range 1000 to 1100 cm-1.}}$$

The O-Parameter is defined in U.S. Patent No. 8,067,070, which claims an O-parameter value of most broadly from 0.4 to 0.9. It can be measured from physical analysis of an FTIR amplitude versus wave number plot to find the numerator and denominator of the above expression, as shown in FIG. 6, which is the same as FIG. 5 of U.S. Patent No. 8,067,070, except annotated to show interpolation of the wave number and absorbance scales to arrive at an absorbance at 1253 cm-1 of .0424 and a maximum absorbance at 1000 to 1100 cm-1 of 0.08, resulting in a calculated O-parameter of 0.53. The O-Parameter can also be measured from digital wave number versus absorbance data.

U.S. Patent No. 8,067,070 asserts that the claimed O-parameter range provides a superior pH protective coating or layer, relying on experiments only with HMDSO and HMDSN, which are both non-cyclic siloxanes. Surprisingly, it has been found by the present inventors that if the PECVD precursor is a cyclic siloxane, for example OMCTS, O- parameters outside the ranges claimed in U.S. Patent No. 8,067,070, using OMCTS, provide even better results than are obtained in U.S. Patent No. 8,067,070 with HMDSO.

Alternatively in the embodiment of FIGS. 1-5, the O-parameter has a value of from 0.1 to 0.39, or from 0.15 to 0.37, or from 0.17 to 0.35.

Even another aspect of the invention is a composite material as just described, exemplified in FIGS. 1-5, wherein the passivation layer shows an N-Parameter measured with attenuated total reflection (ATR) of less than 0.7, measured as:

$$\text{N-Parameter} = \frac{\text{Intensity at 840 cm-1}}{\text{Intensity at 799 cm-1.}}$$

The N-Parameter is also described in U.S. Patent No. 8,067,070, and is measured analogously to the O-Parameter except that intensities at two specific wave numbers are used - neither of these wave numbers is a range. U.S. Patent No. 8,067,070 claims a passivation layer with an N-Parameter of 0.7 to 1.6. Again, the present inventors have made better coatings employing a pH protective coating or layer 286 having an N-Parameter lower than 0.7, as described above. Alternatively, the N-parameter has a value of at least 0.3, or from 0.4 to 0.6, or at least 0.53.

The rate of erosion, dissolution, or leaching (different names for related concepts) of the pH protective coating or layer 286, if directly contacted by the fluid 218, is less than the rate of erosion of the barrier coating or layer 288, if directly contacted by the fluid 218.

The thickness of the pH protective coating or layer is contemplated in any embodiment to be from 50-500 nm, with a preferred range of 100-200 nm.

The pH protective coating or layer 286 is effective to isolate the fluid 218 from the barrier coating or layer 288, at least for sufficient time to allow the barrier coating to act as a barrier during the shelf life of the pharmaceutical package or other vessel 210.

The inventors have further found that certain pH protective coatings or layers of $SiO_xC_y$ or $Si(NH)_xC_y$ formed from polysiloxane precursors, which pH protective coatings or layers have a substantial organic component, do not erode quickly when exposed to fluids, and in fact erode or dissolve more slowly when the fluids have higher pHs within the range of 5 to 9. For example, at pH 8, the dissolution rate of a pH protective coating or layer made from the precursor octamethylcyclotetrasiloxane, or OMCTS, is quite slow. These pH protective coatings or layers of $SiO_xC_y$ or $Si(NH)_xC_y$ can therefore be used to cover a barrier layer of $SiO_x$, retaining the benefits of the barrier layer by protecting it from the fluid in the pharmaceutical package. The protective layer is applied over at least a portion of the $SiO_x$ layer to protect the $SiO_x$ layer from contents stored in a vessel, where the contents otherwise would be in contact with the $SiO_x$ layer.

Although the present invention does not depend upon the accuracy of the following theory, it is further believed that effective pH protective coatings or layers for avoiding erosion can be made from siloxanes and silazanes as described in this disclosure. $SiO_xC_y$ or $Si(NH)_xC_y$ coatings deposited from cyclic siloxane or linear silazane precursors, for example octamethylcyclotetrasiloxane (OMCTS), are believed to include intact cyclic siloxane rings and longer series of repeating units of the precursor structure. These coatings are believed to be nanoporous but structured and hydrophobic, and these properties are believed to contribute to their success as pH protective coatings or layers, and also protective coatings or layers. This is shown, for example, in U.S. Pat. No. 7,901,783.

$SiO_xC_y$ or $Si(NH)_xC_y$ coatings also can be deposited from linear siloxane or linear silazane precursors, for example hexamethyldisiloxane (HMDSO) or tetramethyldisiloxane (TMDSO).

Optionally an FTIR absorbance spectrum of the pH protective coating or layer 286 of any embodiment has a ratio greater than 0.75 between the maximum amplitude of the Si- O-Si symmetrical stretch peak normally located between about 1000 and 1040 cm-1, and the maximum amplitude of the Si-O-Si assymmetric stretch peak normally located between about 1060 and about 1100 cm-1. Alternatively in any embodiment, this ratio can be at least 0.8, or at least 0.9, or at least 1.0, or at least 1.1, or at least 1.2. Alternatively in any embodiment, this ratio can be at most 1.7, or at most 1.6, or at most 1.5, or at most 1.4, or at most 1.3. Any minimum ratio stated here can be combined with any maximum ratio stated here, as an alternative embodiment of the invention of FIGS. 1-5.

Optionally, in any embodiment the pH protective coating or layer 286, in the absence of the medicament, has a non-oily appearance. This appearance has been observed in some instances to distinguish an effective pH protective coating or layer from a lubricity layer, which in some instances has been observed to have an oily (i.e. shiny) appearance.

Optionally, for the pH protective coating or layer 286 in any embodiment, the silicon dissolution rate by a 50 mM potassium phosphate buffer diluted in water for injection, adjusted to pH 8 with concentrated nitric acid, and containing 0.2 wt. % polysorbate-80 surfactant, (measured in the absence of the medicament, to avoid changing the dissolution reagent), at 40°C, is less than 170 ppb/day. (Polysorbate-80 is a common ingredient of pharmaceutical preparations, available for example as Tween®-80 from Uniqema Americas LLC, Wilmington Delaware.)

Optionally, for the pH protective coating or layer 286 in any embodiment, the silicon dissolution rate is less than 160 ppb/day, or less than 140 ppb/day, or less than 120 ppb/day, or less than 100 ppb/day, or less than 90 ppb/day, or less than 80 ppb/day. Optionally, in any embodiment of Figures 24-26 the silicon dissolution rate is more than 10 ppb/day, or more than 20 ppb/day, or more than 30 ppb/day, or more than 40 ppb/day, or more than 50 ppb/day, or more than 60 ppb/day. Any minimum rate stated here can be combined with any maximum rate stated here for the pH protective coating or layer 286 in any embodiment.

Optionally, for the pH protective coating or layer 286 in any embodiment the total silicon content of the pH protective coating or layer and barrier coating, upon dissolution into a test composition with a pH of 8 from the vessel, is less than 66 ppm, or less than 60 ppm, or less than 50 ppm, or less than 40 ppm, or less than 30 ppm, or less than 20 ppm.

The inventors offer the following theory of operation of the pH protective coating or layer described here. The invention is not limited by the accuracy of this theory or to the embodiments predictable by use of this theory.

The dissolution rate of the $SiO_x$ barrier layer is believed to be dependent on SiO bonding within the layer. Oxygen bonding sites (silanols) are believed to increase the dissolution rate.

It is believed that the pH protective coating or layer bonds with the silanol sites on the $SiO_x$ barrier layer to "heal" or passivate the $SiO_x$ surface and thus dramatically reduces the dissolution rate. In this hypothesis, the thickness of the pH protective layer is not the primary means of protection - the primary means is passivation of the $SiO_x$ surface. It is contemplated in any embodiment that a pH protective coating or layer as described in this specification can be improved by increasing the crosslink density of the pH protective coating or layer.

**Lubricity Layer**

A "lubricity layer" according to the present invention is a coating which has a lower frictional resistance than the uncoated surface. In other words, it reduces the frictional resistance of the coated surface in comparison to a reference surface which is uncoated. The present lubricity layers are primarily defined by their lower frictional resistance than the uncoated surface and the process conditions providing lower frictional resistance than the uncoated surface, and optionally can have a composition according to the empirical composition $Si_wO_xC_yH_z$, as defined in the Definition Section. "Frictional resistance" can be static frictional resistance and/or kinetic frictional resistance. One of the optional embodiments of the present invention is a syringe part, e.g. a syringe barrel or plunger, coated with a lubricity layer. In this contemplated embodiment, the relevant static frictional resistance in the context of the present invention is the breakout force as defined herein, and the relevant kinetic frictional resistance in the context of the present invention is the plunger sliding force as defined herein. For example, the plunger sliding force as defined and determined herein is suitable to determine the presence or absence and the lubricity characteristics of a lubricity layer in the context of the present invention whenever the coating is applied to any syringe or syringe part, for example to the inner wall of a syringe barrel. The breakout force is of particular relevance for evaluation of the coating effect on a prefilled syringe, i.e. a syringe which is filled after coating and can be stored for some time, e.g. several months or even years, before the plunger is moved again (has to be "broken out").

The "plunger sliding force" in the context of the present invention is the force required to maintain movement of a plunger in a syringe barrel, e.g. during aspiration or dispense. It can advantageously be determined using the ISO 7886-1:1993 test described herein and known in the art. A synonym for "plunger sliding force" often used in the art is "plunger force" or "pushing force".

The "breakout force" in the context of the present invention is the initial force required to move the plunger in a syringe, for example in a prefilled syringe.

Both "plunger sliding force" and "breakout force" and methods for their measurement are described in more detail in subsequent parts of this description.

"Slidably" means that the plunger is permitted to slide in a syringe barrel.

The plunger sliding force test is a specialized test of the coefficient of sliding friction of the plunger within a syringe, accounting for the fact that the normal force associated with a coefficient of sliding friction as usually measured on a flat surface is addressed by standardizing the fit between the plunger or other sliding element and the tube or other vessel within which it slides. The parallel force associated with a coefficient of sliding friction as usually measured is comparable to the plunger sliding force measured as described in this specification. Plunger sliding force can be measured, for example, as provided in the ISO 7886-1:1993 test.

The plunger sliding force test can also be adapted to measure other types of frictional resistance, for example the friction retaining a stopper within a tube, by suitable variations on the apparatus and procedure. In one embodiment, the plunger can be replaced by a closure and the withdrawing force to remove or insert the closure can be measured as the counterpart of plunger sliding force.

Also or instead of the plunger sliding force, the breakout force can be measured. The breakout force is the force required to start a stationary plunger moving within a syringe barrel, or the comparable force required to unseat a seated, stationary closure and begin its movement. The breakout force is measured by applying a force to the plunger that starts at zero or a low value and increases until the plunger begins moving. The breakout force tends to increase with storage of a syringe, after the prefilled syringe plunger has pushed away the intervening lubricant or adhered to the barrel due to decomposition of the lubricant between the plunger and the barrel. The breakout force is the force needed to overcome "sticktion," an industry term for the adhesion between the plunger and barrel that needs to be overcome to break out the plunger and allow it to begin moving.

Some utilities of coating a vessel in whole or in part with a lubricity layer, such as selectively at surfaces contacted in sliding relation to other parts, is to ease the insertion or removal of a stopper or passage of a sliding element such as a plunger in a syringe or a stopper in a sample tube. The vessel can be made of glass or a polymer material such as polyester, for example polyethylene terephthalate (PET), a cyclic olefin copolymer (COC), an olefin such as polypropylene, or other

materials. Applying a lubricity layer by PECVD can avoid or reduce the need to coat the vessel wall or closure with a sprayed, dipped, or otherwise applied organosilicon or other lubricant that commonly is applied in a far larger quantity than would be deposited by a PECVD process.

The power (in Watts) used for PECVD also has an influence on the coating properties. Typically, an increase of the power will increase the barrier properties of the coating, and a decrease of the power will increase the lubricity of the coating. E.g., for a coating on the inner wall of syringe barrel having a volume of about 3 ml, a power of less than 30 W will lead to a coating which is predominantly a barrier layer, while a power of more than 30 W will lead to a coating which is predominantly a lubricity layer.

A further parameter determining the coating properties is the ratio of $O_2$ (or another oxidizing agent) to the precursor (e.g. organosilicon precursor) in the gaseous reactant used for generating the plasma. Typically, an increase of the $O_2$ ratio in the gaseous reactant will increase the barrier properties of the coating, and a decrease of the $O_2$ ratio will increase the lubricity of the coating.

If a lubricity layer is desired, then $O_2$ is optionally present in a volume-volume ratio to the gaseous reactant of from 0:1 to 5:1, optionally from 0:1 to 1:1, even optionally from 0:1 to 0.5:1 or even from 0:1 to 0.1:1. Most advantageously, essentially no oxygen is present in the gaseous reactant. Thus, the gaseous reactant will in some embodiments comprise less than 1 vol % $O_2$, for example less than 0.5 vol % $O_2$, and optionally is $O_2$-free.

A process is contemplated for applying a lubricity layer characterized as defined in the Definition Section on a substrate, for example the interior of the barrel of a syringe, comprising applying one of the described precursors on or in the vicinity of a substrate at a thickness of 1 to 5000 nm, optionally 10 to 1000 nm, optionally 10-200 nm, optionally 20 to 100 nm thick and crosslinking or polymerizing (or both) the coating, optionally in a PECVD process, to provide a lubricated surface.

A coating of $Si_wO_xC_y$ as defined in the Definition Section optionally can be very thin, having a thickness of at least 4 nm, or at least 7 nm, or at least 10 nm, or at least 20 nm, or at least 30 nm, or at least 40 nm, or at least 50 nm, or at least 100 nm, or at least 150 nm, or at least 200 nm, or at least 300 nm, or at least 400 nm, or at least 500 nm, or at least 600 nm, or at least 700 nm, or at least 800 nm, or at least 900 nm. The coating can be up to 1000 nm, or at most 900 nm, or at most 800 nm, or at most 700 nm, or at most 600 nm, or at most 500 nm, or at most 400 nm, or at most 300 nm, or at most 200 nm, or at most 100 nm, or at most 90 nm, or at most 80 nm, or at most 70 nm, or at most 60 nm, or at most 50 nm, or at most 40 nm, or at most 30 nm, or at most 20 nm, or at most 10 nm, or at most 5 nm thick. Specific thickness ranges composed of any one of the minimum thicknesses expressed above, plus any equal or greater one of the maximum thicknesses expressed above, are expressly contemplated.

A lubricity layer, characterized as defined in the Definition Section, can be applied as a subsequent coating after applying any combination of layers described herein to the interior surface 88 of the vessel 80 to provide a lubricity layer.

Optionally, after the lubricity layer is applied, it can be post-cured after the PECVD process. Radiation curing approaches, including UV-initiated (free radial or cationic), electron-beam (E-beam), and thermal as described in Development Of Novel Cycloaliphatic Siloxanes For Thermal And UV-Curable Applications (Ruby Chakraborty Dissertation, can 2008) be utilized.

A lubricity layer, characterized as defined in the Definition Section, is particularly contemplated for the internal surface of a syringe barrel as further described below. A lubricated internal surface of a syringe barrel can reduce the plunger sliding force needed to advance a plunger in the barrel during operation of a syringe, or the breakout force to start a plunger moving after the prefilled syringe plunger has pushed away the intervening lubricant or adhered to the barrel, for example due to decomposition of the lubricant between the plunger and the barrel.

Thus, the coating 90 can comprise a barrier layer of SiOx or a trilayer and a lubricity layer, characterized as defined in the Definition Section. The lubricity layer of $Si_wO_xC_yH_z$ can be deposited between the layer of $SiO_x$ or the trilayer and the vessel lumen.

Another embodiment is a lubricity layer, characterized as defined in the Definition Section, on the inner wall of a syringe barrel. The coating is produced from a PECVD process using the following materials and conditions. A cyclic precursor is optionally employed, selected from a monocyclic siloxane, a polycyclic siloxane, or a combination of two or more of these, as defined elsewhere in this specification for lubricity layers. One example of a suitable cyclic precursor comprises octamethylcyclotetrasiloxane (OMCTS), optionally mixed with other precursor materials in any proportion. Optionally, the cyclic precursor consists essentially of octamethylcyclotetrasiloxane (OMCTS), meaning that other precursors can be present in amounts which do not change the basic and novel properties of the resulting lubricity layer, i.e. its reduction of the plunger sliding force or breakout force of the coated surface.

Optionally, at least essentially no oxygen is added to the process. In the context of the present invention, "essentially no oxygen" or (synonymously) "substantially no oxygen" is added to the gaseous reactant in some embodiments. This means that some residual atmospheric oxygen can be present in the reaction space, and residual oxygen fed in a previous step and not fully exhausted can be present in the reaction space, which are defined here as essentially no oxygen present. Essentially no oxygen is present in the gaseous reactant if the gaseous reactant comprises less than 1 vol % $O_2$, for example less than 0.5 vol % $O_2$, and optionally is $O_2$-free. If no oxygen is added to the gaseous reactant, or if no

oxygen at all is present during PECVD, this is also within the scope of "essentially no oxygen."

A sufficient plasma generation power input, for example any power level successfully used in one or more working examples of this specification or described in the specification, is provided to induce coating formation.

The materials and conditions employed are effective to reduce the syringe plunger sliding force or breakout force moving through the syringe barrel at least 25 percent, alternatively at least 45 percent, alternatively at least 60 percent, alternatively greater than 60 percent, relative to an uncoated syringe barrel. Ranges of plunger sliding force or breakout force reduction of from 20 to 95 percent, alternatively from 30 to 80 percent, alternatively from 40 to 75 percent, alternatively from 60 to 70 percent, are contemplated.

Another embodiment is a syringe including a plunger, a syringe barrel, and a lubricity layer, characterized as defined in the Definition Section. The syringe barrel includes an interior surface receiving the plunger for sliding. The lubricity layer is disposed on the interior surface of the syringe barrel. The lubricity layer is less than 1000 nm thick and effective to reduce the breakout force or the plunger sliding force necessary to move the plunger within the barrel. Reducing the plunger sliding force is alternatively expressed as reducing the coefficient of sliding friction of the plunger within the barrel or reducing the plunger force; these terms are regarded as having the same meaning in this specification.

Optionally an FTIR absorbance spectrum of the lubricity coating or layer of any embodiment has a ratio of at most 0.9 between the maximum amplitude of the Si- O-Si symmetrical stretch peak normally located between about 1000 and 1040 cm-1, and the maximum amplitude of the Si-O-Si assymmetric stretch peak normally located between about 1060 and about 1100 cm-1. Alternatively in any embodiment, this ratio can be at most 0.85, or at most 0.8, or at most 0.75, or less than 0.75.

Optionally, in any embodiment the lubricity coating or layer, in the absence of the medicament, may have an oily (i.e. shiny) appearance. This appearance has been observed in some instances to distinguish a lubricity coating or layer from a pH protective coating or layer.

### Hydrophobic Layer

The protective or lubricity coating or layer of $Si_wO_xC_y$ or its equivalent $SiO_xC_y$ also can have utility as a hydrophobic layer, independent of whether it also functions as a pH protective coating or layer Suitable hydrophobic coatings or layers and their application, properties, and use are described in U.S. Patent No. 7,985,188. Dual functional protective / hydrophobic coatings or layers having the properties of both types of coatings or layers can be provided for any embodiment of the present invention.

An embodiment can be carried out under conditions effective to form a hydrophobic pH protective coating or layer on the substrate. Optionally, the hydrophobic characteristics of the pH protective coating or layer can be set by setting the ratio of the O2 to the organosilicon precursor in the gaseous reactant, and/or by setting the electric power used for generating the plasma. Optionally, the pH protective coating or layer can have a lower wetting tension than the uncoated surface, optionally a wetting tension of from 20 to 72 dyne/cm, optionally from 30 to 60 dynes/cm, optionally from 30 to 40 dynes/cm, optionally 34 dyne/cm. Optionally, the pH protective coating or layer can be more hydrophobic than the uncoated surface.

Use of a coating or layer according to any described embodiment is contemplated in any embodiment as (i) a lubricity coating having a lower frictional resistance than the uncoated surface; and/or (ii) a pH protective coating or layer preventing dissolution of the barrier coating in contact with a fluid, and/or (iii) a hydrophobic layer that is more hydrophobic than the uncoated surface.

### Atomic Layer Deposition Coating of Vessels

One or more of the layers described herein may be applied by atomic layer deposition coating. Coatings applied by atomic layer deposition are structurally (though not necessarily chemically) distinct from those applied by CVD or PECVD. In contrast to coatings applied by CVD or PECVD, coatings applied by atomic layer deposition consist of a plurality of monolayers of the deposited compound. Because each step deposite only a single monolayer, defects of the sort that can develop due to non-uniform growth during CVD or PECVD are avoided. The result is a coating having significantly higher density than that of a coating (of generally the same chemical composition) applied by CVD or PECVD. Because the coating consists of a plurality of monolayers of the deposited compound, the coating may also have a higher degree of compositional purity and consistency than coatings applied by PECVD.

In an atomic layer deposition process, sources, i.e., precursors, may be sequentially introduced in non-overlapping timeframes to deposit one material at a time. Once each possible adsorption site is occupied in a particular precursor flow, the precursor may be halted and a purge process may be completed before the next source material is introduced, with one timeframe for each precursor comprising one cycle. As the chamber is typically under a 1-20 mbar vacuum, the remaining precursor may be evacuated upon stopping flow. In this manner, the deposition process continues in a self-limited way in that there are only a finite number of sites on which the reactant can adsorb, so once they are filled,

the growth stops until the next precursor is introduced, where the total material thickness is controlled by the number of cycles. This process may continue for each precursor, resulting in a coating or layer being deposited one atomic layer at a time. Accordingly, ALD is capable of growing very thin conformal films with excellent thickness uniformity and control, as well as increased density compared to other deposition techniques. Furthermore, precise composition control is enabled by the ALD process.

A plasma may be optionally utilized to enhance the material deposition, i.e., plasma enhanced atomic layer deposition (PEALD), also sometimes referred to as plasma-assisted atomic layer deposition, where the precursor dissociation may be increased using a plasma, enabling a lower growth temperature, which may be useful when applying coatings to certain thermoplastics.

ALD is useful for depositing high-density layers with low defect density. In an example, a thin SiOx film may be deposited by thermal and/or plasma enhanced ALD. The deposition temperature may be in the range of 30°C to 120°C. For instance, where thermal ALD is used, the deposition temperature may be in the range of 70-120°C, desirably 100 °C or less, desirably 80 °C or less. Where PEALD is used, the temperature may be at least 30°C, e.g. between 30 °C and 80 °C or between 30 °C and 60 °C, desirably 80 °C or less, desirably 60 °C or less.

Precursors for the deposition of an SiOx (e.g. $SiO_2$) film by ALD or PEALD include one or more silicon-containing precursor and one or more oxygen precursors. The silicon precursors may include, for example, aminosilanes; alkylaminosilanes, such as tetradimethyl-aminosilicon; 1,2-bis(diisopropylamino)disilane; diisopropylaminosilane; tris(dimethylamino)silane; bis(ethylmethyl-amino)silane; alkylaminosilylamines (e.g. ORTHRUS® sold by AIR LIQUIDE); Hexakis(ethylamino)disilane $Si_2(NHEt)_6$ (AHEAD); $SiCl_4$ (Silicon tetrachloride); $SiCl_4$ (Silicon tetrachloride) / Pyridine; Alkylchlorosilane; tetraethoxysilane (TEOS); 1,2-Bis(diisopropylamino)disilane (BDIPADS); AP-LTO®330; bis(diethylamino)silane, (BDEAS); diisopropylaminosilane (DIPAS); tris(dimethylamino)silane (TDMAS); 3-aminopropyltriethoxysilane (APTES); bis(ethylmethylamino)silane (BEMAS); Bis-dimethylaminosilane (BDMAS); bis(ethylmethylamino)silane; di(sec-butylamino)silane (DSBAS); and combinations thereof. ozone ($O_3$), $O_2$, a mixture of $O_3$ and $O_2$, $H_2O$, or a combination thereof may be used as an oxygen precursor.. In some embodiments, a catalyser such as $NH_3$, trimethylamine, or pyridine may also be provided.

Further, the silicon precursor (or precursors) may be pulsed to control the growth rate.

In another example, a thin aluminum oxide film may be deposited by ALD or plasma enhanced ALD. The deposition may be carried out at a temperature in the range of 25°C to 120°C. In some embodiments, the temperature may be at least 30°C, e.g. between 30 °C and 80 °C or between 30 °C and 60 °C, desirably 100 °C or less, desirably 80 °C or less, desirably 60 °C or less. Precursors for the deposition of an aluminum oxide film include one or more aluminum-containing precursors and one or more oxygen precursors. The aluminum-containing precursor may comprise or consist of, for example, trimethylaluminum (TMA). The oxygen precursor may comprise ozone ($O_3$), $O_2$, a mixture of $O_3$ and $O_2$, $H_2O$, or a combination thereof. An example schematic of a process for the deposition of an aluminum oxide coating is shown in Figure 26. The schematic of Figure 26 also illustrates that the coating formed by ALD (or PEALD) is made up of a plurality of monolayers of the deposited compound, in this case monolayers of aluminum oxide.

In another example, a zirconium oxide (ZrO2) film may be deposited by ALD or plasma-enhanced ALD. The deposition may be carried out at a temperature in the range of 25°C to 120°C. In some embodiments, the temperature may be at least 30°C, e.g. between 30 °C and 80 °C or between 30 °C and 60 °C, desirably 100 °C or less, desirably 80 °C or less, desirably 60 °C or less. Precursors for the deposition of a zirconium oxide film include one or more zirconium-containing precursors and one or more oxygen precursors. The zirconium-containing precursor may comprise or consist of, for example, tetrakis(ethylmethylamino)zirconium (TEMAZ). The oxygen precursor may comprise ozone ($O_3$), $O_2$, a mixture of $O_3$ and $O_2$, $H_2O$, or a combination thereof.

In another example, ALD and/or PEALD may be utilized to deposit other barrier layer materials such as silicon nitrides, silicon carbides, and aluminum oxides, or other such materials which may improve the gas barrier and/or material dissociation capabilities. Due to the slow and controlled growth rate of ALD, which may result in increased material adhesion, tie layers may not be needed.

The coating of pharmaceutical vessels such as syringes, vials, and the like, gives rise to a variety of issues. For instance, syringes and vials typically have curved and otherwise non-flat surfaces. Further, it is generally desirable to apply the coating only on a single surface of the vessel, e.g. on the interior surface of the wall (adjacent to the lumen) or on the outer surface of the wall. Moreover, syringes typically have a high aspect ratio, e.g. up to 1/20, which can complicate the atomic layer deposition process, particularly when the coating is to be applied to the interior surface of the wall (adjacent to the lumen).

To account for these issues, the atomic layer deposition process must be carefully controlled with respect to at least (a) the residence time of gas during the deposition, including the potential use of longer than conventional deposition times, and (b) the gas flow inside the reaction chamber to ensure gas is going through the high aspect ratio parts and reacting evenly along the surface area of the interior surface of the wall that defines a small diameter lumen.

Further, because disposable pharmaceutical vessels such as plastic syringes, vials, and the like must both be manufacturable in large quantities and highly consistent from unit to unit, it is important that the oxygen barrier coating

and/or the water vapor barrier coating may be applied to a number of vessels simultaneously, i.e. during a single coating process in a reactor, and with a high degree of consistency, i.e. that the thicknesses of the coatings applied to the vessels within the reactor has a high degree of consistency (that the coating thickness has a low standard deviation).

Accordingly, a plurality of vessels, e.g. at least 20 vessels, alternatively at least 50 vessels, alternatively at least 100 vessels, alternatively at least 200 vessels, may be placed and arranged in a reactor and the ALD or PEALD process may be carried out such that a substantially uniform flow of the precursor gases to each of the vessels is achieved. As a result, layers of the coating may build-up substantially uniformly across each of the plurality of vessels within the reactor. Examples of reactors that can be used for this process include the PICOSUN™ P-1000 line of reactors, such as the PICOSUN™ P-1000B PRO. To coat a large number of vessels simultaneously, the vessels may be arranged in a multi-level rack positioned within the reactor.

**Water Vapor Barrier**

In embodiments of the present disclosure, a vessel made from a thermoplastic material/resin may be coated with a water vapor barrier coating or layer in order to provide a package, e.g. a drug primary package, a vial, a syringe, or an evacuated blood tube, having a water vapor transmission rate that is lower than an identical package in which the vessel is made from the same thermoplastic material/resin but which lacks the water vapor barrier coating or layer, optionally at least 5% lower, optionally at least 10% lower, optionally at least 20% lower, optionally at least 30% lower, optionally at least 40% lower, optionally at least 50% lower, optionally at least 60% lower, optionally at least 70% lower, optionally at least 80% lower, optionally at least 90% lower, optionally as determined at 40.0 °C and 75.0% RH using the Water Vapor Transmission Rate Protocol described herein. The vessels may optionally include one or more additional coatings, such as an oxygen barrier coating or layer, a tie coating or layer, a pH protective coating or layer, a lubricity coating or layer, or any combination thereof.

In embodiments of the present disclosure, a vessel made from a thermoplastic material/resin may be coated with a water vapour barrier coating or layer that, in combination with an oxygen barrier coating or layer as described herein, provides a package, e.g. a drug primary package such as a vial or pre-filled syringe or an evacuated blood tube, having a shelf life of at least 3 months, alternatively at least 6 months, alternatively at least 9 months, alternatively at least 1 year, alternatively at least 1.5 years, alternatively at least 2 years, alternatively at least 2.5 years, alternatively at least 3 years. In some embodiments, the drug primary package may be a vial containing a lyophilized drug product.

In some embodiments of the present disclosure, a vessel made from a commodity resin may be coated with a water vapour barrier coating or layer so as to produce a reduced water vapour transmission rate as described above. As described above, the specialty COP and COC resins that are used to produce pharmaceutical vessels such as vials and syringes have been selected in large part due to the low water vapor transmission rate of the COP and COC vessel walls. By embodiments of the present invention, it has been found that close to an equivalent, an equivalent, or a better, i.e. lower, water vapor transmission rate may be obtained using a lower-cost and more readily available commodity resin, which may itself (i.e., the vessel without any additional coating) have a water vapor transmission rate that is at least double that of COP, alternatively at least three times that of COP, alternatively at least four times that of COP, alternatively at least 5 times that of COP.

In some embodiments, for example, the vessel prepared from a commodity resin and coated with the water vapour barrier coating or layer may have a water vapour transmission rate that is within a commercially feasible range for one or more drug products, i.e. a range sufficient to provide the finished drug primary package with a commercially suitable shelf life. In some embodiments, for example, a vial such as a 10 mL thermoplastic vial may be coated with a barrier coating or layer as described herein and the vial (with its associated stopper) may have a WVTR less than 2.0 mg/package/day, alternatively less than 1.5 mg/package/day, alternatively less than 1.0 mg/package/day, alternatively less than 0.9 mg/package/day, alternatively less than 0.8 mg/package/day, alternatively less than 0.7 mg/package/day, alternatively less than 0.6 mg/package/day, alternatively less than 0.5 mg/package/day, alternatively less than 0.4 mg/package/day, alternatively less than 0.3 mg/package/day, , e.g. as determined at 40.0 °C and 75.0% RH using the Water Vapor Transmission Rate Protocol described herein. In some embodiments, the thermoplastic vial may comprise a polycarbonate vessel wall. In other embodiments, the thermoplastic vial may comprise a vessel wall made from a cyclic block copolymer (CBC) as described herein.

In some embodiments, the vessel coated with the water vapor barrier coating or layer may have a water vapor transmission rate that is at least equivalent to the water vapor transmission rate of an identical vessel made from COP resin and lacking the water vapor barrier coating or layer, optionally a water vapor transmission rate that is lower than the water vapor transmission rate of an identical vessel made from COP resin and lacking the water vapor barrier coating or layer, optionally at least 5% lower, optionally at least 10% lower, optionally at least 20% lower, optionally at least 30% lower, optionally at least 40% lower, optionally at least 50% lower, optionally at least 60% lower, optionally at least 70% lower, optionally at least 80% lower, optionally at least 90% lower.

In some embodiments, for example, an evacuated blood tube such as a 9mL blood tube having a vessel wall

made from a commodity resin may be coated with a barrier coating or layer as described herein and the vial (with its associated stopper) may have a WVTR less than 0.5 mg/package/day, alternatively less than 0.4 mg/package/day, alternatively less than 0.3 mg/package/day, alternatively less than 0.2 mg/package/day, alternatively 0.1 mg/package/day or less, e.g. as determined at 40.0 °C and 75.0% RH using the Water Vapor Transmission Rate Protocol described herein. For reference, an uncoated 9mL blood tube made from COP may have a WVTR of about 0.1 mg/package/day. In some embodiments, the blood tube may comprise a vessel wall made from a cyclic block copolymer (CBC) as described herein.

Further, by applying a water vapor barrier layer such as aluminum oxide to a vessel, e.g. a syringe or vial or blood tube, having its wall made from a commodity resin, it has presently been found that the water vapor transmission rate (WVTR) may be less than 0.050 mg/vessel/day, alternatively less than 0.040 mg/vessel/day, alternatively less than 0.030 mg/vessel/day, alternatively less than 0.020 mg/vessel/day, alternatively less than 0.010 mg/vessel/day, at 60 °C and 40% relative humidity. In contrast, the water vapor transmission rate of the same vessel without the water vapor barrier layer may be greater than 1.0 g/container/day, optionally greater than 2.0 g/container/day, optionally greater than 3.0 g/container/day.

In other embodiments, a vessel made from COP or COC resin may be coated with a water vapor barrier coating or layer in order to provide a water vapor transmission rate that is lower than an identical vessel made from COP or COC resin and lacking the water vapor barrier coating or layer, optionally at least 5% lower, optionally at least 10% lower, optionally at least 20% lower, optionally at least 30% lower, optionally at least 40% lower, optionally at least 50% lower, optionally at least 60% lower, optionally at least 70% lower, optionally at least 80% lower, optionally at least 90% lower. The vessels may optionally include one or more additional coatings, such as an oxygen barrier coating or layer, a tie coating or layer, a pH protective coating or layer, a lubricity coating or layer, or any combination thereof.

In some embodiments, for instance, a vial such as a 10 mL COP vial may be coated with a barrier coating or layer as described herein and the vial (with its associated stopper) may have a WVTR less than 0.25 mg/package/day, alternatively less than 0.22 mg/package/day, alternatively less than 0.22 mg/package/day, alternatively less than 0.20 mg/package/day, alternatively 0.18 mg/package/day or less, alternatively less than 0.16 mg/package/day or less, alternatively 0.15 mg/package/day or less, alternatively 0.13 mg/package/day or less, alternatively 0.12 mg/package/day or less, e.g. as determined at 40.0 °C and 75.0% RH using the Water Vapor Transmission Rate Protocol described herein. For reference, an uncoated 10 mL vial made from COP may have a WVTR of about 0.25 mg/package/day at 40.0 °C and 75.0% RH.

In some embodiments, for instance, a vial such as a 10 mL COC vial may be coated with a barrier coating or layer as described herein and the vial (with its associated stopper) may have a WVTR less than 0.25 mg/package/day, alternatively less than 0.22 mg/package/day, alternatively less than 0.22 mg/package/day, alternatively less than 0.20 mg/package/day, alternatively 0.18 mg/package/day or less, alternatively less than 0.16 mg/package/day or less, alternatively 0.15 mg/package/day or less, alternatively 0.13 mg/package/day or less, alternatively 0.12 mg/package/day or less, e.g. as determined at 40.0 °C and 75.0% RH using the Water Vapor Transmission Rate Protocol described herein.

In some embodiments, for example, an evacuated blood tube such as a 9mL blood tube with a vessel wall made from COP, may be coated with a barrier coating or layer as described herein and the vial (with its associated stopper) may have a WVTR less than 0.1 mg/package/day, alternatively less than 0.09 mg/package/day, alternatively less than 0.08 mg/package/day, alternatively less than 0.07 mg/package/day, alternatively 0.06 mg/package/day or less, e.g. as determined at 40.0 °C and 75.0% RH using the Water Vapor Transmission Rate Protocol described herein. For reference, an uncoated 9mL blood tube made from COP may have a WVTR of about 0.1 mg/package/day.

The water vapor transmission rate of the vessels may be determined using a variety of test procedures. In some embodiments, the moisture content of a lyophilized composition stored within the lumen of the (sealed) vessel may be measured at various points in time in order to determine the rate at which the moisture content of the lyophilized composition increases over a defined period of time. For instance, the moisture content of the lyophilized composition may be measured for samples over a number of sequential days, e.g. for at least one day, for at least two days, for at least three days, for at least four days, for at least five days, for at least six days, etc., as needed to have a representative amount of data. The moisture vapor transmission rate may be stated in terms of mg/vessel/day.

The conditions used in that testing, i.e. the conditions under which the vessels are stored, may vary. In some embodiments, the vessels may be stored at 60 °C and 40% relative humidity. In some embodiments, the vessels may be stored at 40 °C and 75% relative humidity. In some embodiments, the vessels may be stored at room temperature (20 - 22 °C) and 75% relative humidity. In some embodiments, the vessels may be stored under refrigeration, e.g. at 3 - 8 °C and 75% relative humidity.

In some embodiments, the measurements may be performed in accordance with USP <921>. In particular, USP <921> describes Method 1a, which is the titrimetric determination of water based upon the quantitative reaction of water with an anhydrous solution of sulfur dioxide and iodine in the presence of a buffer that reacts with hydrogen ions. This method is also known as Karl Fischer titration. Any of a variety of Karl Fischer titration systems may be used to carry out this process, including for example, those produced by METTLER TOLEDO under the Volumetric Compact Karl Fischer Titrators line, Compact Coulometric Karl Fischer Titrator line, or Titration Excellence line.

In other embodiments, the measurements may be performed using the Computrac® Vapor Pro® system or a similar system that operates on the same principles. The Computrac® Vapor Pro® system uses a thermoset polymer capacitance relative humidity sensor to detect changes in the relative humidity of a temperature controlled sensor chamber caused by thermal evolution of sample moisture. Using this system, the sample, which can be kept in the vial (where the vessel is a vial), is heated in a sealed temperature controlled oven. The thermally evolved gasses are transported by a dry inert gas stream to a temperature controlled sensor chamber, which houses the relative humidity sensor. This system can provide an accurate and precise moisture analysis of samples within lyophilization vials, while limiting exposure of the sample to atmospheric moisture.

In other embodiments, the measurements may be performed in accordance with USP <731**>.** In particular, USP <731> describes a procedure for determining the amount of volatile matter that is driven off of a sample under specified conditions. This procedure is known as a "loss on drying" test and is a thermogravimetric method in which the moisture content percentage is determined from the difference in weight before and after drying. Although USP <731> describes a method that utilizes a drying oven, the loss on drying test may be more efficiently performed using a halogen moisture analyzer in which a sample is heated through absorption of IR radiation from a halogen radiator and the mass is monitored continually during the drying process. The loss on drying test can be performed using any of a variety of halogen moisture analyzer devices, including for example, those produced by METTLER TOLEDO. Note that unlike the two methods described above, the "loss on drying" method is not specific to water content, meaning that the presence of other volatiles in the sample may affect the accuracy of the results.

The water vapor barrier coating or layer may be applied to the outer surface of the vessel and/or to the inner surface of the vessel. In some embodiments, the water vapor barrier coating or layer may even be applied as an intermediate step in the preparation of the vessel itself, in which case the water vapor barrier coating or layer may be nested between layers of resin and positioned between the inner and outer surfaces of the vessel wall. In some embodiments, the thickness of the water vapor coating or layer, e.g. an aluminum oxide coating or layer applied by ALD or PEALD, may be between 5 and 50 nm, alternatively between 5 and 40 nm, alternatively between 5 and 30 nm, alternatively between 5 and 20 nm, alternatively between 10 and 50 nm, alternatively between 10 and 40 nm, alternatively between 10 and 30 nm, alternatively between 10 and 20 nm.

The water vapor barrier coating or layer of the present disclosure may also have a number of additional advantages when it comes to pharmaceutical packages such as syringes, vials, and the like. Namely, in contrast to many known moisture barrier materials, the present water vapor barrier coating may be inorganic. The composition of the present water vapor barrier coating may also be tightly controlled, yielding a coating that consists of a single compound and which is free from impurities and/or other potentially undesirable elements, compounds, and the like. Thus, for instance, there are no organics, impurities, or other undesirable elements to be taken up by a liquid drug formulation that comes into contact with the coating.

By providing a plastic pharmaceutical package, such as a vial or syringe, with a suitable water vapor barrier layer, the present inventors may avoid the use of expensive specialty resins, such as COP and COC. It is believed that embodiments of the present invention enable the preparation and use of vessels made from commodity plastics, which themselves may have poor water vapor transmission properties. In other embodiments, the present inventors may provide a plastic pharmaceutical package, such as a vial or syringe, in which the vessel wall is made from COP or COC and in which a water vapour barrier layer provides the package with improved water vapour transmission properties. In some embodiments, for instance, a vessel such as a vial, syringe, or blood tube may be provided in which the vessel wall is made from COP or COC and in which a water vapour barrier layer provides the package with a water vapour transmission rate that is equivalent or substantially equivalent to those of a the same vessel having its (uncoated) wall made from glass.

Further, because blood tubes are maintained in an evacuated state prior to use, it is desirable to prevent the ingress of environmental gases, including water vapor, through the wall of the blood tube and into the evacuated lumen. It has presently been found that the shelf life of an evacuated blood tube can be significantly improved by applying a water vapor barrier layer to the blood tube, as it prevents the ingress of environmental water vapor, and thereby provides a longer lasting vacuum within the lumen of the blood tube.

The application of a water vapor barrier layer to a blood tube may also improve the shelf life of an evacuated blood tube by preventing, or reducing, solvent loss from a blood preservative contained within the lumen of the blood tube.

## Nitrogen Barrier Coating or Layer

Because many biologic drugs can be prone to oxidation, the headspace of a drug primary package, such as a vial or syringe, containing a biologic drug may be purged with an inert gas during filling. The result is a sealed (e.g. with a stopper or plunger) drug primary package in which the lumen of the vessel contains not only the drug product, but also a headspace that consists essentially of the inert gas as a blanketing gas. Over time, however, the inert gas in the headspace of the sealed package may effuse through the wall of the vessel, leaving the headspace with a reduced inert gas content. Among the more common inert gases that may be used are nitrogen and argon.

Further, because blood tubes are maintained in an evacuated state prior to use, it is desirable to prevent the ingress of environmental gases, including nitrogen, through the wall of the blood tube and into the evacuated lumen.

Embodiments of the present disclosure are directed to a vessel having a barrier coating or layer configured to prevent an inert gas, such as nitrogen or argon, from effusing through the vessel wall. In embodiments of the present disclosure, therefore, the gas barrier coating or layer may comprise one or more nitrogen barrier coatings or layers, the nitrogen barrier coatings or layers being effective to reduce the ingress of nitrogen into the lumen or, as described above, reduce the egress of nitrogen out of the lumen.

The nitrogen barrier coating or layer optionally can be deposited by atomic layer deposition (ALD), plasma enhanced chemical vapor deposition (PECVD) or other chemical vapor deposition processes on the vessel of a pharmaceutical package. Desirably, the nitrogen barrier coating or layer may be deposited by atomic layer deposition (ALD).

The nitrogen barrier coating or layer optionally comprises an SiOx coating, and contains silicon, oxygen, and optionally other elements, in which x, the ratio of oxygen to silicon atoms, is from about 1.5 to about 2.9, or 1.5 to about 2.6, or about 2. The nitrogen barrier coating or layer optionally comprises a metal oxide coating, e.g. aluminum oxide.

The nitrogen barrier coating or layer may be applied to the outer surface of the vessel and/or to the inner surface of the vessel. In some embodiments, the nitrogen barrier coating or layer may even be applied as an intermediate step in the preparation of the vessel itself, in which case the nitrogen barrier coating or layer may be nested between layers of resin and positioned between the inner and outer surfaces of the vessel wall.

In embodiments of the present disclosure, a vessel made from a thermoplastic material/resin may be coated with a nitrogen barrier coating or layer in order to provide a package, e.g. a drug primary package, a vial, a syringe, or an evacuated blood tube, having a nitrogen transmission rate that is lower than an identical package in which the vessel is made from the same thermoplastic material/resin but which lacks the nitrogen barrier coating or layer, optionally at least 5% lower, optionally at least 10% lower, optionally at least 20% lower, optionally at least 30% lower, optionally at least 40% lower, optionally at least 50% lower, optionally at least 60% lower, optionally at least 70% lower, optionally at least 80% lower, optionally at least 90% lower. The vessels may optionally include one or more additional coatings, such as an oxygen barrier coating or layer, a water vapour barrier coating or layer, a tie coating or layer, a pH protective coating or layer, a lubricity coating or layer, or any combination thereof. In some embodiments, the vessel wall may consist essentially of a COP or COC resin while in other embodiments the vessel wall may consist essentially of a commodity resin, e.g. a CBC resin.

By applying a nitrogen barrier layer such to a vessel, e.g. a syringe or vial, it has presently been found that the nitrogen transmission rate (NTR) may be less than 0.0003 $d^{-1}$; optionally less than 0.0002 $d^{-1}$; optionally less than 0.0001 $d^{-1}$, optionally less than 0.00008 $d^{-1}$; optionally less than 0.00006 $d^{-1}$; optionally less than 0.00004 $d^{-1}$, optionally less than 0.00003 $d^{-1}$; optionally less than 0.00002 $d^{-1}$; optionally less than 0.00001 $d^{-1}$.

The nitrogen barrier coating or layer may be effective to reduce the ingress of nitrogen into the lumen of a drug primary package or the egress of nitrogen out of the lumen of the drug primary package to less than 0.0002 cc/package/day at 25 °C, 60% relative humidity and 0.21 bar, optionally less than 0.00015 cc/package/day at 25 °C, 60% relative humidity and 0.21 bar, optionally less than 0.0001 cc/package/day at 25 °C, 60% relative humidity and 0.21 bar, optionally less than 0.00005 cc/package/day at 25 °C, 60% relative humidity and 0.21 bar, optionally less than 0.00002 cc/package/day at 25 °C, 60% relative humidity and 0.21 bar, optionally less than 0.00001 cc/package/day at 25 °C, 60% relative humidity and 0.21 bar. Accordingly, embodiments of vials comprising a nitrogen barrier layer may allow for an ingress of nitrogen into the lumen or an egress of nitrogen out of the lumen of less than 0.0002 cc/package/day at 25 °C, 60% relative humidity and 0.21 bar, optionally less than 0.00015 cc/package/day at 25 °C, 60% relative humidity and 0.21 bar, optionally less than 0.0001 cc/package/day at 25 °C, 60% relative humidity and 0.21 bar, optionally less than 0.00005 cc/package/day at 25 °C, 60% relative humidity and 0.21 bar, optionally less than 0.00002 cc/package/day at 25 °C, 60% relative humidity and 0.21 bar, optionally less than 0.00001 cc/package/day at 25 °C, 60% relative humidity and 0.21 bar.

By applying a nitrogen barrier layer to a vessel, e.g. a syringe or vial, it has presently been found that the nitrogen blanketing gas located in the headspace of the vessel can be maintained for a longer period of time, thereby improving the shelf life of the drug primary package.

It has also presently been found that the shelf life of an evacuated blood tube can be significantly improved by applying a nitrogen barrier layer to the blood tube, as it prevents the ingress of environmental nitrogen, and thereby providing a longer lasting vacuum within the lumen of the blood tube.

**Carbon Monoxide Barrier Coating or Layer**

Low doses of carbon monoxide of are finding increased interest as a therapeutic agent. Low dose carbon monoxide has been shown to exhibit therapeutic properties, including antiinflammatory and anti-apoptotic properties in sickle cell disease, kidney transplant, and Parkinson's disease. Similarly, CO has been demonstrated to act as an effective antiinflammatory agent in preclinical animal models of inflammation, acute lung injury, sepsis, ischemia/reperfusion injury,

and organ transplantation. Additional experimental indications for this gas include pulmonary fibrosis, pulmonary hypertension, metabolic diseases, and preeclampsia. Low doses of carbon monoxide can be provided orally, intravenously, or as an inhalation agent. Because CO is a gas, inhalation is the natural consideration for administration. Gas inhalation, however, has a variety of issues. Consequently, formulations that allow for CO delivery via oral, intravenous, intraperitoneal, subcutaneous, or other routes are under investigation and development. As with other drug products, these formulations must be stored in vessels, e.g. vials, pre-filled syringes, and the like. Over time, the carbon monoxide in the sealed packages may effuse through the wall of the vessel, leaving the drug product with reduced carbon monoxide content.

Embodiments of the present disclosure are directed to a vessel having a barrier coating or layer configured to prevent carbon monoxide from effusing through the vessel wall. In embodiments of the present disclosure, therefore, the gas barrier coating or layer may comprise one or more carbon monoxide barrier coatings or layers, the carbon monoxide coatings or layers being effective to reduce the ingress of carbon monoxide into the lumen or, more typically as described above, reduce the egress of carbon monoxide out of the lumen.

The carbon monoxide barrier coating or layer optionally can be deposited by atomic layer deposition (ALD), plasma enhanced chemical vapor deposition (PECVD) or other chemical vapor deposition processes on the vessel of a pharmaceutical package. Desirably, the carbon monoxide barrier coating or layer may be deposited by atomic layer deposition (ALD).

The carbon monoxide barrier coating or layer optionally comprises an SiOx coating, and contains silicon, oxygen, and optionally other elements, in which x, the ratio of oxygen to silicon atoms, is from about 1.5 to about 2.9, or 1.5 to about 2.6, or about 2. The carbon monoxide barrier coating or layer optionally comprises a metal oxide coating, e.g. aluminum oxide.

The carbon monoxide barrier coating or layer may be applied to the outer surface of the vessel and/or to the inner surface of the vessel. In some embodiments, the carbon monoxide barrier coating or layer may even be applied as an intermediate step in the preparation of the vessel itself, in which case the carbon monoxide barrier coating or layer may be nested between layers of resin and positioned between the inner and outer surfaces of the vessel wall.

In embodiments of the present disclosure, a vessel made from a thermoplastic material/resin may be coated with a carbon monoxide barrier coating or layer in order to provide a package, e.g. a drug primary package, a vial, a syringe, or an evacuated blood tube, having a carbon monoxide transmission rate that is lower than an identical package in which the vessel is made from the same thermoplastic material/resin but which lacks the carbon monoxide barrier coating or layer, optionally at least 5% lower, optionally at least 10% lower, optionally at least 20% lower, optionally at least 30% lower, optionally at least 40% lower, optionally at least 50% lower, optionally at least 60% lower, optionally at least 70% lower, optionally at least 80% lower, optionally at least 90% lower. The vessels may optionally include one or more additional coatings, such as an oxygen barrier coating or layer, a water vapour barrier coating or layer, a tie coating or layer, a pH protective coating or layer, a lubricity coating or layer, or any combination thereof. In some embodiments, the vessel wall may consist essentially of a COP or COC resin while in other embodiments the vessel wall may consist essentially of a commodity resin, e.g. a CBC resin.

By applying a carbon monoxide barrier layer such to a vessel, e.g. a syringe or vial, it has presently been found that the carbon monoxide transmission rate (COTR) may be less than 0.0003 $d^{-1}$; optionally less than 0.0002 $d^{-1}$; optionally less than 0.0001 $d^{-1}$, optionally less than 0.00008 $d^{-1}$; optionally less than 0.00006 $d^{-1}$; optionally less than 0.00004 $d^{-1}$, optionally less than 0.00003 $d^{-1}$; optionally less than 0.00002 $d^{-1}$; optionally less than 0.00001 $d^{-1}$.

The carbon monoxide barrier coating or layer may be effective to reduce the ingress of carbon monoxide into the lumen of a drug primary package or the egress of carbon monoxide out of the lumen of the drug primary package to less than 0.0002 cc/package/day at 25 °C, 60% relative humidity and 0.21 bar, optionally less than 0.00015 cc/package/day at 25 °C, 60% relative humidity and 0.21 bar, optionally less than 0.0001 cc/package/day at 25 °C, 60% relative humidity and 0.21 bar, optionally less than 0.00005 cc/package/day at 25 °C, 60% relative humidity and 0.21 bar, optionally less than 0.00002 cc/package/day at 25 °C, 60% relative humidity and 0.21 bar, optionally less than 0.00001 cc/package/day at 25 °C, 60% relative humidity and 0.21 bar. Accordingly, embodiments of vials comprising a carbon monoxide barrier layer may allow for an ingress of carbon monoxide into the lumen or an egress of carbon monoxide out of the lumen of less than 0.0002 cc/package/day at 25 °C, 60% relative humidity and 0.21 bar, optionally less than 0.00015 cc/package/day at 25 °C, 60% relative humidity and 0.21 bar, optionally less than 0.0001 cc/package/day at 25 °C, 60% relative humidity and 0.21 bar, optionally less than 0.00005 cc/package/day at 25 °C, 60% relative humidity and 0.21 bar, optionally less than 0.00002 cc/package/day at 25 °C, 60% relative humidity and 0.21 bar, optionally less than 0.00001 cc/package/day at 25 °C, 60% relative humidity and 0.21 bar.

By applying a carbon monoxide barrier layer to a vessel, e.g. a syringe or vial, it has presently been found that carbon monoxide located in the drug product and/or in the headspace of the vessel can be maintained for a longer period of time, thereby improving the shelf life of the drug primary package.

## Carbon Dioxide Barrier Coating or Layer

Some drug products comprise a liquid formulation in which the active agent (and excipients) are dissolved in a carbon dioxide-containing medium. This may be done, for example, to help stabilize the active agent or, in some instances, simply to create a carbonated, or fizzy, composition. Regardless, where carbon dioxide is present in the formulation, and the drug is packaged in a vessel (e.g. a vial or pre-filled syringe), it may be desirable to prevent the egress of $CO_2$ out of the drug product and through the vessel walls.

Further, because blood tubes are maintained in an evacuated state prior to use, it is desirable to prevent the ingress of environmental gases, including carbon dioxide, through the wall of the blood tube and into the evacuated lumen.

Embodiments of the present disclosure are therefore directed to a vessel having a barrier coating or layer configured to prevent carbon dioxide from effusing through the vessel wall. In embodiments of the present disclosure, therefore, the gas barrier coating or layer may comprise one or more carbon dioxide barrier coatings or layers, the carbon dioxide barrier coatings or layers being effective to reduce the ingress of carbon dioxide into the lumen or, as described above, reduce the egress of carbon dioxide out of the lumen.

The carbon dioxide barrier coating or layer optionally can be deposited by atomic layer deposition (ALD), plasma enhanced chemical vapor deposition (PECVD) or other chemical vapor deposition processes on the vessel of a pharmaceutical package. Desirably, the carbon dioxide barrier coating or layer may be deposited by atomic layer deposition (ALD).

The carbon dioxide barrier coating or layer optionally comprises an SiOx coating, and contains silicon, oxygen, and optionally other elements, in which x, the ratio of oxygen to silicon atoms, is from about 1.5 to about 2.9, or 1.5 to about 2.6, or about 2. The carbon dioxide barrier coating or layer optionally comprises a metal oxide coating, e.g. aluminum oxide.

The carbon dioxide barrier coating or layer may be applied to the outer surface of the vessel and/or to the inner surface of the vessel. In some embodiments, the carbon dioxide barrier coating or layer may even be applied as an intermediate step in the preparation of the vessel itself, in which case the carbon dioxide barrier coating or layer may be nested between layers of resin and positioned between the inner and outer surfaces of the vessel wall.

In embodiments of the present disclosure, a vessel made from a thermoplastic material/resin may be coated with a carbon dioxide barrier coating or layer in order to provide a package, e.g. a drug primary package, a vial, a syringe, or an evacuated blood tube, having a carbon dioxide transmission rate that is lower than an identical package in which the vessel is made from the same thermoplastic material/resin but which lacks the carbon dioxide barrier coating or layer, optionally at least 5% lower, optionally at least 10% lower, optionally at least 20% lower, optionally at least 30% lower, optionally at least 40% lower, optionally at least 50% lower, optionally at least 60% lower, optionally at least 70% lower, optionally at least 80% lower, optionally at least 90% lower. The vessels may optionally include one or more additional coatings, such as an oxygen barrier coating or layer, a water vapour barrier coating or layer, a tie coating or layer, a pH protective coating or layer, a lubricity coating or layer, or any combination thereof. In some embodiments, the vessel wall may consist essentially of a COP or COC resin while in other embodiments the vessel wall may consist essentially of a commodity resin, e.g. a CBC resin.

By applying a carbon dioxide barrier layer such to a vessel, e.g. a syringe or vial, it has presently been found that the carbon dioxide transmission rate ($CO_2TR$) may be less than 0.005 $d^{-1}$; optionally less than 0.004 $d^{-1}$; optionally less than 0.002 $d^{-1}$; optionally less than 0.001 $d^{-1}$; optionally less than 0.0008 $d^{-1}$, optionally less than 0.0006 $d^{-1}$; optionally less than 0.0005 $d^{-1}$; optionally less than 0.0004 $d^{-1}$, optionally less than 0.0003 $d^{-1}$; optionally less than 0.0002 $d^{-1}$; optionally less than 0.0001 $d^{-1}$.

The carbon dioxide barrier coating or layer may be effective to reduce the ingress of carbon dioxide into the lumen of a drug primary package or the egress of carbon dioxide out of the lumen of the drug primary package to less than 0.005 cc/package/day at 25 °C, 60% relative humidity and 0.21 bar, optionally less than 0.004 cc/package/day at 25 °C, 60% relative humidity and 0.21 bar, optionally less than 0.003 cc/package/day at 25 °C, 60% relative humidity and 0.21 bar, optionally less than 0.002 cc/package/day at 25 °C, 60% relative humidity and 0.21 bar, optionally less than 0.001 cc/package/day at 25 °C, 60% relative humidity and 0.21 bar, optionally less than 0.0008 cc/package/day at 25 °C, 60% relative humidity and 0.21 bar, optionally less than 0.0006 cc/package/day at 25 °C, 60% relative humidity and 0.21 bar. Accordingly, embodiments of vials comprising a carbon dioxide barrier layer may allow for an ingress of carbon dioxide into the lumen or an egress of carbon dioxide out of the lumen of less than 0.005 cc/package/day at 25 °C, 60% relative humidity and 0.21 bar, optionally less than 0.004 cc/package/day at 25 °C, 60% relative humidity and 0.21 bar, optionally less than 0.003 cc/package/day at 25 °C, 60% relative humidity and 0.21 bar, optionally less than 0.002 cc/package/day at 25 °C, 60% relative humidity and 0.21 bar, optionally less than 0.001 cc/package/day at 25 °C, 60% relative humidity and 0.21 bar, optionally less than 0.0008 cc/package/day at 25 °C, 60% relative humidity and 0.21 bar, optionally less than 0.0006 cc/package/day at 25 °C, 60% relative humidity and 0.21 bar.

By applying a carbon dioxide barrier layer to a vessel, e.g. a syringe or vial, it has presently been found that the carbon dioxide contained within a drug product and/or located in the headspace of the vessel can be maintained for a

longer period of time, thereby improving the shelf life of the drug primary package.

It has also presently been found that the shelf life of an evacuated blood tube can be significantly improved by applying a carbon dioxide barrier layer to the blood tube, as it prevents the ingress of environmental carbon dioxide, and thereby provides a longer lasting vacuum within the lumen of the blood tube.

**Improved Oxygen Barrier**

In some embodiments, an oxygen barrier coating or layer may be applied by atomic layer deposition. The oxygen barrier coating or layer may comprise SiOx wherein x is from 1.5 to 2.9. In contrast to application of the oxygen barrier coating or layer by PECVD, it has presently been found that using atomic layer deposition, the oxygen barrier coating or layer may provide improved barrier performance when compared to the same coating, i.e., an oxygen barrier coating or layer having substantially the same chemical composition and substantially the same thickness, applied by PECVD. Without being bound by theory, it is believed that the high density of the oxygen barrier coating or layer produced by atomic layer deposition creates an improved barrier to oxygen ingress. Accordingly, by using atomic layer deposition to deposit the oxygen barrier coating or layer in accordance with aspects of the present disclosure, the performance of the coating may be increased relative to the same coating deposited by PECVD and the thickness of the coating may be significantly decreased.

In some embodiments, for example, the vessel having an oxygen barrier coating or layer applied by ALD or PEALD may have an oxygen transmission rate constant that is less than the oxygen transmission rate constant of an otherwise equivalent vessel in which an oxygen barrier coating or layer having substantially the same composition and thickness is applied by PECVD, optionally at least 10% less, optionally at least 20% less, optionally at least 30% less, optionally at least 40% less, optionally at least 50% less, optionally at least 60% less, optionally at least 70% less, optionally at least 80% less, optionally at least 90% less. This effect may be particularly present at low coating thicknesses, such as between 1 nm and 20 nm, optionally between 1 nm and 15 nm, optionally between 1 nm and 10 nm, optionally between 2 nm and 20 nm, optionally between 2 nm and 15 nm, optionally between 2 nm and 10 nm.

The oxygen barrier coating or layer may also provide an oxygen transmission rate that is suitable for the vessel to store liquid drug formulations over a period of time, even where the thickness of the oxygen barrier coating or layer is significantly reduced from that which may be required of an oxygen barrier coating or layer applied by PECVD. For example, the vessel comprising an oxygen barrier coating or layer applied by atomic layer deposition may have an oxygen transmission rate constant less than 0.0010 $d^{-1}$; optionally less than 0.0008 $d^{-1}$; optionally less than 0.0006 $d^{-1}$; optionally less than 0.0004 $d^{-1}$; optionally less than 0.0003 $d^{-1}$; optionally less than 0.0002 $d^{-1}$; optionally less than 0.0001 $d^{-1}$. This may be achieved at a relatively small thickness, such as between 1 nm and 20 nm, optionally between 1 nm and 15 nm, optionally between 1 nm and 10 nm, optionally between 2 nm and 20 nm, optionally between 2 nm and 15 nm, optionally between 2 nm and 10 nm, optionally between 3 nm and 20 nm, optionally between 3 nm and 15 nm, optionally between 3 nm and 10 nm, optionally between 4 nm and 20 nm, optionally between 4 nm and 15 nm, optionally between 4 nm and 10 nm.

**Vials**

Vials 400 of the present disclosure may include a bottom wall 401, a side wall extending 402 upward from the bottom wall, a curved lower edge joining the bottom wall and the side wall 403, a radially inwardly extending shoulder 404 formed at the top of the side wall, and a neck 405 extending upwardly from the shoulder, the neck defining an opening 406 at the top thereof, the opening leading to the vial interior, i.e. lumen 212.

<u>Improved lyophilization</u>

Embodiments of the vials of the present disclosure are configured to provide an improved, e.g. more efficient and more consistent, lyophilization process, and a vial 400 configured to enable an improved lyophilisation process.

For many drug products, after the drug product is filled into the vial, the filled vial is subjected to lyophilization in order to freeze-dry the drug product. As part of the lyophilization process, a filled vial, usually a set of filled vials seated in a tray having a plurality of vial seating receptacles with open bottoms such as that described in WO 2014/130349, is placed on a freezer plate. After freezing, the filled vial is then subjected to drying, which is typically performed in two steps: primary drying and secondary drying.

In some embodiments of vials 400 according to the present disclosure, the lower surface of the bottom wall 407 may be flat or substantially flat (as described below). A flat or substantially flat lower surface 407 provides for a consistent lyophilization of the drug product contained within the lumen 212 of the vial. By providing a vial 400 with a flat or substantially flat lower surface 407, heat transfer from the freezer plate to the drug product is improved. The improved heat transfer may lead to lower cycle times. For example, due to the improved heat transfer provided by the flat or substantially

flat lower surface 407, the cycle time of the lyophilization process may be reduced by at least 10%, alternatively at least 20%, alternatively at least 30%, alternatively at least 40%, alternatively 50% or more relative to a conventional plastic vial having a lower surface comprising an outer rim. Similarly, the cycle time of the process may be reduced by at least 5%, alternatively 10% or more relative to a conventional glass vial (which also has a lower surface comprising an outer rim). An example of a vial 400 have a flat or substantially flat lower surface 407 is shown in Figure 30.

The flatness of the lower surface of the bottom wall 407 may be tested by an ink-blot test. An embodiment of a vial 400 having a substantially flat lower surface 407 and a conventional vial, which has a curved lower surface, were each placed on a colored (here, blue) ink and then placed on a clean, white surface. As shown in Figure 31, for example, a conventional vial having a lower surface comprising an outer rim produced an ink blot that corresponded with the outer rim, producing a coverage of less than 20 percent of the footprint of the vial (see ink blot on the left). The vial 400 having a substantially flat lower surface 407 in accordance with an embodiment of the present disclosure on the other hand produced an ink blot that spanned substantially the entire footprint of the vial, i.e. had no major gaps between the circumference and the center, and covered about 75-80% of that footprint (see ink blot on the right). To be substantially flat, embodiments of the vials 400 disclosed herein must produce an ink blot that covers at least 50% of the footprint of the vial. In some embodiments, the vials disclosed herein may produce an ink blot that covers at least 60% of the footprint of the vial, optionally at least 70%, optionally at least 75%, optionally at least 80%, optionally at least 85%, optionally at least 90%.

The improvements in heat transfer provided by a vial 400 having a flat or substantially flat lower surface 407 can be seen, for example, in Table A1, which show the results of a heat transfer study designed to determine the heat transfer coefficients (Kv) of various vial types for lyophilization (Kv is a critical parameter used to design an optimal lyophilization cycle for a particular drug formulation). Heat transfer coefficient depends on the wall 401, 402 thickness and mass of the vial 400, the thermal conductivity of the materials that make up the vial, and the contact between the base, e.g. lower surface 407, of the vial and the lyophilizer shelf.

10 mL vials 400 made of (i) type 1 glass, (ii) uncoated COP, (iii) trilayer-coated COP, and (iv) trilayer-coated COP configured to have a substantially flat lower surface 407 were provided. The vials were each filled with 3 mL of water and trays 408 were filled with each vial type. Each tray 408 was placed on a lyophilizer shelf and the lyophilization cycle shown in Figure 33A was run. A sample of each type of 10 mL vials 400 were selected from each tray 408, ensuring that the vials were selected from the same positions in each 240 count tray, as shown in Figure 33B, and the following were measured:

$$\dot{m} = \frac{(mass\ of\ vial + water\ before\ cycle) - (mass\ of\ vial + ice\ after\ cycle)}{time\ spent\ in\ vacuum}$$

$$T_{bottom} = unknown - measure\ experimentally\ with\ thermocouple$$

The measured values were then plugged into the following equation to determine the heat transfer coefficient (Kv) for each type of vial:

$$K_v = \frac{\Delta H_{sub} * \dot{\mathrm{m}}}{\mathrm{Area} * (T_{shelf} - T_{bottom})}$$

$$\Delta H_{sub} = 660\ cal/g\ (obtained\ from\ Pikal\ 1983\ article)$$

$$A = \pi \left(\frac{d}{2}\right)^2$$ *where d = 24 mm, the outer diameter of the vial bottom*

$$T_{shelf} = -5\mathrm{°C}$$

The results, shown below in Table A1, demonstrate that COP vials 400 molded in accordance with the present disclosure to have a substantially flat lower surface 407 produced a heat transfer coefficient (Kv x $10^4$) of 3.56 cal/s/$cm^2$/°C whereas COP vials molded in accordance with the present disclosure but lacking a flat or substantially flat lower surface produced a heat transfer of only 3.18 cal/s/$cm^2$/°C. Thus, the inclusion of a substantially flat lower surface produced an almost 12% increase in the heat transfer coefficient (Kv) of the COP vials.

**Table A1**

| Vial Type | Kv x $10^4$ (cal/s/cm$^2$/°C) | Standard Deviation |
|---|---|---|
| Glass | 4.23 | ±0.19 |
| Coated COP | 3.18 | ±0.07 |
| Coated COP (Flat Bottom) | 3.56 | ±0.07 |

Further, embodiments of the vials 400 of the present disclosure also provide a more consistent heat transfer than conventional glass vials due to being molded to have particularly tight dimensional tolerances. For instance, embodiments of vials 400 molded in accordance with the present disclosure may have a low variation in mass, a low dimensional variation, or both.

As shown in Table A2, for example, (twenty unfilled) vials 400 molded from COP in accordance with the present disclosure were weighed and found to have masses with a standard deviation of 0.005 g, whereas (twenty unfilled) conventional glass vials were found to have masses with a standard deviation of 0.085 g.

**Table A2**

| Vial Type | Mass (g) |
|---|---|
| Glass | 11.708 ± 0.085 |
| COP | 6.726 ± 0.006 |
| Coated COP | 6.728 ± 0.005 |
| Coated COP (Flat Bottom) | 6.726 ± 0.005 |

In some embodiments of the present disclosure, the masses of vials 400 may have a standard deviation of less than 0.010 g, optionally less than 0.009 g, optionally less than 0.008 g, optionally less than 0.007 g, optionally less than 0.006, optionally 0.005 g or less.

Similarly, as shown in Figure 32, the outside diameters of (twenty unfilled) vials 400 molded from COP in accordance with the present disclosure were measured and plotted as 409, while the outside diameters of (twenty unfilled) conventional glass vials were measured and the results plotted as 410. The COP vials 400 prepared in accordance with the present disclosure had a variance of about 0.13 mm (and a standard deviation of 0.0026 mm). The conventional glass vials had a variance of about 0.66 mm (and a standard deviation of 0.050 mm), which is about five times greater than the variation of the COP vials produced in accordance with the present disclosure.

In some embodiments of the present disclosure, the outer diameters of a plurality of vials may vary (±) from the nominal value by less than 0.50 mm, optionally less than 0.40 mm, optionally less than 0.30 mm, optionally less than 0.20 mm, optionally less than 0.15 mm. In some embodiments of the present disclosure, the outer diameters of vials 400 may have a standard deviation of less than 0.04 mm, optionally less than 0.03 mm, optionally less than 0.02 mm, optionally less than 0.01 mm, optionally less than 0.008 mm, optionally less than 0.005 mm.

At least in part due to the tight mass and dimensional tolerances of embodiments of the present disclosure, vials 400 produced in accordance with the present disclosure may produce a more consistent heat transfer during the lyophilization process. For instance, the results of the testing shown in Table A1 demonstrated that while conventional glass vials produced a heat transfer (Kv x $10^4$) of 4.23 cal/s/cm$^2$/°C with a standard deviation of 0.19, COP vials produced in accordance with embodiments of the present disclosure produced a heat transfer (Kv x $10^4$) of 3.56 cal/s/cm$^2$/°C with a standard deviation of 0.07. In some embodiments of the present disclosure, when subjected to lyophilization heat transfer testing as described above, the vials 400 may produce a heat transfer (Kv x $10^4$) of at least 3.3 cal/s/cm$^2$/°C, alternatively at least 3.4 cal/s/cm$^2$/°C, alternatively at least 3.5 cal/s/cm$^2$/°C, with a standard deviation of less than 0.15 cal/s/cm$^2$/°C, alternatively less than 0.12 cal/s/cm$^2$/°C, alternatively less than 0.10 cal/s/cm$^2$/°C, alternatively less than 0.08 cal/s/cm$^2$/°C.

Cold Chain / Cryogenic

Many drug products are temperature sensitive and require refrigeration storage and transportation. Others require cryogenic storage and transportation, e.g. temperatures below 0 °C and often significantly lower temperatures such as below -15 °C, below -35 °C, below -65 °C, below -120 °C, below -150 °C, and the like. These cold chain drugs require specific storage conditions, many of which utilize liquid nitrogen which can have a temperature as low as -196 °C. In general, drug products storage conditions can be divided into the following categories: refrigerated (e.g., 2°C to 8°C),

freezer (e.g., -25°C to -10°C), ultra-low freezer (e.g., -70°C to - 90°C), vapor-phase liquid nitrogen (e.g., -135°C to -196°C), and liquid-phase liquid nitrogen (e.g., -195°C). Storage of drug products at these extreme temperatures places significant stresses on the drug primary package, e.g. the vial 400 and the rubber stopper or plug 411.

In particular, the materials that make up the vial 400 and stopper 411 will expand and/or contract differently at these low temperatures, which can lead to gaps in the seal between the vial and the stopper through which environmental gases, e.g. oxygen, can enter the lumen and negatively impact the drug product. Further, glass vials have been known to crack or break as a result of the mechanical stresses that it undergoes at extreme low temperatures, including for example stresses caused by expansion of a liquid drug product within the lumen.

Embodiments of the vials 400 and stoppers 411, which may together and optionally including an additional cap 412, typically made of a metal such as aluminium, that is crimped over the top of the stopper and neck flange of the vial, be referred to as a vial package of the present disclosure may be configured to withstand temperatures down to -196 °C without a loss of container closure integrity (CCI), without any resulting loss in gas barrier properties that would be caused by a loss of CCI, and without risk of cracking or breaking.

Testing of the container closure integrity was performed on vials 400 produced in accordance with embodiments of the present disclosure in combination with commercial stoppers 411. As positive control samples, vials provided with leaks by insertion of a 5μm, 10mm long capillary tube was also tested. For the CCI testing, the samples were sealed and stored on a bed of dry ice. Then, at various times the headspace $CO_2$ partial pressure (mbar) of each sample was measured using an FMS-Carbon Dioxide ($CO_2$) Headspace Analyzer by Lighthouse Instruments. Changes in $CO_2$ partial pressure in the vial headspace was found to serve as confirmation of a breach in Container Closure Integrity (CCI).

More particularly, the samples were each sealed with a rubber stopper 411 and aluminum crimp 412 at ambient conditions and characterized via residual seal force (RSF) measurements at Genesis Packaging Technologies. A low, mid, and high compression setting was chosen for each vial-stopper combination. The headspace $CO_2$ partial pressure (mbar) of all sealed vials, including the positive controls, were then measured via FMS-Carbon Dioxide ($CO_2$) Headspace Analyzer by Lighthouse Instruments. The FMS-Carbon Dioxide Headspace Analyzer employs tunable diode laser absorption spectroscopy (TDLAS) to provide rapid and non-invasive gas analysis of the headspace within sealed containers. Additional details on the FMS-Carbon Dioxide Headspace Analyzer can be found in Victor KG, Levac L, Timmins M, Veale, J., Method Development for Container Closure Integrity Evaluation via Headspace Gas Ingress by Using Frequency Modulation Spectroscopy, J Pharm. Sci. Technol. 71(6), at pages 429-453 (Nov.-Dec. 2017).

After that t = 0 measurement, the samples were incubated at -80 °C by being stored in a freezer with a bed of dry ice, which created a $CO_2$ rich atmosphere. After storage for various times, 20 samples per compression setting for each vial-stopper combination and 5 positive controls were pulled from cold storage and allowed to equilibrate to room temperature. The headspace $CO_2$ partial pressure (mbar) of each sample was then measured again using an FMS-Carbon Dioxide ($CO_2$) Headspace Analyzer by Lighthouse Instruments.

The results are shown in Figure 34. The results demonstrate that vials 400 produced in accordance with embodiments of the present disclosure can be stored at -80 °C without any loss of container closure integrity (CCI). In contrast, the control sample showed significant losses in CCI after one week of storage at -80 °C. The test is presently ongoing, with additional measurements to be performed until, and possibly after, one year of storage. Based on the results thus far, it is expected that the test vials will show no loss of CCI after six months of storage, after nine months of storage, and after one year of storage at -80 °C.

Embodiments of vials 400 of the present disclosure may maintain container closure integrity for at least 3 months, optionally for at least 6 months, optionally for at least 9 months, optionally for at least 12 months when stored at -80 °C.

These results are due, at least in part, to the tight dimensional tolerances to which vials 400 of embodiments of the present disclosure are molded. For instance, the dimensional consistency of the flange at the mouth of the vial ensures that the rubber stopper will have a consistent surface-to-surface contact with the flange.

An additional test was done to determine whether the barrier coatings 288 described herein, and in particular a SiOx barrier coating as described herein, would be negatively affected by extreme cryogenic conditions. In this test, a sample of a COP vial 400 produced in accordance with the present disclosure and containing a trilayer coating set 285 (with each layer being applied by PECVD) was immersed in liquid nitrogen, which has a temperature of -196 °C. Oxygen transmission rate of the vial 400 was then measured and the result plotted in Figure 35. As shown in Figure 35, the oxygen transmission constant of the trilayer-coated COP vial was substantially the same after immersion in liquid nitrogen as the oxygen transmission rate of a trilayer-coated COP vial maintained at room temperature (and well below that of a COP vial lacking a gas barrier coating).

Embodiments of vials 400 of the present disclosure comprise gas barrier coatings 288 that are configured to demonstrate substantially no loss of barrier properties even when subjected to extreme temperatures as low as -196 °C, including for example -80 °C or lower, -100 °C or lower, -120 °C or lower, -140 °C or lower, -160 °C or lower, -180 °C or lower, or even -196 °C. Relatedly, embodiments of vials 400 of the present disclosure are configured to have an oxygen transmission rate constant less than 0.005 $d^{-1}$, optionally less than 0.004 $d^{-1}$, optionally less than 0.003 $d^{-1}$, optionally less than 0.002 $d^{-1}$, optionally less than 0.001 $d^{-1}$, optionally less than 0.0005 $d^{-1}$ even after immersion of the vial in liquid

nitrogen.

As it has been shown that the gas barrier coating 288 itself does not lose effectiveness when subjected to extreme low temperatures, based on the CCI testing described above it can be said that embodiments of vials 400 of the present disclosure may have an oxygen transmission rate constant less than 0.005 $d^{-1}$, optionally less than 0.004 $d^{-1}$, optionally less than 0.003 $d^{-1}$, optionally less than 0.002 $d^{-1}$, optionally less than 0.001 $d^{-1}$, optionally less than 0.0005 $d^{-1}$ after storage at -80 °C for at least 3 months, optionally for at least 6 months, optionally for at least 9 months, optionally for at least 12 months.

Low Particles

The present of foreign materials, e.g. particles, in a liquid drug product can negatively affect the immunogenicity of the drug and promote protein aggregation. Moreover, the presence of foreign materials within injectable and ophthalmic drug products can have significant effects on product quality and safety. USP<788> and USP <789> are tests for the release of injectable and ophthalmic drug products, respectively. When administering a drug intravitreally, it is extremely important to minimize the introduction of particles into the vitreous body of the eye, which may be seen as floaters or otherwise interfere with the patient's vision. Therefore, the standards limiting the amount and size of particles in formulations for intravitreal injection - for example <789> or Ph. Eur 5.7.1 - are stringent.

Embodiments of vials 400 of the present disclosure may produce very low particle contents in the drug products contained therein. PECVD coating processes can introduce a small number of particles into the lumen of the vessel. By using automated molding and coating cells, maintaining tight in-process controls during molding and coating, and on-line particle inspection, the number of visible and subvisible particles can be minimized and effectively eliminated. Atomic layer deposition (ALD) coating, on the other hand, is a particle-free coating process. Accordingly, it is believed that applying one or more gas barrier coatings or layers 288 using ALD in place of PECVD, the number of particles that are introduced into a drug product from the vessel may be further reduced down to effectively zero.

For instance, embodiments of the vials 400 of the present disclosure were tested for particles using standard light obscuration (LO) test procedures in accordance with USP 787 and USP 788 and industry accepted micro flow imaging (MFI) test procedures. The results of this testing is shown in Figures 39-40. Specifically, Figure 39 shows the results of LO testing of six production lots of trilayer-coated 6mL vials. Notably, no particles having sizes 50 μm or greater were found in any of the six lots, no particles having sizes 25 μm or greater were found in any of the six lots, the six lots averaged less than two, indeed less than one, particles/mL having sizes 10 μm or greater, and the six lots averaged less than 15, indeed less than 10, particles/mL having sizes 2 μm or greater. Figure 40 shows the results of a comparative MFI testing of those same trilayer-coated 6 mL vials against a number of commercial vials. Notably, the trilayer-coated 6 mL vials were found to have about 10 particles/mL of 2 μm in size or greater, while the commercial vials contained between about 30 and about 225 particles of 2 μm in size or greater.

Embodiments of the vials 400 of the present disclosure may, using standard LO or MFI testing, have less than 50 particles/mL of 2 μm in size or greater, optionally less than 40 particles/mL of 2 μm in size or greater, optionally less than 30 particles/mL of 2 μm in size or greater, optionally less than 25 particles/mL of 2 μm in size or greater, optionally less than 20 particles/mL of 2 μm in size or greater, optionally less than 15 particles/mL of 2 μm in size or greater, optionally less than 12 particles/mL of 2 μm in size or greater, optionally less than 10 particles/mL of 2 μm in size or greater.

**Syringes**

Embodiments of the syringes 500 disclosed herein include staked needle syringes and luer lock syringes. Some embodiments of the syringes 500 disclosed herein may be configured for inclusion in auto-injector systems. Indeed, because of the improved dimensional consistency described herein, embodiments of the syringes 500 of the present disclosure are particularly suitable and configurable for use in auto-injectors.

Dimensional Consistency

Embodiments of the syringes 500 and syringe barrels 501 of the present disclosure may provide significantly improved dimensional consistency between individual units. Large dimensional tolerances decrease dose accuracy and lead to inconsistent device performance. By improving dimensional consistency, therefore, embodiments of the present disclosure may increase dose accuracy, provide for a more consistent drug filling operation, reduce costs associated with drug overfilling, improve efficiencies on automated lines and handling equipment, and reduce the risk of failures in the field particularly with autoinjectors and pens.

Improved dimensional consistency applies across a variety of dimensions, including for instance syringe barrel inner diameter 502, syringe barrel needle or Luer hub outer diameter 503, syringe barrel length 504, and flange outer diameter 505. Weight consistency may also be measured and used as a general correlation for dimensional consistency.

For each of these, the consistency of embodiments of syringes 500 of the present disclosure were measured and compared against a commercial glass syringe product and the results plotted in Figures 41-47.

Embodiments of syringes 500 and syringe barrels 501 according to the present disclosure may be produced with a very narrow inner diameter 502 tolerance range. Though the syringes 500 are produced in high volumes, no syringe may have an inner diameter 502 that varies (±) from the nominal value by more than 0.05 mm. Acordingly, a syringe 500 of embodiments of the present disclosure may be said to comprise an inner diameter 502 having a degree of precision within plus-or-minus (±) 0.05 mm.

As shown in Figures 41-42, embodiments of syringes 500 according to the present disclosure are configured to have barrel inner diameters 502 that fall within a tight distribution peak having a standard deviation of 0.0035 mm. In contrast, commercial glass syringes have barrel inner diameters that are spread across a much wider curve and have a standard deviation of 0.0504 mm. Compared to glass, the barrel inner diameters of PET plastic syringes fall within a relatively tight peak, but one that is significantly broader than the peak obtained by embodiments of the present disclosure. Embodiments of syringes 500 according to the present disclosure have thus been demonstrated to have significantly more consistent barrel inner diameters 502 than conventional syringes.

Embodiments of syringes 500 and syringe barrels 501 according to the present disclosure may have barrel inner diameters 502 that are consistent and tightly grouped, with a standard deviation less than 0.03 mm, optionally less than 0.02 mm, optionally less than 0.01 mm, optionally less than 0.008 mm, optionally less than 0.006 mm, optionally less than 0.005 mm, optionally less than 0.004 mm.

Importantly, these tight dimensional tolerances, and in particular a tight dimensional tolerance for the inner diameter 502, provide the syringes 500 with enhanced dose accuracy amongst a variety of other benefits.

Embodiments of syringes 500 and syringe barrels 501 according to the present disclosure may also be produced with a very narrow needle hub or Luer hub outer diameter 503 tolerance range. Though the syringes 500 are produced in high volumes, no syringe may have a needle hub or Luer hub outer diameter 503 that varies (±) from the nominal value by more than 0.07 mm, optionally 0.05 mm. Acordingly, a syringe 500 of embodiments of the present disclosure may be said to comprise a needle hub or Luer hub outer diameter 503 having a degree of precision within plus-or-minus (±) 0.07 mm, optionally within plus-or-minus (±) 0.05 mm.

As shown in Figure 43, embodiments of syringes 500 according to the present disclosure are configured to have needle hub or Luer hub outer diameters 503 that fall within a tight distribution peak having a standard deviation of 0.0047 mm. In contrast, the needle or Luer hub outer diameter of commercial glass syringes are spread across a much wider curve and have a standard deviation of 0.2003 mm. Embodiments of syringes 500 and syringe barrels 501 according to the present disclosure have thus been demonstrated to have significantly more consistent needle hub outer diameters 503 than conventional syringes.

Embodiments of syringes 500 and syringe barrels 501 according to the present disclosure may have needle hub outer diameters 503 that are consistent and tightly grouped, with a standard deviation less than 0.15 mm, optionally less than 0.10 mm, optionally less than 0.08 mm, optionally less than 0.05 mm, optionally less than 0.02 mm, optionally less than 0.008 mm, optionally less than 0.005 mm.

Importantly, these tight dimensional tolerances, and in particular a tight dimensional tolerance for the needle hub outer diameter 503, provide the syringes 500 with a consistent and enhanced sealing, such as with a rigid needle shield, which leads to improved CCI, amongst a variety of other benefits.

Embodiments of syringes 500 and syringe barrels 501 according to the present disclosure may be produced with a very narrow syringe barrel length 504 tolerance range. Though the syringes 500 are produced in high volumes, no syringe barrel may have a length 504 that varies (±) from the nominal value by more than 0.20 mm. Acordingly, a syringe barrel 501 of embodiments of the present disclosure may be said to comprise a length 504 having a degree of precision within plus-or-minus (±) 0.20 mm.

As shown in Figures 44-45, embodiments of syringes 500 and syringe barrels 501 according to the present disclosure are configured to have overall lengths 504 that fall within a tight distribution peak having a standard deviation of 0.0096 mm. In contrast, commercial glass syringes have overall lenghts that are spread across a much wider curve and have a standard deviation of 0.0724 mm. Embodiments of syringes according to the present disclosure have thus been demonstrated to have significantly more consistent lengths than conventional syringes.

Embodiments of syringes 500 and syringe barrels 501 according to the present disclosure may have overall lengths 504 that are consistent and tightly grouped, with a standard deviation less than 0.06 mm, optionally less than 0.05 mm, optionally less than 0.04 mm, optionally less than 0.03 mm, optionally less than 0.02 mm, optionally less than 0.01 mm.

Embodiments of syringes 500 and syringe barrels 501 according to the present disclosure may be produced with a very narrow flange diameter 505 tolerance range. Though the syringes 500 are produced in high volumes, no syringe may have a flange outer diameter 505 that varies (±) from the nominal value by more than 0.10 mm. Accordingly, a syringe 500 of embodiments of the present disclosure may be said to comprise a flange outer diameter 505 having a degree of precision within plus-or-minus (±) 0.10 mm.

As shown in Figure 46, embodiments of syringes 500 according to the present disclosure are configured to have flange outer diameters 505 that fall within a tight distribution peak having a standard deviation of 0.0104 mm. In contrast, commercial glass syringes have flange outer diameters that are spread across a much wider curve and have a standard deviation of 0.0662 mm. Embodiments of syringes 500 and syringe barrels 501 according to the present disclosure have thus been demonstrated to have significantly more consistent flange outer diameters 505 than conventional syringes.

Embodiments of syringes 500 and syringe barrels 501 according to the present disclosure may have flange outer diameters 505 that are consistent and tightly grouped, with a standard deviation less than 0.06 mm, optionally less than 0.05 mm, optionally less than 0.04 mm, optionally less than 0.03mm, optionally less than 0.02 mm, optionally less than 0.015 mm.

As shown in Figure 47, embodiments of syringes 500 and syringe barrels 501 according to the present disclosure are configured to have weights that fall within a tight distribution peak having a standard deviation of 0.0003 g. In contrast, the commercial glass syringes have weights that are spread across a much wider curve and have a standard deviation of 0.0289 g. Embodiments of syringes 500 and syringe barrels 501 according to the present disclosure have thus been demonstrated to have significantly more consistent weights than conventional syringes.

Embodiments of syringes 500 and syringe barrels 501 according to the present disclosure may have weights that are consistent and tightly grouped, with a standard deviation less than 0.025 g, optionally less than 0.020 g, optionally less than 0.015 g, optionally less than 0.010 g, optionally less than 0.0075 g, optionally less than 0.005 g.

Low Particles

The present of foreign materials, e.g. particles, in a liquid drug product can negatively affect the immunogenicity of the drug and promote protein aggregation. Moreover, the presence of foreign materials within injectable and ophthalmic drug products can have significant effects on product quality and safety. USP<788> and USP <789> are tests for the release of injectable and ophthalmic drug products, respectively. When administering a drug intravitreally, it is extremely important to minimize the introduction of particles into the vitreous body of the eye, which may be seen as floaters or otherwise interfere with the patient's vision. Therefore, the standards limiting the amount and size of particles in formulations for intravitreal injection - for example <789> or Ph. Eur 5.7.1 - are stringent.

Silicone oil is commonly used as a lubricant on conventional syringes. However, that silicone oil contributes heavily to the particle content of a drug product delivered via a syringe. While the thermal fixation of silicone oil on the glass surface in a process called baked-on siliconization reduces this effect, baked-on silicone still contributes heavily to particle formation. Inclusion of a lubricating coating or layer in accordance with embodiments of the present disclosure provide syringes that have no silicone oil or baked-on silicone. By overcoming the need for silicone oil or baked-on silicone, embodiments of the syringes of the present disclosure may provide a drug primary package in which the drug product has a significantly lower particle count than would otherwise be the case with, e.g., a siliconized glass syringe.

The ability of a lubricating coating as described herein to reduce the particle content of a liquid contained within the lumen of a syringe is demonstrated by testing in which a syringe having no lubricating layer or additive ("Blank" or "Control"), a COP syringe having an OMCTS-based lubricity coating prepared in accordance with the present disclosure ("OMCTS"), and a glass syringe on which silicone oil was sprayed to produce a siliconized surface ("Siliconized Glass") were each filled with Milli-Q ("MQ") water, subjected to a variety of stresses, and then tested for particle count using resonant mass measurement (RMM), standard light obscuration (LO) testing, and FlowCAM® microflow digital imaging (FlowCAM® is a registered trademark of Fluid Imaging Technologies Inc.). The stresses included inversion for 2 hours at 50 rpm, incubation for two weeks at 4 °C, and five cycles of freeze thawing between 20 °C and -40 °C. The results of the testing is shown in Figures 48-50.

As shown in Figure 48, resonant mass measurement (RMM) was performed to quantify the amount of particles sized 300 nm or greater present in the Milli-Q water contained within each syringe after each of the stresses. The RMM results demonstrated that, after each of the three stresses, the COP syringes having an OMCTS-based lubricity coating in accordance with the present disclosure contained less than 300,000 (the LOQ of the test) particles sized 300 nm or greater. In contrast, the siliconized glass syringes demonstrated significantly higher numbers of particles, including over 1,000,000 particles sized 300 nm or greater after being subjected to the incubation and freeze-thawing stresses. Embodiments of the syringes of the present disclosure may be configured so that, when filled with Milli-Q water and subjected to any one or more of the three stresses described herein, the contents of the syringe has less than 500,000 particles sized 300 nm or higher, alternatively less than 400,000 particles sized 300 nm or higher, alternatively less than 300,000 particles sized 300 nm or higher when tested with resonant mass measurement.

As shown in Figures 49 and 50, the FlowCAM® microflow digital imaging and light obscuration testing were each performed to quantify the amount of particles sized 2 μm or higher present in the Milli-Q water contained within each syringe after each of the stresses. Both tests demonstrated that, after each of the three stresses, the COP syringes having an OMCTS-based lubricity coating in accordance with the present disclosure contained significantly few particles than siliconized glass syringes.

For example, after inversion for two hours at 50 rpm, the Milli-Q water contained in the syringes having an OMCTS-based lubricity coating in accordance with the present disclosure contained about 140 particles sized 2 μm or higher when tested with FlowCAM® microflow digital imaging and about 80 particles sized 2 μm or higher when tested with standard light obscuration methods. In contrast, the Milli-Q water contained in the siliconized glass syringes contained about 16,537 particles sized 2 μm or higher when tested with FlowCAM® microflow digital imaging and about 5,938 particles sized 2 μm or higher when tested with standard light obscuration methods. Embodiments of the syringes of the present disclosure may be configured so that, when filled with Milli-Q water and inverted for two hours at 50 rpm, the contents of the syringe has less than 500 particles sized 2 μm or higher, alternatively less than 400 particles sized 2 μm or higher, alternatively less than 300 particles sized 2 μm or higher, alternatively less than 200 particles sized 2 μm or higher when tested with FlowCAM® microflow digital imaging, light obscuration testing, or both.

Similarly, after a two week incubation at 4 °C, the Milli-Q water contained in the syringes having an OMCTS-based lubricity coating in accordance with the present disclosure contained about 861 particles sized 2 μm or higher when tested with FlowCAM® microflow digital imaging and about 340 particles sized 2 μm or higher when tested with standard light obscuration methods. In contrast, the Milli-Q water contained in the siliconized glass syringes contained about 11,247 particles sized 2 μm or higher when tested with FlowCAM® microflow digital imaging and about 5,441 particles sized 2 μm or higher when tested with standard light obscuration methods. Embodiments of the syringes of the present disclosure may be configured so that, when filled with Milli-Q water and incubated for two weeks at 4 °C, the contents of the syringe has less than 2,000 particles sized 2 μm or higher, alternatively less than 1,000 particles sized 2 μm or higher, alternatively less than 900 particles sized 2 μm or higher, alternatively less than 800 particles sized 2 μm or higher, alternatively less than 700 particles sized 2 μm or higher, alternatively less than 600 particles sized 2 μm or higher, alternatively less than 500 particles sized 2 μm or higher when tested with FlowCAM® microflow digital imaging, light obscuration testing, or both.

Similarly, after five cycles of freeze thawing between 20 °C and -40 °C, the Milli-Q water contained in the syringes having an OMCTS-based lubricity coating in accordance with the present disclosure contained about 1,794 particles sized 2 μm or higher when tested with FlowCAM® microflow digital imaging and about 220 particles sized 2 μm or higher when tested with standard light obscuration methods. In contrast, the Milli-Q water contained in the siliconized glass syringes contained about 140,292 particles sized 2 μm or higher when tested with FlowCAM® microflow digital imaging and about 34,491 particles sized 2 μm or higher when tested with standard light obscuration methods. Embodiments of the syringes of the present disclosure may be configured so that, when filled with Milli-Q water and subjected to five cycles of freeze thawing between 20 °C and -40 °C, the contents of the syringe has less than 20,000 particles sized 2 μm or higher, alternatively less than 10,000 particles sized 2 μm or higher, alternatively less than 5,000 particles sized 2 μm or higher, alternatively less than 2,000 particles sized 2 μm or higher, alternatively less than 1,000 particles sized 2 μm or higher, alternatively less than 500 particles sized 2 μm or higher, alternatively less than 300 particles sized 2 μm or higher when tested with FlowCAM® microflow digital imaging, light obscuration testing, or both.

In some embodiments, the liquid drug product within the drug primary package of the present disclosure may have low particle content sufficient for injection or parenteral infusion, e.g. satisfy the requirements of USP <788>. Optionally, the liquid drug product within the primary drug package of the present disclosure may also have low particle content sufficient for use intravitreally, e.g. satisfy the requirements of USP <789>. In some embodiments, in particular, it comprises less than 50 particles having a size of more than 10 μm after the vessel has been rotated at 40°C for five minutes, two weeks or four weeks after three freeze-thaw cycles from +5°C to -20°C with 1°C per minute, or after storage of the vessel at 5°C, 25°C and 60% relative humidity or 40°C and 75% relative humidity for three months. Alternatively or additionally, the liquid drug product within the drug primary package of the present disclosure may comprise less than 5 particles having a size of more than 25 μm after the vessel has been rotated at 40°C for five minutes, two weeks or four weeks, or after three freeze-thaw cycles from +5°C to -20°C with 1°C per minute, or after storage of the vessel at 5°C, 25°C/60% relative humidity or 40°C/75% relative humidity for three months. Hence, the drug primary package may meet the requirements of United States Pharmacopoeia USP789 for ophthalmic solutions with respect to these particle sizes.

As an alternative to a lubricating coating, embodiments of the syringes 500 of the present disclosure may comprise a plunger 509 having a lubricious gasket. Like the use of a lubricity coating as described herein, the inclusion of a plunger 509 having a lubricious gasket, e.g. as described below, produces low particle counts. For instance the 0.5 mL syringe and the 1.0 mL syringe shown in Figures 52 and 53 and having lubricious plunger gaskets were filled with Milli-Q water and tested for particle counts. The results are shown in Table A4.

**Table A4**

| Coating | Syringe Type | Particle Counts per mL of Water | | | |
|---|---|---|---|---|---|
| | | ≥ 2 μm | ≥ 10 μm | ≥ 25 μm | ≥ 50 μm |
| Lubricant Free | 0.5 mL Syringe | 30 | 1.3 | 0 | 0 |
| | 1 mL Syringe | 33 | 1.5 | 0 | 0 |

(continued)

| Coating | Syringe Type | Particle Counts per mL of Water | | | |
|---|---|---|---|---|---|
| | | ≥ 2 μm | ≥ 10 μm | ≥ 25 μm | ≥ 50 μm |
| USP-789 | Allowed Particulate Load | N/A | 50 | 5 | 1 |
| USP-788 | Allowed Particulate Load | N/A | 6000 | 60 | N/A |

Embodiments of the syringe 500 of the present disclosure may comprise a plunger 509 having a lubricious gasket and having less than 50 particles, optionally less than 40 particles, optionally less than 30 particles, optionally less than 20 particles, optionally less than 10 particles, optionally less than 5 particles, optionally less than 3 particles, optionally less than 2 particles having sizes of 10 μm or greater per mL of water. Embodiments of the syringes 500 of the present disclosure may comprise a plunger 509 having a lubricious gasket and having less than 60 particles, optionally less than 50 particles, optionally less than 40 particles, optionally less than 35 particles having sizes of 2 μm or greater per mL of water. Embodiments of the syringe of the present disclosure may comprise a plunger having a lubricious gasket and having less than 5 particles, optionally less than 4 particles, optionally less than 3 particles, optionally less than 2 particles, optionally no particles having sizes of 25 μm or greater per mL of water. Embodiments of the syringes 500 of the present disclosure may comprise a plunger 509 having a lubricious gasket and having particle counts within the limits imposed by USP-788 and/or USP-789.

Syringes 500 comprising a plunger 509 having a lubricious gasket, e.g. as described below, were also tested to determine whether they could produce a consistent break loose force and/or a consistent glide force, even after aging. Namely a sample of 1 mL staked needle syringes 500, such as that shown in Figure 52 and coated with a trilayer coating set 285, were filled with Mille-Q water and the plungers 509 (having lubricious gaskets) were vacuum loaded. The filled syringe assemblies were then stored at 4 °C for three months. At various times during those three months, the plunger speed of a number of the syringes were tested at 300 mm/min. The results of those measurements are shown in Figure 54.

As illustrated in Figure 54, the syringes produced a consistent break loose force in the range of about 4 to 5N and a consistent glide force in the range of about 5 to 6 N. Moreover, the results were largely consistent between syringes and over time, indicating that the syringes were not subjected to any plunger force aging over the three month test duration.

In addition to the low particles described herein, embodiments of syringes 500 of the present disclosure may have a plunger break loose force between 3 N and 6 N, optionally between 4 N and 5 N, without the use of silicone oil or baked-on silicone (or a PECVD lubricity coating). In addition to the low particles described herein, embodiments of syringes 500 of the present disclosure may have a plunger glide force between 4 N and 7 N, optionally between 5 N and 6 N, without the use of silicone oil or baked-on silicone (or a PECVD lubricity coating). Embodiments of syringes 500 of the present disclosure may also maintain the plunger break loose force and/or the plunger glide force within any of the above-identified ranges even after aging at 4 °C for at least one month, optionally at least two months, optionally at least three months.

Terminal Sterilization

Pre-filled syringes 500, and particularly those for intravitreal injection, typically are terminally sterilized using oxidizing gases such as ethylene oxide to reduce the risk of microbial infection of the eye. Syringe barrels 501 made from plastic typically have not been suitable for terminal sterilization because the plastic is permeable by the gases used for sterilization. Gases which enter into the pre-filled syringe may chemically react with the drug contained in the syringe and may thus significantly reduce the stability of the drug.

Embodiments of thermoplastic syringes of the present disclosure comprise a gas barrier coating or layer that is effective to reduce the ingress of ethylene oxide sterilization gas into the lumen compared to an uncoated syringe and/or to prevent ethylene oxide from permeating the thermoplastic wall and thus entering the lumen of the container. For example, embodiments of thermoplastic syringes of the present disclosure may have a barrier to ethylene oxide (EO) that is equivalent to a glass syringe of the same general size, as demonstrated for example, by the test results shown in Figure 51. Embodiments of the thermoplastic syringes of the present disclosure may be configured so that, when filled with Milli-Q water and subjected to conventional ethylene oxide sterilization, the contents of the syringe has less than 0.1 ppm ethylene oxide and less than 0.1 ppm ethylene chlorohydrin one month after sterilization, alternatively two months after sterilization, alternatively three months after sterilization, alternatively six months after sterilization, alternatively nine months after sterilization.

Optionally in any embodiment, a pre-filled pharmaceutical package, e.g. syringe, can be provided which is suitable for terminal sterilization by a sterilizing gas, optionally ethylene oxide EO gas, optionally at a pressure of 16.6 in. Hg (= 42.2 cm. Hg, 56 kilopascal, 560 mbar) for 10 hours at 120°F (49°C).

Lubricious and/or CCI-enhancing Plunger Gasket

In some embodiments of the present disclosure, the syringes 500 may be compatible with any of a variety of commercially available plungers. In other embodiments, however, the plunger 509 may be produced to have a tight dimensional tolerance with the syringe barrel 501 and/or to include a lubricious plunger gasket that also maintains CCI, such as those described in the below embodiments.

Pre-filled parenteral containers are typically sealed with a rubber gasket that is secured at the distal end to a plunger, which that provides closure integrity over the shelf life of the container's contents. Seals provided by rubber gaskets in the barrel of the syringe typically involve the rubber of the gasket being pressed against the interior surface of the barrel. Typically, the maximum diameter of the rubber gasket is larger in diameter than the smallest internal diameter of the barrel. Thus, to displace the rubber gasket and its attached plunger when the injection product is to be dispensed from the syringe requires overcoming this pressing force of the rubber gasket. Moreover, not only does this pressing force provided by the rubber seal typically need to be overcome when initially moving the gasket secured to the plunger, but this force also needs to continue to be overcome as the rubber gasket is displaced along the barrel during the dispensing of the injection product. The need for relatively elevated forces to advance the gasket and plunger in the syringe may increase the user's difficulty in administering the injection product from the syringe. This is particularly problematic for auto-injection systems where the syringe is placed into the auto-injection device and the gasket is advanced by a fixed spring. Accordingly, primary considerations concerning the use of a gasket secured to a plunger in a pre-filled parenteral container include: (1) container closure integrity ("CCI", defined below) and liquid/gas-tightness; and (2) plunger force (defined below) required to dispense syringe contents.

In practice, maintaining CCI/liquid or gas-tightness and providing desirable plunger force tend to be competing considerations. In other words, absent other factors, the tighter the fit between the gasket and the interior surface of the container to maintain adequate CCI/liquid or gas-tightness, the greater the force necessary to advance the gasket in use. In the field of syringes, it is important to ensure that the gasket secured to the plunger can move at a substantially constant speed and with a substantially constant and relatively low force when advanced in the barrel. In addition, the force necessary to initiate plunger movement and then continue advancement of the plunger should be low enough to enable comfortable administration by a user and prevent jolting or unnecessarily high pressing force that can cause patient discomfort.

To reduce friction and thus improve plunger force, lubrication is traditionally applied to the barrel-contacting engagement surface of the gasket secured to the plunger, the interior surface of the barrel, or both. However, as described above, use of flowable lubricant between the gasket and the barrel is not desired. As an alternative (or in addition) to flowable lubricants, gaskets have been developed from materials having lubricious properties or to include friction-reduced coatings or films on their exterior surface. However, such gaskets have experienced failures in CCI due to film wrinkling, defects in the film and/or film delamination from the rubber gasket may also have inferior gas-barrier properties. Accordingly, a conventional fluoropolymer film laminated gasket alone may not be a viable solution for a pre-filled syringe that houses product which is sensitive to certain gases. Moreover, such syringe and gasket systems have inferior CCI.

In some embodiments, the disclosure of this application provides gaskets with non-continuous channels for use in matched syringe-plunger systems. A plurality of non-continuous channels may be included in or in some embodiments through a film residing on at least a part of a circumferential outer surface portion of the gasket. The plurality of non-continuous channels of some of the embodiments also include non-channel portions disposed around the circumferential outer surface portion of the gasket. The non-continuous channels may be approximately parallel to each other with each channel including in some embodiment a non-channel portion disposed to be non-aligned with each other. The silicone oil-free syringe and gasket systems of some embodiments of this disclosure, preferably pre-filled plastic syringe systems, have superior container closure integrity (CCI), avoid high break loose forces and liquid/gas leakage, produce consistent delivery performance over time, provide protection of the enclosed product, minimize interaction of the gasket with the product, and maintain efficacy and sterility during the shelf life of the product, and have improved product shelf life. The syringe and gasket systems of some embodiments also produce reduced sub-visible particles and can protect complex or sensitive biologics contained within the syringe from silicone oil-induced aggregation and particulate formation.

In some embodiments, the disclosure also provides a process for producing silicone oil free syringe and gasket systems that has fewer than 300 particles of 2 micron size or more, measured using light obscuration (LO) or microflow imaging (MFI). Further, in some embodiments, the syringe system of the present disclosure incorporates a process of improving the sealability provided by the built-in lubrication film on a gasket that eliminates the need to use a lubricated syringe barrel. In other embodiments, the present disclosure provides tight dimensional control of the gasket and corresponding syringe and channels, thereby enabling a highly consistent compression of the assembled syringe and gasket system optimized for container closure integrity and plunger forces.

In some embodiments, the gasket may comprise:

(a) a main body made of an elastic material and having a circumferential surface portion and an internal cavity, the

cavity being defined by an inner surface portion of the gasket and being open-ended at one end;

(b) a film residing on at least a part of a circumferential outer portion of the gasket; and

(c) a plurality of non-continuous channels in or through the film being approximately parallel to the other non-continuous channels, each non-continuous channel of the plurality of non-continuous channels extending around the circumferential outer surface of the gasket and having a non-channel portion interrupting the non-continuous channel,

wherein the non-channel portion of each non-continuous channel is positioned along the circumferential outer surface portion of the gasket such that it is not aligned with the non-channel portion of the immediately adjacent one or more non-continuous channels.

The gasket may also have one or more of the following characteristics:

(i) maintaining container closure integrity (CCI) over a two-year shelf life as measured by one or more of a liquid migration and a helium leak detection test method;

(ii) when assembled within a matched syringe and plunger system a container closure integrity (CCI) with a defect rate of no more than 6-sigma;

(iii) a break loose force between 4 and 20 Newtons (N), alternatively 4 and 10 Newtons (N), alternatively 4 and 8 Newtons (N) when assembled within a matched syringe and plunger system;

(iv) a glide force between 4 and 20 Newtons (N), alternatively 4 and 10 Newtons (N), alternatively 4 and 8 Newtons (N) when assembled within a matched syringe and plunger system; and

(v) wherein the break loose force or glide force changes less than about 10-30% over two-years of storage life.

In some embodiments of the present disclosure, the gasket comprises two materials: a bromobutyl rubber base gasket and a film, preferably a PTFE film, that resides on the outside surface. Examples of a bromobutyl rubber include: Sumitomo LAG 5010-50 and West 4023. The PTFE film in preferred embodiments substantially covers the outer surface of the gasket. Gasket manufacturing comprises the following processes, which pertain to some embodiments of this disclosure:

(a) Molding: The PTFE film is treated to promote adhesion with the bromobutyl rubber of the gasket. A typical treatment is corona treatment. In some embodiments, chemical treatments may also be used. The PTFE film is placed into a multi-cavity gasket mold. Bromobutyl rubber is poured/injected into the multi-cavity mold. The mold is closed, the PTFE film and bromobutyl rubber are formed into the gasket. The mold opens, and the gaskets are removed from the mold. The gaskets thus produced have a substantially uniform wall thickness and comprise rubber and PTFE. The gaskets are trimmed via die cutting to remove the excess material. In some embodiments, the multi-cavity mold produces gaskets that are not threaded within the internal cavity.

(b) Laser-cut the PTFE or other film: The process of the disclosure comprises the following steps: (1) inserting a portion of one end of a mandrel into the open end of the gasket cavity of the gaskets manufactured in step (a); (2) positioning the mandrel and gasket in proximity to a laser; and (3) applying a laser beam emitted from a precision laser to one or more selected locations on a surface portion of the film residing on the circumferential outer surface portion of the gasket while rotating the mandrel and the gasket along the mandrel's longitudinal axis to form plurality of non-continuous channels in the film, the channels extending partially around the circumferential outer surface of the gasket. This process produces plurality of non-continuous channels in the PTFE or other film circumferentially on the outer surface of the gasket. The precision of the channels produced by the laser beam is directly related to the securing of the gasket on the mandrel, the position of the laser beam, and the dimensional tolerance of gaskets used in the process.

The resultant channels create a physical separation in the PTFE or other films on the gasket. In particular, without being bound by theory, it is believed that the laser treatment melts the PTFE or other film, and pushes the PTFE material to either side of the channel. During the laser treatment, the PTFE or other film material is 'piled' on either side of the channel creating two sealing ribs or peaks (micro projections). The PTFE or other film sealing ribs on either side of the channel are capable of maintaining CCI - both a liquid barrier and a sterile barrier. Assuming the PTFE film thickness is uniform and 'defect free', the height and angle of the sealing ribs are dependent on the alignment and position control of the laser beam

(relative to the rotating gasket on the mandrel).

The following reference characters are used in Figures 55 to 60, which show embodiments of a lubricious gasket according to the present disclosure.

| | |
|---|---|
| 14 | Gasket |
| 16 | Film |
| 18 | Gasket core |
| 20 | First non-continuous channel |
| 20a | First non-channel portion |
| 21 | Second non-continuous channel |
| 21a | Second non-channel portion |
| 22 | First lip of channel |
| 23 | Third non-continuous channel |
| 23a | Third non-channel portion |
| 24 | Second lip of channel |

A syringe of the present disclosure includes a hollow cylindrical syringe barrel, a plunger combined with the syringe barrel and reciprocally movable in the syringe barrel, and a gasket 14 attached to a distal end of the plunger 26.

As used in this subsection, the term "gasket" in the context of the present disclosure is a shaped piece or ring made of an elastomeric material that can be used to mechanically seal the space between two opposing inner surfaces of a syringe barrel. The gasket has a circumferential surface portion to be kept in substantially gas-tight and liquid-tight contact with an inner peripheral surface of the syringe barrel. A gasket of the present disclosure may be a gasket comprising a main body made of an elastic material and a film residing on at least a circumferential surface of the main body, the gasket having a circumferential surface portion and an internal cavity (IC) in its center, the cavity being defined by the inner surface of the gasket and being open at one end. In some embodiments, the internal cavity of the gasket is not threaded.

The "elastic material" may be rubber or an elastomer. Particularly, preferred types of rubber include butyl rubbers, chlorinated butyl rubbers and brominated butyl rubbers. Other types of elastic material may include thermosetting rubbers and dynamically cross-linkable thermoplastic elastomers having crosslinking sites are which make them heat-resistant. These polymer components of such elastomers include ethylene - propylene - diene rubbers and butadiene rubbers.

As used in this subsection, the term "film" is a material residing on at least a circumferential outer surface portion of the main body of the gasket. Preferably, it coats or resides on substantially all of the outer surfaces of the gasket. The film may have an optional thickness of under about 100 micrometers (μm or microns), optionally from about 10-30 microns, about 15-35 microns, or about 20-50 microns. Most preferably, the film is about 20 microns in thickness. A variety of different materials may be employed for the film, such as, for example, an inert fluoropolymer, including, fluorinated ethylene propylene (FEP), ethylene tetrafluoroethylene (ETFE), polytetrafluoroethylene (PTFE), ethylene perfluoroethylenepropylene (EFEP), ethylene chlorotrifluoroethylene (ECTFE), Polychlorotrifluoroethene (PCTFE), perfluoroalkoxy (PFA), among other coatings. Preferably, the film is an ultrahigh molecular weight polyethylene film (UHMWPE) or a fluoropolymer film. Fluoropolymer films such as polytetrafluoroethylene (PTFE) are preferred because of their excellent slidability and chemical stability. The type of the film to be provided on the surface of the main body of the gasket is not particularly limited, as long as the film is capable of preventing migration of substances from the crosslinked rubber (main body) and has a slidability, i.e., a smaller friction coefficient, as compared to the main body of the gasket.

Optionally, the film may comprise CPT fluoropolymer. CPT is a modified perfluoroalkoxy (PFA) that generally comprises the addition of PCTFE side chains to a PFA main chain during polymerization.

Optionally, additives may also be added to the film material for the film, such as additives that may improve the adhesion of the film to the underlying portion of the gasket to make a liquid sealing section and/or decrease the friction between that section and the sidewall of the syringe barrel. Additionally, according to certain embodiments, an adhesion promoting coating or process may be employed, such as, for example, a corona treatment or a chemical treatment. Corona treatment or air plasma is a surface modification technique that uses a low temperature corona discharge plasma to impart changes in the properties of a surface. The corona plasma is generated by the application of high voltage to an electrode that has a sharp tip. For some applications, it may be desirable to coextrude different materials to form the film. For example, coextruded film combinations may include a cyclic olefin copolymer (COC) with Aclar, Polyethylene (PE) with

Aclar and FEP with PE, among other combinations.

As used in this subsection, the term "mandrel" refers to a device or tool which may be attached at its distal end to a base that keeps the body of the mandrel steady and secured but one that allows the mandrel to rotate along its longitudinal axis. The proximal portion of the mandrel has a shape similar to the male portion of a two-part mold, which can be inserted and secured within the internal cavity (the corresponding female portion) of a gasket. In some embodiments, the mandrel is a shaped bar of metal or steel, such as a cylindrical rod. The proximal end of the mandrel may be continuous with the body of the mandrel or may have a smaller or larger circumferential portion than distal sections of the mandrel. In preferred embodiments, the proximal end of the mandrel is secured to the gasket using "press-fit assembly" in which is the gasket is secured to the mandrel by friction after the parts are pushed together, rather than by any other means of fastening (such as screwing). In some embodiments, the diameter of at least a part of the mandrel portion that is inserted into the internal cavity of the gasket is greater than the inner diameter of the cavity.

As used in this subsection, the term "channel" refers to a cut in the film residing on the surface of the gasket by the laser cut. The term channel may be used interchangeably with the term "cut." In the present disclosure, the term "cut" may also refer to the process of using one or more laser beams to create a nick or separation of the film residing on at least a circumferential outer surface portion of a gasket. In some embodiments, the channel is cut in the surface portion of the film. In more preferred embodiments, the channel extends through the film into the outer surface of the gasket. One or more such channels can be produced, each encircling a part of the gasket. Each channel has a non-channel portion where the channel/cut is not formed. For example, the channel can encircle 350 degrees of the gasket and the non-channel portion can encircle the remaining 10 degrees of a 360 degree circle on the gasket. When more than one channel is present, they are preferably axially spaced from one another. The non-channel portions of the more than one channels are not aligned on the gasket. For example, the non-channel portion can be disposed on one side, and a second non-channel portion can be disposed on another side of the gasket. Each channel has two lips. The term "lip" refers to the structure created due to the pile-up of film material along either side of the channel that is created by the laser beam cut. The channel lips 22 and 24 are shown in Figure 60 for example. Each lip is a raised rib positioned to seal against the barrel’s inner surface. Thus, each channel has two lips comprising two sealing ribs or peaks. In the present disclosure, the terms "lip", "rib" "peak" and "micro projections" are interchangeable.

The laser cut and the resulting channels are characterized by various dimensions, including laser-cut depth, radial depth, peak width, axial width, and peak height. The "laser-cut depth" is measured from the surface of the uncut gasket film down to the lowest point in the trough of the channel. The laser-cut depth for the one or more channels is independently selected from the following ranges: 30-60 microns, 40-50 microns, 50-60 microns, 40-45 microns, 45-50 microns, 50-55 microns and 55-60 microns. The "radial depth" is measured from the uncut outer surface of the gasket up to the lowest trough in the channel. The radial depth for the one or more channels that may be independently selected from the following ranges: 0 to 100 microns, 5 to 50 microns, 10 to 30 microns, and 15 to 25 microns. The "peak width" is the distance between two peaks of two lips on either side of a channel. Peak width is measured from the top of the peaks. The peak width may be one of the following ranges: 200-1,000 micron, 275-550 microns, 300-400 microns, and 450-500 microns.

The circumferential non-continuous channel of the present disclosure has axially opposed "first and second side walls" and a "floor." The floor of the channel may be either a film surface or, more preferably a gasket surface, depending on the thickness of the film and the depth of the cut. The "axial width" is measured from the first side wall to the second side wall of the channel across the breadth of the channel floor. In other words, the "axial width" is measured from one end of a channel to the other end of the channel across its breadth at the baseline level, i.e., at the laser uncut outer surface level of the film or gasket. The one or more channel independently has an axial width between the side walls of one of the following ranges: 1 to 100 microns, 5 to 50 microns, 10 to 30 microns, and 15 to 25 microns.

The "peak height" is measured from the surface of the uncut gasket film up to the highest peak of the lip created by the laser beam along the central axis of the peak, i.e., perpendicular to the surface of the film. The peak height of the lip on one or more of the channels is independently selected from one of the following ranges: 10-100 microns, 15-60 microns, 20-50 microns, and 30-40 microns.

As used in this subsection, the term "Container closure integrity" or "CCI" refers to the ability of a container closure system, e.g., a plunger attached to a gasket disposed in a syringe barrel, preferably a pre-filled syringe barrel, to provide protection and maintain efficacy and sterility during the shelf life of a sterile product contained in the container. In some embodiments, the container closure integrity is related to the sealability of a syringe system of the present disclosure. The one or more channels created by the laser in the film is intended to enhance the CCI of the plunger attached the gasket when assembled into a pre-filled syringe, by providing a physical break in the film that prevents defects in the film (such as delamination, tearing, or wrinkling) from adversely affecting the seal integrity between the gasket and the syringe. Container Closure Integrity (CCI) must be substantially maintained throughout the shelf life of a syringe of the present disclosure. CCI is an important characteristic of a pre-filled syringe for parenteral drug products contained within the syringe. One important element of CCI is maintaining a sterile barrier. The improved process of the present disclosure for producing one or more channels on a film reduces the likelihood of a CCI failure (breach of sterility), and/or facilitates a

longer shelf life.

As used herein, the term "break loose force" refers to the force required to initiate movement of the plunger attached to a gasket in a syringe, for example in a pre-filled syringe. It is the maximum force required to break the static friction of the gasket attached to a plunger. Break loose force is synonymous with "plunger force", "plunger breakout force", "breakout force", "initiation force" and "Fi" in the context of the present disclosure.

As used herein, the term "glide force" refers to the force required to maintain plunger movement (when the plunger is attached to a gasket of the present disclosure) in a syringe barrel once static friction has been overcome, e.g., during aspiration or dispense. Glide force is synonymous with "pushing force", "plunger sliding force", "maintenance force", and "Fm" in the context of the present disclosure.

As used herein, the terms "break loose force" "glide force", are collectively referred to as "BLGF forces", i.e., the various forces of the plunger and attached gasket of the present disclosure. The BLGF forces can be measured using any well-known test in the art, such as ISO 7886-1:1993. For example, the BLGF forces can be tested by filling a syringe of the disclosure with 1ml of a liquid (such as water) and thereafter vacuum loading the stopper. The plunger force can be tested with a plastic threaded (or unthreaded) rod at 300 mm/min. In the present disclosure, the improved process of producing channels on the surface of gaskets prevents plunger force aging (i.e., an increase in break loose force over time). A matched syringe-plunger system of the present disclosure maintains a break loose force and a glide force of between 4 and 20 Newtons (N), optionally from about 4 and 10 Newtons (N), or about 4 and 8 Newtons (N). Most preferably, the glide force is about 4 and 8 Newtons (N) and that changes less than about 10%-30% over a two-year storage life. The process of the present disclosure provides consistent break loose and glide forces by incorporating manufacturing process control and 100% inspection systems.

Figure 55 shows the inner diameter (ID) of the barrel and the outer diameter (OD) of the gasket 14 that are matched to be within a predetermined tolerance between them, and also showing a film 16 on the outer surface of a gasket core 18, a first non-continuous channel 20 extending around the circumferential outer surface of the gasket core 18 and a second non-continuous channel 21 extending around the circumferential outer surface of the gasket core 18 and approximately parallel to first non-continuous channel 20. The channel has lips 22 and 24, as shown in Figure 57.

Figure 56 shows a schematic sectional view taken along section lines 3A-3A of Figure 55, showing the gasket core 18 within internal cavity (IC), film 16, and first non-continuous channel 20 in the surface of the film (in some embodiments channel 20 extends through the film into the outer surface of the gasket (not shown)) and second non-continuous channel 21 in the surface of the film (in some embodiments channel 21 extends through the film into the outer surface of the gasket (not shown)) and approximately parallel to first non-continuous channel 20. Figure 57 shows a fragmentary detail view of the structure of Figure 56, showing one embodiment of each of the first non-continuous channel 20 and the first non-continuous channel 21 extending around the circumferential outer surface of the gasket core 18 and approximately parallel to first non-continuous channel 20 and lips 22 and 24 on the respective sides of the channel 20.

Figure 58 shows a top view of the gasket 14 and the approximate geometric distribution of the first non-continuous channel 20 extending around the circumferential outer surface of the gasket core 18 and the approximate geometric distribution of the second non-continuous channel 21 extending around the circumferential outer surface of the gasket core 18. Each of the non-continuous layers includes a non-channel portion (21a, 20a) associated with the non-continuous layers. The non-channel portions (21a, 20a) are not aligned with each other on the circumferential outer surface portion of the gasket.

The top view is intended to be illustrative of the position of the non-channel portion for each non-continuous channel of the plurality of non-continuous channels. Referring to Figure 58, the non-channel portion of the first non-continuous channel is not aligned with the non-channel portion of the second non-continuous channel. Although Figure 58 illustrates the non-channel portion of the first non-continuous channel is positioned 180 degrees from the non-channel portion of the adjacent non-continuous channel around the circumference of the gasket, the non-channel portions may be positioned around the circumference of the gasket as long as the adjacent non-channel portions are not aligned, for example, the non-channel portion of the first non-continuous channel may be positioned in a range of 90 to 270 degrees from the adjacent non-channel portion around the circumference of the gasket, with the adjacent non-channel portions not aligned. In a preferred embodiment, the non-channel portion of the first non-continuous channel may be positioned approximately 180 degrees from the adjacent non-channel portion around the circumference of the gasket.

In another embodiment, each non-continuous channel may include a plurality of non-channel portions arranged around the circumference such that the non-continuous channel may be a "dashed line" of channel and non-channel portions. An adjacent non-continuous channel may also be a "dashed line" channel of channel and non-channel portions. The channel and non-channel portions of adjacent non-continuous channels may be positioned around the circumference of the gasket as long as the adjacent non-channel portions are not aligned. For example, the non-channel portions may be out of phase with adjacent non-channel portions.

Figure 59 shows a top view of the gasket 14 and the approximate geometric distribution of three non-continuous channels 20, 21, 23 extending around the circumferential outer surface of the gasket core 18. The three non-continuous channels 20, 21, 23 are axially spaced from each other and are arranged such that non-continuous channel 20 is proximal

to the top of the plunger relative to the other two non-continuous channels 21 and 23, non-continuous channel 21 is adjacent to non-continuous channel 20 and distal to the top of the plunger relative to non-continuous channel 20, and non-continuous channel 23 is adjacent to non-continuous channel 21 and distal to the top of the plunger relative to non-continuous channel 21. Each of the non-continuous channels 20, 21, 23 includes a non-channel portion (20a, 21a, 23a) associated with the non-continuous channels 20, 21, 23. The non-channel portions (20a, 21a, 23a) are positioned such that they are not aligned with the non-channel portion of the adjacent non-continuous channels along the circumferential outer surface portion of the gasket.

The top view is intended to be illustrative of the position of the non-channel portion for each non-continuous channel 20, 21, 23 of the plurality of non-continuous channels. Each of the channels extends around the circumferential surface of the gasket and each having a portion that does not include a channel. The three non-continuous channels 20, 21, 23 are axially spaced from each other and are arranged such that non-continuous channel 20 is proximal to the top of the plunger relative to the other two non-continuous channels 21 and 23, non-continuous channel 21 is adjacent to non-continuous channel 20 and distal to the top of the plunger relative to non-continuous channel 20, and non-continuous channel 23 is adjacent to non-continuous channel 21 and distal to the top of the plunger relative to non-continuous channel 21. Referring to Figure 59, the non-channel portions (20a, 21a, 23a) are positioned such that they are not aligned with the non-channel portion of the adjacent non-continuous channels along the circumferential outer surface portion of the gasket. In some embodiments, the non-channel portion of one non-continuous channel may be aligned with the non-channel portion of a non-adjacent non-continuous channel.

Figure 60 shows a fragmentary detail view of an embodiment of a first non-continuous channel 20 in the surface of the film with lips 22 and 24 on the respective sides of the first non-continuous channel 20 and the various dimensions of the first non-continuous channel 20 and lips 22 and 24 (peak width, axial width, laser-cut depth and radial depth). In other, more preferred embodiments, first non-continuous channel 20 extends into the outer surface of the gasket (not shown).

In some embodiments, moreover, the syringe and plunger may be matched so that the syringe barrel has a wall including an inner surface defining a generally cylindrical lumen, the barrel having an inner diameter; the gasket has a leading face, a side surface, a trailing portion, and an outer diameter; the gasket is configured to be received within the barrel with the gasket outer diameter located within and movable with respect to the barrel inner diameter; and the barrel and gasket of the system are respectively sized to provide spacing between the smallest barrel inner diameter and largest gasket outer diameter, when assembled, deviating from the nominal spacing by no more than ± 100 microns, ± 50 microns, ± 35 microns, ± 25 microns, ± 20 microns, ± 15 microns, ± 10 microns, ± 5 microns or ± 2 microns.

Plunger anti-backout feature

In some embodiments of the present disclosure, the syringe 500 may be configured to withstand cold-chain drug storage conditions without the plunger 509 moving axially rearward, which may break CCI. For instance, embodiments of the syringes 500 of the present disclosure may comprise a plunger anti-backout feature 520.

A pre-filled syringe 500 is filled with a liquid drug and is stoppered with a plunger 509. There is typically a small air bubble between the liquid and the plunger. When the filled syringe is subjected to temperature below freezing, as is typically the case for cold-chain drugs, including DNA-based and RNA-based vaccines, the liquid in the lumen of the syringe expands. This expansion can cause the plunger to move axially rearward, e.g. such that the plunger may extend farther (by a small distance) out of the back end of the syringe barrel. This axially rearward movement of the plunger may be referred to as "plunger backout."

Movement of the plunger 509 axially rearward can cause the plunger to move into a non-sterile space, and the subsequent return of the plunger to its original position may create a pathway for microbial/bacterial ingress, i.e. a break in the CCI of the syringe. For purposes of maintaining container closure integrity (CCI), therefore, it is important to prevent the plunger 509 from moving rearward during the life cycle of the pre-filled syringe 500, which often includes storage at temperatures such as -20 °C, -70°C, or the like. Movement of the plunger axially rearward may also be a concern if the syringe undergoes significant changes in air pressure, such as during unpressurized air shipment or when transported between high and low altitude locations.

To test whether or not a plunger 509 will move axially rearward during the life cycle of a cold-chain drug, a syringe 500 may be subjected to freeze-thaw cycles such as those described herein. Embodiments of the syringes 500 of the present disclosure may be configured so that the plunger 509 does not move axially when the package or syringe is cycled between -20 °C and 10 °C, optionally when cycled between -20 °C and 20 °C, optionally when cycled between -20 °C and 30 °C, optionally when cycled between -20 °C and 40 °C, optionally when cycled between - 40 °C and 10 °C, optionally when cycled between -40 °C and 20 °C, optionally when cycled between -40 °C and 30 °C, optionally when cycled between -40 °C and 40 °C, optionally when cycled between -70 °C and 10 °C, optionally when cycled between -70 °C and 20 °C, optionally when cycled between -70 °C and 30 °C, optionally when cycled between -70 °C and 40 °C. Movement of the plunger can be determined by precise measurement of the distance between, e.g., the back end (or other feature or marking) of the plunger and the back end of the syringe.

In some embodiments of the present disclosure, the syringe 500 may comprise a plunger anti-backout feature 520. For example, the syringe 500 may be configured with an anti-backout feature 520 which is movable between a locked position, in which the plunger 509 is prevented from moving axially (either in a single direction, i.e. rearward, or in both rear and forward directions), and an unlocked position, in which the plunger 509 is able to move axially. An example of such a plunger anti-backout feature 520 is shown in Figure 64.

As shown in the embodiment illustrated in Figure 64, the syringe 500 comprises a finger flange 521 at its back end. The finger flange 521 may be a separate element that is attachable to the syringe barrel, as illustrated. In other embodiments, the finger flange 521 may be integral with the syringe barrel. The plunger rod 510 may pass through a central aperture of the finger flange and be rotatable therein. By rotation of the plunger rod 510 within the central aperture of the finger flange 521, the plunger 509 may be moved between a locked position and an unlocked position. In the locked position, for instance, the plunger rod 510 may be rotated into locked engagement with a portion of the finger flange 521.

In the illustrated embodiment, the plunger rod 510 comprises a backstop engagement feature 523, e.g. one or more radially extending elements, that are configured to fit through the central aperture 522 of the finger flange 521 if the plunger rod is rotated to an unlocked position (such that the one or more radially extending elements are aligned with portions of the aperture). The finger flange 521 may comprise one or more backstops 524, which, when the plunger rod 510 is rotated to a locked position, abut against the one or more radially extending elements 523 of the plunger rod, thereby preveting the plunger 509 from moving radially rearward. For instance, once the plunger rod 510 is inserted into the central aperture 522 of the finger flange 521, the plunger rod may be rotated so that at least one of the one or more radially extending elements 523 of the plunger rod may be aligned with and located forward of (i.e. closer to the front of the syringe than) the one or more backstops 524. Axially rearward movement of the plunger 509 may thus be prevented by the abutment of the radially extending element 523 of the plunger rod with the backstop 524.

Another embodiment of an anti-backout feature 520 is shown in Figures 65 through 67. In this embodiment, the system is not moved between locked and unlocked positions, but does use the interaction between a backstop 524 and a backstop engagement feature 523, e.g. one or more radially protruding elements, on the plunger rod 510.

As shown in Figure 65, a backstop element 524, e.g. one comprising a finger flange 521, is positioned about the rear flange 508 of the syringe barrel 501. While the finger flange 521 is an optional feature, it has been found useful in controlling the insertion of the plunger rod 510 during injection. As illustrated, the backstop element 524 may be a separate element that is attachable to the syringe barrel 501. In other embodiments, however, the backstop element 524 may be integral with the syringe barrel.

The plunger rod 510 comprises a backstop engagement feature 523. As illustrated, the backstop engagement feature 523 may comprise a radially-projecting, continuous ring. In other embodiments, however, the ring may not be continuous but may instead comprise one or more gaps. In some embodiments, for instance, rather than a continuous ring, the backstop engagement feature 523 may comprise a discontinuous ring made up of multiple portions of a ring separated from one another by gaps. Moreover, though the backstop engagement feature 523 is shown in Figure 67 as having an outer surface 525 that is parallel with the plunger rod 510, in other embodiments, the outer surface of the backstop engagement feature may be angled downward, such that the thickest portion of the projection is at the top (e.g. rear) and the thinnest portion is at the bottom (e.g. front). Such a wedge-shape would help facilitate movement of the backstop engagement feature 523 through the aperture 522 of the backstop element 524 during insertion of the plunger rod 510 into the syringe barrel 501.

The backstop element 524 comprises an aperture 522 which is aligned with the lumen 212 of the syringe barrel 501. As illustrated in Figure 67, the aperture 522 is defined by an interior wall 526 that is angled inward (moving toward the front of the syringe barrel 501). This allows the backstop engagement feature 523 to be pushed fully through the aperture 522 as the plunger rod 510 is inserted into the lumen 212 of the syringe barrel 501. In other embodiments, however, such as where the backstop engagement feature 523 is angled, the interior wall 526 that defines the aperture 522 may be parallel or substantially parallel with the longitudinal axis of the syringe barrel 501.

Once the plunger rod 510 is inserted to its stop position and the backstop engagement feature 523 has been pushed through the aperture 522, the lower edge of the interior wall 526 of the backstop element 524 acts to prevent the backstop engagement feature of the plunger rod from entering the aperture. In particular, a contact surface 527 of the backstop element 524 contacts the backstop engagement feature 523 of the plunger rod 510 and prevents rearward movement of the plunger rod. As shown in the illustrated embodiment, and in particular Figure 67, the contact surface 527 may be an underside (or lower surface) of the backstop 524 positioned adjacent the bottom of the central aperture 522.

The backstop engagement feature 523 may be positioned at a location along the length of the plunger rod 510 that places in close proximity to (immediately below) the contact surface 527 of the backstop 524 when the plunger 509 is in its stop position within the syringe barrel 501. For instance, the plunger rod 510 may be customized so that the backstop engagement feature 523 is positioned at a location along the length of the plunger rod that is immediately adjacent the contact surface 527 of the backstop 524, e.g. within about 1.5 mm, alternatively within about 1.0 mm, alternatively within about 0.75 mm, alternatively within about 0.5 mm, alternatively within about 0.25 mm, when the plunger is at a stop position that is determined and defined by the fill volume of a specific pre-filled syringe assembly. Put another way, the location of

the backstop engagement feature 523 along the length of the plunger rod 510 may be coordinated with the plunger 509 insertion depth in the syringe barrel 501 that corresponds to the specific fill volume of a filled and fully assembled syringe 500. In this manner, the plunger rod 510 may be customized for a particular pre-filled syringe assembly 500.

Pre-filled syringes 500, including for instance those containing cold chain drugs, may have precise fill volumes which require that the plunger 509 insertion depth be within tight tolerances. This, in turn, may require that the backstop engagement feature 523 be positioned adjacent the contact surface 527 of the backstop 524 within tight tolerances (i.e. have little variance from unit to unit). Accordingly, in some embodiments, the backstop engagement feature 523 may repeatably and consistently be provided at a precise location relative to the backstop contact surface 527 (and which is coordinated with the fill volume of a pre-filled syringe assembly), e.g. at a position that varies by less than 1 mm, alternatively at a position that varies by less than 0.75 mm, alternatively at a position that varies by less than 0.5 mm, alternatively at a position that varies by less than 0.25 mm from unit to unit. By ensuring that the backstop engagement feature 523 is consistently within a tight tolerance of the backstop contact surface 527 when the plunger 509 is at its stop position for the fill volume of a particular syringe assembly 500, embodiments of the present disclosure consistently (i.e. across substantially all units) prevent the plunger from moving into unsterile regions of the syringe barrel 501 during cold storage while also ensuring that the headspace between the liquid and the plunger is maintained within tight tolerances throughout the cold chain cycle.

Another embodiment of an anti-backout 520 feature is shown in Figures 68 through 75. This embodiment provides the additional benefits of preventing movement of the plunger 509 bidirectionally, i.e. in both rear and forward directions. This embodiment also comprises components (including a plunger rod 510) that may be used in pre-filled syringes 500 having varying fill volumes (and thus varying plunger insertion depths) while consistently preventing the plunger 509 from moving into unsterile regions of the syringe barrel 501 during cold storage. In other words, no customization of parts for a particular drug formulation is necessary. Rather, this embodiment provides for the precise positioning of the plunger rod 510 before it is locked in place. Relatedly, this embodiment also does not require any backstop engagement feature 523 to be present on the plunger rod 510. Further, this embodiment provides a user-friendly mechanism by which a health care provider can easily move the plunger 509 between locked and unlocked positions, e.g. to unlock the plunger prior to injection.

As shown in Figure 68, this embodiment comprises a syringe barrel 501, a plunger rod 510, a backstop element 524 comprising a locking collet 528, a threaded housing element 529, and a twist lock thumb nut 530. As shown in Figure 69, the plunger rod 510 may be a conventional plunger rod that does not contain any specific backstop engagement features.

An example of a backstop element according to this embodiment is shown in Figures 70 to 72. As shown in the illustrated embodiment, the backstop element 524 comprising a finger flange 521 may be positioned about the rear flange 508 of the syringe barrel 501. While the finger flange 521 is an optional feature, it has been found useful in controlling the insertion of the plunger 509 during injection. As illustrated, the backstop element may be a separate element that is attachable to the syringe barrel. For example, the backstop element 524 may be made of a polymeric material, such as polypropylene, and molded in a traditional injection molding process. In other embodiments, however, the backstop element 524 may be integral with the syringe barrel.

As shown in Figures 70 to 72, the backstop element 524 comprises an aperture 522 which is aligned with the lumen 212 of the syringe barrel 501 and which is sized so that the plunger rod 510 may pass through the aperture. At least a portion of this aperture 522 is defined by a locking collet 528, which is flexible such that it may be compressed to a reduced diameter, by which the interior surface 531 of the locking collet may be pressed against a portion of the plunger rod 510 contained within the aperture to lock the plunger rod in place. In the illustrated embodiment, this locking collet 528 extends from the upper (or rearward) surface of the backstop element 524.

Optionally, as illustrated, an upper portion of the exterior surface of the locking collet 528 may be drafted, i.e. angled, outward such that the drafted portion 532 of the exterior surface has an increased diameter moving downward from the top of the locking collet. The drafted portion 532 of the locking collet 528 is configured so that the twist lock thumb nut 530 interfaces with the drafted portion to compress the locking collett inward and up against the plunger rod 510 as the twist lock thumb nut is brought into increased engagement with the threaded housing 529 (and thus into contact with the increased diameters present moving downward along the drafted portion, thus compressing the locking collet inward).

As illustrated, the locking collet 528 is divided into a plurality of sections, here four quadrants, by circumferential gaps, to provide a desired degree of flexibility. In other embodiments, the locking collett 528 may be divided into any number of sections by any number of gaps, so long as the locking collet maintains both sufficient flexibility to be pressed inward and sufficient surface area in contact with the plunger rod 510 to prevent movement of the plunger rod. In other embodiments, the locking collet 528 may be continuous (e.g. depending on the thickness of the material).

An example of the threaded housing 529 is shown in Figure 73. As illustrated, the threaded housing 529 comprises a central aperture 533 defined by an interior wall 534. The central aperture 533 is sized and configured to surround the locking collet 528 of the backstop. The interior wall comprises a threading - here shown as female threads (though male threads could be present instead). The threading is configured to engage threads on the twist lock thumb nut

530, which are shown in the illustrated embodiment as being male threads (but which could alternatively be female threads). The lower end of the threaded housing 529 is configured to engage with the backstop element 524 to secure the threaded housing in place. In the illustrated embodiment, for instance, the lower end of the threaded housing 529 comprises a male mating element that mates with the female mating element of the backstop 524 to provide a snap-on connection. In the illustrated embodiment, the lower end of the threaded housing 529 also comprises a male locating feature 535 that is inserted into the female cutout 536 on the backstop 524 and which prevents rotation of the threaded housing. As noted above, however, other mating mechanisms are also contemplated. The threaded housing 529 may be made of a polymeric material, such as polypropylene, and molded in a traditional injection molding process. In other embodiments, it is contemplated that the threaded housing 529 may be integral with the backstop element 524.

The backstop element 524 may also comprise a mating element that mates with the threaded housing 529 so as to secures the threaded housing in place. As illustrated, the backstop element 524 comprises a recess, or undercut, on its upper (or rearward) surface into which a male portion of the threaded housing may be inserted and secured, e.g. by a snap-on connection. The mating elements should be configured so that the threaded housing 529 does not rotate during operation of the backstop assembly. For instance, in the illustrated embodiment, the recess also comprises a female cutout 536 configured to align and mate with a male locating feature 535 on the threaded housing 529, thereby preventing rotation of the threaded housing. In alternative embodiments (not illustrated), the recess and/or female cutout 536 may be present on the threaded housing 529 and the male portion of the mating assembly and/or male locating feature 535 may be present on the backstop element 524. Other mating elements are also contemplated, so long as the threaded housing 529 is securable to the backstop element 524 and rotation of the threaded housing prevented. In other embodiments, it is also contemplated that the threaded housing 529 may be integral with the backstop element 524.

An example of the twist lock thumb nut 530 is shown in Figures 74A and 74B. The twist lock thumb nut 530 comprises a central aperture 537 through which the plunger rod 510 may pass. The twist lock thumb nut 530 also comprises a downward-extending portion comprising a wall 538, an exterior surface of which comprises threading - here shown as male threads - that is configured to engage with the threading on the interior wall 534 of the threaded housing 529. At least a portion of the central aperture 537 is defined by an interior surface of the downward-extending wall 538. Optionally, as shown in Figure 74B, the bottom edge of the wall 538 may be drafted, i.e. angled, outward such that the diameter of the central aperture 537 decreases moving upward from the bottom edge of the wall. The drafted portion 539 of the twist lock thumb nut 530 is configured to interface with the locking collet 528 to compress the locking collett inward and up against the plunger rod 510 as the exterior threading of the twist lock thumb nut is brought into increased engagement with the interior threading of the threaded housing 529 (bringing the decreased diameter interior surface of the twist lock thumb nut into contact with the locking collet to compress it inward).

The twist lock thumb nut 530 may also comprise a relatively large diameter gripping portion 540 that may easily be gripped and rotated by a user. In some embodiments, including the illustrated embodiment for example, the gripping portion 540 may comprise a plurality of ribs that provide an improved grip when twisting. Although not illustrated, the twist lock thumb nut 530, e.g. the upper surface of the twist lock thumb nut, may be provided with a visual indicator identifying a first direction of rotation corresponding to a locked position and a second direction of rotation corresponding to an unlocked position. The twist lock thumb nut 530 may be made of a polymeric material, such as polypropylene, and molded in a traditional injection molding process.

The interaction between these various elements that shown in Figure 75. As illustrated in Figure 75, the backstop element 524 comprising the locking collet 528 is affixed to the rear flange 508 of the syringe barrel 501. The threaded housing 529 is secured to the backstop element 524, e.g. by the rotation-preventing, snap-on connection. The exterior threads on the twist lock thumb nut 530 are brought into intial engagement with the interior threads of the threaded housing 539 to thereby secure the twist lock thumb nut in place. When assembled in this manner, the central apertures 212, 522, 533, 537 of the syringe barrel 501, the backstop element 524 and in particular the locking collet 528, the threaded housing 529, and the twist lock thumb nut 530 are aligned. The plunger rod 510 is inserted through the aligned apertures and to a desired stop position within the centraul lumen 212 of the syringe barrel 510. Once the plunger rod 510 has reached the desired stop position, the twist lock thumb nut 530 is rotated in the direction corresponding with a locked position. As the twist lock thumb nut 530 is rotated, the exterior threads of the twist lock thumb nut further engage the interior threads of the threaded housing 529 such that the bottom of the twist lock thumb nut travels farther into the threaded housing. As the bottom of the twist lock thumb nut 530 moves farther into the threaded housing 529 (i.e. downward), (i) the drafted portion 539 of the twist lock thumb nut 530 contacts the locking collet 528, (ii) the twist lock thumb nut contacts the drafted portion 532 of the locking collet, or (iii) the drafted portion of the twist lock thumb nut contacts the drafted portion of the locking collet (as illustrated), compressing the locking collet inward so that the interior surface 531 of the locking collet presses up against the plunger rod 510 and secures it in place, e.g. by an interference fit. When it becomes difficult or impossible to rotate the twist lock thumb nut 530 any farther, the assembly has been brought to a locked configuration in which the plunger rod 510 is prevented from axial movement. When in the locked position, the backstop assembly 520 prevents the plunger 509 from moving into unsterile regions of the syringe barrel 501 during cold storage. It also prevents any undesirable forward movement of the plunger 509.

In order to use the syringe, the twist lock thumb nut 530 may simply be rotated in the direction corresponding with an unlocked position. Rotation in that direction causes the bottom of the twist lock thumb nut 530 to travel toward the top of the threaded housing 529. As the bottom of the twist lock thumb nut 530 moves farther out of the threaded housing 529 (i.e. upward), (i) the drafted portion 539 of the twist lock thumb nut at least partially disengages the locking collet 528, (ii) the twist lock thumb nut at least partially disengages the drafted portion 532 of the locking collet, or (iii) the drafted portion of the twist lock thumb nut at least partially disengages the drafted portion of the locking collet (as illustrated), allowing the locking collet to return to its natural, or rest, position so that the interior surface 531 of the locking collet no longer presses up against the plunger rod 510 and the plunger rod is slidable within the assembly. Because most users may already be familiar with rotating a thumb nut 530 between tightened, i.e. locked, and loosened, i.e. unlocked, positions, the backstop assembly 520 is easily operable by health care professionals.

When in the unlocked position, the interior surface 531 of the locking collet 528 may still have some degree of contact with the plunger rod 510 or alternatively the interior surface of the locking collet may not contact the plunger rod. In some embodiments, it may be desirable that, when in the unlocked position, the backstop assembly (i.e. the combination of the backstop element 524 and in particular the locking collet 528, threaded housing 529, and twist lock thumb nut 530) produce no resistance or substantially no resistance to plunger 509 sliding beyond that produced by the syringe barrel 510 and plunger without the backstop assembly.

Another embodiment of an anti-backout feature 520 is shown in Figures 76 through 85. This embodiment provides a user-friendly mechanism by which a health care provider can easily move the plunger 509 between locked and unlocked positions, e.g. to unlock the plunger prior to injection. Further, if desired, this embodiment may provide the additional benefits of preventing movement of the plunger 509 bidirectionally, i.e. in both rear and forward directions.

Some embodiments of the anti-backout feature 520 shown in Figures 76 through 85 also comprise components (including a plunger rod 510) that may be used in pre-filled syringes 500 having varying fill volumes (and thus varying plunger insertion depths) while consistently preventing the plunger 509 from moving into unsterile regions of the syringe barrel 5015 during cold storage. In other words, no customization of parts for a particular drug formulation is necessary. Such an embodiment is shown in Figure 76 to 78, for example. Note that in the illustrated embodiment, the plunger rod 510 comprises a plurality of backstop engagement features 523 positioned at various locations along the length of the rod. In this way, without any need for customization, it is contemplated that one of the plurality of backstop engagement features 523 will be positioned at a location along the length of the plunger rod 510 that places it in close proximity to (e.g., immediately below) a contact surface 527 of the backstop 524 when the plunger 509 is in its stop position within the syringe barrel 501.

In other embodiments, however, the plunger rod 510 may be customized for a particular pre-filled syringe 500 assembly, such as described above with respect to the embodiment illustrated in Figures 65 through 67. In some embodiments, the plunger rod 510 may only comprise a single backstop engagement feature 523 to prevent undesired rearward movement of the plunger 509. That backstop engagement feature 523 may be precisely positioned at a location along the length of the plunger rod 510 that places below and in close proximity to (e.g., immediately below) a contact surface 527 of the backstop 524 when the plunger 509 is in a stop position within the syringe barrel 501 that is determined and defined by the fill volume of a specific pre-filled syringe assembly 500. For instance, the plunger rod 510 may be customized so that the backstop engagement feature 523 is positioned at a location along the length of the plunger rod that places it below and immediately adjacent the contact surface 527 of the backstop 524, e.g. within about 1.5 mm, alternatively within about 1.0 mm, alternatively within about 0.75 mm, alternatively within about 0.5 mm, alternatively within about 0.25 mm, when the plunger 509 is at a stop position that is determined and defined by the fill volume of a specific pre-filled syringe assembly 500. Put another way, the location of the backstop engagement feature 523 along the length of the plunger rod 510 may be coordinated with the plunger insertion depth in the syringe barrel that corresponds to the specific fill volume of a filled and fully assembled syringe. In this manner, the plunger rod 510 may be customized for a particular pre-filled syringe assembly 500.

In some embodiments, in addition to the backstop engagement feature 523 described in the previous paragraph, the plunger rod 410 may also comprise at least a second backstop engagement feature to prevent undesired forward movement of the plunger. That backstop engagement feature 523 may be precisely positioned at a location along the length of the plunger rod 510 that places it above and in close proximity to (e.g., immediately above) an upper contact surface of the backstop 524 when the plunger 509 is in a stop position within the syringe barrel 501 that is determined and defined by the fill volume of a specific pre-filled syringe assembly 500. For instance, the plunger rod 510 may be customized so that the backstop engagement feature 523 is positioned at a location along the length of the plunger rod that is immediately adjacent an upper contact surface of the backstop 524, e.g. within about 1.5 mm, alternatively within about 1.0 mm, alternatively within about 0.75 mm, alternatively within about 0.5 mm, alternatively within about 0.25 mm, when the plunger 509 is at a stop position that is determined and defined by the fill volume of a specific pre-filled syringe assembly 500.

In any case, the one or more backstop engagement features 523 may repeatably and consistently be provided at a precise location relative to the backstop contact surface 527 (and which is coordinated with the fill volume of a pre-filled

syringe assembly), e.g. at a position that varies by less than 1 mm, alternatively at a position that varies by less than 0.75 mm, alternatively at a position that varies by less than 0.5 mm, alternatively at a position that varies by less than 0.25 mm from unit to unit. By ensuring that the backstop engagement feature 523 is consistently within a tight tolerance of the backstop contact surface 527 when the plunger 509 is at its stop position for the fill volume of a particular syringe assembly 500, embodiments of the present disclosure consistently (i.e. across substantially all units) prevent the plunger from moving into unsterile regions of the syringe barrel 501 during cold storage while also ensuring that the headspace between the liquid and the plunger is maintained within tight tolerances throughout the cold chain cycle.

As shown in Figures 76 to 77, a backstop element 524, e.g. one comprising a finger flange 521, is positioned about the rear flange 508 of the syringe barrel 501. While the finger flange 521 is an optional feature, it has been found useful in controlling the insertion of the plunger 509 during injection. As illustrated, the backstop element 524 may be a separate element that is attachable to the syringe barrel 501. In other embodiments, however, the backstop element 524 may be integral with the syringe barrel 501.

As shown in Figures 76 to 78, the plunger rod 510 comprises at least one backstop engagement feature 523, and optionally a plurality of backstop engagement features. As illustrated, the backstop engagement feature 523 may comprise a radially-projecting, continuous ring. In other embodiments, however, the ring may not be continuous but may instead comprise one or more gaps. In some embodiments, for instance, rather than a continuous ring, the backstop engagement feature 523 may comprise a discontinuous ring made up of multiple portions of a ring separated from one another by gaps.

As shown in Figures 79 to 80, the backstop element 524 comprises an aperture 522 which is aligned with the lumen 212 of the syringe barrel 501 and thus referred to as a central aperture. The plunger rod 510 is inserted into the syringe barrel 501 through the central aperture 522 and the portion of the plunger rod comprising the backstop engagement feature 523 travels through the central aperture as the plunger rod is inserted into the lumen 212 of the syringe barrel 501 to its stop position. In contrast to some other embodiments, the central aperture 522 may not need to have a diameter that is close to the outer diameter of the plunger rod 510 or a backstop engagement feature 523 of the plunger rod. Instead, the central aperture 522 can be oversized, facilitating and potentially simplifying assembly of the pre-filled syringe 500.

The backstop element 524 also comprises a locking block cavity 541, which spans between a first side of the backstop element and a second side of the backstop element, is transverse to the central aperture 522, and crosses over the central aperture. The locking block cavity 541 is configured to receive a locking block 542. The locking block 542 is configured to slide within the locking block cavity 541 between a first unlocked position and a second, locked position. An embodiment of a locking block 542 is shown in Figure 81.

The locking block 542 comprises an aperture 543, which spans the locking block vertically, and which has two distinct portions: a larger cross-section portion 544 and a smaller cross-section portion 545. During operation, the locking block 542 slides within the locking block cavity 541 so that either the larger cross-section portion 544 of the aperture is aligned with the central aperture 522, as shown in Figures 84 to 85, or the smaller cross-section portion 545 of the aperture is aligned with the central aperture, as shown in Figures 82 to 83. When the larger cross-section portion 544 of the aperture is aligned with the central aperture 522, the plunger rod 510 including the backstop engagement feature(s) 523 may readily move through the locking block aperture 543. When the smaller cross-section portion 545 of the aperture is aligned with the central aperture 522, the backstop engagement feature(s) 523 on the plunger rod 510 may not move past a contact surface 527 of the locking block 542. With reference to Figure 82, for instance, the backstop engagement feature 523 that is positioned just below the locking block 542 abuts against an underside, or lower contact surface 527, of the locking block adjacent the smaller cross-section portion 545 of the aperture, preventing rearward movement of the plunger rod 510. Similarly, if present, the backstop engagement feature 523 that is positioned just above the locking block 542 abuts against an upperside, or top contact surface 547, of the locking block 542 adjacent the smaller cross-section portion 545 of the aperture, preventing forward movement of the plunger rod 510.

In some embodiments, including that illustrated for example, the smaller cross-section portion 545 of the locking block aperture may have a substantially circular cross-section and a radius of curvature that is substantially the same as the plunger rod 510. The larger cross-section portion 544 of the locking block aperture may have any of a variety of shapes, though shown as rectangular in the illustrated embodiment, so long as the plunger rod 510, including any backstop engagement features 523, may readily pass through it without interference from any surfaces of the locking block 542.

In some embodiments, the larger cross-section portion 544 and the smaller cross-section portion 545 may be separated from one another by at least one inward-extending rib 546. In the illustrated embodiment, for example, the larger cross-section portion 544 and the smaller cross-section portion 545 are separated from one another by two ribs 546, one extending inward from each side wall such that they directly oppose one another. The one or more ribs 546 may form the boundary between the larger cross-section portion 544 and the smaller cross-section portion 545. As shown, each rib 546 may comprise a first angled or curved surface facing the larger cross-section portion 544 of the aperture and a second angled or curved surface facing the smaller cross-section 545 portion of the aperture. The first angled or curved surface may facilitate movement of the rib surface over a portion of the plunger rod 510 when moving the locking block 542 from an

unlocked position to a locked position. The second angled or curved surface may facilitate movement of the rib surface over a portion of the plunger rod 510 when moving the locking block 542 from a locked position to an unlocked position. The second angled or curved surface may also be configured to correspond or substantially correspond to the curvature of the plunger rod 510 so as to form part of the lower 527 (and if application, upper 547) contact surface.

In other (non-illustrated) embodiments, the plunger rod 510 may not include any backstop engagement feature 523. Rather, when the locking block 542 is moved to the locked position, the smaller cross-section portion 545 of the aperture may be designed such that the surfaces of the locking block 542 that define that portion of the aperture press inward on the plunger rod 510 itself to create an interference fit and thereby prevent movement of the plunger 509 due to changes in pressure common to the cold-chain cycle. For instance, the dimensions of the smaller cross-section portion 545 of the aperture may be tightly aligned with the dimensions of the plunger rod 510 to create a sort of friction-fit which prevents plunger 509 movement.

As shown in the illustrated embodiment, the locking block 542 may have a first end that is marked to identify that pressing the first end surface 548 will bring the locking block to its unlocked position and a second end that is marked to identify that pressing the second end surface 549 will bring the locking block to its locked position. As illustrated, the markings may be molded directly into the end surfaces 548, 549 of the locking block 542, though alternative markings and means of providing those markings are contemplated.

The backstop assembly 520 may also comprise a retention mechanism that retains the locking bar 542 in either the locked position or the unlocked position until a user applies a sufficient force to overcome the retention mechanism.

For instance, the backstop element 524 may comprise one or more retention ribs 550 that extend into the locking block cavity 541 on a first side of the central aperture 522 and/or one or more retention ribs that extend into the locking block cavity on a second side of the central aperture. As shown in Figures 82 and 83, for example, a first retention rib 550 extends downward from an upper surface of the locking block cavity 541 and a second, opposing retention rib extends upward from a lower surface of the locking block cavity on a first side of the central aperture 522. Similarly, a first retention rib 550 extends downward from an upper surface of the locking block cavity 541 and a second, opposing retention rib extends upward from a lower surface of the locking block cavity on a second side of the central aperture 522. Though shown as present on the upper and lower surfaces that define the locking block cavity 541, ribs 550 could just as easily be placed on the opposing side surfaces.

The locking block 542 may one or more indents 551 that align with and receive one or more retention ribs 550 when the locking block is in its locked position and/or one or more indents that align with and receive one or more retention ribs when the locking block is in its unlocked position. For instance, a first indent or set of indents 551 may be located on a first side of the aperture 543 and a second indent or set of indents may be located on a second side of the aperture. As shown in Figure 81, each indent 551 may be a channel that runs continuously around the locking block 542. Alternatively, indents 551 such as but not limited to channels may be present only on opposing surfaces of the locking block 542 (e.g. on the top and bottom surfaces, though again it could just as easily be the side surfaces).

As shown in Figure 82, when the locking block 542 has been brought into a locked position, the retention ribs 550 on one side of the central aperture 522 are received by the indents 551 on one end of the locking block 542, thereby retaining the locking block in that position until a user presses the locking block in the "unlock" direction (shown here as being to the right) with enough force to overcome the interaction of the rib and indent. Similarly, as shown in Figure 84, when the locking block 542 has been brought into an unlocked position, the retention ribs 550 on the other side of the central aperture 522 are received by the indents 551 on the other end of the locking block, thereby retaining the locking block in that position until a user presses the locking block in the "lock" direction (shown here as being to the left) with enough force to overcome the interaction of the rib and indent. In other embodiments, the backstop assembly 520 may be configured so that a retention mechanism only operates to retain the locking block 542 in the locked position or so that retention mechanism only operates to retain the locking block in the unlocked position.

It is also contemplated that the retention ribs 550 may be present on an exterior surface of the locking block 542 and the indents 551 may be present on an interior surface that defines the locking block channel 541, i.e. the reverse of the above-described and illustrated embodiment.

An anti-backout feature 520 such as that shown in Figures 76 through 85 may be incredibly easy and natural for a user to operate, e.g. with a user's thumb while the user's fingers are stably on the finger flange 521, to move between locked and unlocked positions. Moreover, by including markings on the locking bar 542 (and/or on the backstop element 524), there may be little opportunity for confusion as to whether the assembly is in a locked or unlocked position.

In some embodiments, and using any of the mechanisms described herein, when the backstop assembly 520 is in the unlocked configuration, the plunger sliding force of a syringe bearing the backstop assembly may be the same or substantially the same as the plunger sliding force of the same syringe but without the backstop assembly. For instance, when the backstop assembly 520 is in the unlocked configuration, the plunger sliding force of a syringe bearing the backstop assembly may be within 20%, alternatively within 15%, alternatively within 10%, alternatively within 8%, alternatively within 5%, alternatively within 3% of the plunger sliding force of the same syringe but without the backstop assembly.

In some embodiments, and using any of the mechanisms described herein, when the backstop assembly 520 is in the unlocked configuration, the breakout force of a syringe bearing the backstop assembly may be the same or substantially the same as the breakout force of the same syringe but without the backstop assembly. For instance, when the backstop assembly 520 is in the unlocked configuration, the breakout force of a syringe bearing the backstop assembly may be within 20%, alternatively within 15%, alternatively within 10%, alternatively within 8%, alternatively within 5%, alternatively within 3% of the breakout force of the same syringe but without the backstop assembly.

The plunger anti-backout feature 520 of the present disclosure is not limited to the illustrated embodiments, and it is contemplated that the same may be achieved in a variety of different manners using the same or a similar concept to that disclosed herein.

**Maintenance of CCI during freeze-thaw cycling**

Many drugs, including for example many biologic drugs and vaccines, and in particular DNA-based and RNA-based vaccines, are highly sensitive to temperature and require storage and transportation under carefully controlled low temperatures, e.g. in refrigerators or freezers that in some cases provide extremely low temperatures. These drugs are classified as cold-chain drugs. In general, drug products storage conditions can be divided into the following categories: refrigerated (e.g., 2°C to 8°C), freezer (e.g., -25°C to -10°C), ultra-low freezer (e.g., -70°C to - 90°C), vapor-phase liquid nitrogen (e.g., -135°C to -196°C), and liquid-phase liquid nitrogen (e.g., -195°C). Typical freezers used for this purpose may include those that produce temperatures at or near -20 °C and those that produce temperatures at or near -70 °C. Storage of drug products at these extreme temperatures places significant stresses on the drug primary package.

These stresses are exacerbated by changes in the temperature of the package, which are common throughout the life cycle of a drug primary package such as a vial or pre-filled syringe. As illustrated in Figure 61, for instance, vials used cold storage applications are at risk of being exposed to multiple freeze-thaw cycles during the life cycle of each vial, from preconditioning, to fill/finish, to transport, to drug administration. Vials and pre-filled syringes are often stored at low temperatures and then brought up to room temperature for patient administration. Yet it is desirable that CCI is maintained throughout the entire life cycle of the package. Accordingly, embodiments of the present disclosure are directed to drug primary packages, and in particular vials and syringes, configured to maintain CCI throughout their entire life cycle, taking into account the temperature changes that will occur within the life cycle of a primary package for a cold-chain drug.

For vials 400, for instance, the materials that make up the vial and stopper 411 will expand and/or contract differently in response to changes in temperature, which can lead to gaps in the seal between the vial and the stopper through which environmental gases can enter the lumen and negatively impact the drug product and/or through which microbial/bacterial ingress may occur. Those gaps represent a break in the CCI of the filled vial. Further, glass vials have been known to crack or break as a result of the mechanical stresses that it undergoes at extreme low temperatures, including for example stresses caused by expansion of a liquid drug product within the lumen.

Pre-filled syringes 500 are subject to the same material stresses as vials during thermal cycling. The maintenance of CCI of a pre-filled syringe 500 is further complicated by the lumen 212 of the syringe barrel 501 having two openings, one of which is sealed by the plunger 509 and the other of which is sealed by, for instance, a rigid needle shield 511 in the case of a staked needle syringe or a luer cap in the case of a luer lock syringe. For a staked needle syringe, in particular, the rigid needle shield 511 may generally be made of an elastomeric material, which like the stopper of a vial, may expand and/or contract differently from the thermoplastic syringe barrel, leading to gaps in the seal. Similarly, the plunger 509 may comprise a gasket that is made from a very different material than the thermoplastic syringe barrel 501 and thus be subject to the same differences in expansion and/or contraction that can lead to breaks in the CCI of the pre-filled syringe. The presence of moving components also complicates the maintenance of CCI for a pre-filled syringe 500. As described in more detail above, for instance, expansion of the liquid contents of the lumen under cold temperatures can cause the plunger 509 to move axially rearward, which can create a break in the CCI of the pre-filled syringe.

As a way of testing whether a package will maintain CCI through the life cycle of a cold-chain drug, samples of the package may be subjected to a freeze-thaw study. In a freeze-thaw study, a vial or syringe is filled with high purity water (e.g. Milli-Q water) and sealed. For a vial 400, this typically involves a stopper 411 and, optionally, an aluminum crimp cap 412. For a syringe 500, this typically involves both a plunger 509 and either a rigid needle shield 511 or a luer cap. The filling and assembly typically takes place at room temperature. The filled and sealed packages are then placed in a freezer, which is configured to provide a specific lower temperature, e.g. about -20 °C, about -30 °C, about -40 °C, about -50 °C, about -60 °C, about - 70 °C, about -80 °C, about -90 °C, about -135°C, about -195 °C, etc. The lower temperature may be selected depending on the specific storage requirements for a particular drug for which the vial or syringe is to be used (though the selected temperature need not match up exactly with the storage requirement; rather in some instances, the CCI testing may be performed at a temperature that lower than the storage requirement for the drug).

The packages are held in the freezer for a defined period of time, known as a soak time. For example, the packages may be held in the freezer for 24 hours. After the soak time, the packages are removed from the freezer and placed in an environment that is maintained at the upper temperature, which is typically room temperature or a temperature

that is slightly elevated above room temperature. The packages are held at the upper temperature for a defined period of time, i.e. soak time, that is typically the same as the soak time at the lower temperature. For example, the packages may be held in the upper temperature environment for 24 hours. Once the package has been subjected to a soak time at both the lower and upper temperatures, a cycle is complete. The package may be subjected to any number of cycles. In some embodiments, for example, the package may be subjected to three or more cycles, optionally to three cycles.

Samples of the packages may be removed after each cycle and tested for CCI. CCI testing may be performed in a number of ways. For example, the most obvious loss of CCI would be one that can seen by a simple visual inspection, such as a breakage or crack, a displaced plunger, needle shield, or stopper, or something of that nature. Packages may also be tested for loss of CCI using a headspace gas analyzer. This process may be similar to (or the same as) the vial headspace $CO_2$ partial pressure analysis described elsewhere herein using a FMS-Carbon Dioxide ($CO_2$) Headspace Analyzer. Alternatively, packages may be tested using any suitable conventional leak testing technology, as would be known and understood by persons of skill in the art.

In some embodiments of the present disclosure, a syringe 500 or vial 400 may be configured to maintain container closure integrity (CCI) when cycled between -20 °C and 10 °C, optionally when cycled between -20 °C and 20 °C, optionally when cycled between -20 °C and 30 °C, optionally when cycled between -20 °C and 40 °C, optionally when cycled between -40 °C and 10 °C, optionally when cycled between -40 °C and 20 °C, optionally when cycled between - 40 °C and 30 °C, optionally when cycled between -40 °C and 40 °C, optionally when cycled between -70 °C and 10 °C, optionally when cycled between -70 °C and 20 °C, optionally when cycled between -70 °C and 30 °C, optionally when cycled between -70 °C and 40 °C. During each cycle, the syringe or vial may be held both at the lower temperature for 24 hours or more and at the upper temperature for 24 hours or more. In some embodiments, the syringe or vial may be subjected to three or more cycles.

In order to combat the potential of cracking or breakage of glass vials and syringes, the lumen of a glass vial or syringe may provided with a volume of liquid, the fill volume, that is significantly less than the nominal volume of the vial or syringe. For instance, a 2 mL syringe may be filled with only 1 mL of liquid. Embodiments of the vials 400 and syringes 500 of the present disclosure are configured to maintain CCI when subjected to a freeze-thaw cycle such as those described above, during which the fill volume of the package is within 40% of the nominal volume of the syringe or vial, optionally in which the fill volume of the package is within 30% of the nominal volume of the syringe or vial, optionally in which the fill volume of the package is within 20% of the nominal volume of the syringe or vial, optionally in which the fill volume of the package is within 10% of the nominal volume of the syringe or vial, optionally in which the fill volume of the package is within 5% of the nominal volume of the syringe or vial.

In some embodiments, for instance, the syringe 500 may have a nominal fill volume between 0.25 and 10 mL, optionally between 0.5 and 5 mL, optionally between 0.5 and 1 mL, optionally 0.5 mL, optionally 1 mL, optionally 2.25 mL. Similarly, in some embodiments, the vial 400 may have a nominal volume of either 10 mL or 2 mL, optionally a nominal volume of 10 mL, optionally a nominal volume of 2 mL. By maintaining CCI at a desired fill volume that is close to the nominal volume of the syringe or vial, embodiments of the present disclosure may enable more efficient drug storage packages.

Embodiments of vials of the present disclosure were subjected to a freeze-thaw cycle and visually examined for defects. First, embodiments of 2 mL vials and 10 mL vials of the present disclosure were filled with different fill volumes of high purity water. Specifically, one hundred 2 mL vials were filled with 1.0 mL of water and one hundred 2 mL vials were filled with 2.0 mL of water. One hundred of a first set of 10 mL vials were filled with 6.5 mL of water and one hundred of a second set of 10 mL vials were filled with 6.5 mL of water. The filled and sealed vials where then subjected to the freeze-thaw cycles shown in Figure 62. Namely, a first half of the vials were cycled between a lower temperature of -20 °C and an upper temperature of 30 °C while a second half of the vials were cycled between a lower temperature of -70 °C and an upper temperature of about 30 °C. The samples were provided with a 24 hour soak time at each of the lower and upper temperatures. The samples were subjected to three cycles and visually examined for failures after each cycle. The results are shown in Figure 63. As is shown in Figure 63, no defects were observed in any of the samples.

**Customizable surface energy**

The pH protective coating or layer described herein provides the additional benefit that the interior surfaces of the vessel walls, e.g. the inner surface of the vial side wall and the upper surface of the vial bottom wall, can be provided with any water contact angle between, for example, 25° (hydrophilic) and 105° (hydrophobic).

Different drug products interact with surfaces in different ways. As a result, it may be desirable that a drug primary package for one drug product, e.g. drug product A, has a first surface energy and a drug primary package for another drug product, e.g. drug product B, has a second, and significantly different, surface energy. For instance, it may be desirable that drug primary packages for some protein-based drug products are hydrophilic, so that the proteins or peptides do not stick to the surface of the vessel. At the same time, it may be desirable that drug primary packages for other drug products be hydrophobic, which can yield a more efficient dispensing or expelling of the drug product and thus less unused drug

product (which consequently can lead to the vessel being filled with less drug product).

Embodiments of the present disclosure provide vessels and drug primary packages in which the surface tension of the interior surfaces of the vessel, i.e. the surfaces that define the lumen that holds the drug product, is tailored/customized for a particular drug product. In some embodiments, for instance, the pH protective coating or layer may comprise a lumen-facing surface having a predetermined degree of hydrophilicity or hydrophobicity, as demonstrated by a selected water contact angle. The water contact angle may fall within the range between 25° and 105°. In some embodiments, the water contact angle may fall within a hydrophilic range such as between 25° and 60°, alternatively between 25° and 50°, alternatively between 30° and 60°, alternatively between 30° and 50°, alternatively between 40° and 60°, alternatively between 40° and 50°. In other embodiments, the water contact angle may fall within a hydrophobic range such as between 70° and 105°, alternatively between 75° and 105°, alternatively between 80° and 105°, alternatively between 85° and 105°, alternatively between 90° and 105°, alternatively between 95° and 105°. In other embodiments, the water contact angle may fall within a more neutral range, such as between 50° and 80°, alternatively between 55° and 75°, alternatively between 60° and 70°.

The surface free energy of the lumen-facing surface of the pH protective layer may also be determined from contact angle measurements for water, diiodomethane, and ethylene glycol (as probe liquids) using the Kitazaki-Hata Method. Surface free energy is the excessive energy existing on the surface due to variations of intermolecular forces among the molecules of the solid surface. These forces are comprised of dispersion, polar, and hydrogen-bond components. Using the Kitazaki-Hata Method, samples of hydrophilic and hydrophobic pH protective layers were tested and the results shown in Table A3 and Figure 38.

**Table A3**

| **Liquid Contact Angle Measurements for Each Coating Configuration** | | | |
|---|---|---|---|
| **Surface** | **Water** | **Diiodomethane** | **Ethylene Glycol** |
| | Average | Average | Average |
| **Trilayer** | 90.1 ± 7.2 | 62.3 ± 0.6 | 61.1 ± 5.1 |
| **Hydrophobic Trilayer** | 100.4 ± 2.5 | 62.3 ± 4.6 | 69.5 ± 7.3 |
| **Hydrophilic Trilayer** | 41.2 ± 7.2 | 47.2 ± 3.6 | 23.4 ± 7.3 |
| **Bilayer** | 38.1 ± 7.2 | 49.2 ± 3.1 | 26.7 ± 6.5 |
| **Carbon-Coated Trilayer** | 30.0 ± 9.8 | 58.0 ± 4.0 | 22.3 ± 8.5 |

In some embodiments, the pH protective coating or layer may comprise a lumen-facing surface having a predetermined surface free energy, e.g. within the range between 20 mJ/m$^2$ and 120 mJ/m$^2$. For instance, in some embodiments, the surface free energy may be between 20 mJ/m$^2$ and 50 mJ/m$^2$, alternatively between 25 mJ/m$^2$ and 50 mJ/m$^2$, alternatively between 20 mJ/m$^2$ and 45 mJ/m$^2$, alternatively between 25 mJ/m$^2$ and 45 mJ/m$^2$, alternatively between 20 mJ/m$^2$ and 40 mJ/m$^2$, alternatively between 25 mJ/m$^2$ and 40 mJ/m$^2$. In other embodiments, the surface free energy may be between 60 mJ/m$^2$ and 100 mJ/m$^2$, alternatively between 60 mJ/m$^2$ and 90 mJ/m$^2$, alternatively between 65 mJ/m$^2$ and 100 mJ/m$^2$, alternatively between 65 mJ/m$^2$ and 90 mJ/m$^2$, alternatively between 70 mJ/m$^2$ and 100 mJ/m$^2$, alternatively between 70 mJ/m$^2$ and 90 mJ/m$^2$.

### Blood Tubes

Embodiments of the present disclosure are also directed to evacuated blood tubes, an example of which is shown in Figure 93. An evacuated blood tube comprises a lumen defined at least in part by a thermoplastic side wall, the thermoplastic side wall having an interior surface facing the lumen and an outer surface. The blood tube also has a top defining an opening to the lumen. The lumen of the blood tube is evacuated to create a vacuum level within the lumen, relative to ambient pressure at sea level, sufficient to draw blood from a patient's vein into the lumen. A stopper is seated within the opening and seals the lumen in its evacuated state. Embodiments of the blood tubes of the present disclosure further comprise a gas barrier coating supported by at least one of the interior surface and the outer surface of the side wall, at least a portion of the gas barrier coating consisting essentially of a plurality of atomic monolayers of a pure element or compound.

The gas barrier may be effective to reduce the ingress of environmental gases into the lumen, including for example oxygen, nitrogen, water vapor, carbon dioxide, or any combination thereof.

By reducing the ingress of environmental gases into the lumen, embodiments of the blood tubes of the present disclosure may be effective to maintain a vacuum level within the lumen, relative to ambient pressure at sea level, sufficient

to draw blood from a patient's vein into the lumen for at least 28 months, optionally at least 30 months, optionally at least 32 months, optionally at least 34 months, optionally at least 36 months.

The shelf life of a blood tube is defined by the amount of time after evacuation the tube maintains a draw volume capacity of at least 90% of the draw volume capacity of a newly evacuated vessel of the same kind. The shelf life of an uncoated thermoplastic blood tube is typically about six months. By reducing the ingress of environmental gases into the lumen, embodiments of the blood tubes of the present disclosure may be effective to extend the shelf life of the evacuated blood tube to at least 28 months, optionally at least 30 months, optionally at least 32 months, optionally at least 34 months, optionally at least 36 months, in which the shelf life is defined by the amount of time after evacuation the tube maintains a draw volume capacity of at least 90% of the draw volume capacity of a newly evacuated vessel of the same kind.

Typically a blood preservative is contained within the lumen. Over time, however, the solvent for the blood preservative, which typically comprises water, may effuse out of the tube. Loss of solvent in this way can result in a damaged and ineffective blood preservative. By applying a water vapor barrier coating or layer to the wall of the blood tube, the gas barrier coating may be effective to reduce the amount of solvent loss of the blood preservative, e.g. over the shelf life of the blood tube.

**Vials and Syringes for DNA-based and/or mRNA-based vaccines**

Embodiments of the present disclosure are directed to vials and pre-filled syringes that contain DNA-based or mRNA-based vaccines.

**EXAMPLES**

**Examples 1-4 - Conditions for Production of pH Protective Layer**

Some conditions used for production of pH Protective Layers are shown in Table 1.

**TABLE 1: OMCTS-BASED PLASMA pH PROTECTIVE COATING OR LAYER MADE WITH CARRIER GAS**

| Example | pH protective coating or layer Type | pH protective Monomer | pH protective coating or layer Time (sec) | Protective OMCTS Flow Rate (sccm) | Protective O2 Flow Rate (sccm) | Carrier Gas (Ar) Flow Rate (sccm) | pH protective coating or layer Power (Watts) |
|---|---|---|---|---|---|---|---|
| 1 (Control) | Uncoated COC | n/a | n/a | n/a | n/a | n/a | n/a |
| 2 (Industry Standard) | Silicon oil on COC | n/a | n/a | n/a | n/a | n/a | n/a |
| 3 (without Oxygen) | L3 lubricity coating or layer over SiOx on COC | OMCTS | 10 sec | 3 | 0 | 65 | 6 |
| 4 (with Oxygen) | L2 pH protective coating or layer over SiOx on COC | OMCTS | 10 sec | 3 | 1 | 65 | 6 |

**Examples 5-8**

Syringe samples were produced as follows. A COC 8007 extended barrel syringe was produced according to the Protocol for Forming COC Syringe Barrel. An $SiO_x$ barrier coating or layer was applied to the syringe barrels according to the Protocol for Coating COC Syringe Barrel Interior with $SiO_x$. A pH protective coating or layer was applied to the $SiO_x$ coated syringes according to the Protocol for Coating COC Syringe Barrel Interior with OMCTS, modified as follows. Argon carrier gas and oxygen were used where noted in Table 2. The process conditions were set to the following, or as indicated in Table 2:

- OMCTS -3 sccm (when used)

- Argon gas -7.8 sccm (when used)
- Oxygen 0.38 sccm (when used)
- Power - 3 watts
- Power on time - 10 seconds

Syringes of Examples 5, 6, and 7 were tested to determine total extractable silicon levels (representing extraction of the organosilicon-based PECVD pH protective coating or layer) using the Protocol for Measuring Dissolved Silicon in a Vessel, modified and supplemented as shown in this example.

The silicon was extracted using saline water digestion. The tip of each syringe plunger was covered with PTFE tape to prevent extracting material from the elastomeric tip material, then inserted into the syringe barrel base. The syringe barrel was filled with two milliliters of 0.9% aqueous saline solution via a hypodermic needle inserted through the Luer tip of the syringe. This is an appropriate test for extractables because many prefilled syringes are used to contain and deliver saline solution. The Luer tip was plugged with a piece of PTFE beading of appropriate diameter. The syringe was set into a PTFE test stand with the Luer tip facing up and placed in an oven at 50°C for 72 hours.

Then, either a static or a dynamic mode was used to remove the saline solution from the syringe barrel. According to the static mode indicated in Table 2, the syringe plunger was removed from the test stand, and the fluid in the syringe was decanted into a vessel. According to the dynamic mode indicated in Table 2, the Luer tip seal was removed and the plunger was depressed to push fluid through the syringe barrel and expel the contents into a vessel. In either case, the fluid obtained from each syringe barrel was brought to a volume of 50ml using 18.2MΩ-cm deionized water and further diluted 2x to minimize sodium background during analysis. The CVH barrels contained two milliliters and the commercial barrels contained 2.32 milliliters.

Next, the fluid recovered from each syringe was tested for extractable silicon using the Protocol for Measuring Dissolved Silicon in a Vessel. The instrument used was a Perkin Elmer Elan DRC II equipped with a Cetac ASX-520 autosampler. The following ICP-MS conditions were employed:

- Nebulizer: Quartz Meinhardt
- Spray Chamber: Cyclonic
- RF (radio frequency) power: 1550 Watts
- Argon (Ar) Flow: 15.0 L/min
- Auxiliary Ar Flow: 1.2 L/min
- Nebulizer Gas Flow: 0.88 L/min
- Integration time: 80 sec
- Scanning mode: Peak hopping
- RPq (The RPq is a rejection parameter) for Cerium as CeO (m/z 156: < 2 %

Aliquots from aqueous dilutions obtained from Syringes E, F, and G were injected and analyzed for Si in concentration units of micrograms per liter. The results of this test are shown in Table 2. While the results are not quantitative, they do indicate that extractables from the pH protective coating or layer are not clearly higher than the extractables for the $SiO_x$ barrier layer only. Also, the static mode produced far less extractables than the dynamic mode, which was expected.

**TABLE 2: OMCTS PH PROTECTIVE COATING OR LAYER (E and F)**

| Example | OMCTS (sccm) | $O_2$ (sccm) | Ar (sccm) |
|---|---|---|---|
| 5 | 3.0 | 0.38 | 7.8 |
| 6 | 3.0 | 0.38 | 7.8 |
| 7 ($SiO_x$only) | n/a | n/a | n/a |
| 8 (silicon oil) | n/a | n/a | n/a |

**Examples 9-11**

Syringe Examples 9, 10, and 11, employing three different pH protective coatings or layers, were produced in the same manner as for Examples 5-8 except as follows or as indicated in Table 3:

- OMCTS -2.5 sccm
- Argon gas -7.6 sccm (when used)

- Oxygen 0.38 sccm (when used)
- Power - 3 watts
- Power on time - 10 seconds

Syringe Example 9 had a three-component pH protective coating or layer employing OMCTS, oxygen, and carrier gas. Syringe Example 10 had a two component pH protective coating or layer employing OMCTS and oxygen, but no carrier gas. Syringe Example 11 had a one-component pH protective coating or layer (OMCTS only). Syringes of Examples 9-11 were then tested for lubricity as described for Examples 5-8.

The pH protective coatings or layers produced according to these working examples are also contemplated to function as protective coatings or layers to increase the shelf life of the vessels, compared to similar vessels provided with a barrier coating or layer but no pH protective coating or layer.

**TABLE 3: OMCTS pH protective coating or layer**

| |
|---|
| • OMCTS -2.5 sccm |
| • Argon gas -7.6 sccm (when used) |
| • Oxygen 0.38 sccm (when used) |
| • Power - 3 watts |
| • Power on time - 10 seconds |

**Examples 12-14**

Examples 9-11 using an OMCTS precursor gas were repeated in Examples 12-14, except that HMDSO was used as the precursor in Examples 12-14. The results are shown in Table 4. The coatings produced according to these working examples are contemplated to function as pH protective coatings or layers, and also as protective coatings or layers to increase the shelf life of the vessels, compared to similar vessels provided with a barrier coating or layer but no pH protective coating or layer.

**TABLE 4: HMDSO pH protective coating or layer**

| Example | HMDSO(sccm) | $O_2$ (sccm) | Ar (sccm) |
|---|---|---|---|
| 12 | 2.5 | 0.38 | 7.6 |
| 13 | 2.5 | 0.38 | - |
| 14 | 2.5 | - | - |

The pH protective coatings or layers produced according to these working examples are also contemplated to function as protective coatings or layers to increase the shelf life of the vessels, compared to similar vessels provided with a barrier coating or layer but no pH protective coating or layer.

**TABLE 5**

<table>
<tr><th>Example</th><th>OMCTS (sccm)</th><th>Ar/O2 (sccm)</th><th>Power (Watts)</th><th>Dep. Time (sec)</th><th colspan="2">AFM RMS (nanometers)</th></tr>
<tr><td>15</td><td rowspan="3">2.0</td><td rowspan="3">10/0.38</td><td rowspan="3">3.5</td><td rowspan="3">10</td><td></td><td></td></tr>
<tr><td>16</td><td></td><td></td></tr>
<tr><td>17</td><td></td><td>19.6, 9.9, 9.4 (Average=13.0)</td></tr>
<tr><td colspan="7"></td></tr>
<tr><td>21</td><td rowspan="3">2.0</td><td rowspan="3">10/0.38</td><td rowspan="3">4.5</td><td rowspan="3">10</td><td></td><td></td></tr>
<tr><td>22</td><td>FIG. 7</td><td></td></tr>
<tr><td>23</td><td></td><td>12.5, 8.4, 6.1 (Average=6.3)</td></tr>
<tr><td colspan="7"></td></tr>
</table>

(continued)

| Example | OMCTS (sccm) | Ar/O2 (sccm) | Power (Watts) | Dep. Time (sec) | | AFM RMS (nanometers) |
|---|---|---|---|---|---|---|
| **24** | 2.0 | 10/0 | 3.4 | 10 | | |
| **25** | | | | | | 1.9, 2.6, 3.0 (Average=2.3) |

**TABLE 6**

| | SiOx/Lub | Coater | Mode | Siloxane Feed | Ar/O2 | Power (W) | Dep. Time (Sec.) |
|---|---|---|---|---|---|---|---|
| **Example 18 $SiO_x$/Baseline OMCTS Lub** | **$SiO_x$:** | Auto-Tube | Auto | HMDSO 52.5 in, 133.4 cm. | 0 sccm Ar, 90 sccm $O_2$ | 37 | 7 |
| | **Lubricity:** | Auto-S | Same | OMCTS, 2.0 sccm | 10 sccm Ar 0.38 sccm $O_2$ | 3,4 | 10 |
| | | | | | | | |
| **Example 19 $SiO_x$/High Pwr OMCTS Lub** | **$SiO_x$:** | Same | Same | Same | Same | 37 | 7 |
| | **Lubricity:** | Same | Same | Same | Same | 4,5 | 10 |
| | | | | | | | |
| **Example 20 $SiO_x$/No $O_2$ OMCTS Lub** | **SiOx:** | Auto-Tube | Same | Same | 0 sccm Ar, 90 sccm O2 | 37 | 7 |
| | **Lubricity:** | Auto-S | Same | Same | 10 sccm Ar 0 sccm O2 | 3,4 | 10 |

**Summary of Lubricity and/or Protective Measurements**

Table 8 shows a summary of the above OMCTS coatings or layers

**TABLE 8: Summary Table of OMCTS PH PROTECTIVE COATING OR LAYER from Tables 1, 2, 3 and 5**

| Example | OMCTS(sccm) | O2 (sccm) | Ar (sccm) | Power (Watt) | Dep Time (sec) |
|---|---|---|---|---|---|
| **3** | 3.0 | 0.00 | 65 | 6 | 10 |
| **4** | 3.0 | 1.00 | 65 | 6 | 10 |
| **5** | 3.0 | 0.38 | 7.8 | 6 | 10 |
| **6** | 3.0 | 0.38 | 7.8 | 6 | 10 |
| **9** | 2.5 | 0.38 | 7.6 | 6 | 10 |
| **10** | 2.5 | 0.38 | 0.0 | 6 | 10 |
| **11** | 2.5 | 0.00 | 0.0 | 6 | 10 |
| **15** | 2.0 | 0.38 | 10 | 3.5 | 10 |
| **16** | 2.0 | 0.38 | 10 | 4.5 | 10 |
| **16A** | 2.0 | 0.00 | 10 | 3.4 | 10 |
| **18** | 2.0 | 0.38 | 10 | 3.4 | 10 |
| **19** | 2.0 | 0.38 | 10 | 4.5 | 10 |
| **20** | 2.0 | 0.00 | 10 | 3.4 | 10 |

**Comparative Example 26: Dissolution of SiOx Coating Versus pH**

The Protocol for Measuring Dissolved Silicon in a Vessel is followed, except as modified here. Test solutions - 50 mM buffer solutions at pH 3, 6, 7, 8, 9, and 12 are prepared. Buffers are selected having appropriate pKa values to provide the pH values being studied. A potassium phosphate buffer is selected for pH 3, 7, 8 and 12, a sodium citrate buffer is utilized for pH 6 and tris buffer is selected for pH 9. 3 ml of each test solution is placed in borosilicate glass 5 ml pharmaceutical vials and $SiO_x$ coated 5 ml thermoplastic pharmaceutical vials. The vials are all closed with standard coated stoppers and crimped. The vials are placed in storage at 20 - 25°C and pulled at various time points for inductively coupled plasma spectrometer (ICP) analysis of Si content in the solutions contained in the vials, in parts per billion (ppb) by weight, for different storage times.

The Protocol for Determining Average Dissolution Rate Si content is used to monitor the rate of glass dissolution, except as modified here. The data is plotted to determine an average rate of dissolution of borosilicate glass or $SiO_x$ coating at each pH condition. Representative plots at pH 6 through 8 are FIGS 6-8.

The rate of Si dissolution in ppb is converted to a predicted thickness (nm) rate of Si dissolution by determining the total weight of Si removed, then using a surface area calculation of the amount of vial surface (11.65 cm2) exposed to the solution and a density of $SiO_x$ of 2.2 g/cm3. FIG. 9 shows the predicted initial thickness of the $SiO_x$ coating required, based on the conditions and assumptions of this example (assuming a residual $SiO_x$ coating of at least 30 nm at the end of the desired shelf life of two years, and assuming storage at 20 to 25°C). As FIG. 9 shows, the predicted initial thickness of the coating is about 36 nm at pH 5, about 80 nm at pH 6, about 230 nm at pH 7, about 400 nm at pH 7.5, about 750 nm at pH 8, and about 2600 nm at pH 9.

The coating thicknesses in FIG. 9 represent atypically harsh case scenarios for pharma and biotech products. Most biotech products and many pharma products are stored at refrigerated conditions and none are typically recommended for storage above room temperature. As a general rule of thumb, storage at a lower temperature reduces the thickness required, all other conditions being equivalent.

The following conclusions are reached, based on this test. First, the amount of dissolved Si in the $SiO_x$ coating or glass increases exponentially with increasing pH. Second, the $SiO_x$ coating dissolves more slowly than borosilicate glass at a pH lower than 8. The $SiO_x$ coating shows a linear, monophasic dissolution over time, whereas borosilicate glass tends to show a more rapid dissolution in the early hours of exposure to solutions, followed by a slower linear dissolution. This may be due to surface accumulation of some salts and elements on borosilicate during the forming process relative to the uniform composition of the $SiO_x$ coating. This result incidentally suggests the utility of an $SiO_x$ coating on the wall of a borosilicate glass vial to reduce dissolution of the glass at a pH lower than 8. Third, PECVD applied barrier coatings for vials in which pharmaceutical preparations are stored will need to be adapted to the specific pharmaceutical preparation and proposed storage conditions (or vice versa), at least in some instances in which the pharmaceutical preparation interacts with the barrier coating significantly.

**Example 27**

An experiment is conducted with vessels coated with $_{SiOx}$ coating + OMCTS pH protective coating or layer, to test the pH protective coating or layer for its functionality as a protective coating or layer. The vessels are 5 mL vials (the vials are normally filled with product to 5 mL; their capacity without headspace, when capped, is about 7.5 mL) composed of cyclic olefin co-polymer (COC, Topas® 6013M-07).

Sixty vessels are coated on their interior surfaces with an $SiO_x$ coating produced in a plasma enhanced chemical vapor deposition (PECVD) process using a HMDSO precursor gas according to the Protocol for Coating Tube Interior with $SiO_x$ set forth above, except that equipment suitable for coating a vial is used. The following conditions are used.

- HMDSO flow rate: 0.47 sccm
- Oxygen flow rate: 7.5 sccm
- RF power: 70 Watts
- Coating time: 12 seconds (includes a 2-sec RF power ramp-up time)

Next the $SiO_x$ coated vials are coated over the $SiO_x$ with an $SiO_xC_y$ coating produced in a PECVD process using an OMCTS precursor gas according to the Protocol for Coating COC Syringe Barrel Interior with OMCTS Lubricity Coating set forth above, except that the same coating equipment is used as for the $SiO_x$ coating. Thus, the special adaptations in the protocol for coating a syringe are not used. The following conditions are used.

- OMCTS flow rate: 2.5 sccm
- Argon flow rate: 10 sccm
- Oxygen flow rate: 0.7 sccm
- RF power: 3.4 Watts
- Coating time: 5 seconds

Eight vials are selected and the total deposited quantity of PECVD coating ($SiO_x$ + $SiO_xC_y$) is determined with a Perkin Elmer Optima Model 7300DV ICP-OES instrument, using the Protocol for Total Silicon Measurement set forth above. This measurement determines the total amount of silicon in both coatings, and does not distinguish between the respective $SiO_x$ and $SiO_xC_y$ coatings. The results are shown below.

| Vial | Total Silicon ug/L |
|---|---|
| 1 | 13844 |
| 2 | 14878 |
| 3 | 14387 |
| 4 | 13731 |
| 5 | 15260 |
| 6 | 15017 |
| 7 | 15118 |
| 8 | 12736 |
| **Mean** | **14371** |
| **Std Dev** | **877** |

*Quantity of $SiO_x$ + Lubricity layer on Vials*

In the following work, except as indicated otherwise in this example, the Protocol for Determining Average Dissolution Rate is followed. Two buffered pH test solutions are used in the remainder of the experiment, respectively at pH 4 and pH 8 to test the effect of pH on dissolution rate. Both test solutions are 50 mM buffers using potassium phosphate as the buffer, diluted in water for injection (WFI) (0.1 um sterilized, filtered). The pH is adjusted to pH 4 or 8, respectively, with concentrated nitric acid.

25 vials are filled with 7.5 ml per vial of pH 4 buffered test solution and 25 other vials are filled with 7.5 ml per vial of pH 4 buffered test solution (note the fill level is to the top of the vial - no head space). The vials are closed using prewashed butyl stoppers and aluminum crimps. The vials at each pH are split into two groups. One group at each pH containing 12 vials is stored at 4°C and the second group of 13 vials is stored at 23°C

The vials are sampled at Days 1, 3, 6, and 8. The Protocol for Measuring Dissolved Silicon in a Vessel is used, except as otherwise indicated in this example.The analytical result is reported on the basis of parts per billion of silicon in the buffered test solutions of each vial. A dissolution rate is calculated in terms of parts per billion per day as described above in the Protocol for Determining Average Dissolution Rate. The results at the respective storage temperatures follow:

| | **Shelf Life Conditions 23º C** | |
|---|---|---|
| | **Vial $SiO_x$ + Lubricity Coating at pH4** | **Vial $SiO_x$ + Lubricity Coating at pH8** |
| Si Dissolution Rate(PPB/day) | 31 | 7 |
| | | |
| | **Shelf Life Conditions 4º C** | |
| | **Vial $SiO_x$ + Lubricity Coating at pH4** | **Vial $SiO_x$ + Lubricity Coating at pH8** |
| Si Dissolution Rate (PPB/day) | 7 | 11 |

The observations of Si dissolution versus time for the OMCTS-based coating at pH8 and pH 4 indicate the pH 4 rates are higher at ambient conditions. Thus, the pH 4 rates are used to determine how much material would need to be

initially applied to leave a coating of adequate thickness at the end of the shelf life, taking account of the amount of the initial coating that would be dissolved. The results of this calculation are:

| | Vial $SiO_x$ + Lubricity Coating at pH4 |
|---|---|
| Si Dissolution Rate (PPB/day) | 31 |
| Mass of Coating Tested (Total Si) | 14,371 |
| Shelf Life (days) at 23º C | 464 |
| Shelf Life (years) at 23º C | 1.3 |
| Required Mass of Coating (Total Si) - 2 years | 22,630 |
| Required Mass of Coating (Total Si) - 3 years | 33,945 |

*Shelf Life Calculation*

Based on this calculation, the OMCTS protective layer needs to be about 2.5 times thicker - resulting in dissolution of 33945 ppb versus the 14,371 ppb representing the entire mass of coating tested - to achieve a 3-year calculated shelf life.

**Example 28**

The results of Comparative Example 26 and Example 27 above can be compared as follows, where the "pH protective coating or layer" is the coating of $SiO_xC_y$ referred to in **Example BB.**

| | Shelf Life Conditions - - pH8 and 23º C | |
|---|---|---|
| | Vial $SiO_x$ | Vial $SiO_x$ + Lubricity Coating |
| Si Dissolution Rate (PPB/day) | 1,250 | 7 |

This data shows that the silicon dissolution rate of $SiO_x$ alone is reduced by more than 2 orders of magnitude at pH 8 in vials also coated with $SiO_xC_y$ coatings.

**Example 29**

Another comparison is shown by the following data from several different experiments carried out under similar accelerated dissolution conditions, of which the 1- day data is also presented in FIG. 10.

| | Silicon Dissolution with pH 8 at 40°C (ug/L) | | | | | | |
|---|---|---|---|---|---|---|---|
| Vial Coating Description | 1day | 2days | 3days | 4 days | 7 days | 10days | 15 days |
| A. $SiO_X$ made with HMDSO Plasma + $Si_WO_XCy$ or its equivalent $SiO_XC_y$ made with OMCTS Plasma | 165 | 211 | 226 | 252 | 435 | 850 | 1,364 |
| B. SiwOxCy or its equivalent SiOxCy made with OMCTS Plasma | 109 | 107 | 76 | 69 | 74 | 158 | 198 |
| C. SiOx made with HMDSO Plasma | 2,504 | 4,228 | 5,226 | 5,650 | 9,292 | 10,177 | 9,551 |
| D. SiOx made with HMDSO Plasma + SiwOxCy or its equivalent $SiO_xC_y$ made with HMDSO Plasma | 1,607 | 1,341 | 3,927 | 10,182 | 18,148 | 20,446 | 21,889 |
| E. $Si_wO_xC_y$ or its equivalent $SiO_xC_y$ made with HMDSO Plasma | 1,515 | 1,731 | 1,813 | 1,743 | 2,890 | 3,241 | 3,812 |

FIG. 10 and Row A ($SiO_x$ with OMCTS coating) versus C ($SiO_x$ without OMCTS coating) show that the OMCTS pH protective coating or layer is also an effective protective coating or layer to the $SiO_x$ coating at pH 8. The OMCTS coating reduced the one-day dissolution rate from 2504 ug/L ("u" or μ or the Greek letter "mu" as used herein are identical, and are abbreviations for "micro") to 165 ug/L. This data also shows that an HMDSO-based $Si_wO_xC_y$ (or its equivalent $SiO_xC_y$) overcoat(Row D) provided a far higher dissolution rate than an OMCTS-based $Si_wO_xC_y$ (or its equivalent $SiO_xC_y$) overcoat (Row A). This data shows that a substantial benefit can be obtained by using a cyclic precursor versus a linear one.

**Example 30**

Samples 1-6 as listed in Table 9 were prepared as described in Example AA, with further details as follows.
A cyclic olefin copolymer (COC) resin was injection molded to form a batch of 5ml vials. Silicon chips were adhered with double-sided adhesive tape to the internal walls of the vials. The vials and chips were coated with a two layer coating by plasma enhanced chemical vapor deposition (PECVD). The first layer was composed of $SiO_x$ with barrier properties as defined in the present disclosure, and the second layer was an $SiO_xC_y$ pH protective coating or layer.
A precursor gas mixture comprising OMCTS, argon, and oxygen was introduced inside each vial. The gas inside the vial was excited between capacitively coupled electrodes by a radio-frequency (13.56 MHz) power source. The monomer flow rate (Fm) in units of sccm, oxygen flow rate (Fo) in units of sccm, argon flowrate in sccm, and power (W) in units of watts are shown in Table 9.
A composite parameter, W/FM in units of kJ/kg, was calculated from process parameters W, Fm, Fo and the molecular weight, M in g/mol, of the individual gas species. W/FM is defined as the energy input per unit mass of polymerizing gases. Polymerizing gases are defined as those species that are incorporated into the growing coating such as, but not limited to, the monomer and oxygen. Non-polymerizing gases, by contrast, are those species that are not incorporated into the growing coating, such as but not limited to argon, helium and neon.
In this test, PECVD processing at high W/FM is believed to have resulted in higher monomer fragmentation, producing organosiloxane coatings with higher cross- link density. PECVD processing at low W/FM, by comparison, is believed to have resulted in lower monomer fragmentation producing organosiloxane coatings with a relatively lower cross-link density.
The relative cross-link density of samples 5, 6, 2, and 3 was compared between different coatings by measuring FTIR absorbance spectra. The spectra of samples 5, 6, 2, and 3 are provided in FIGS. 13 to 16. In each spectrum, the ratio of the peak absorbance at the symmetric stretching mode (1000-1040 cm-1) versus the peak absorbance at the asymmetric stretching mode (1060-1100 cm-1) of the Si-O-Si bond was measured, and the ratio of these two measurements was calculated, all as shown in Table 9. The respective ratios were found to have a linear correlation to the composite parameter W/FM as shown in FIG. 11.
A qualitative relation - whether the coating appeared oily (shiny, often with irridescence) or non-oily (non-shiny) when applied on the silicon chips - was also found to correlate with the W/FM values in Table 9. Oily appearing coatings deposited at lower W/FM values, as confirmed by Table 9, are believed to have a lower crosslink density, as determined by their lower sym/asym ratio, relative to the non-oily coatings that were deposited at higher W/FM and a higher cross-link density. The only exception to this general rule of thumb was sample 2 in Table 9. It is believed that the coating of sample
2 exhibited a non-oily appearance because it was too thin to see. Thus, an oilyness observation was not reported in Table 9 for sample 2. The chips were analyzed by FTIR in transmission mode, with the infrared spectrum transmitted through the chip and sample coating, and the transmission through an uncoated null chip subtracted.
Non-oily organosiloxane layers produced at higher W/FM values, which protect the underlying $SiO_x$ coating from aqueous solutions at elevated pH and temperature, were preferred because they provided lower Si dissolution and a longer shelf life, as confirmed by Table 9. For example, the calculated silicon dissolution by contents of the vial at a pH of 8 and 40°C was reduced for the non-oily coatings, and the resulting shelf life was 1381 days in one case and 1147 days in another, as opposed to the much shorter shelf lives and higher rates of dissolution for oily coatings. Calculated shelf life was determined as shown for Example AA. The calculated shelf life also correlated linearly to the ratio of symmetric to asymmetric stretching modes of the Si-O- Si bond in organosiloxane pH protective coatings or layers.
Sample 6 can be particularly compared to Sample 5. An organosiloxane, pH protective coating or layer was deposited according to the process conditions of sample 6 in Table 9. The coating was deposited at a high W/FM. This resulted in a non-oily coating with a high Si-O-Si sym/asym ratio of 0.958, which resulted in a low rate of dissolution of 84.1ppb/day (measured by the Protocol for Determining Average Dissolution Rate) and long shelf life of 1147 days (measured by the Protocol for Determining Calculated Shelf Life). The FTIR spectra of this coating is shown in Figure 35, which exhibits a relatively similar asymmetric Si-O-Si peak absorbance compared to the symmetric Si-O-Si peak absorbance. This is an indication of a higher cross-link density coating, which is a preferred characteristic for pH protection and long shelf life.
An organosiloxane pH protective coating or layer was deposited according to the process conditions of sample 5

in Table 9. The coating was deposited at a moderate W/FM. This resulted in an oily coating with a low Si-O-Si sym/asym ratio of 0.673, which resulted in a high rate of dissolution of 236.7 ppb/day (following the Protocol for Determining Average Dissolution Rate) and shorter shelf life of 271 days (following the Protocol for Determining Calculated Shelf Life). The FTIR spectrum of this coating is shown in FIG. 13, which exhibits a relatively high asymmetric Si-O-Si peak absorbance compared to the symmetric Si-O-Si peak absorbance. This is an indication of a lower cross-link density coating, which is contemplated in any embodiment to be an unfavorable characteristic for pH protection and long shelf life.

Sample 2 can be particularly compared to Sample 3. A pH protective coating or layer was deposited according to the process conditions of sample 2 in Table 9. The coating was deposited at a low W/FM. This resulted in a coating that exhibited a low Si- O-Si sym/asym ratio of 0.582, which resulted in a high rate of dissolution of 174ppb/day and short shelf life of 107 days. The FTIR spectrum of this coating is shown in Figure 36, which exhibits a relatively high asymmetric Si-O-Si peak absorbance compared to the symmetric Si-O-Si peak absorbance. This is an indication of a lower cross-link density coating, which is an unfavorable characteristic for pH protection and long shelf life.

An organosiloxane, pH pH protective coating or layer was deposited according to the process conditions of sample 3 in Table 9. The coating was deposited at a high W/FM. This resulted in a non-oily coating with a high Si-O-Si sym/asym ratio of 0.947, which resulted in a low rate of Si dissolution of 79.5ppb/day (following the Protocol for Determining Average Dissolution Rate) and long shelf life of 1381 days (following the Protocol for Determining Calculated Shelf Life). The FTIR spectrum of this coating is shown in Figure 37, which exhibits a relatively similar asymmetric Si-O-Si peak absorbance compared to the symmetric Si-O-Si peak absorbance. This is an indication of a higher cross-link density coating, which is a preferred characteristic for pH protection and long shelf life.

**TABLE 9**

| | Process Parameters | | | | | Si Dissolution @ pH8/40ºC | | | FTIR Absorbance | | | |
|---|---|---|---|---|---|---|---|---|---|---|---|---|
| Flow Rate Samples | OMCTS | Ar | 02 Flow Rate | Power (W) | W/FM (kJ/kg) | Total Si (ppb) | Shelf life (days) | Rate of Dissolution (ppb/day) | Si-0-Si sym stretch (1000-1040 $cm^{-1}$) | Si-0-Si asym stretch (1060-1100 $cm^{-1}$) | Ratio Si-O-Si (sym/asym) | Oilyness |
| 1 | 3 | 10 | 0.5 | 14 | 21613 | 43464 | 385 | 293.18 | 0.153 | 0.219 | 0.700 | YES |
| 2 | 3 | 20 | 0.5 | 2 | 3088 | 7180 | 107 | 174.08 | 0.011 | 0.020 | 0.582 | NA |
| 3 | 1 | 20 | 0.5 | 14 | 62533 | 42252.17 | 1381 | 79.53 | 0.098 | 0.098 | 0.947 | NO |
| 4 | 2 | 15 | 0.5 | 8 | 18356 | 27398 | 380 | 187.63 | 0.106 | 0.141 | 0.748 | YES |
| 5 | 3 | 20 | 0.5 | 14 | 21613 | 24699 | 271 | 236.73 | 0.135 | 0.201 | 0.673 | YES |
| 6 | 1 | 10 | 0.5 | 14 | 62533 | 37094 | 1147 | 84.1 | 0.134 | 0.140 | 0.958 | NO |

**Example 31**

An experiment similar to Example 27 was carried out, modified as indicated in this example and in Table 10 (where the results are tabulated). 100 5 mL COP vials were made and coated with an $SiO_x$ barrier layer and an OMCTS-based pH protective coating or layer as described previously, except that for Sample PC194 only the pH protective coating or layer was applied. The coating quantity was again measured in parts per billion extracted from the surfaces of the vials to remove the entire pH protective coating or layer, as reported in Table 10.

In this example, several different coating dissolution conditions were employed. The test solutions used for dissolution contained either 0.02 or 0.2 wt.% polysorbate-80 surfactant, as well as a buffer to maintain a pH of 8. Dissolution tests were carried out at either 23°C or 40°C.

Multiple syringes were filled with each test solution, stored at the indicated temperature, and analyzed at several intervals to determine the extraction profile and the amount of silicon extracted. An average dissolution rate for protracted storage times was then calculated by extrapolating the data obtained according to the Protocol for Determining Average Dissolution Rate. The results were calculated as described previously and are shown in Table 10. Of particular note, as shown on Table 10, were the very long calculated shelf lives of the filled packages provided with a PC 194 pH protective coating or layer:

- 21045 days (over 57 years) based on storage at a pH of 8, 0.02 wt.% polysorbate-80 surfactant, at 23°C;
- 38768 days (over 100 years) based on storage at a pH of 8, 0.2 wt.% polysorbate-80 surfactant, at 23°C;
- 8184 days (over 22 years) based on storage at a pH of 8, 0.02 wt.% polysorbate-80 surfactant, at 40°C; and
- 14732 days (over 40 years) based on storage at a pH of 8, 0.2 wt.% polysorbate-80 surfactant, at 40°C.

Referring to Table 10, the longest calculated shelf lives corresponded with the use of an RF power level of 150 Watts and a corresponding high W/FM value. It is believed that the use of a higher power level causes higher cross-link density of the pH protective coating or layer.

**TABLE 10**

| **Sample** | **OMCTS Flow Rate (sccm)** | **Argon Flow Rate (sccm)** | **$O_2$ Flow Rate (sccm)** | **Power (W)** | **Plasma Duration (sec)** | **W/FM (kJ/kg)** | **Total Si (ppb) (OMCTS) layer)** | **Calculated Shelf-life (days)** | **Average Rate of Dissolution (ppb/day)** |
|---|---|---|---|---|---|---|---|---|---|
| | | | | | | | | | |
| | Process Parameters | | | | | | Si Dissolution @ pH8/23ºC/0.02% Tween®-80 | | |
| PC194 | 0.5 | 20 | 0.5 | 150 | 20 | 1223335 | 73660 | 21045 | 3.5 |
| 018 | 1.0 | 20 | 0.5 | 18 | 15 | 77157 | 42982 | 1330 | 32.3 |
| | | | | | | | | | |
| | Process Parameters | | | | | | Si Dissolution @ pH8/23ºC/0.2% Tween®-80 | | |
| PC194 | 0.5 | 20 | 0.5 | 150 | 20 | 1223335 | 73660 | 38768 | 1.9 |
| 018 | 1.0 | 20 | 0.5 | 18 | 15 | 77157 | 42982 | 665 | 64.6 |
| 048 | 4 | 80 | 2 | 35 | 20 | 37507 | 56520 | 1074 | 52.62 |
| | | | | | | | | | |
| | Process Parameters | | | | | | Si Dissolution @ pH8/40ºC/0.02% Tween®-80 | | |
| PC194 | 0.5 | 20 | 0.5 | 150 | 20 | 1223335 | 73660 | 8184 | 9 |
| 018 | 1.0 | 20 | 0.5 | 18 | 15 | 77157 | 42982 | 511 | 84 |
| | | | | | | | | | |
| | Process Parameters | | | | | | Si Dissolution @ pH8/40ºC/0.2% Tween®-80 | | |
| PC194 | 0.5 | 20 | 0.5 | 150 | 20 | 1223335 | 73660 | 14732 | 5 |

(continued)

| Sample | OMCTS Flow Rate (sccm) | Argon Flow Rate (sccm) | $O_2$ Flow Rate (sccm) | Power (W) | Plasma Duration (sec) | W/FM (kJ/kg) | Total Si (ppb) (OMCTS) layer) | Calculated Shelf-life (days) | Average Rate of Dissolution (ppb/day) |
|---|---|---|---|---|---|---|---|---|---|
| 018 | 1.0 | 20 | 0.5 | 18 | 15 | 77157 | 42982 | 255 | 168 |

**Example 32**

Another series of experiments similar to those of Example 31 are run, showing the effect of progressively increasing the RF power level on the FTIR absorbance spectrum of the pH protective coating or layer. The results are tabulated in Table 11, which in each instance shows a symmetric / assymmetric ratio greater than

between the maximum amplitude of the Si-O-Si symmetrical stretch peak normally located between about 1000 and 1040 cm-1, and the maximum amplitude of the Si-O-Si assymmetric stretch peak normally located between about 1060 and about 1100 cm-1. Thus, the symmetric / assymmetric ratio is 0.79 at a power level of 20 W, 1.21 or 1.22 at power levels of 40, 60, or 80 W, and 1.26 at 100 Watts under otherwise comparable conditions.

The 150 Watt data in Table 11 is taken under somewhat different conditions than the other data, so it is not directly comparable with the 20 - 100 Watt data discussed above. The FTIR data of samples 6 and 8 of Table 11 was taken from the upper portion of the vial and the FTIR data of samples 7 and 9 of Table 11 was taken from the lower portion of the vial. Also, the amount of OMCTS was cut in half for samples 8 and 9 of Table 11, compared to samples 6 and 7. Reducing the oxygen level while maintaining a power level of 150 W raised the symmetric / asymmetric ratio still further, as shown by comparing samples 6 and 7 to samples 8 and 9 in Table 11.

It is believed that, other conditions being equal, increasing the symmetric / asymmetric ratio increases the shelf life of a vessel filled with a material having a pH exceeding 5.

Table 12 shows the calculated O-Parameters and N-Parameters (as defined in U.S. Pat. No. 8,067,070) for the experiments summarized in Table 11. As Table 12 shows, the O-Parameters ranged from 0.134 to 0.343, and the N-Parameters ranged from 0.408 to 0.623 - all outside the ranges claimed in U.S. Pat. No. 8,067,070.

**TABLE 11**

| Samples ID | OMCTS Flow Rate (sccm) | Argon Flow Rate (sccm) | $O_2$ Flow Rate (sccm) | Power (W) | Plasma Duration (sec) | W/FM (kJ/kg) | Symmetric Stretch Peak at 1000-1040 $cm^{-1}$ | Assymetric Stretch Peak at 1060-1100 $cm^{-1}$ | Symmetric / Assymetric Ratio |
|---|---|---|---|---|---|---|---|---|---|
| | | | | | | | | | |
| | Process Parameters | | | | | | FTIR Results | | |
| 1 | 1 | 20 | 0.5 | 20 | 20 | 85,730 | 0.0793 | 0.1007 | 0.79 |
| 2 | 1 | 20 | 0.5 | 40 | 20 | 171,460 | 0.0619 | 0.0507 | 1.22 |
| 3 | 1 | 20 | 0.5 | 60 | 20 | 257,190 | 0.1092 | 0.0904 | 1.21 |
| 4 | 1 | 20 | 0.5 | 80 | 20 | 342,919 | 0.1358 | 0.1116 | 1.22 |
| 5 | 1 | 20 | 0.5 | 100 | 20 | 428,649 | 0.209 | 0.1658 | 1.26 |
| 6 | 1 | 20 | 0.5 | 150 | 20 | 642,973 | 0.2312 | 0.1905 | 1.21 |
| 7 | 1 | 20 | 0.5 | 150 | 20 | 642,973 | 0.2324 | 0.1897 | 1.23 |
| 8 | 0.5 | 20 | 0.5 | 150 | 20 | 1,223,335 | 0.1713 | 0.1353 | 1.27 |
| 9 | 0.5 | 20 | 0.5 | 150 | 20 | 1,223,335 | 0.1475 | 0.1151 | 1.28 |

TABLE 12

| Samples ID | OMCTS Flow Rate (sccm) | Argon Flow Rate (sccm) | $O_2$ Flow Rate (sccm) | Power (W) | Plasma Duration (sec) | W/FM (kJ/kg) | O-Parameter | N-Parameter |
|---|---|---|---|---|---|---|---|---|
| Process Parameters | | | | | | | | |
| 1 | 1 | 20 | 0.5 | 20 | 20 | 85,730 | 0.343 | 0.436 |
| 2 | 1 | 20 | 0.5 | 40 | 20 | 171,460 | 0.267 | 0.408 |
| 3 | 1 | 20 | 0.5 | 60 | 20 | 257,190 | 0.311 | 0.457 |
| 4 | 1 | 20 | 0.5 | 80 | 20 | 342,919 | 0.270 | 0.421 |
| 5 | 1 | 20 | 0.5 | 100 | 20 | 428,649 | 0.177 | 0.406 |
| 6 | 1 | 20 | 0.5 | 150 | 20 | 642,973 | 0.151 | 0.453 |
| 7 | 1 | 20 | 0.5 | 150 | 20 | 642,973 | 0.151 | 0.448 |
| 8 | 0.5 | 20 | 0.5 | 150 | 20 | 1,223,335 | 0.134 | 0.623 |
| 9 | 0.5 | 20 | 0.5 | 150 | 20 | 1,223,335 | 0.167 | 0.609 |

**Example 33**

The purpose of this example was to evaluate the recoverability or drainage of a slightly viscous aqueous solution from glass, COP and coated vials,

This study evaluated the recovery of a 30 cps (centipoise) carbohydrate solution in water-for-injection from (A) an uncoated COP vial, (B) an $SiO_x$ + pH protective layer coated COP vial prepared according to the above Protocol for Coating Syringe Barrel Interior with $SiO_x$, followed by the Protocol for Coating Syringe Barrel Interior with OMCTS PH protective Coating or Layer, and (C) a glass vial.

2.0 ml of the carbohydrate solution was pipetted into 30 vials each of glass, COP and pH protective coated vials. The solution was aspirated from the vials with a 10 ml syringe, through a 23 gauge, 1.5" needle. The vials were tipped to one side as the solution was aspirated to maximize the amount recovered. The same technique and similar withdrawal time was used for all vials. The vials were weighed empty, after placing 2.0 ml of the solution to the vial and at the conclusion of aspirating the solution from the vial. The amount delivered to the vial (A) was determined by subtracting the weight of the empty vial from the weight of the vial with the 2.0 ml of solution. The weight of solution not recovered (B) was determined by subtracting the weight of the empty vial from the weight of the vials after aspirating the solution from the vial. The percent unrecovered was determined by dividing B by A and multiplying by 100.

It was observed during the aspiration of drug product that the glass vials remained wetted with the solution. The COP vial repelled the liquid and as the solution was aspirated from the vials. This helped with recovery but droplets were observed to bead on the sidewalls of the vials during the aspiration. The pH protective coated vials also repelled the liquid during aspiration but no beading of solution on the sidewalls was observed.

The conclusion was that pH protective coated vials do not wet with aqueous solutions as do glass vials, leading to superior recovery of drug product relative to glass. pH protective coated vials were not observed to cause beading of solution on sidewall during aspiration of aqueous products therefore coated vials performed better than uncoated COP vials in product recovery experiments.

**Example 34**

Syringe samples were produced as follows. A COC 8007 extended barrel syringe was produced according to the Protocol for Forming COC Syringe Barrel. An $SiO_x$ coating or layer was applied to some of the syringes according to the Protocol for coating COC Syringe Barrel Interior with $SiO_x$. A pH protective coating or layer was applied to the $SiO_x$ coated syringes according to the Protocol for Coating COC Syringe Barrel Interior with OMCTS Lubricity Coating, modified as follows. The OMCTS was supplied from a vaporizer, due to its low volatility. Argon carrier gas was used. The process conditions were set to the following:

- OMCTS - 3 sccm
- Argon gas - 65 sccm
- Power - 6 watts

- Time - 10 seconds

The coater was later determined to have a small leak while producing the samples identified in the Table, which resulted in an estimated oxygen flow of 1.0 sccm. The samples were produced without introducing oxygen.
The coatings produced according to these working examples are contemplated to function as primer coatings or layers, and also as protective coatings or layers to increase the shelf life of the vessels, compared to similar vessels provided with a barrier coating or layer but no pH protective coating or layer.

**PECVD Process for Trilayer Coating**

The PECVD trilayer coating described in this specification can be applied, for example, as follows for a 1 to 5 mL vessel. Two specific examples are 1 mL thermoplastic resin syringe and a 5 mL thermoplastic resin drug vial. Larger or smaller vessels will call for adjustments in parameters that a person of ordinary skill can carry out in view of the teaching of this specification.
The apparatus used is the PECVD apparatus with rotating quadrupole magnets as described generally in this specification.
The general coating parameter ranges, with preferred ranges in parentheses, for a trilayer coating for a 1 mL syringe barrel are shown in the PECVD Trilayer Process General Parameters Tables (1 mL syringe and 5 mL vial).

| PECVD Trilayer Process General Parameters Table (1 mL syringe) | | | | |
|---|---|---|---|---|
| **Parameter** | **Units** | **Tie** | **Barrier** | **pH Protective** |
| Power | W | 40-90 (60-80) | 140 | 40-90 (60-80) |
| TMDSO Flow | sccm | 1-10 (3-5) | None | 1-10 (3-5) |
| HMDSO Flow | sccm | None | 1.56 | None |
| $O_2$ Flow | sccm | 0.5-5 (1.5-2.5) | 20 | 0.5-5 (1.5-2.5) |
| Argon Flow | sccm | 40-120 (70-90) | 0 | 40-120 (70-90) |
| Ramp Time | seconds | None | None | None |
| Deposition Time | seconds | 0.1-10 (1-3) | 20 | 0.1-40 (15-25) |
| Tube Pressure | Torr | 0.01-10 (0.1-1.5) | 0.59 | 0.01-10 (0.1-1.5) |

| PECVD Trilayer Process General Parameters Table (5 mL vial) | | | | |
|---|---|---|---|---|
| **Parameter** | **Units** | **Adhesion** | **Barrier** | **Protection** |
| Power | W | 40-90 (60-80) | 140 | 40-90 (60-80) |
| TMDSO Flow | sccm | 1-10 (3-5) | None | 1-10 (3-5) |
| HMDSO Flow | sccm | None | 1.56 | None |
| $O_2$ Flow | sccm | 0.5-5 (1.5-2.5) | 20 | 0.5-5 (1.5-2.5) |
| Argon Flow | sccm | 40-120 (70-90) | 0 | 40-120 (70-90) |
| Ramp Time | seconds | None | None | None |
| Deposition Time | seconds | 0.1-10 (1-3) | 20 | 0.1-40 (15-25) |
| Tube Pressure | Torr | 0.01-10 (0.1-1.5) | 0.59 | 0.01-10 (0.1-1.5) |

**Example 35**

Examples of specific coating parameters that have been used for a 1 mL syringe and 5 mL vial are shown in the PECVD Trilayer Process Specific Parameters Tables (1 mL syringe and 5 mL vial):

| PECVD Trilayer Process Specific Parameters Table (1 mL syringe) | | | | |
|---|---|---|---|---|
| **Parameter** | **Units** | **Tie** | **Barrier** | **pH Protective** |
| Power | W | 70 | 140 | 70 |
| TMDSO Flow | sccm | 4 | None | 4 |
| HMDSO Flow | sccm | None | 1.56 | None |
| $O_2$ Flow | sccm | 2 | 20 | 2 |
| Argon Flow | sccm | 80 | 0 | 80 |
| Ramp Time | seconds | None | None | None |
| Deposition Time | seconds | 2.5 | 20 | 10 |
| Tube Pressure | Torr | 1 | 0.59 | 1 |

| PECVD Trilayer Process Specific Parameters Table (5 mL vial) | | | | |
|---|---|---|---|---|
| **Parameter** | **Units** | **Adhesion** | **Barrier** | **Protection** |
| Power | W | 20 | 40 | 20 |
| TMDSO Flow | sccm | 2 | 0 | 2 |
| HMDSO Flow | sccm | 0 | 3 | 0 |
| $O_2$ Flow | sccm | 1 | 50 | 1 |
| Argon Flow | sccm | 20 | 0 | 20 |
| Ramp Time | seconds | 0 | 2 | 2 |
| Deposition Time | seconds | 2.5 | 10 | 10 |
| Tube Pressure | Torr | 0.85 | 1.29 | 0.85 |

The O-parameter and N-parameter values for the pH protective coating or layer applied to the 1 mL syringe as described above are 0.34 and 0.55, respectively.

The O-parameter and N-parameter values for the pH protective coating or layer applied to the 5 mL vial are 0.24 and 0.63, respectively.

**Example 36**

Referring to Fig. 18 and Table, Example 36, the thickness uniformity at four different points along the length of a 1 mL syringe with a staked needle (present during PECVD deposition) and the indicated trilayer coating (avg. thicknesses: 38 nm adhesion or tie coating or layer; 55 nm barrier coating or layer, 273 nm pH protective coating or layer) is shown. The table shows individual layer thicknesses at the four marked points, showing adequate thickness of each layer at each point along the high profile syringe barrel.

**TABLE, Example 36**

| Syringe Location | Adhesion | Barrier | Protection |
|---|---|---|---|
| 1 | 46 | 75 | 343 |
| 2 | 38 | 55 | 273 |
| 3 | 86 | 47 | 493 |
| 4 | 42 | 25 | 287 |

Referring to FIG. 19, the plot maps the coating thickness over the portion of the cylindrical inner surface of the barrel shown in FIG. 18, as though unrolled to form a rectangle. The overall range of thickness of the trilayer coating is 572 plus or minus 89 nm.

FIG. 20 is a photomicrograph showing a cross-section of the trilayer coating on a COP syringe substrate at the

point 2 shown in FIG. 18.

A syringe having a coating similar to the trilayer coating of FIGS. 18-20 is tested for shelf life, using the silicon dissolution and extrapolation method described in this specification, compared to syringes having a bilayer coating (similar to the trilayer coating except lacking the tie coating or layer) and a monolayer coating which is just the pH protective coating or layer directly applied to the thermoplastic barrel of the syringe, with no barrier layer. The test solution was a 0.2% Tween, pH 8 phosphate buffer. The extrapolated shelf lives of the monolayer and trilayer coatings were similar and very long - on the order of 14 years. The shelf life of the syringes having a bilayer coating were much lower - less than two years. In other words, the presence of a barrier layer under the pH protective layer shortened the shelf life of the coating substantially, but the shelf life was restored by providing a tie coating or layer under the barrier layer, sandwiching the barrier coating or layer with respective $SiO_xC_y$ layers. The barrier layer is necessary to establish a gas barrier, so the monolayer coating would not be expected to provide adequate gas barrier properties by itself. Thus, only the trilayer coating had the combination of gas barrier properties and a long shelf life, even while in contact with a solution that would attack an exposed barrier coating or layer.

## Example 37

FIGS. 21 and 22 show a trilayer coating distribution for the 5 mL vial, which is much shorter in relation to its inner diameter and thus easier to coat uniformly, showing very little variation in coating thickness, with the great majority of the surface coated between 150 and 250 nm thickness of the trilayer, with only a small proportion of the container coated with between 50 and 250 nm of the trilayer.

## Example 38

Figure 23 shows the breakdown of coating thickness (nm) by vial location. The Vial Coating Distribution Table shows the uniformity of coating.

| Vial Coating Distribution Table | | | | |
|---|---|---|---|---|
| Vial Location | Adhesion | Barrier | Protection | Total Trilayer,nm |
| 1 | 13 | 29 | 77 | 119 |
| 2 | 14 | 21 | 58 | 93 |
| 3 | 25 | 37 | 115 | 177 |
| 4 | 35 | 49 | 158 | 242 |
| 5 | 39 | 49 | 161 | 249 |
| 6 | 33 | 45 | 148 | 226 |
| 7 | 31 | 29 | 153 | 213 |
| 8 | 48 | 16 | 218 | 282 |
| 9 | 33 | 53 | 155 | 241 |
| 10 | 31 | 29 | 150 | 210 |
| Average | 30 | 36 | 139 | 205 |

## Example 39

Fig. 24 is a visual test result showing the integrity of the trilayer vial coating described above. The three 5 mL cyclic olefin polymer (COC) vials of FIGS. 24 and 24A were respectively:

- uncoated (left vial),
- coated with the bilayer coating described in this specification (a barrier coating or layer plus a pH protective coating or layer - the second and third components of the trilayer coating) (center vial); and
- coated with the trilayer coating as described above (right vial).

The three vials were each exposed to 1 N potassium hydroxide for four hours, then exposed for 24 hours to a ruthenium oxide (RuO4) stain that darkens any exposed part of the thermoplastic vial unprotected by the coatings. The

high pH potassium hydroxide exposure erodes any exposed part of the barrier coating or layer at a substantial rate, greatly reduced, however by an intact pH protective coating or layer. In particular, the high pH exposure opens up any pinholes in the coating system. As FIG. 24 shows, the uncoated vial is completely black, showing the absence of any effective coating. The bilayer coating was mostly intact under the treatment conditions, but on microscopic inspection has many pinholes (illustrated by FIG. 24A) where the ruthenium stain reached the thermoplastic substrate through the coating. The overall appearance of the bilayer coating clearly shows visible "soiled" areas where the stain penetrated. The trilayer coating, however, protected the entire vial against penetration of the stain, and the illustrated vial remains clear after treatment. This is believed to be the result of sandwiching the barrier coating or layer between two layers of $SiO_xC_y$, which both protects the barrier layer against direct etching and against undercutting and removal of flakes of the barrier layer.

**Example 40**

A number of vessels were provided with water vapor barrier coatings or layers and/or oxygen barrier coatings or layers in accordance with embodiments of the present disclosure, and the vessels tested for a variety of performance properties.

Vessels made of COP, COC, polycarbonate, and CBC were divided into three stacks and then each stack was provided with a different coating, as illustrated in Figure 27. Using atomic layer deposition, the vessels in the first stack were provided with an aluminum oxide ($Al_2O_3$) water vapor barrier layer 300 having a thickness of about 15 nm. Using the same atomic layer deposition procedures, the vessels in the second stack were provided with an aluminum oxide water vapor barrier layer 300. However, the atomic layer deposition was ceased once the aluminum oxide water vapor barrier layer 300 had achieved a thickness of 13 to 14 nm. Then, an $SiO_2$ oxygen barrier layer 301 was applied on top of the aluminum oxide water vapor barrier layer 300. The oxygen barrier layer 301 was also applied using atomic layer deposition. The atomic layer deposition of the oxygen barrier layer 301 was ceased once the oxygen barrier layer had achieved a thickness of 1 to 2 nm. The vessels in the third stack were provided with both a water vapor barrier layer 300 and an oxygen barrier layer 301 using atomic layer deposition in the same manner as the vessels in the second stack. However, for the vessels in the third stack, the aluminum oxide water vapor barrier layer 300 was applied only to a thickness of 9 to 10 nm. The $SiO_2$ oxygen barrier layer 301 was once again applied to a thickness of 1 to 2 nm. Finally, a thin top coat of an adhesion layer of SiOx was applied by atomic layer deposition in order to promote adhesion of the pH protective layer. The vessels in the third stack were then provided with a pH protective layer 286, the pH protective layer comprising SiOxCy or SiNxCy wherein x is from about 0.5 to about 2.4 and y is from about 0.6 to about 3, using PECVD.

**Example 41**

The interior, i.e. lumen-facing, surfaces of samples of COP vessels coated with aluminum oxide water vapor barrier layer having a thickness of about 17 nm to about 19 nm, were tested for water vapor transmission rate (WVTR) following the Water Vapor Transmission Rate testing protocols described below and compared against an uncoated COP vessel. The results of water vapor transmission rate testing are shown in Figure 28.

**Example 42**

The interior, i.e. lumen-facing, surfaces of various vessels were coated with an SiOx (wherein x is from 1.5 to 2.9) oxygen barrier layer using atomic layer deposition and tested for oxygen transmission rate (OTR) relative to the same vessel coated with an SiOx (wherein x is from 1.5 to 2.9) oxygen barrier layer coated using PECVD. The containers included in this study were COP 1mL syringes, COP 0.5mL syringes, 10mL vials made with COP and CBC, and COP 2mL vials. The results of oxygen transmission rate testing are shown in Figure 29. These results demonstrate that an SiOx barrier layer produced by ALD may provide a vessel wall with significantly higher barrier properties than an SiOx barrier layer of the same thickness produced by PECVD.

**Example 43**

The interior, i.e. lumen-facing, surfaces of a number of vessels were coated by atomic layer deposition with a combination of $SiO_2$ and $Al_2O_3$ layers according to one of recipes A and B described below. More specifically, following recipe A or recipe B, a barrier coating having layers of SiO2 and layers of $Al_2O_3$ was prepared by thermal ALD at a temperature of 70 °C. Nitrogen gas was used as the carrier gas, though argon or another inert gas could easily be substituted. Both recipes utilized pulses of precursors, also known as "on" times, each of which is immediately followed by a purge time during which the introduced precursor may be fully deposited, also known as an "off" time.

| Recipe | coating structure | Stabilization time (min) | Deposition Temperature (°C) |
|---|---|---|---|
| A | substrate/Al2O3/SiO2 | 120 | 70 |
| B | substrate/SiO2/AL2O3/SiO2 | 40 | 70 |

| RECIPE A | | | | |
|---|---|---|---|---|
| No of stacks | No of Cycles | Precursor | Pulse time (s) **(ON)** | Purge time (s) **(OFF)** |
| 4 | 75 | TMA | 2 | 5 |
| | | TMA | 2 | 10 |
| | | H2O | 2 | 10 |
| | | H2O | 2 | 20 |
| | | | | |
| | 25 | ORTHRUS | 2 | 5 |
| | | ORTHRUS | 3 | 10 |
| | | O3 | 20 | 5 |
| | | O3 | 40 | 10 |
| Total cycles | 400 | | | |

| RECIPE B | | | | |
|---|---|---|---|---|
| No of stacks | No of Cycles | Precursor | Pulse time (s) **(ON)** | Purge time (s) **(OFF)** |
| 1 | 10 | ORTHUS | 3 | 20 |
| | | O3 | 40 | 20 |
| 4 | 50 | TMA | 2 | 2 |
| | | TMA | 2 | 30 |
| | | H2O | 2 | 2 |
| | | H2O | 2 | 30 |
| | | | | |
| | 10 | ORTHRUS | 2 | 2 |
| | | ORTHRUS | 3 | 20 |
| | | O3 | 20 | 2 |
| | | O3 | 40 | 20 |
| Total cycles | 250 | | | |

Vessels coated per one of the above recipes were then tested for oxygen barrier properties and/or water vapour barrier properties using the protocols described below.

**Example 44**

10 mL vials having vessel walls made from polycarbonate (identified as PC in the tables) were coated with SiO2 and Al2O3 layers according to recipes A and B described above. The coated vials, as well as an uncoated polycarbonate control vial, were tested to determine a water vapour transmission rate and an oxygen transmission rate using the protocols described below. For each vessel type, the individual test results were averaged. The results of that testing is shown in the table below and in Figures 86 and 87.

| Article | Material | Coating Recipe | WVTR Averages | | OTR Averages | |
|---|---|---|---|---|---|---|
| | | | WVTR (mg/package/day) | WVTR Std Dev | OTR Constant ($d^{-1}$) | OTR Std Dev |
| 10mL Vial | PC | B | 1.88 | 0.21 | 0.00013 | 0.00004 |
| 10mL Vial | PC | UNC | 8.27 | 0.43 | 0.01026 | 0.00090 |
| 10mL Vial | PC | B | 2.50 | 0.25 | 0.00235 | 0.00068 |
| 10mL Vial | PC | A | 3.47 | 0.42 | 0.00024 | 0.00009 |
| 10mL Vial | N2 plasma Pretreated PC | A | 3.59 | 0.28 | 0.00010 | 0.00002 |
| 10mL Vial | Ar plasma Pretreated PC | A | 3.74 | 0.27 | 0.00009 | 0.00003 |
| 10mL Vial | PC Pre-heated | A | 4.38 | 0.39 | 0.00009 | 0.00003 |

**Example 45**

10 mL vials having vessel walls made from one of cyclic olefin polymer (identified as COP in the tables), cyclic olefin copolymer (identified as COC in the table), or cyclic block copolymer (identified as CBC in the tables) were coated with $SiO_2$ and $Al_2O_3$ layers according to recipes A and B described above. The coated vials, as well as an uncoated COP, COC, and CBC control vials, were tested to determine a water vapour transmission rate and an oxygen transmission rate using the protocols described below. For each vessel type, the individual test results were averaged. The results of that testing is shown in the table below and in Figures 88 and 89.

| Article | Material | Coating Recipe | WVTR Averages | | OTR Averages | |
|---|---|---|---|---|---|---|
| | | | WVTR (mg/package/day) | WVTR Std Dev | OTR Constant ($d^{-1}$) | OTR Std Dev |
| 10mL Vial | COC | UNC | 0.15 | 0.03 | 0.00795 | 0.00095 |
| 10mL Vial | COC | B | 0.24 | 0.02 | 0.00011 | 0.00002 |
| 10mL Vial | COP | UNC | 0.25 | 0.01 | 0.00612 | 0.00028 |
| 10mL Vial | COP | B | 0.18 | 0.03 | 0.00010 | 0.00003 |
| 10mL Vial | COP Pre-heated | A | 0.13 | 0.03 | 0.00008 | 0.00003 |
| 10mL Vial | COC Pre-heated | A | 0.20 | 0.03 | 0.00008 | 0.00002 |
| 10mL Vial | CBC | A | 0.35 | - | 0.00003 | - |
| 10mL Vial | CBC | UNC | 0.92 | - | 0.00250 | - |

**Example 46**

A variety of differently sized syringe barrels - 0.3 mL, 0.5 mL, and 1 mL syringe barrels - having vessel walls made from cyclic olefin polymer (identified as COP in the tables) were coated with $SiO_2$ and $Al_2O_3$ layers according to recipe B described above. The coated syringes, as well as an uncoated control syringe, were tested to determine an oxygen transmission rate using the protocol described below. For each vessel type, the individual test results were averaged. The results of that testing is shown in the table below and in Figure 90.

| Article | Material | Coating | WVTR Averages | | OTR Averages | |
|---|---|---|---|---|---|---|
| | | | WVTR (mg/package/day) | WVTR Std Dev | OTR Constant ($d^{-1}$) | OTR Std Dev |
| 0.5mL Syringe | COP | UNC | - | - | 0.03412 | 0.00570 |
| 0.5mL Syringe | COP | B | - | - | 0.00046 | 0.00010 |

(continued)

| Article | Material | Coating | WVTR Averages | | OTR Averages | |
|---|---|---|---|---|---|---|
| | | | WVTR (mg/package/day) | WVTR Std Dev | OTR Constant ($d^{-1}$) | OTR Std Dev |
| 1mL Syringe | COP | UNC | - | - | 0.02650 | 0.00871 |
| 1mL Syringe | COP | B | - | - | 0.00035 | 0.00012 |
| 0.3mL Syringe | COP | UNC | - | - | 0.03282 | 0.00054 |
| 0.3mL Syringe | COP | B | - | - | 0.00205 | 0.00028 |
| 0.5mL Syringe | COP | B | - | - | 0.00147 | 0.00028 |

**Example 47**

9 mL blood collection tubes having vessel walls made from one of cyclic olefin polymer (identified as COP in the tables) or cyclic block copolymer (identified as CBC in the tables) were coated with $SiO_2$ and $Al_2O_3$ layers according to recipe A described above. The coated blood collection tubes, as well as uncoated COP and CBC control tubes, were tested to determine a water vapour transmission rate and an oxygen transmission rate using the protocols described below. For each vessel type, the individual test results were averaged. The results of that testing is shown in the table below and in Figures 91 and 92.

| Article | Material | Coating | WVTR Averages | | OTR Averages | |
|---|---|---|---|---|---|---|
| | | | WVTR (mg/package/day) | WVTR Std Dev | OTR Constant ($d^{-1}$) | OTR Std Dev |
| 9mL Blood Tubes | COP | A | 0.06 | - | 0.00014 | - |
| 9mL Blood Tubes | COP | UNC | 0.10 | - | 0.01300 | - |
| 9mL Blood Tubes | CBC | A | 0.10 | - | 0.00014 | - |
| 9mL Blood Tubes | CBC | UNC | 0.16 | - | 0.04170 | - |

**Protocol for Total Silicon Measurement**

This protocol is used to determine the total amount of silicon coatings present on the entire vessel wall. A supply of 0.1 N potassium hydroxide (KOH) aqueous solution is prepared, taking care to avoid contact between the solution or ingredients and glass. The water used is purified water, 18 M'Ω quality. A Perkin Elmer Optima Model 7300DV ICP-OES instrument is used for the measurement except as otherwise indicated.

Each device (vial, syringe, tube, or the like) to be tested and its cap and crimp (in the case of a vial) or other closure are weighed empty to 0.001 g, then filled completely with the KOH solution (with no headspace), capped, crimped, and reweighed to 0.001g. In a digestion step, each vial is placed in an autoclave oven (liquid cycle) at 121°C for 1 hour. The digestion step is carried out to quantitatively remove the silicon coatings from the vessel wall into the KOH solution. After this digestion step, the vials are removed from the autoclave oven and allowed to cool to room temperature. The contents of the vials are transferred into ICP tubes. The total Si concentration is run on each solution by ICP/OES following the operating procedure for the ICP/OES.

The total Si concentration is reported as parts per billion of Si in the KOH solution. This concentration represents the total amount of silicon coatings that were on the vessel wall before the digestion step was used to remove it.

The total Si concentration can also be determined for fewer than all the silicon layers on the vessel, as when an SiOx barrier layer is applied, an SiOxCy second layer (for example, a lubricity layer or a primer coating or layer) is then applied, and it is desired to know the total silicon concentration of just the SiOxCy layer. This determination is made by preparing two sets of vessels, one set to which only the SiOx layer is applied and the other set to which the same SiOx layer is applied, followed by the SiOxCy layer or other layers of interest. The total Si concentration for each set of vessels is determined in the same manner as described above. The difference between the two Si concentrations is the total Si concentration of the $SiO_xC_y$ second layer.

## Protocol for Measuring Dissolved Silicon in a Vessel

In some of the working examples, the amount of silicon dissolved from the wall of the vessel by a test solution is determined, in parts per billion (ppb), for example to evaluate the dissolution rate of the test solution. This determination of dissolved silicon is made by storing the test solution in a vessel provided with an $SiO_x$ and/or $SiO_xC_y$ coating or layer under test conditions, then removing a sample of the solution from the vessel and testing the Si concentration of the sample. The test is done in the same manner as the Protocol for Total Silicon Measurement, except that the digestion step of that protocol is replaced by storage of the test solution in the vessel as described in this protocol. The total Si concentration is reported as parts per billion of Si in the test solution

## Protocol for Determining Average Dissolution Rate

The average dissolution rates reported in the working examples are determined as follows. A series of test vessels having a known total total silicon

measurement are filled with the desired test solution analogous to the manner of filling the vials with the KOH solution in the Protocol for Total Silicon Measurement. (The test solution can be a physiologically inactive test solution as employed in the present working examples or a physiologically active pharmaceutical preparation intended to be stored in the vessels to form a pharmaceutical package). The test solution is stored in respective vessels for several different amounts of time, then analyzed for the Si concentration in parts per billion in the test solution for each storage time. The respective storage times and Si concentrations are then plotted. The plots are studied to find a series of substantially linear points having the steepest slope.

The plot of dissolution amount (ppb Si) versus days decreases in slope with time, even though it does not appear thatthe Si layer has been fully digested by the test solution.

For the PC194 test data in Table 10 below, linear plots of dissolution versus time data are prepared by using a least squares linear regression program to find a linear plot corresponding to the first five data points of each of the experimental plots. The slope of each linear plot is then determined and reported as representing the average dissolution rate applicable to the test, measured in parts per billion of Si dissolved in the test solution per unit of time.

## Protocol for Determining Calculated Shelf Life

The calculated shelf life values reported in the working examples are determined by extrapolation of the total silicon measurements and average dissolution rates, respectively determined as described in the Protocol for Total Silicon Measurement and the Protocol for Determining Average Dissolution Rate. The assumption is made that under the indicated storage conditions the $SiO_xC_y$ primer coating or layer will be removed at the average dissolution rate until the coating is entirely removed. Thus, the total silicon measurement for the vessel, divided by the dissolution rate, gives the period of time required for the test solution to totally dissolve the $SiO_xC_y$ coating. This period of time is reported as the calculated shelf life. Unlike commercial shelf life calculations, no safety factor is calculated. Instead, the calculated shelf life is the calculated time to failure.

It should be understood that because the plot of ppb Si versus hours decreases in slope with time, an extrapolation from relatively short measurement times to relatively long calculated shelf lives is believed to be a "worst case" test that tends to underestimate the calculated shelf life actually obtainable.

## Protocol for Measuring Water Vapor Transmission Rate

The water vapour transmission rates of the sealed containers reported in the working examples were measured using modified gravimetric testing based on the methodology set forth in USP <671>. The materails utilized include a desiccant, and in particular 3A 4X8 molecular sieve pellets, an analytical scale with a resolution of at least a tenth of a milligram, and an environmental chamber with the capability of maintaining an atmosphere of 75% relative humidity and 40 °C. Each container is weighed and then an amount of desiccant to fill each container to a level between 1/2 and 2/3 of its volume is weighed (e.g. for a 10 mL vial, the amount of dessicant used is 4 grams). The desiccant pellets are then placed into the container and the container is sealed. The sealed, desiccant-containing container is then weighed and the initial, or day zero, weight of the pellets is registered. Within one hour of weighing, the sealed, desiccant-containing containers are then placed in an environmental chamber that is set at 40.0 °C and 75.0% RH.

At weekly intervals (7 days ± 1 hour), the sealed, desiccant-containing container is removed from the environmental chamber, equilibrated to the weighing temperature and relative humidity for a period of time between about 30 minutes and one hour, and reweighed to determine the new weight of the pellets. The container is then placed back into the environmental chamber, with the total time it is outside of the environmental chamber during the weighing process being less than 2 hours. This weighing process is performed at weekly intervals five times over the course of 35

days in order to obtain steady-state data points for the weight of the pellets, with the time interval from day 0 to day 7 being a period of equilibration. The steady-state data points are plotted to produce the weight gain profile, which is the slope of the weight gain over time.

### Protocol for Measuring Oxygen Transmission Rate

The oxygen transmission rates of the sealed containers reported in the working examples were measured using a noninvasive optical measurement method using the Mocon OpTech $O_2$ Platinum System. An Optech oxygen-sensitive sensor is selected and inserted into the container with the adhesive side of the sensor being placed in contact with the interior surface of the vessel wall. The container is then sealed in a low oxygen environment inside of a glove box, which has been evacuated of oxygen and subjected to a nitrogen purge. For syringes, this involves inserting the plunger to a consistent depth in each syringe (e.g. for 1 mL syringes, the plunger is inserted about 14.0 mm from the flange) and coating the needle (which may be cut first) in epoxy. The sealed container is then removed from the glove box and stored at room temperature, e.g. 20-25 °C.

To obtain an oxygen reading, the OpTech Probe is properly calibrated and then the container is placed into the container nest of the OpTech system, with the probe and the sensor aligned and with the distance between the probe tip and the container wall where the sensor is attached being approximately 5 mm. The OpTech system is then activated and a reading taken. The container is tested in this manner twice per day (at relatively consistent times each day) for a period of about 7 days, producing 14 data points from which an oxygen transmission rate is calculated.

## Claims

**1.** A drug primary package comprising

- a vessel (210) comprising a lumen (212) defined at least in part by a wall (214), the wall (214) having an interior surface facing the lumen (212) and an outer surface (216);
- an oxygen barrier coating or layer (301) supported by at least one of the interior surface and the outer surface (216) of the wall (214), the oxygen barrier coating or layer (301) being effective to reduce the ingress of oxygen into the lumen (212) to less than 0.0005 cc/package/day at 25 °C, 60% relative humidity, and 0.21 bar;
- a water vapor barrier coating or layer (300) supported by at least one of the interior surface and the outer surface (216) of the wall (214), the water vapor barrier coating or layer (300) being effective to reduce the ingress of water vapor into the lumen (212) to less than 0.05 mg/package/day at 60 °C and 40% relative humidity;
- a fluid drug stored in the lumen,

**characterized in that**, at least one of the oxygen barrier coating or layer (301) and the water vapor barrier coating or layer (300) consist of a plurality of atomic monolayers of a pure element or compound.

**2.** The drug primary package of claim 1, in which the water vapor barrier coating or layer and the oxygen barrier coating or layer are located between the interior surface of the wall and the lumen.

**3.** The drug primary package of claim 2, in which the water vapor coating or layer is located between the interior surface of the wall and the oxygen barrier coating or layer, and the oxygen barrier coating or layer is located between the water vapor coating or layer and the lumen.

**4.** The drug primary package of any preceding claim 2 or 3, further comprising a pH protective coating or layer (286) between the lumen and both the water vapor barrier coating or layer and the oxygen barrier coating or layer, the pH protective coating or layer (286) being effective to increase the calculated shelf life of the vessel.

**5.** The drug primary package of claim 4, in which the fluid drug is in contact with the pH protective coating.

**6.** The drug primary package of any preceding claim, in which the oxygen barrier coating or layer (301) consists essentially of a plurality of atomic monolayers of a pure element or compound.

**7.** The drug primary package of any preceding claim, in which the water vapor barrier coating or layer consists essentially of a plurality of atomic monolayers of a pure element or compound.

**8.** The drug primary package of any preceding claim, in which the wall (214) consists essentially of thermoplastic material.

9. The drug primary package of claim 8, in which the thermoplastic material consists essentially of a commodity resin.

10. The drug primary package of claim 9, in which the commodity resin consists essentially of PET, PETG, polypropylene, a polyamide, polystyrene, polycarbonate, TRITAN™, a cyclic block copolymer (CBC) resin, or a thermoplastic olefinic polymer, or any combination thereof.

11. The drug primary package of any preceding claim, in which the pure element or compound of at least one atomic monolayer is Al2O3, AlxTiyOz, HfO2, In2O3, MgO, SiO2, SrTiOx, Ta2O5, TiO2, Y2O3, ZnO, ZnO:Al, ZrO2, La2O3, or CeO2.

12. The drug primary package of any preceding claim 4-11, in which the pH protective coating consists essentially of a plasma enhanced chemical vapor deposition (PECVD) coating of SiOxCy, in which x is from about 0.5 to about 2.4 and y is from about 0.6 to about 3.

13. The drug primary package of any preceding claim further comprising a lubricity coating or layer (288) supported by the interior surface of the wall (214).

14. The drug primary package of claim 13 in which the lubricity coating or layer (288) consists essentially of SiOxCy, in which x is from about 0.5 to about 2.4 and y is from about 0.6 to about 3.

15. The drug primary package of any one of claims 13 to 14, in which the vessel (210) is a syringe barrel (250) and the lubricity coating or layer (288) provides (i) a lower plunger sliding force, (ii) a lower plunger breakout force, or (iii) both (i) and (ii), when compared against the same syringe barrel but lacking the lubricity coating or layer (288).

**Patentansprüche**

1. Arzneimittelprimärverpackung, umfassend

- einen Behälter (210), der ein Lumen (212) umfasst, das zumindest teilweise durch eine Wand (214) definiert ist, wobei die Wand (214) eine Innenfläche, die dem Lumen (212) zugewandt ist, und eine Außenfläche (216) aufweist;
- eine Sauerstoffbarrierebeschichtung oder -schicht (301), die von mindestens einer der Innenfläche und der Außenfläche (216) der Wand (214) getragen wird, wobei die Sauerstoffbarrierebeschichtung oder -schicht (301) wirksam ist, um das Eindringen von Sauerstoff in das Lumen (212) auf weniger als 0,0005 $cm^3$/Verpackung/Tag bei 25 °C, 60 % relativer Feuchtigkeit und 0,21 bar zu reduzieren;
- eine Wasserdampfbarrierebeschichtung oder -schicht (300), die von mindestens einer der Innenfläche und der Außenfläche (216) der Wand (214) getragen wird, wobei die Wasserdampfbarrierebeschichtung oder -schicht (300) wirksam ist, um das Eindringen von Wasserdampf in das Lumen (212) auf weniger als 0,05 mg/Verpackung/Tag bei 60 °C und 40 % relativer Feuchtigkeit zu reduzieren;
- ein flüssiges Medikament, das im Lumen gespeichert ist,

**dadurch gekennzeichnet, dass** mindestens eine der Sauerstoffbarrierebeschichtung oder -schicht (301) und der Wasserdampfbarrierebeschichtung oder -schicht (300) aus einer Vielzahl von atomaren Monoschichten eines reinen Elements oder einer reinen Verbindung besteht.

2. Arzneimittelprimärverpackung nach Anspruch 1, wobei die Wasserdampfbarrierebeschichtung oder -schicht und die Sauerstoffbarrierebeschichtung oder -schicht zwischen der Innenfläche der Wand und dem Lumen angeordnet sind.

3. Arzneimittelprimärverpackung nach Anspruch 2, wobei die Wasserdampfbarrierebeschichtung oder -schicht zwischen der Innenfläche der Wand und der Sauerstoffbarrierebeschichtung oder -schicht angeordnet ist und die Sauerstoffbarrierebeschichtung oder -schicht zwischen der Wasserdampfbarrierebeschichtung oder -schicht und dem Lumen angeordnet ist.

4. Arzneimittelprimärverpackung nach einem der vorhergehenden Ansprüche 2 oder 3, ferner umfassend eine pH-Schutzbeschichtung oder -schicht (286) zwischen dem Lumen und sowohl der Wasserdampfbarrierebeschichtung oder -schicht als auch der Sauerstoffbarrierebeschichtung oder -schicht, wobei die pH-Schutzbeschichtung oder -schicht (286) wirksam ist, um die berechnete Haltbarkeit des Behälters zu erhöhen.

5. Arzneimittelprimärverpackung nach Anspruch 4, wobei das flüssige Arzneimittel in Kontakt mit der pH-Schutzbeschichtung ist.

6. Arzneimittelprimärverpackung nach einem der vorhergehenden Ansprüche, wobei die Sauerstoffbarrierebeschichtung oder - schicht (301) im Wesentlichen aus einer Vielzahl von atomaren Monoschichten eines reinen Elements oder einer reinen Verbindung besteht.

7. Arzneimittelprimärverpackung nach einem der vorhergehenden Ansprüche, wobei die Wasserdampfbarrierebeschichtung oder - schicht im Wesentlichen aus einer Vielzahl von atomaren Monoschichten eines reinen Elements oder einer reinen Verbindung besteht.

8. Arzneimittelprimärverpackung nach einem der vorhergehenden Ansprüche, wobei die Wand (214) im Wesentlichen aus thermoplastischem Material besteht.

9. Arzneimittelprimärverpackung nach Anspruch 8, wobei das thermoplastische Material im Wesentlichen aus einem Harzwerkstoff besteht.

10. Arzneimittelprimärverpackung nach Anspruch 9, wobei der Harzwerkstoff im Wesentlichen aus PET, PETG, Polypropylen, einem Polyamid, Polystyrol, Polycarbonat, TRITAN™, einem cyclischen Blockcopolymer(CBC)-Harz oder einem thermoplastischen Olefinpolymer oder einer Kombination davon besteht.

11. Arzneimittelprimärverpackung nach einem der vorhergehenden Ansprüche, wobei das reine Element oder die reine Verbindung von mindestens einer atomaren Monoschicht Al2O3, AlxTiyOz, HfO2, In2O3,MgO, SiO2, SrTiOx, Ta2O5, TiO2, Y2O3, ZnO, ZnO:Al, Zr02, La203 oder CeO2 ist.

12. Arzneimittelprimärverpackung nach einem der vorhergehenden Ansprüche 4 bis 11, wobei die pH-Schutzbeschichtung im Wesentlichen aus einer plasmaunterstützten chemischen Dampfabscheidungsbeschichtung (PECVD) aus SiOxCy besteht, wobei x etwa 0,5 bis etwa 2,4 und y etwa 0,6 bis etwa 3 beträgt.

13. Arzneimittelprimärverpackung nach einem der vorhergehenden Ansprüche, ferner umfassend eine Schmierfähigkeitsbeschichtung oder -schicht (288), die von der Innenfläche der Wand (214) getragen wird.

14. Arzneimittelprimärverpackung nach Anspruch 13, wobei die Schmierfähigkeitsbeschichtung oder -schicht (288) im Wesentlichen aus SiOxCy besteht, wobei x etwa 0,5 bis etwa 2,4 und y etwa 0,6 bis etwa 3 beträgt.

15. Arzneimittelprimärverpackung nach einem der Ansprüche 13 bis 14, wobei der Behälter (210) ein Spritzenzylinder (250) ist und die Schmierfähigkeitsbeschichtung oder -schicht (288) beim Vergleich mit demselben Spritzenzylinder, bei dem jedoch die Schmierfähigkeitsbeschichtung oder -schicht (288) fehlt, (i) eine niedrigere Kolbengleitkraft, (ii) eine niedrigere Kolbenausbrechkraft oder (iii) sowohl (i) als auch (ii) bereitstellt.

## Revendications

1. Emballage primaire pour médicament comprenant

 • un récipient (210) comprenant une lumière (212) définie au moins en partie par une paroi (214), la paroi (214) ayant une surface intérieure faisant face à la lumière (212) et une surface extérieure (216) ;
 • un enrobage ou une couche de barrière à l’oxygène (301) supportés par au moins l’une parmi la surface intérieure et la surface extérieure (216) de la paroi (214), l’enrobage ou la couche de barrière à l’oxygène (301) étant efficaces pour réduire l’entrée d’oxygène dans la lumière (212) à moins de 0,0005 cm3/emballage/jour à 25 °C, 60 % d’humidité relative et 0,21 bar ;
 • un enrobage ou une couche de barrière à la vapeur d’eau (300) supportés par au moins l’une parmi la surface intérieure et la surface extérieure (216) de la paroi (214), l’enrobage ou la couche de barrière à la vapeur d’eau (300) étant efficaces pour réduire l’entrée de vapeur d’eau dans la lumière (212) à moins de 0,05 mg/emballage/jour à 60 °C et 40 % d’humidité relative ;
 • un médicament fluide stocké dans la lumière,

**caractérisé en ce qu’**au moins l’un parmi l’enrobage ou la couche de barrière à l’oxygène (301) et l’enrobage ou la

couche de barrière à la vapeur d'eau (300) sont constitués d'une pluralité de monocouches atomiques d'un élément ou composé pur.

2. Emballage primaire de médicament selon la revendication 1, dans lequel l'enrobage ou la couche de barrière à la vapeur d'eau et l'enrobage ou la couche de barrière à l'oxygène sont situés entre la surface intérieure de la paroi et la lumière.

3. Emballage primaire de médicament selon la revendication 2, dans lequel l'enrobage ou la couche de vapeur d'eau sont situés entre la surface intérieure de la paroi et l'enrobage ou la couche de barrière à l'oxygène, et l'enrobage ou la couche de barrière à l'oxygène sont situés entre l'enrobage ou la couche de vapeur d'eau et la lumière.

4. Emballage primaire de médicament selon l'une quelconque des revendications précédentes 2 ou 3, comprenant en outre un enrobage ou une couche de protection du pH (286) entre la lumière et à la fois l'enrobage ou la couche de barrière à la vapeur d'eau et l'enrobage ou la couche de barrière à l'oxygène, l'enrobage ou la couche de protection de pH (286) étant efficaces pour augmenter la durée de conservation calculée du récipient.

5. Emballage primaire de médicament selon la revendication 4, dans lequel le médicament fluide est en contact avec l'enrobage de protection de pH.

6. Emballage primaire de médicament selon l'une quelconque des revendications précédentes, dans lequel l'enrobage ou la couche de barrière à l'oxygène (301) sont constitués essentiellement d'une pluralité de monocouches atomiques d'un élément ou composé pur.

7. Emballage primaire de médicament selon l'une quelconque des revendications précédentes, dans lequel l'enrobage ou la couche de barrière à la vapeur d'eau sont constitués essentiellement d'une pluralité de monocouches atomiques d'un élément ou composé pur.

8. Emballage primaire de médicament selon l'une quelconque des revendications précédentes, dans lequel la paroi (214) est constituée essentiellement d'un matériau thermoplastique.

9. Emballage primaire de médicament selon la revendication 8, dans lequel le matériau thermoplastique est constitué essentiellement d'une résine pour usage courant.

10. Emballage primaire de médicament selon la revendication 9, dans lequel la résine pour usage courant est constituée essentiellement de PET, PETG, polypropylène, un polyamide, polystyrène, polycarbonate, TRITAN™, une résine de copolymère séquencé cyclique (CBC), ou un polymère oléfinique thermoplastique, ou toute combinaison de ceux-ci.

11. Emballage primaire de médicament selon l'une quelconque des revendications précédentes, dans lequel l'élément ou composé pur d'au moins une monocouche atomique est Al2O3, AlxTiyOz, HfO2, In2O3,MgO, SiO2, SrTiOx, Ta2O5, TiO2, Y2O3, ZnO, ZnO:Al, Zr02, La203, ou CeO2.

12. Emballage primaire de médicament selon l'une quelconque des revendications précédentes 4 à 11, dans lequel l'enrobage de protection de pH est constitué essentiellement d'un enrobage de SiOxCy par dépôt chimique en phase vapeur assisté par plasma (PECVD), dans lequel x est d'environ 0,5 à environ 2,4 et y est d'environ 0,6 à environ 3.

13. Emballage primaire de médicament selon l'une quelconque des revendications précédentes, comprenant en outre un enrobage ou une couche à pouvoir lubrifiant (288) supportés par la surface intérieure de la paroi (214).

14. Emballage primaire de médicament selon la revendication 13, dans lequel l'enrobage ou la couche à pouvoir lubrifiant (288) sont constitués essentiellement de SiOxCy, dans lequel x est d'environ 0,5 à environ 2,4 et y est d'environ 0,6 à environ 3.

15. Emballage primaire de médicament selon l'une quelconque des revendications 13 et 14, dans lequel le récipient (210) est un cylindre de seringue (250) et l'enrobage ou la couche à pouvoir lubrifiant (288) fournissent (i) une force de coulissement de piston inférieure, (ii) une force d'arrachement de piston inférieure, ou (iii) à la fois (i) et (ii), comparativement au même cylindre de seringue mais dépourvu de l'enrobage ou de la couche à pouvoir lubrifiant (288).

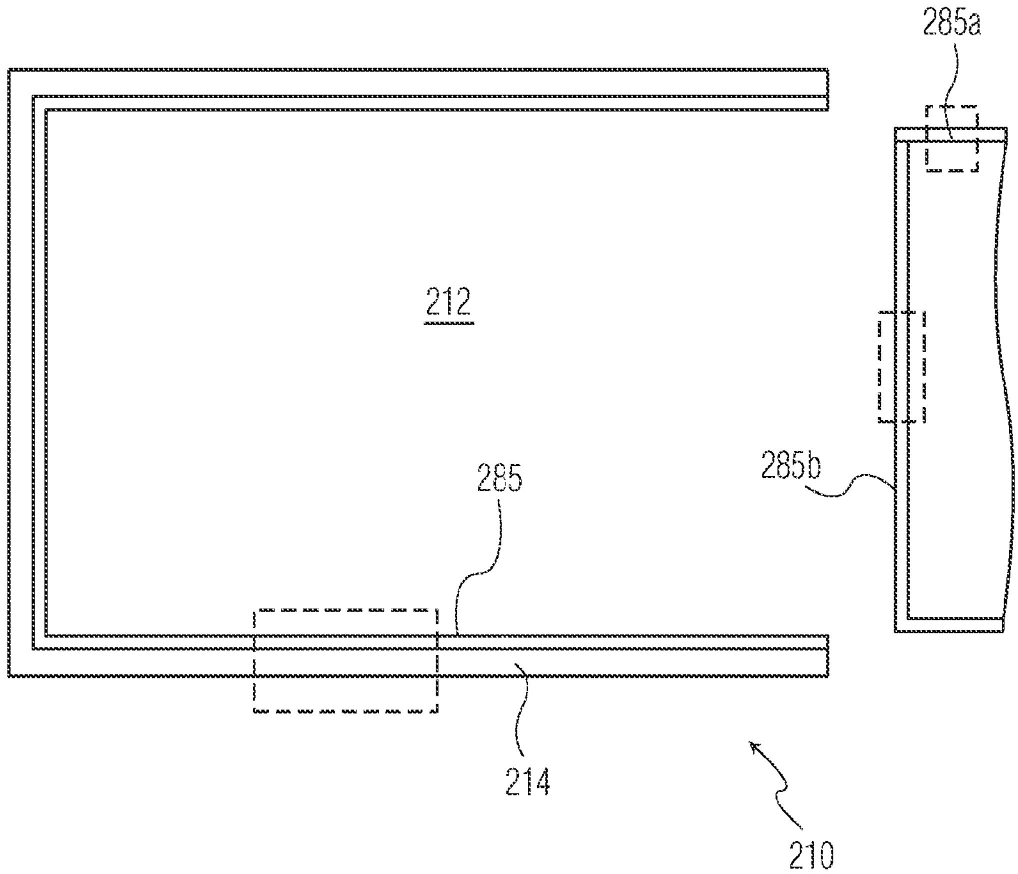


FIG. 1

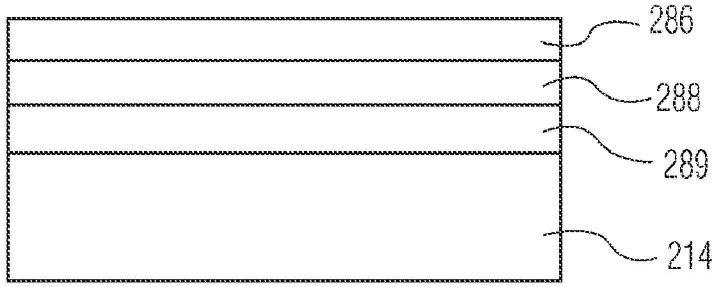


FIG. 2

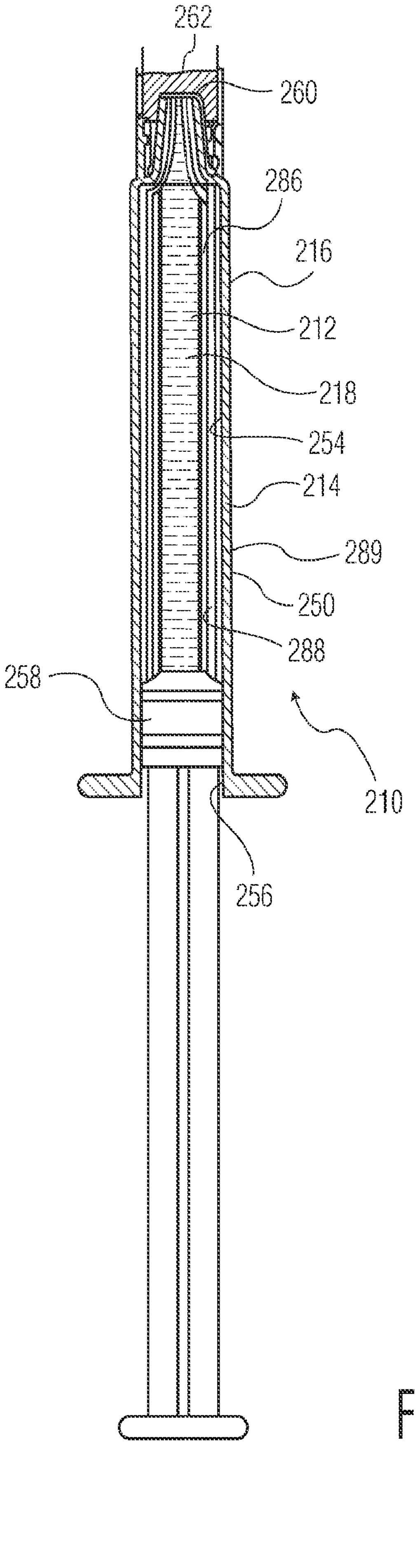


FIG. 3

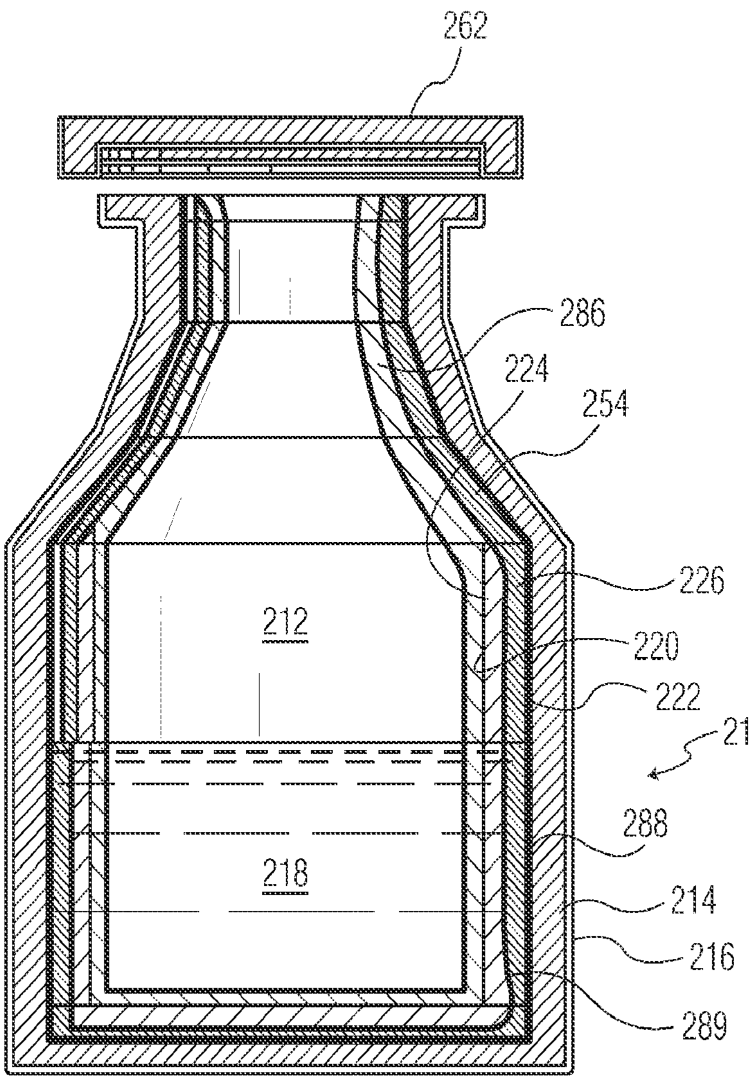
262
286
224
254
226
220
222
210
212
218
288
214
216
289

FIG. 4

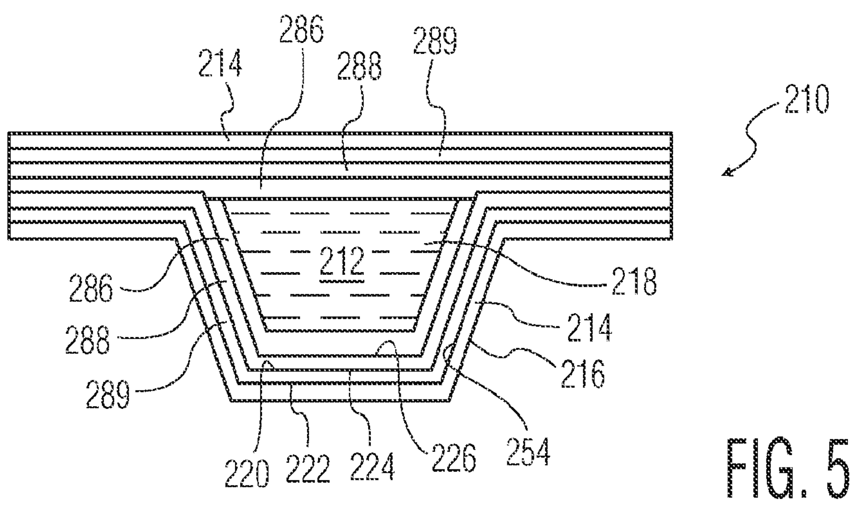
286
214
288
289
210
212
218
286
288
289
214
216
220
222
224
226
254

FIG. 5

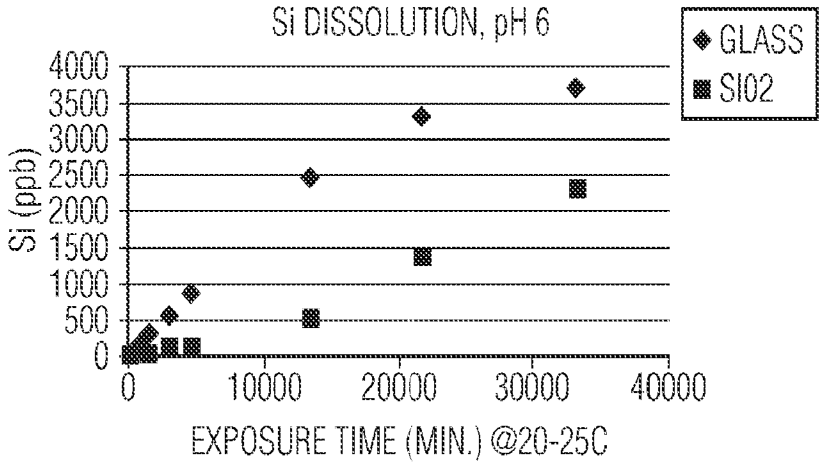
Si DISSOLUTION, pH 6
GLASS
SI02
Si (ppb)
4000
3500
3000
2500
2000
1500
1000
500
0
0
10000
20000
30000
40000
EXPOSURE TIME (MIN.) @20-25C

FIG. 6

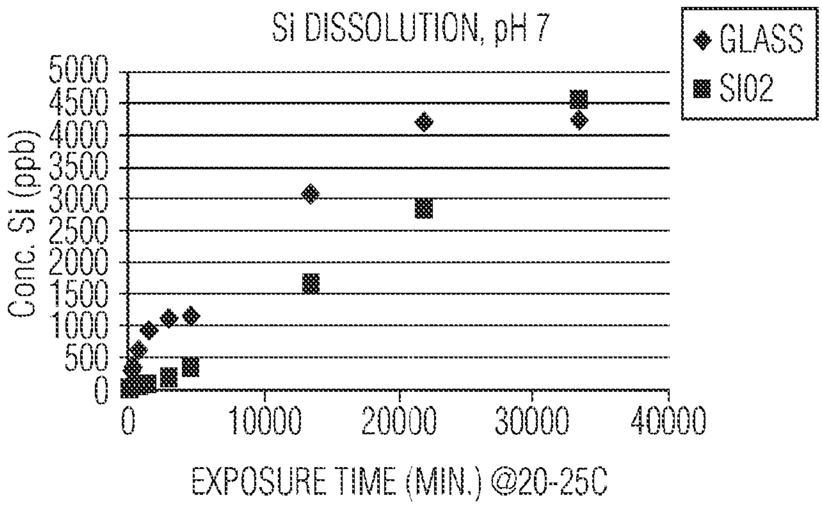
Si DISSOLUTION, pH 7
GLASS
SI02
Conc. Si (ppb)
5000
4500
4000
3500
3000
2500
2000
1500
1000
500
0
0
10000
20000
30000
40000
EXPOSURE TIME (MIN.) @20-25C

FIG. 7

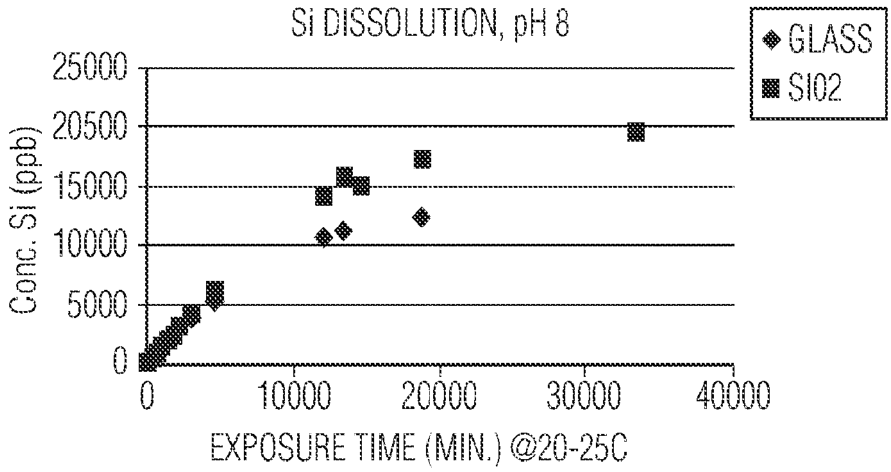
Si DISSOLUTION, pH 8
GLASS
SI02
25000
20500
15000
10000
5000
0
Conc. Si (ppb)
0
10000
20000
30000
40000
EXPOSURE TIME (MIN.) @20-25C

FIG. 8

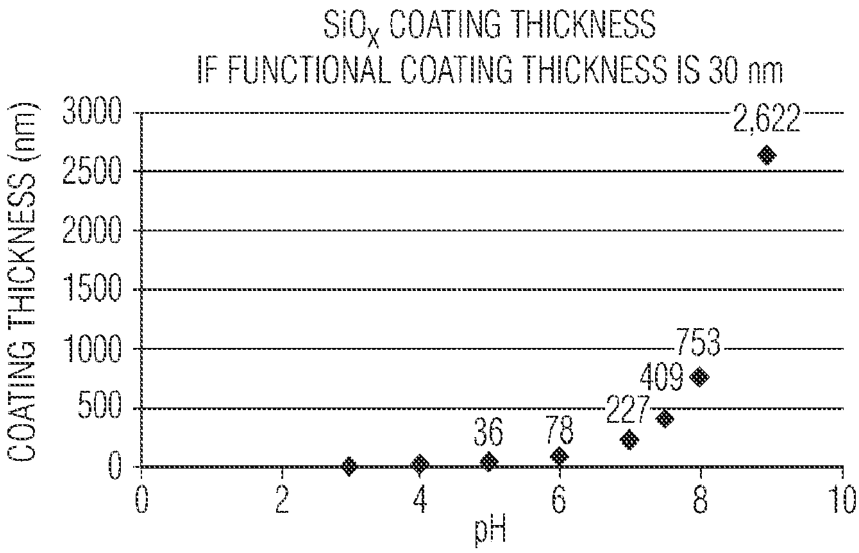
$SiO_x$ COATING THICKNESS
IF FUNCTIONAL COATING THICKNESS IS 30 nm
3000
2500
2000
1500
1000
500
0
COATING THICKNESS (nm)
2,622
753
409
227
78
36
0
2
4
6
8
10
pH

FIG. 9

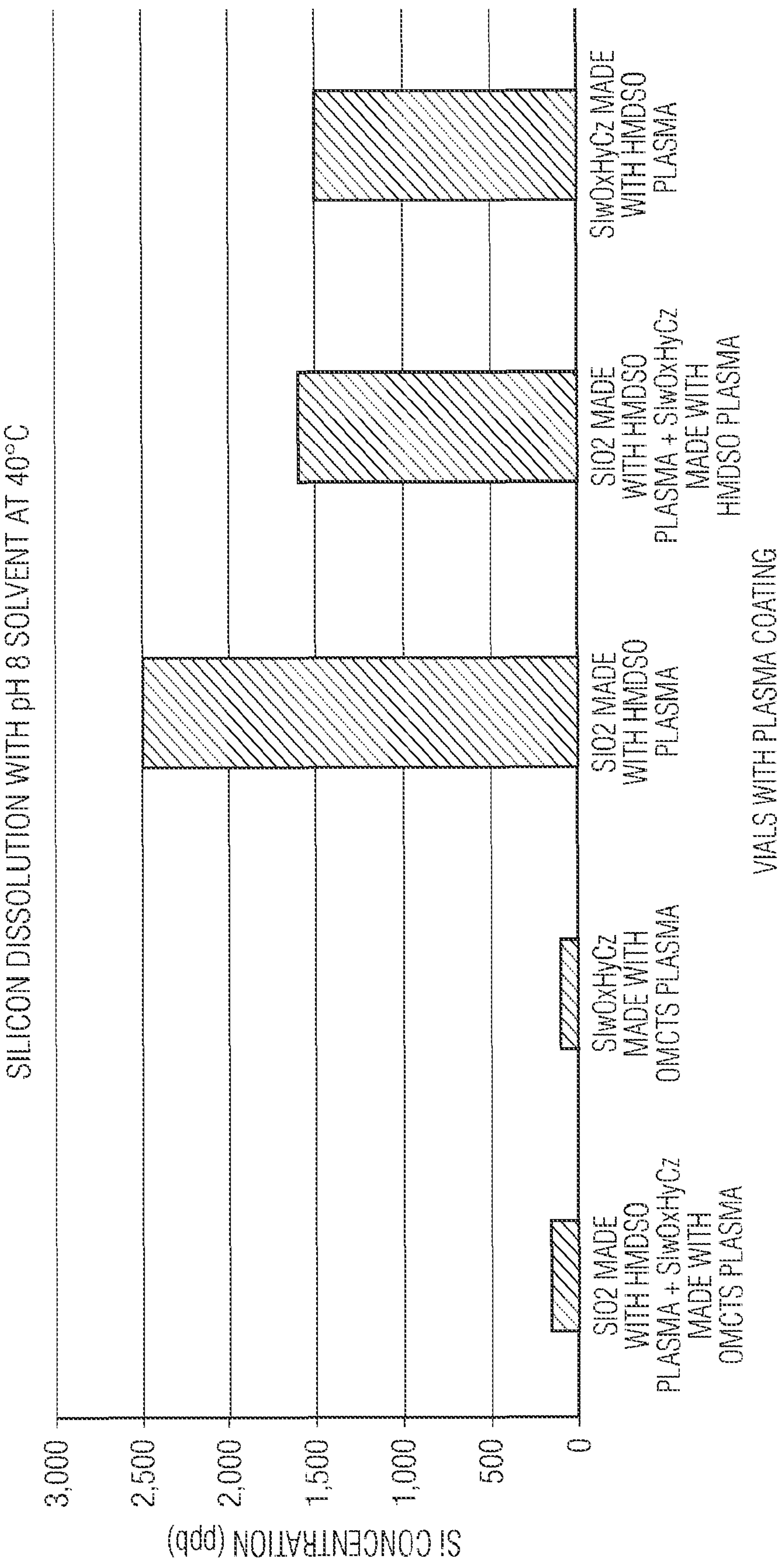
SILICON DISSOLUTION WITH pH 8 SOLVENT AT 40°C
Si CONCENTRATION (ppb)
3,000
2,500
2,000
1,500
1,000
500
0
SIO2 MADE WITH HMDSO PLASMA + SIwOxHyCz MADE WITH OMCTS PLASMA
SIwOxHyCz MADE WITH OMCTS PLASMA
SIO2 MADE WITH HMDSO PLASMA
SIO2 MADE WITH HMDSO PLASMA + SIwOxHyCz MADE WITH HMDSO PLASMA
SIwOxHyCz MADE WITH HMDSO PLASMA
VIALS WITH PLASMA COATING

FIG. 10

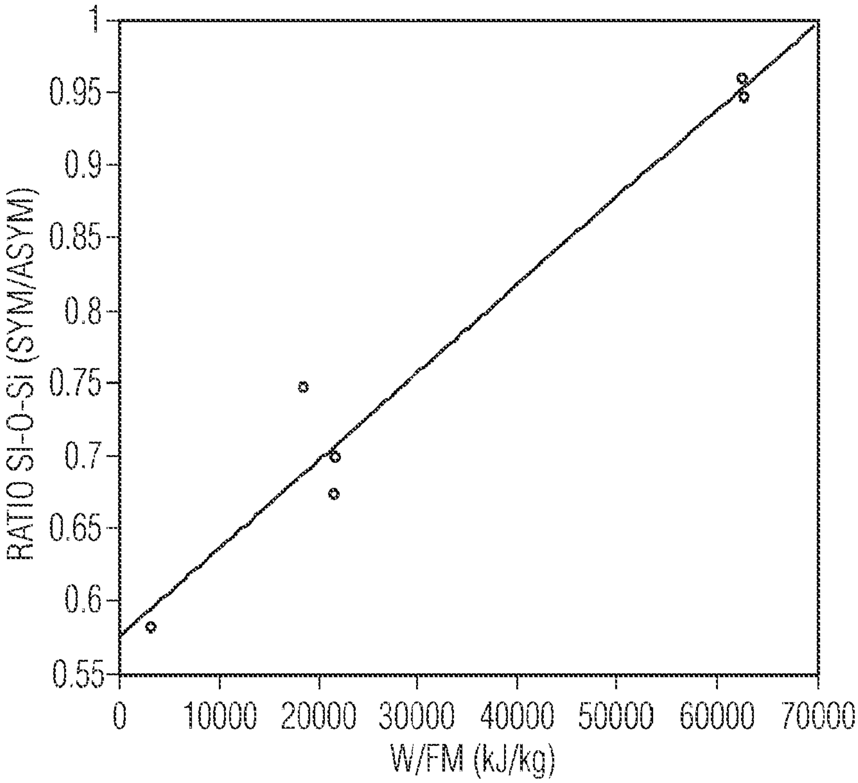
1
0.95
0.9
0.85
0.8
0.75
0.7
0.65
0.6
0.55
RATIO SI-O-SI (SYM/ASYM)
0
10000
20000
30000
40000
50000
60000
70000
W/FM (kJ/kg)

FIG. 11

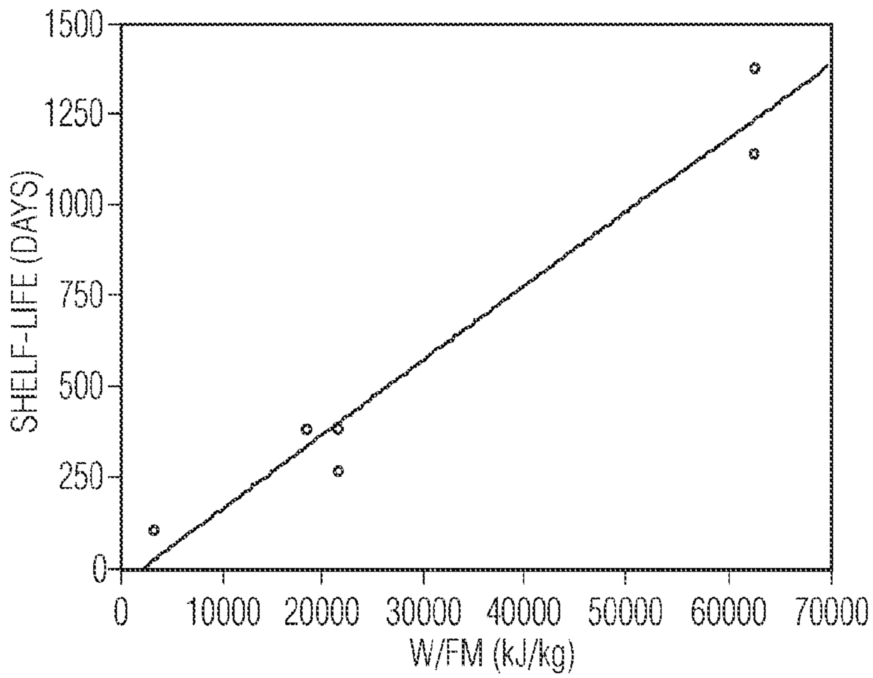
1500
1250
1000
750
500
250
0
SHELF-LIFE (DAYS)
0
10000
20000
30000
40000
50000
60000
70000
W/FM (kJ/kg)

FIG. 12

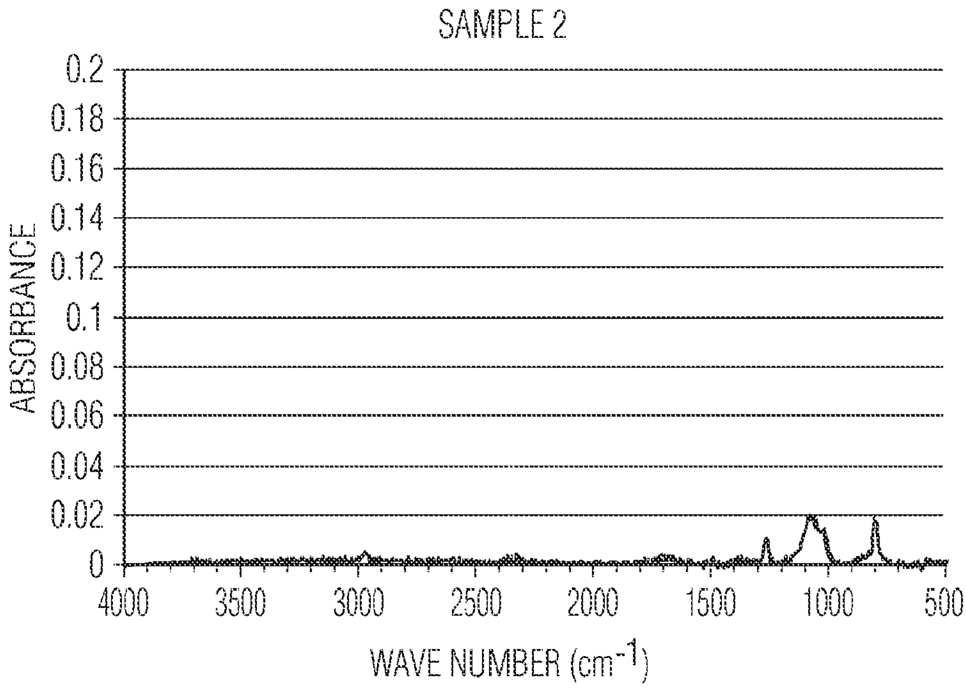
SAMPLE 2
ABSORBANCE
0.2
0.18
0.16
0.14
0.12
0.1
0.08
0.06
0.04
0.02
0
4000
3500
3000
2500
2000
1500
1000
500
WAVE NUMBER ($cm^{-1}$)

FIG. 13

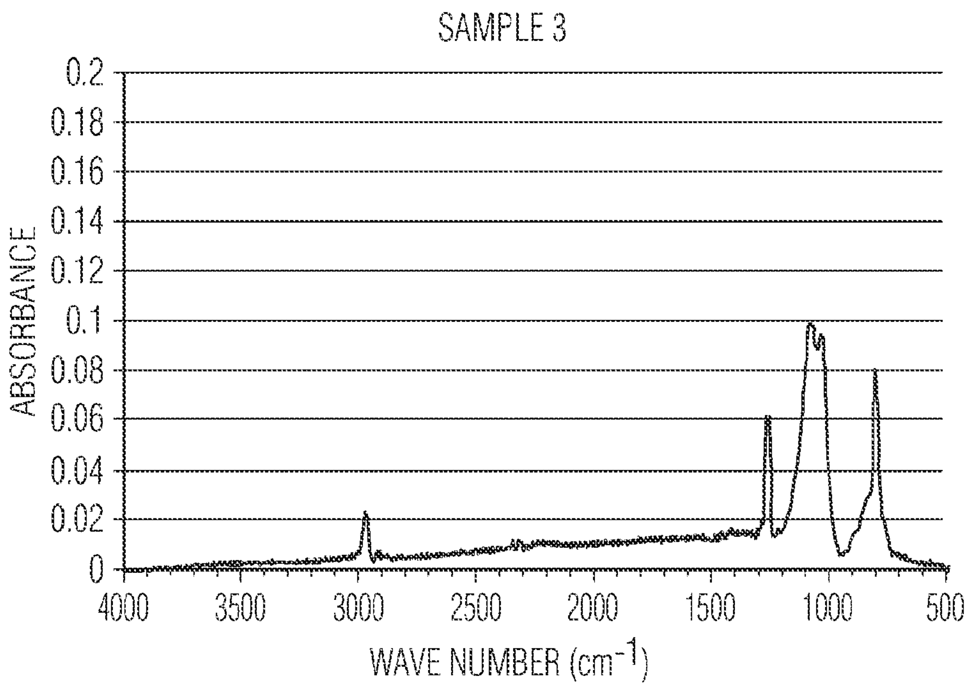
SAMPLE 3
ABSORBANCE
0.2
0.18
0.16
0.14
0.12
0.1
0.08
0.06
0.04
0.02
0
4000
3500
3000
2500
2000
1500
1000
500
WAVE NUMBER ($cm^{-1}$)

FIG. 14

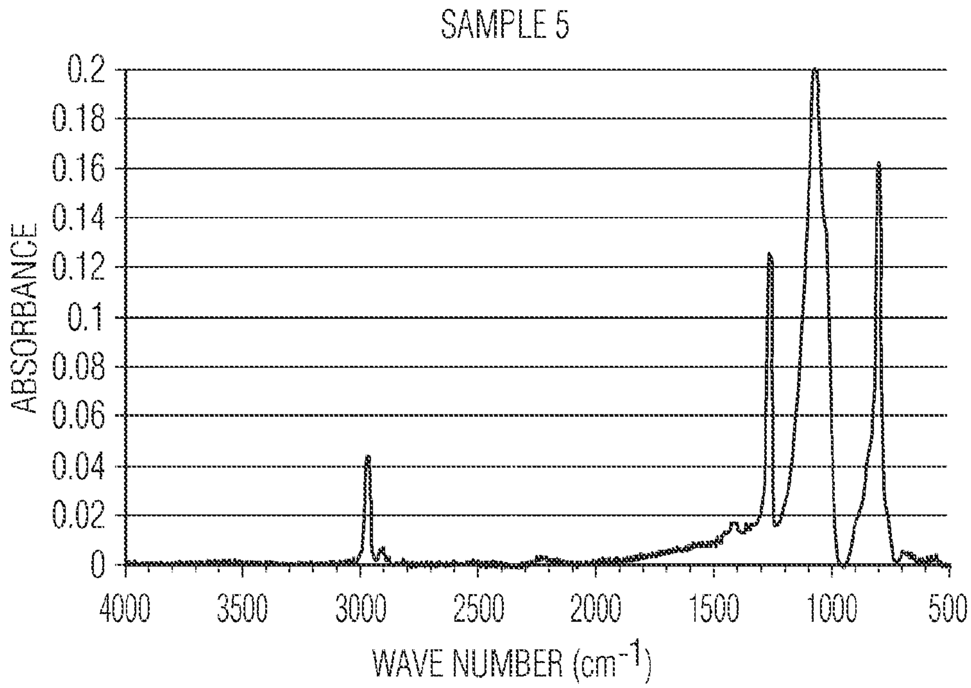
SAMPLE 5
ABSORBANCE
0.2
0.18
0.16
0.14
0.12
0.1
0.08
0.06
0.04
0.02
0
4000
3500
3000
2500
2000
1500
1000
500
WAVE NUMBER (cm-1)

FIG. 15

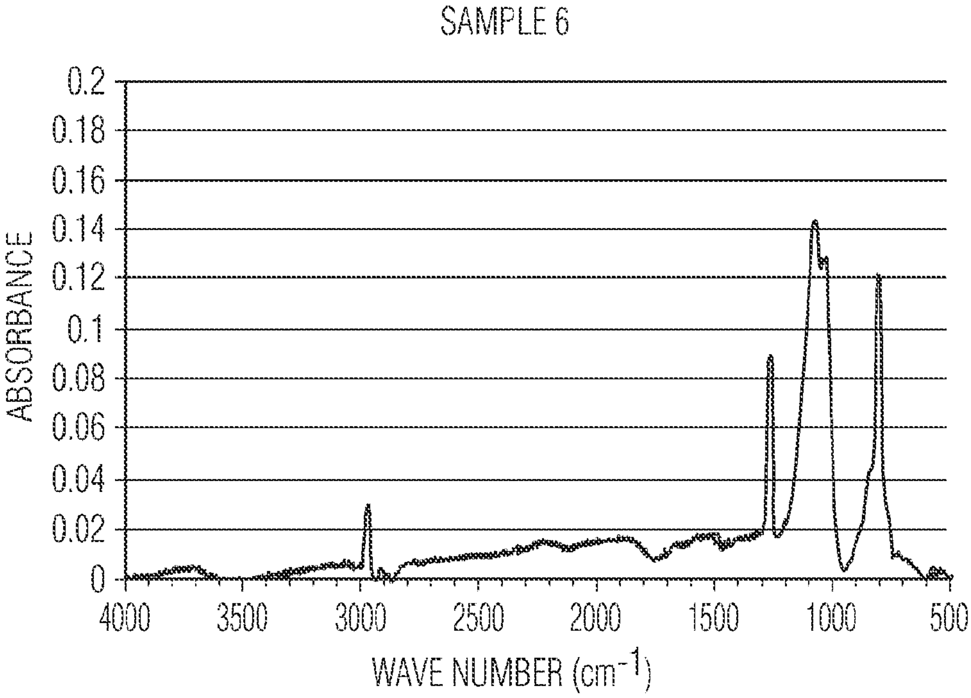
SAMPLE 6
ABSORBANCE
0.2
0.18
0.16
0.14
0.12
0.1
0.08
0.06
0.04
0.02
0
4000
3500
3000
2500
2000
1500
1000
500
WAVE NUMBER (cm-1)

FIG. 16

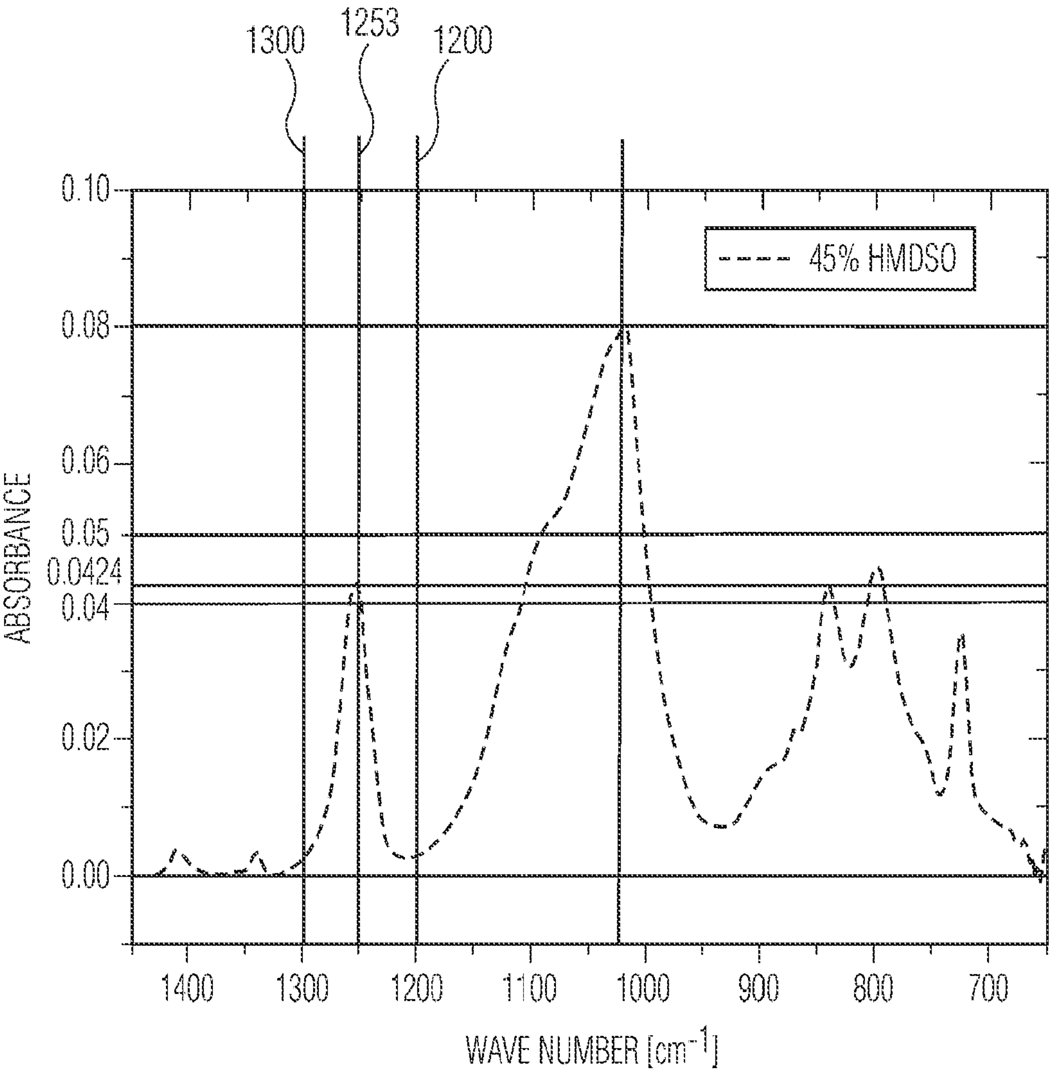


FIG. 17
PRIOR ART
(ANNOTATED)

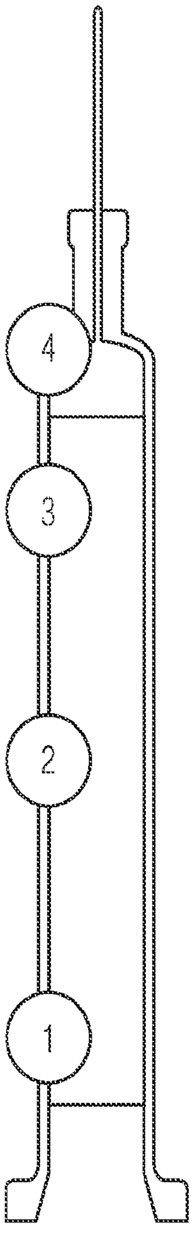


FIG. 18

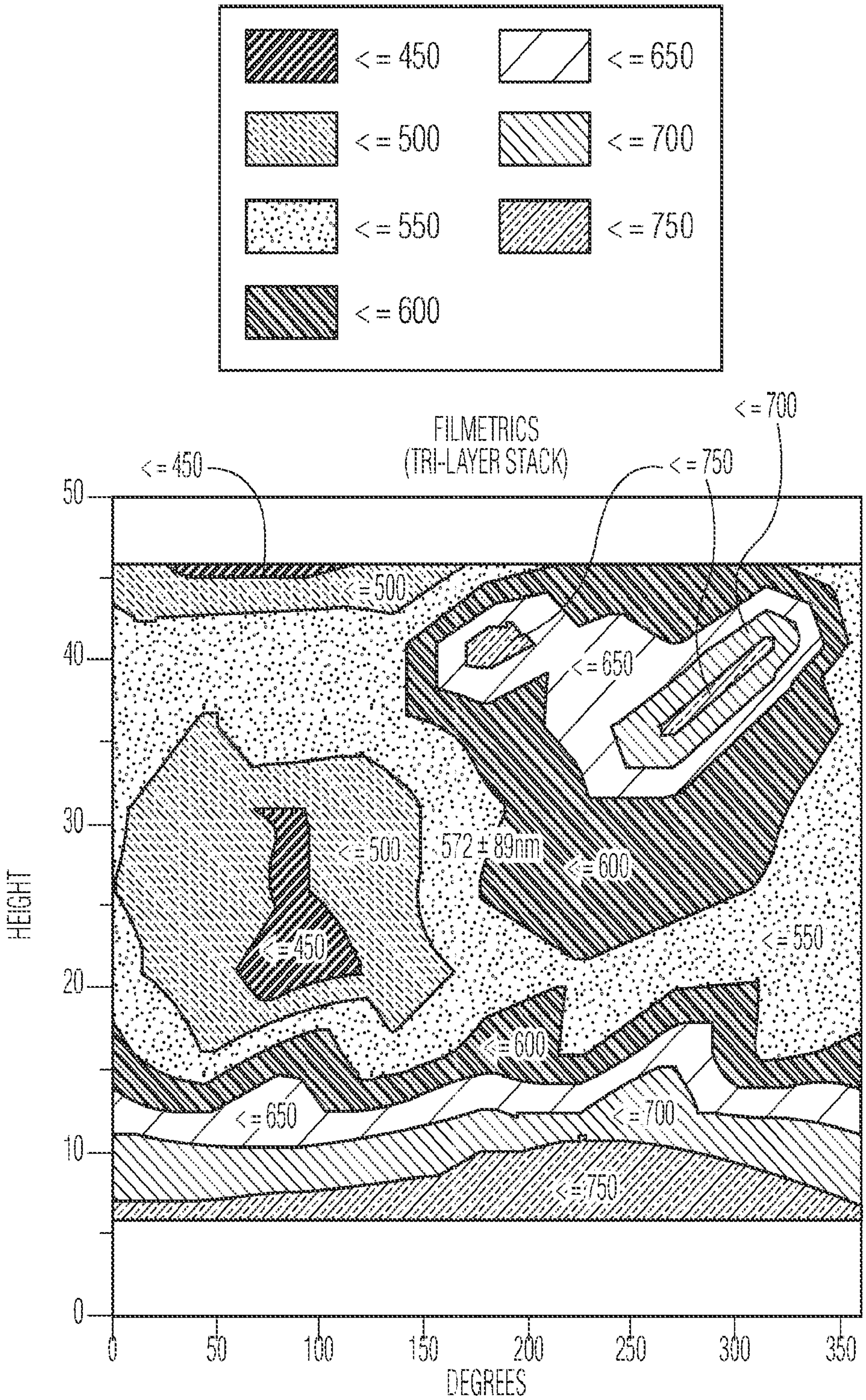

< = 450
< = 500
< = 550
< = 600
< = 650
< = 700
< = 750
FILMETRICS
(TRI-LAYER STACK)
572 ± 89nm
HEIGHT
DEGREES
0
50
100
150
200
250
300
350
10
20
30
40


FIG. 19

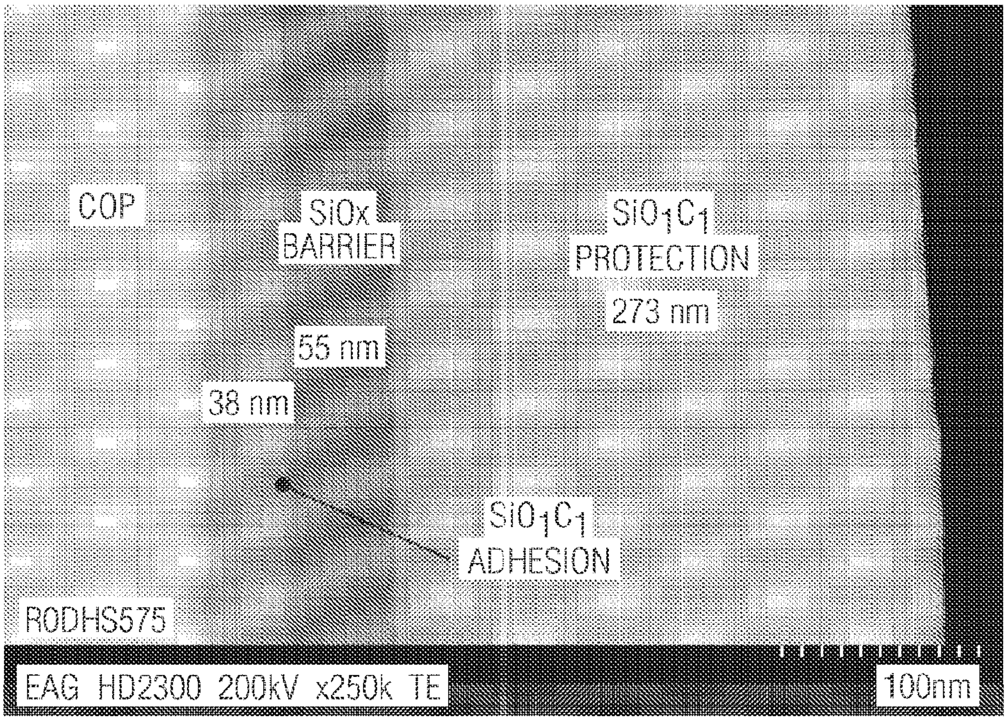
COP
SiOx
BARRIER
SiO1C1
PROTECTION
273 nm
55 nm
38 nm
SiO1C1
ADHESION
R0DHS575
EAG HD2300 200kV x250k TE
100nm

FIG. 20

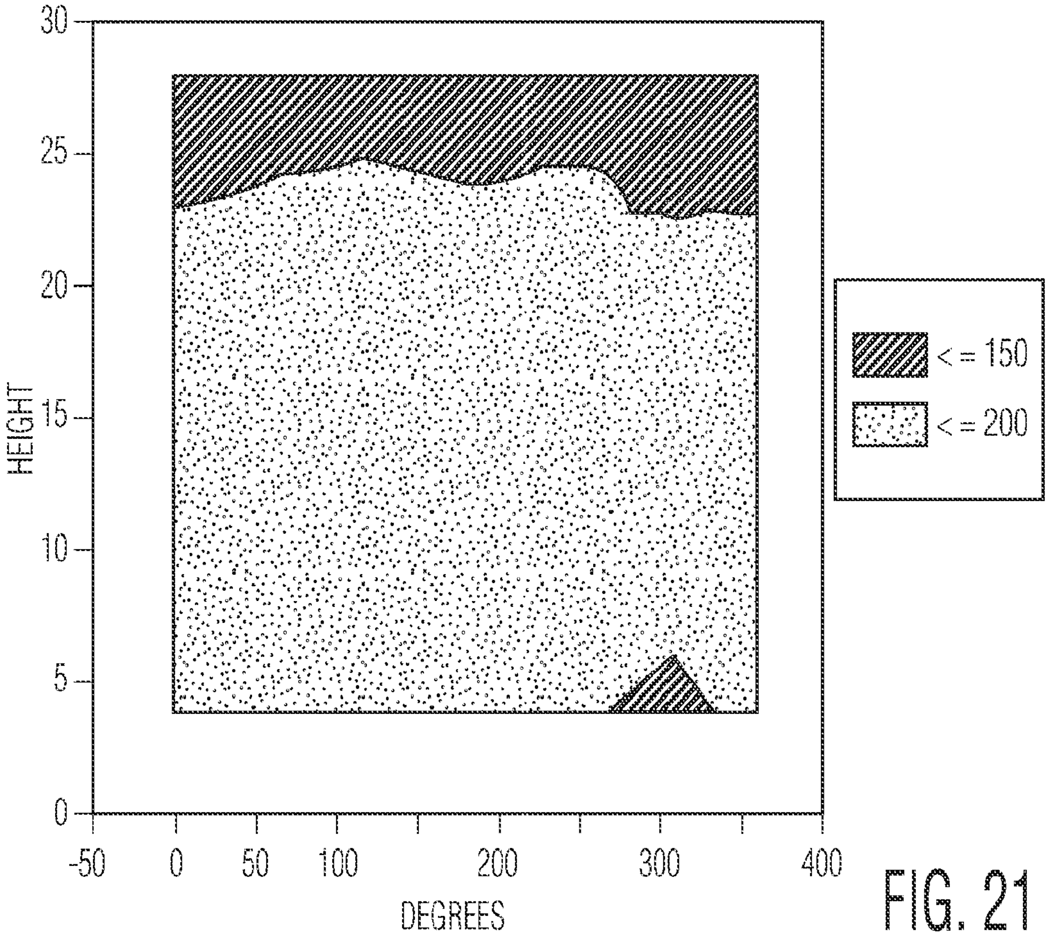
30
25
20
15
10
5
0
HEIGHT
-50
0
50
100
200
300
400
DEGREES
< = 150
< = 200

FIG. 21

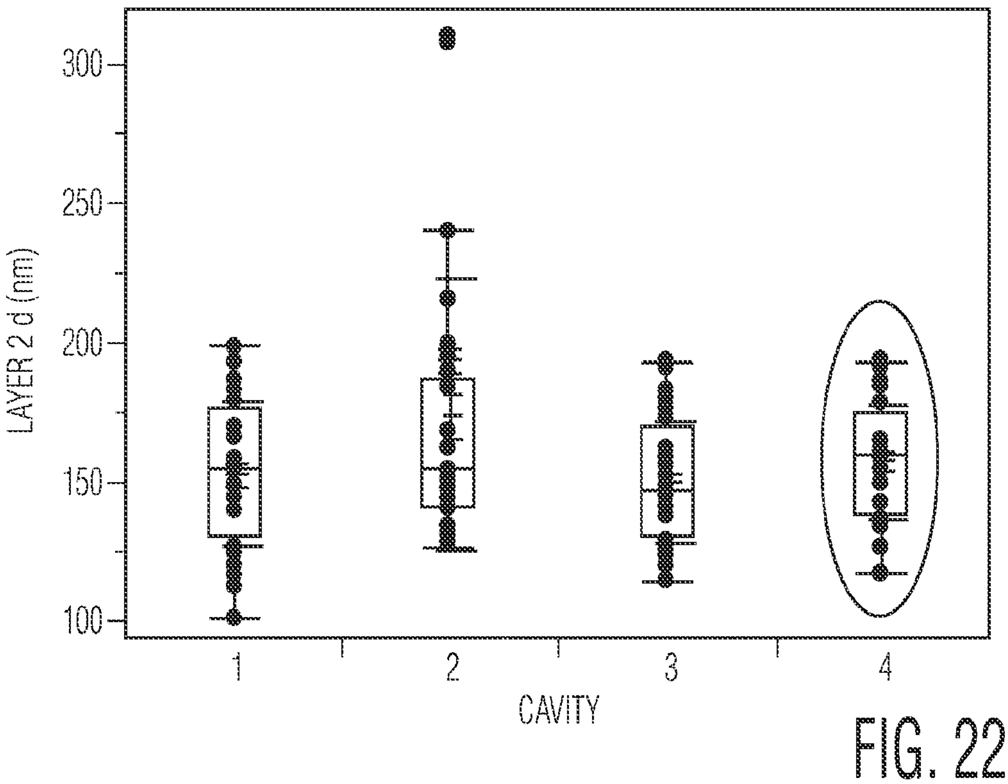
300
250
200
150
100
LAYER 2 d (nm)
1
2
3
4
CAVITY

FIG. 22

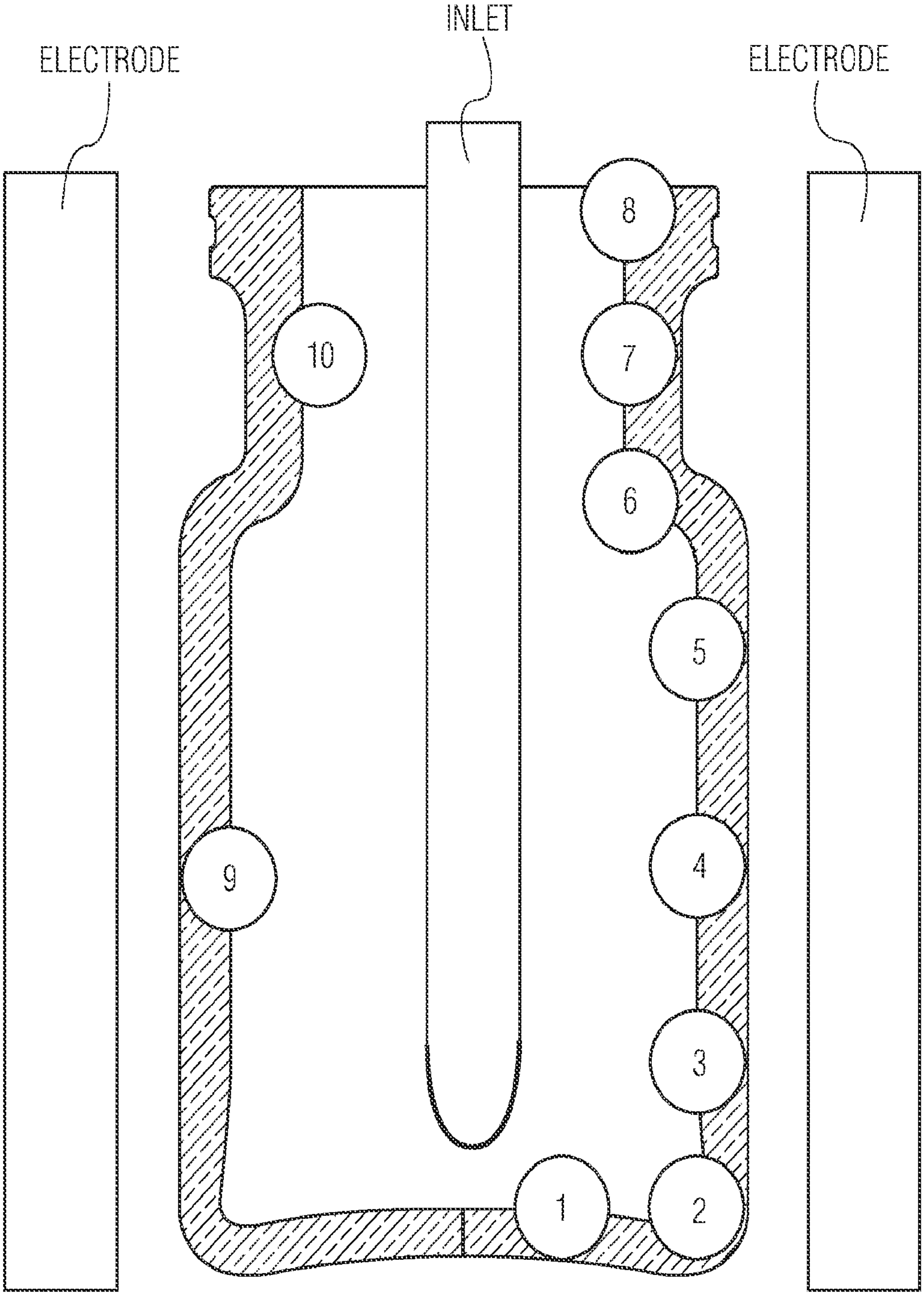


FIG. 23

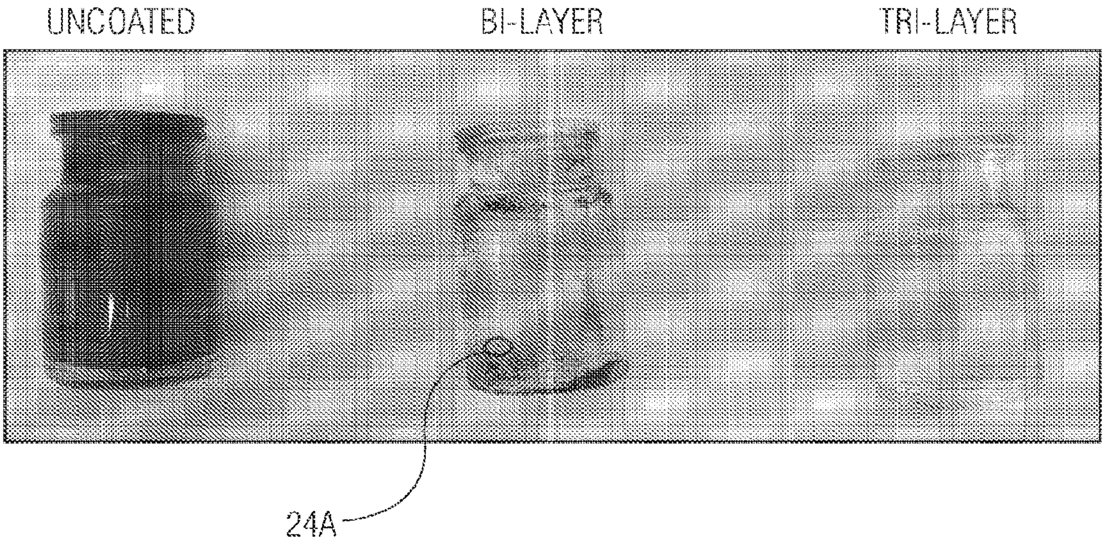


FIG. 24

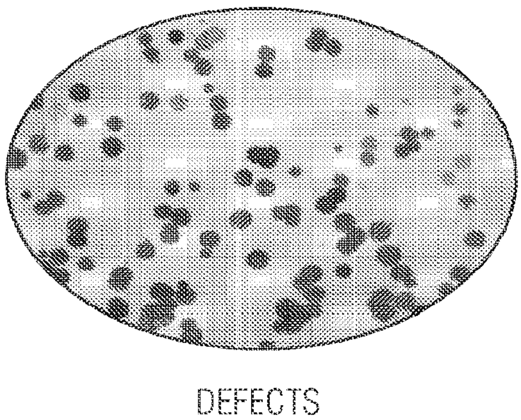


FIG. 24A

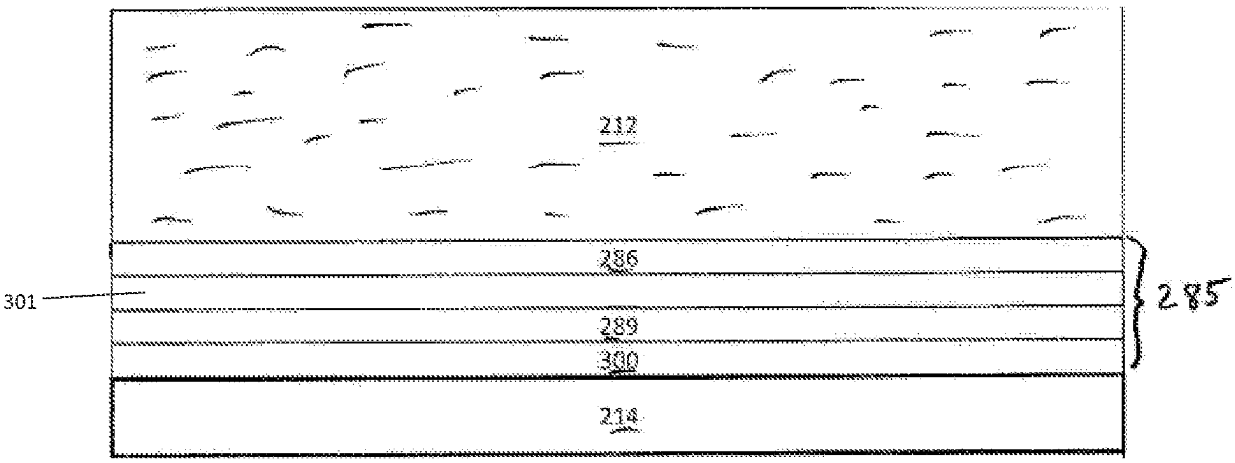


Fig. 25

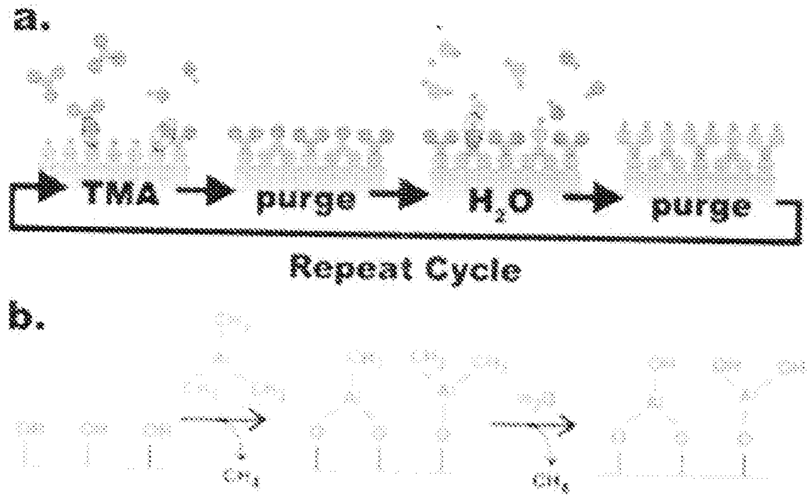


Figure 26

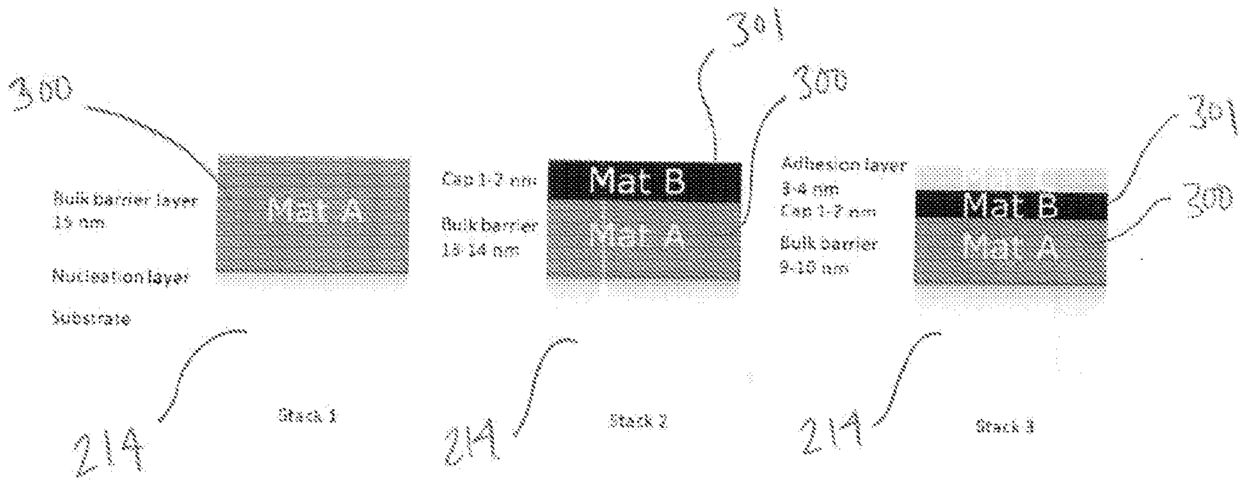


Figure 27

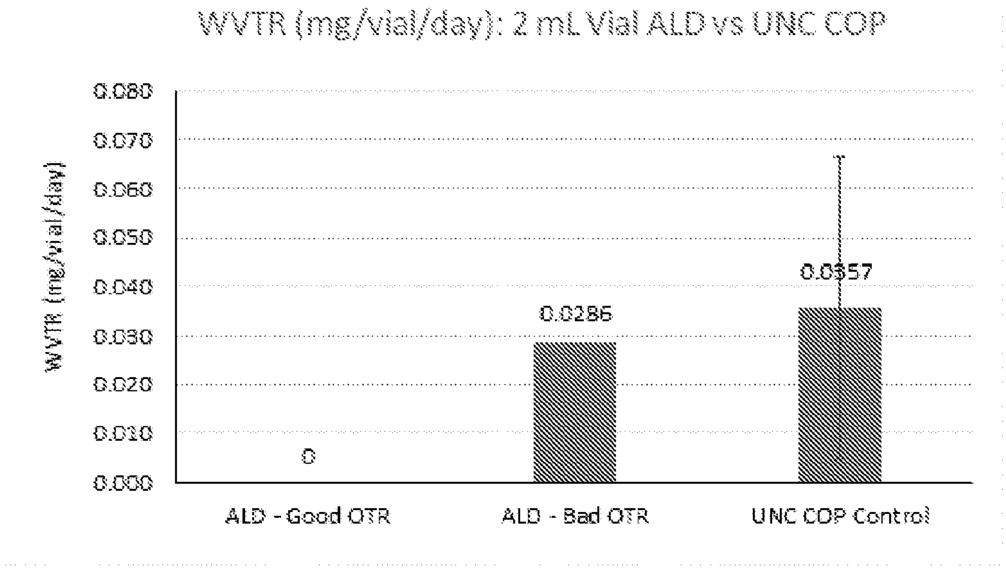


Figure 28

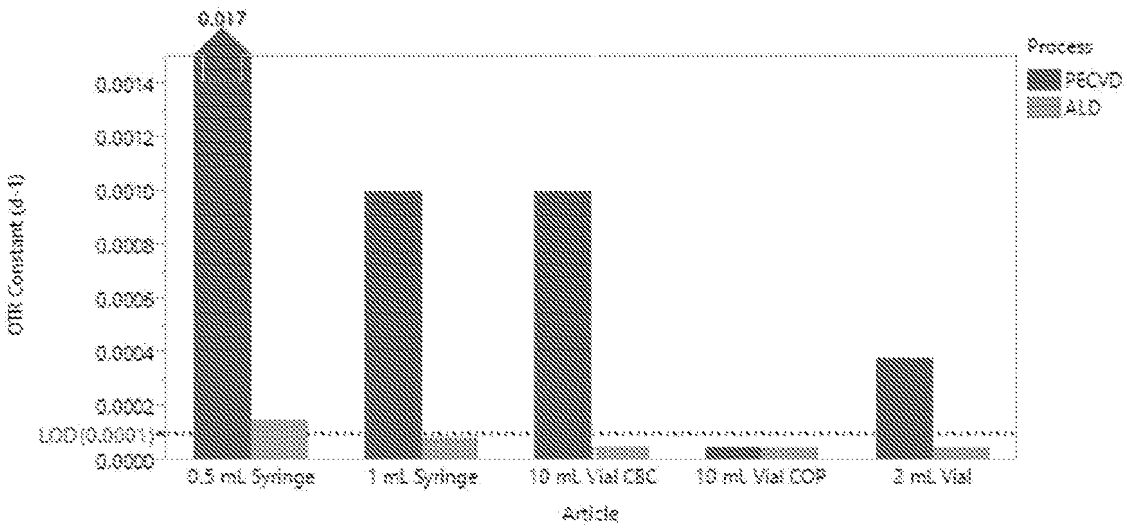


Figure 29

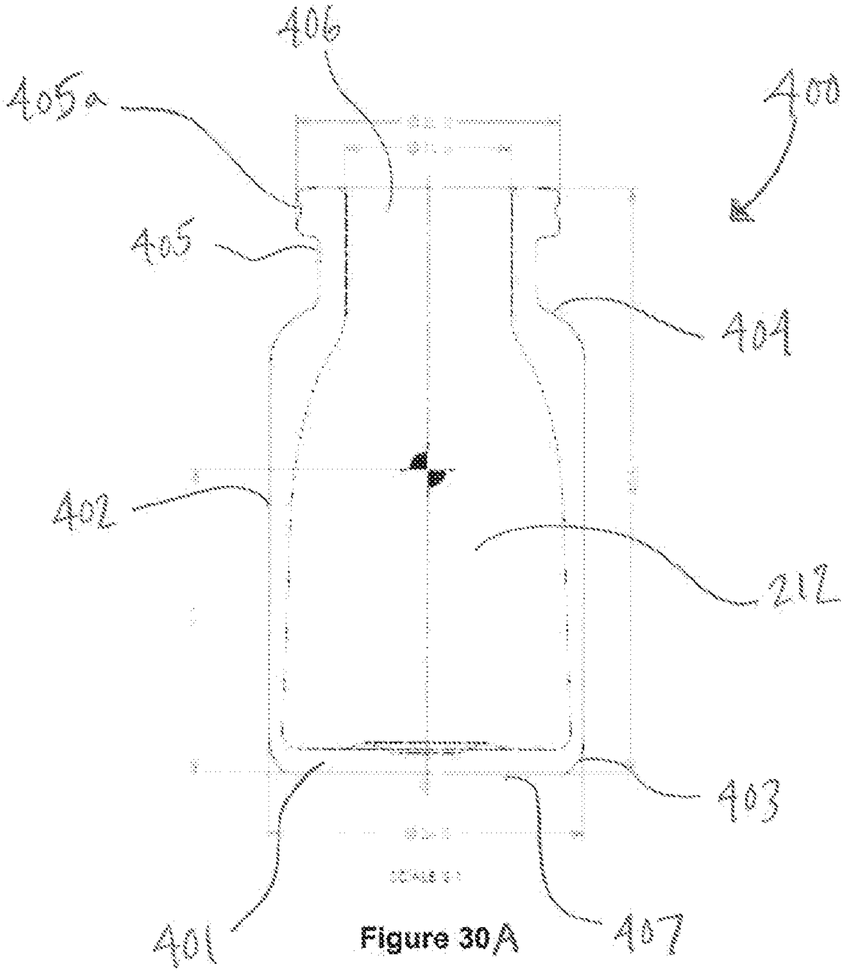


Figure 30A

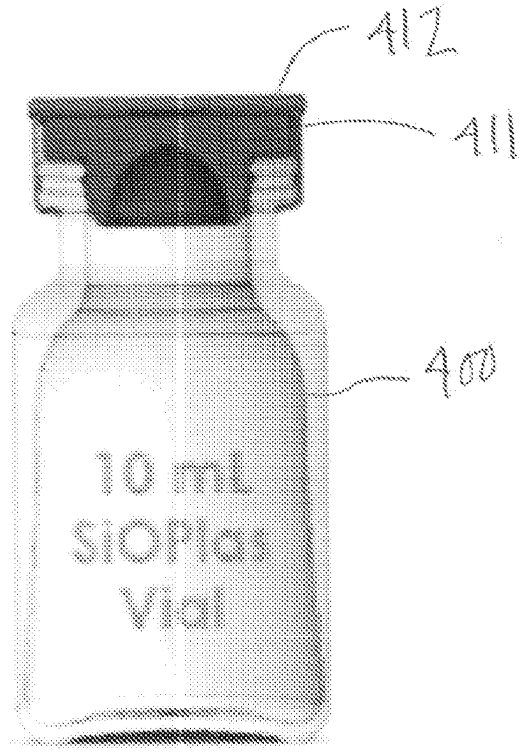


Figure 30B

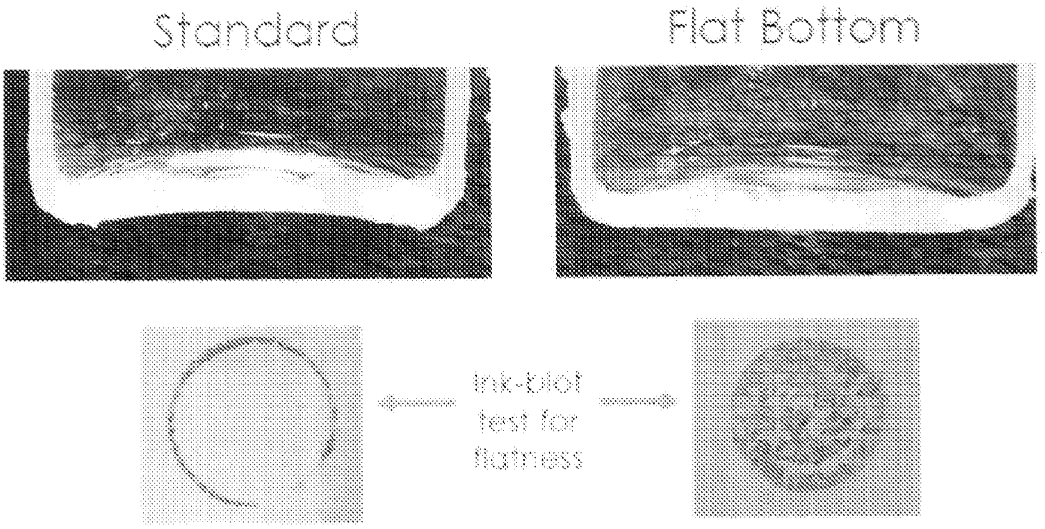


Figure 31

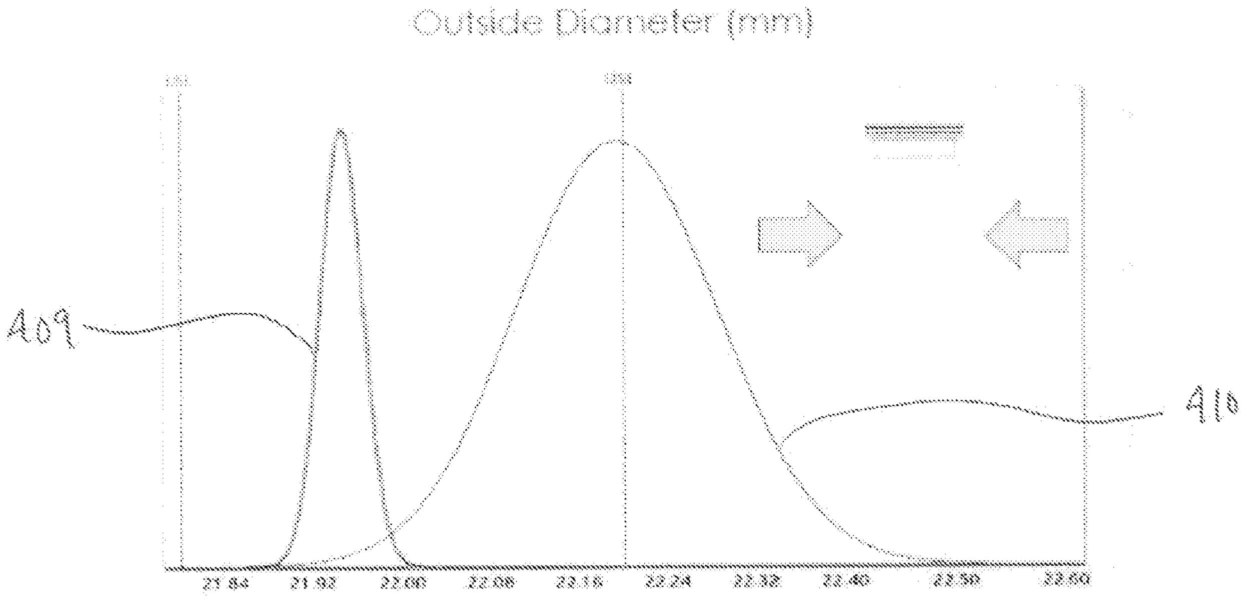


Figure 32

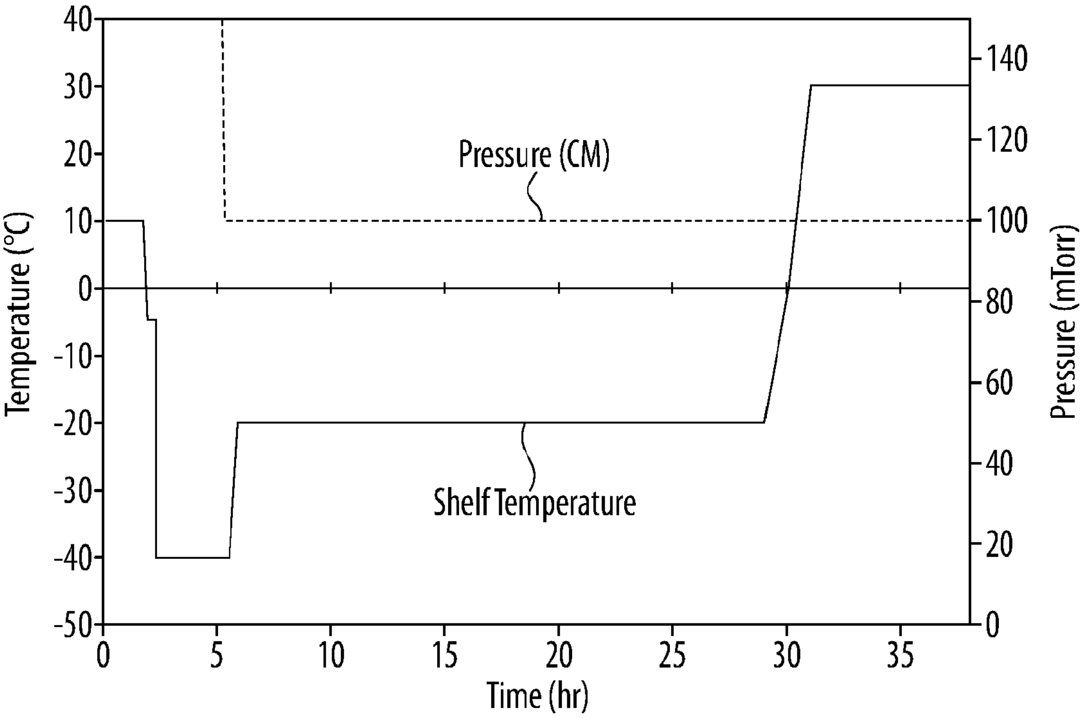


FIG. 33A

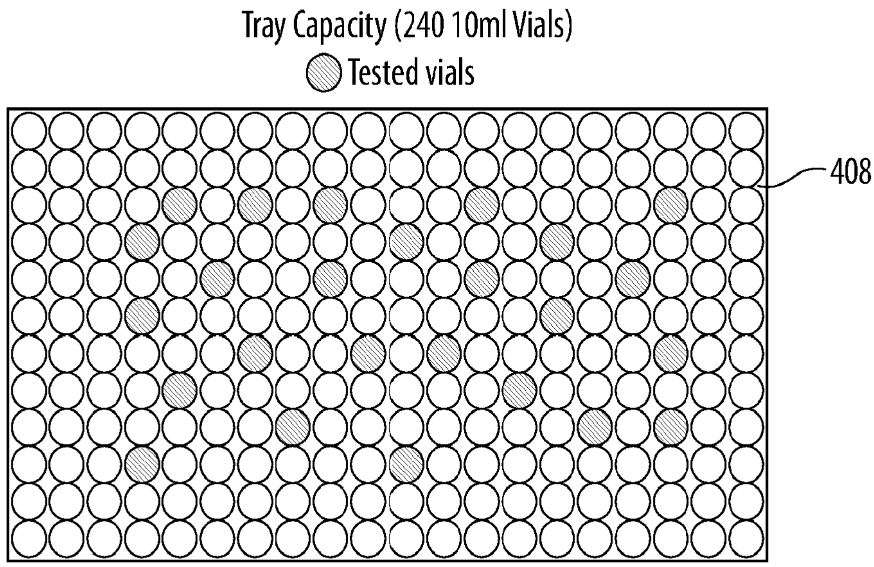


FIG. 33B

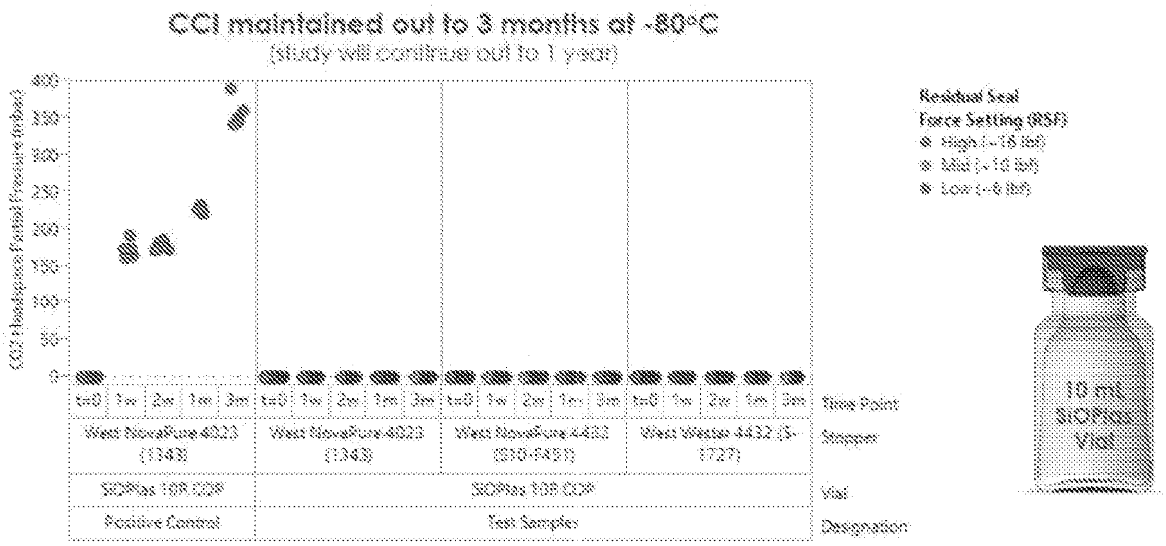


Figure 34

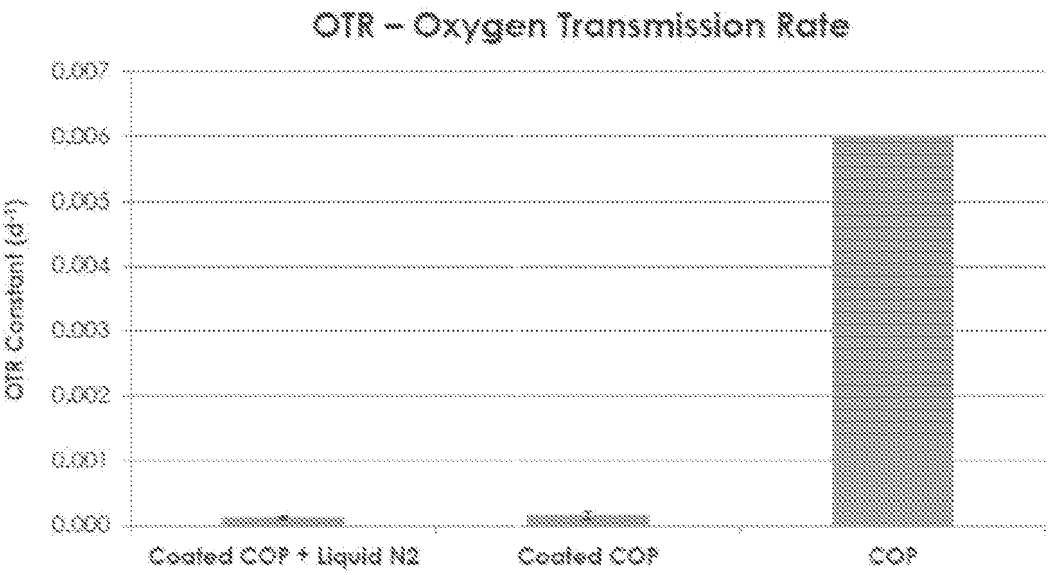


Figure 35

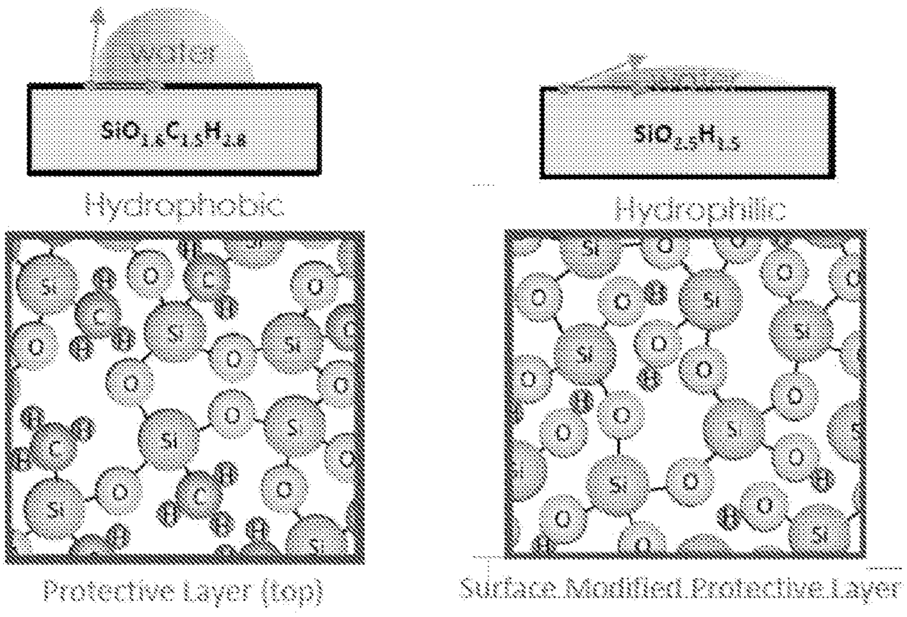


Figure 36

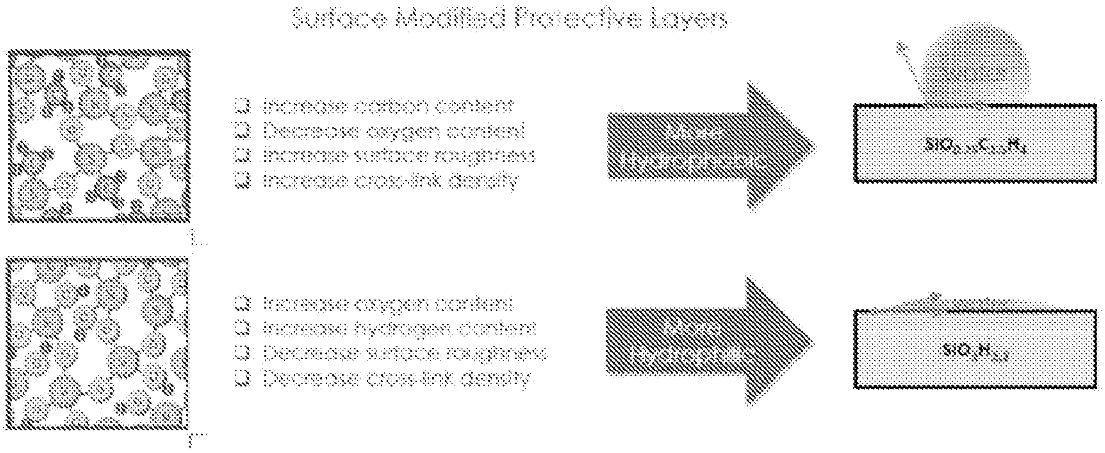


Figure 37

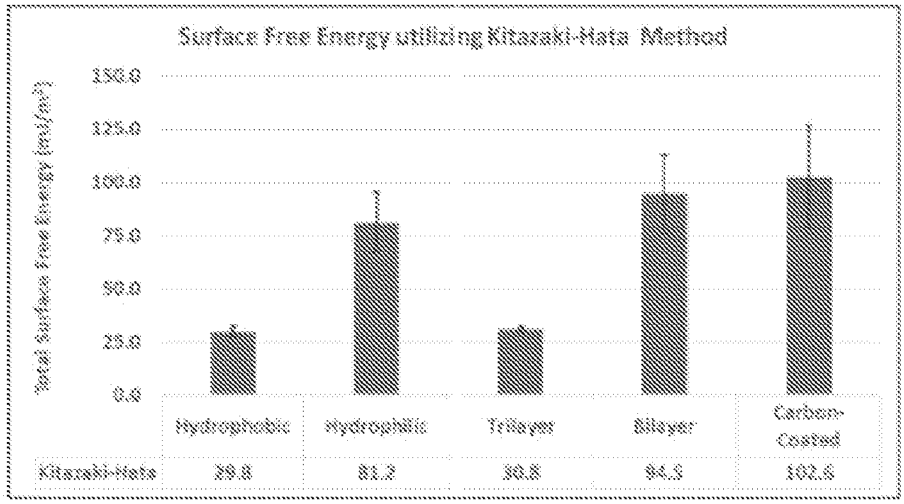
Surface Free Energy utilizing Kitazaki-Hata Method
Total Surface Free Energy (mJ/m²)
150.0
125.0
100.0
75.0
50.0
25.0
0.0
Hydrophobic
Hydrophilic
Trilayer
Bilayer
Carbon-Coated
Kitazaki-Hata
29.8
81.2
30.8
94.3
102.6

Figure 38

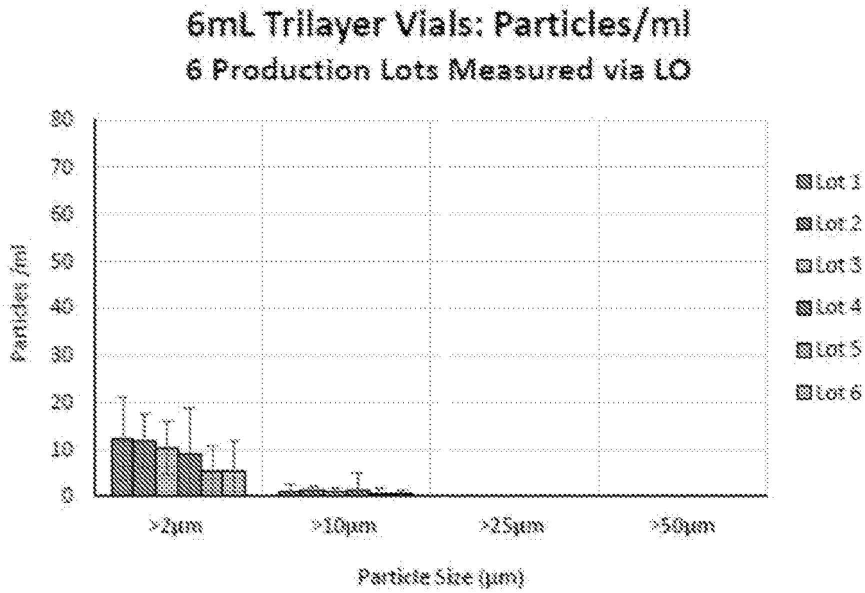


Figure 39

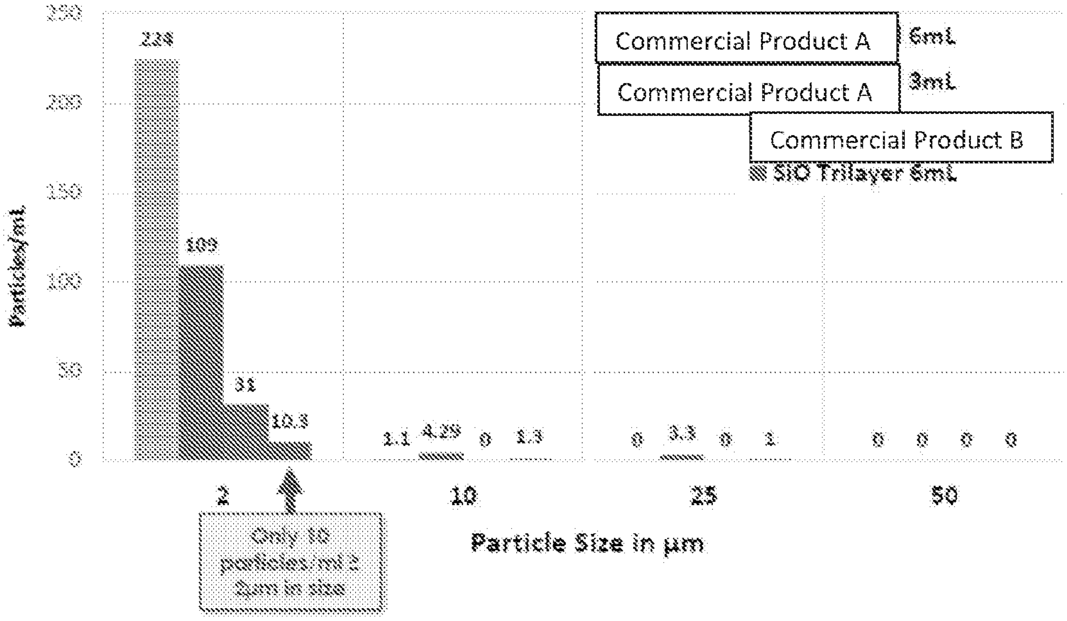


Figure 40

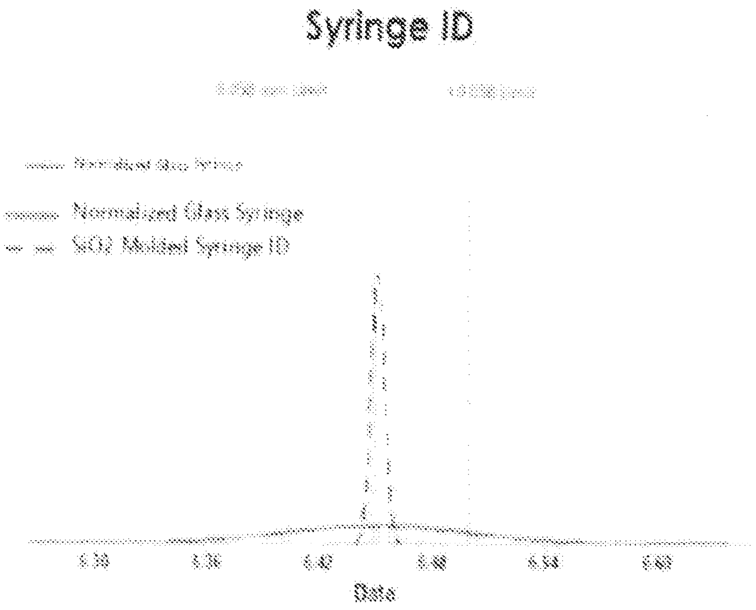


Figure 41

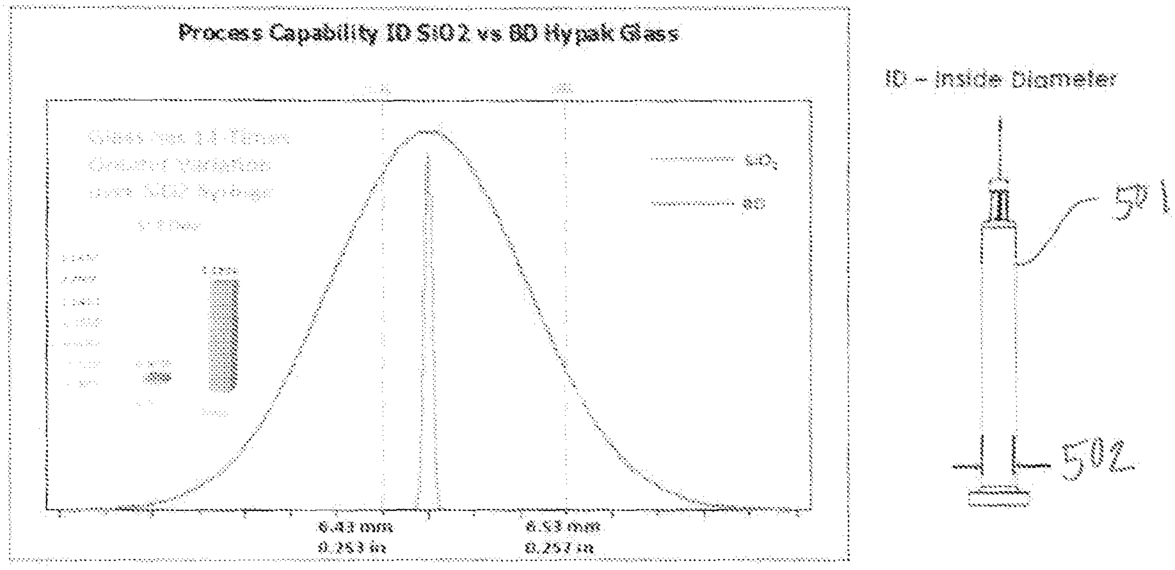


Figure 42

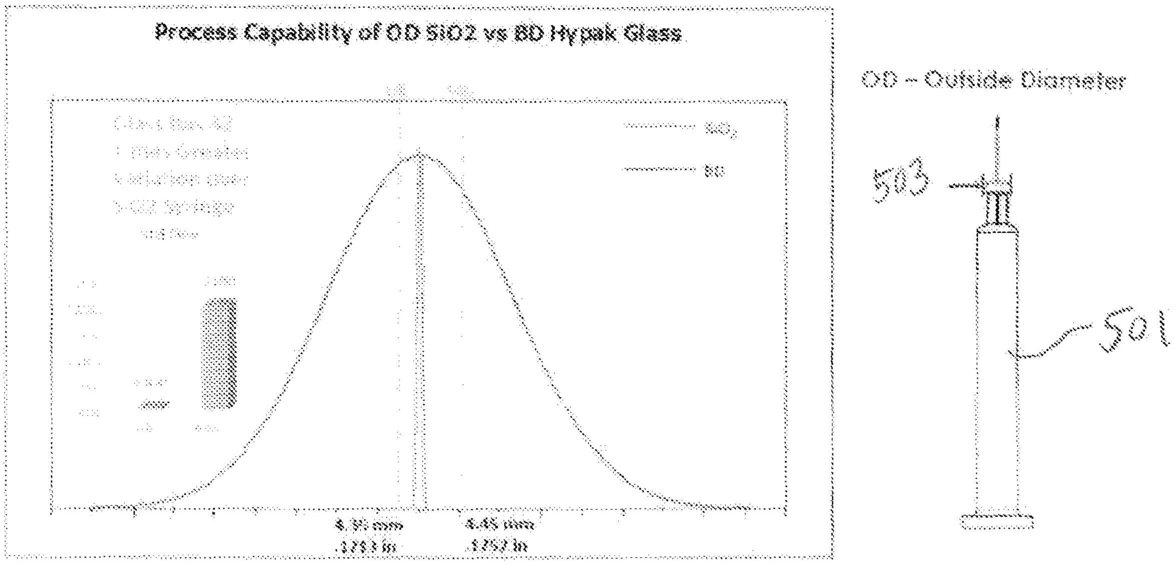


Figure 43

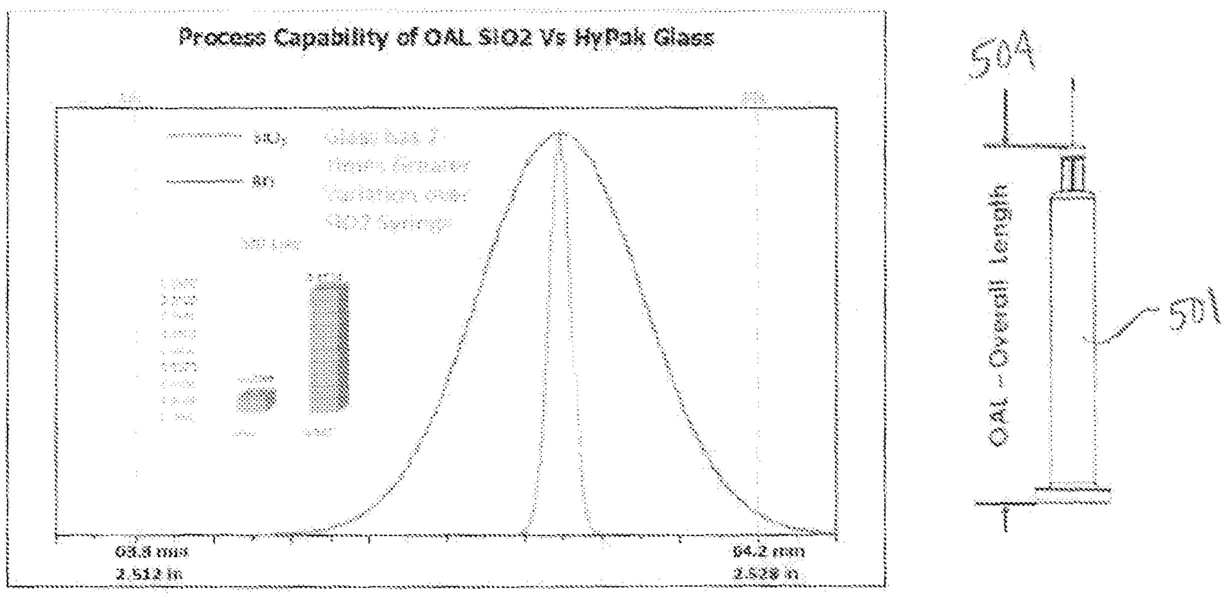


Figure 44

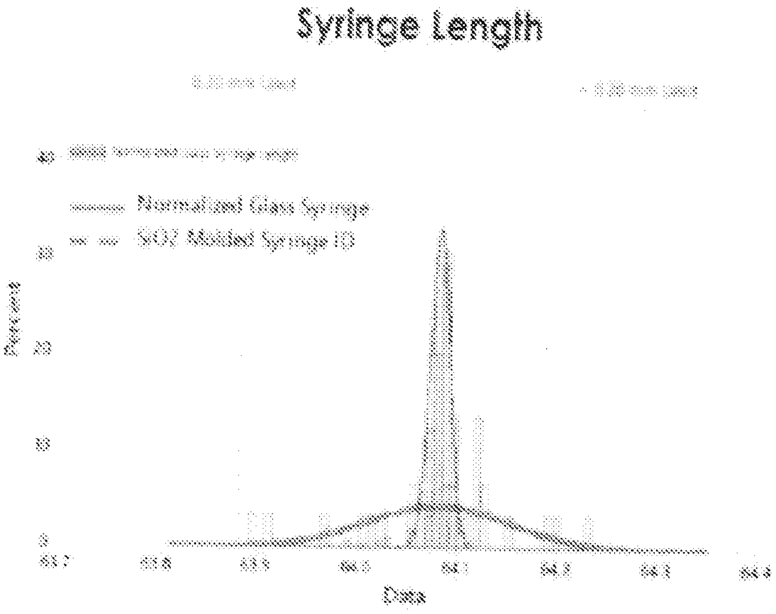


Figure 45

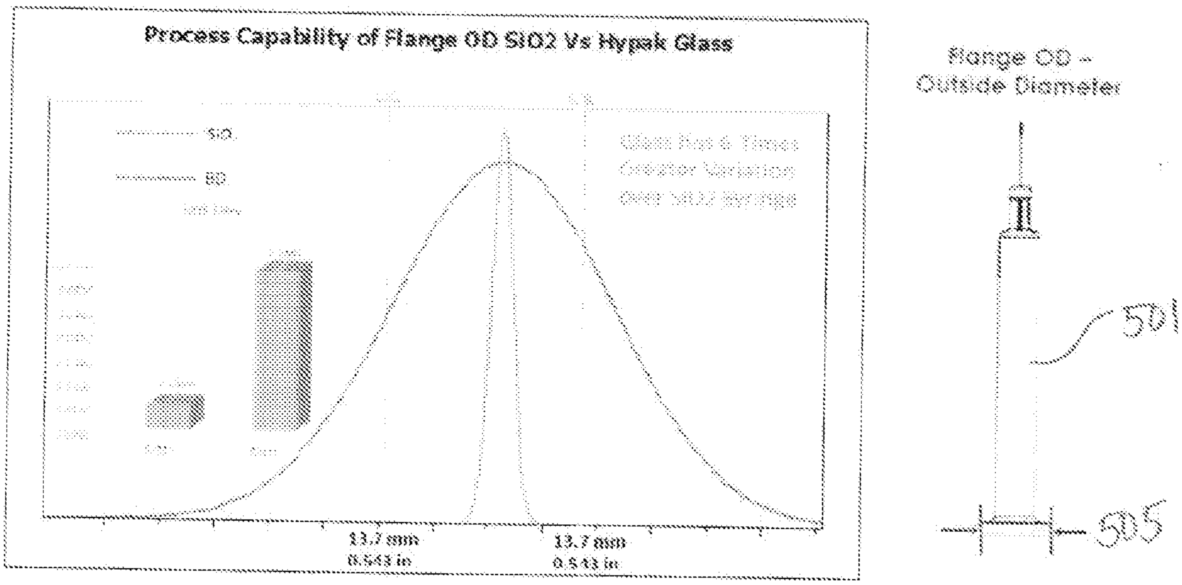


Figure 46

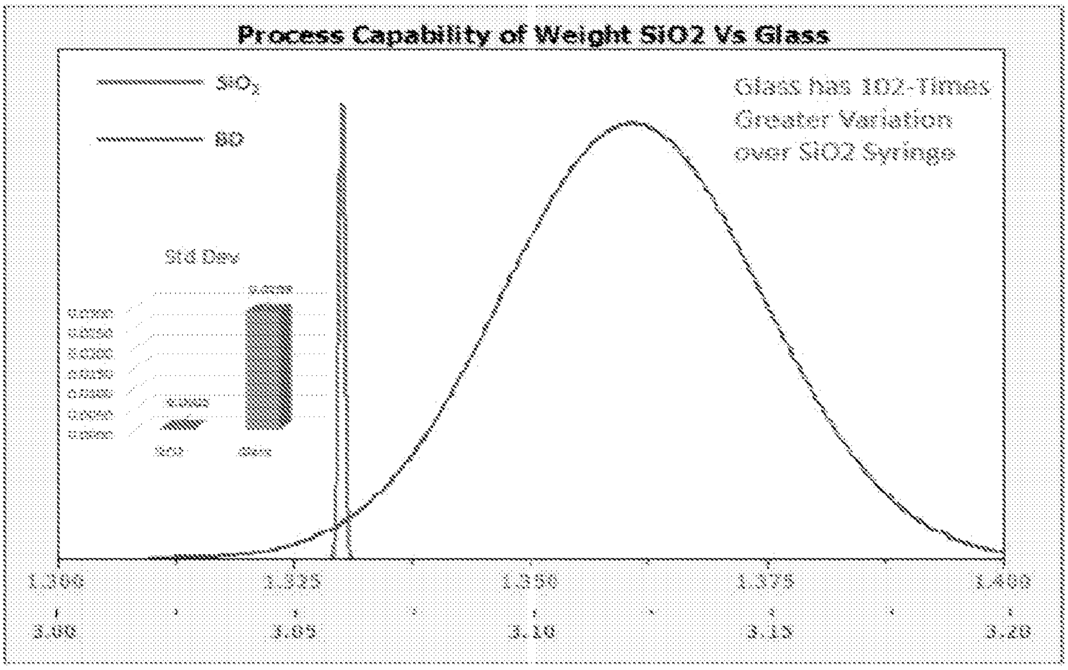
Process Capability of Weight SiO2 Vs Glass
$SiO_2$
BD
Glass has 102-Times
Greater Variation
over SiO2 Syringe
Std Dev
1.300
1.325
1.350
1.375
1.400
3.00
3.05
3.10
3.15
3.20

Figure 47

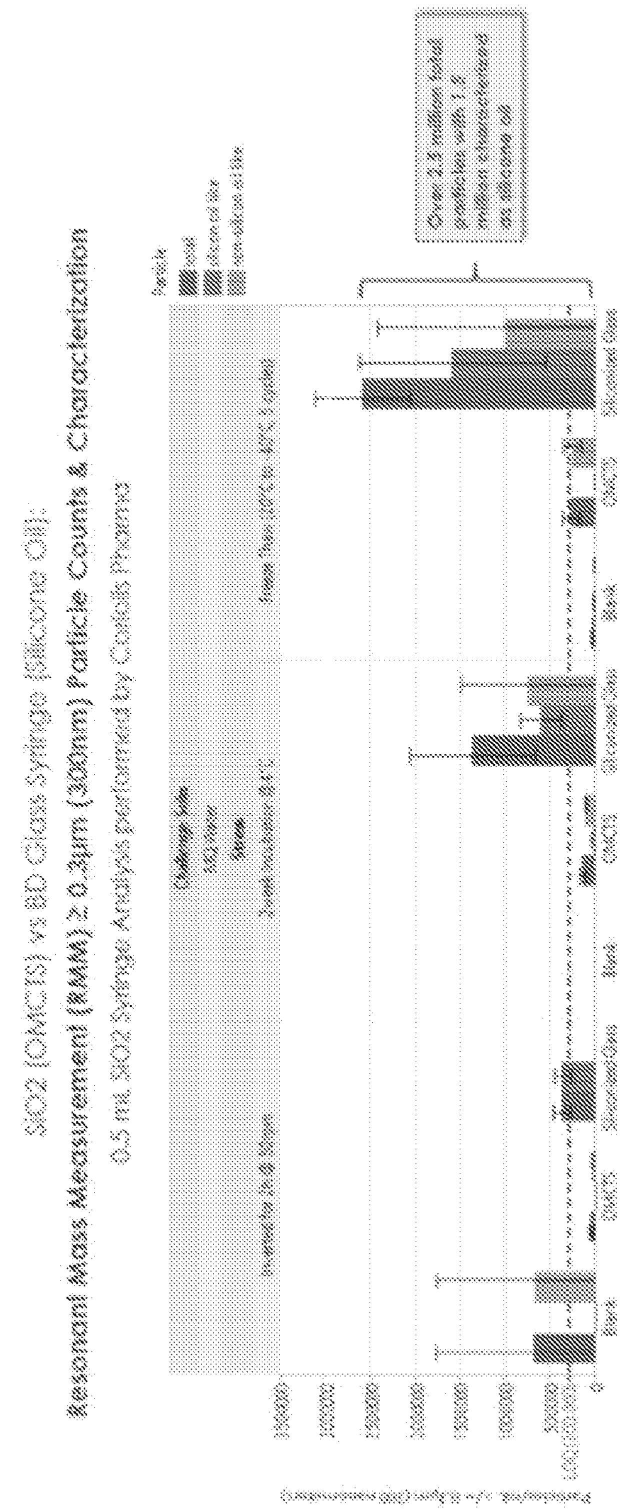
SiO2 (OMCTS) vs BD Glass Syringe (Silicone Oil):
Resonant Mass Measurement (RMM) ≥ 0.3µm (300nm) Particle Counts & Characterization
0.5 mL SiO2 Syringe Analysis performed by Coriolis Pharma

Figure 48

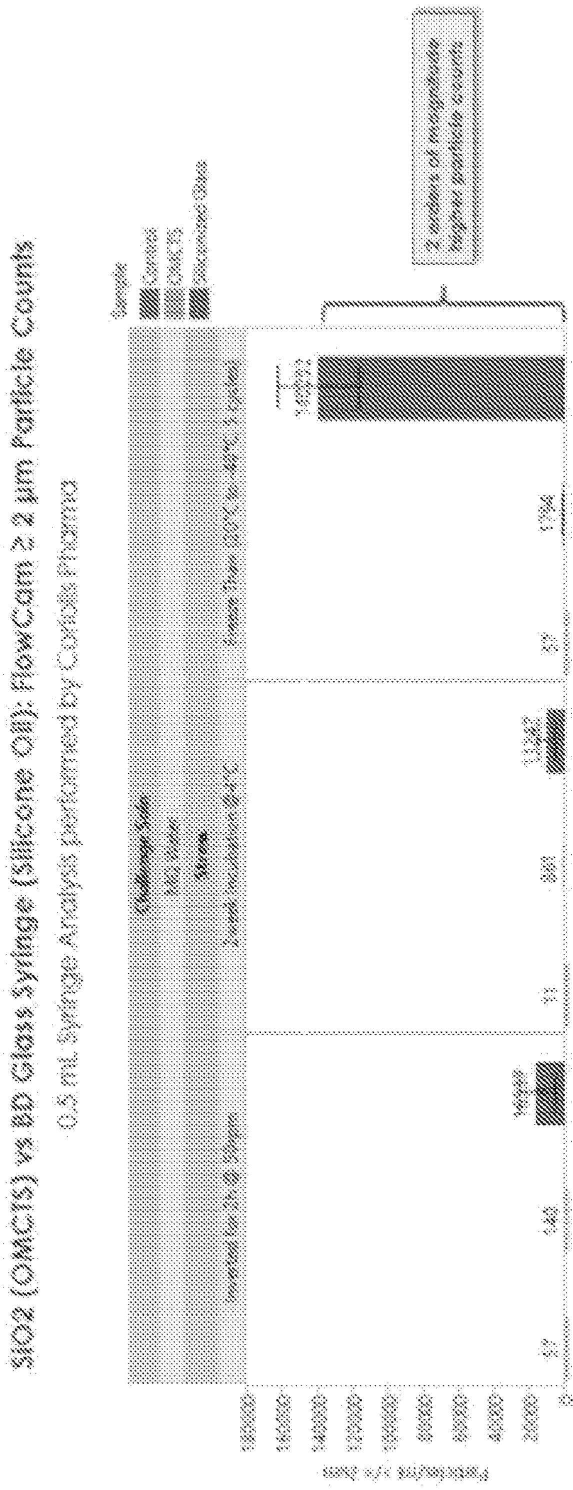
SiO2 (OMCTS) vs BD Glass Syringe (Silicone Oil): FlowCam ≥ 2 µm Particle Counts
0.5 mL Syringe Analysis performed by Coriolis Pharma

Figure 49

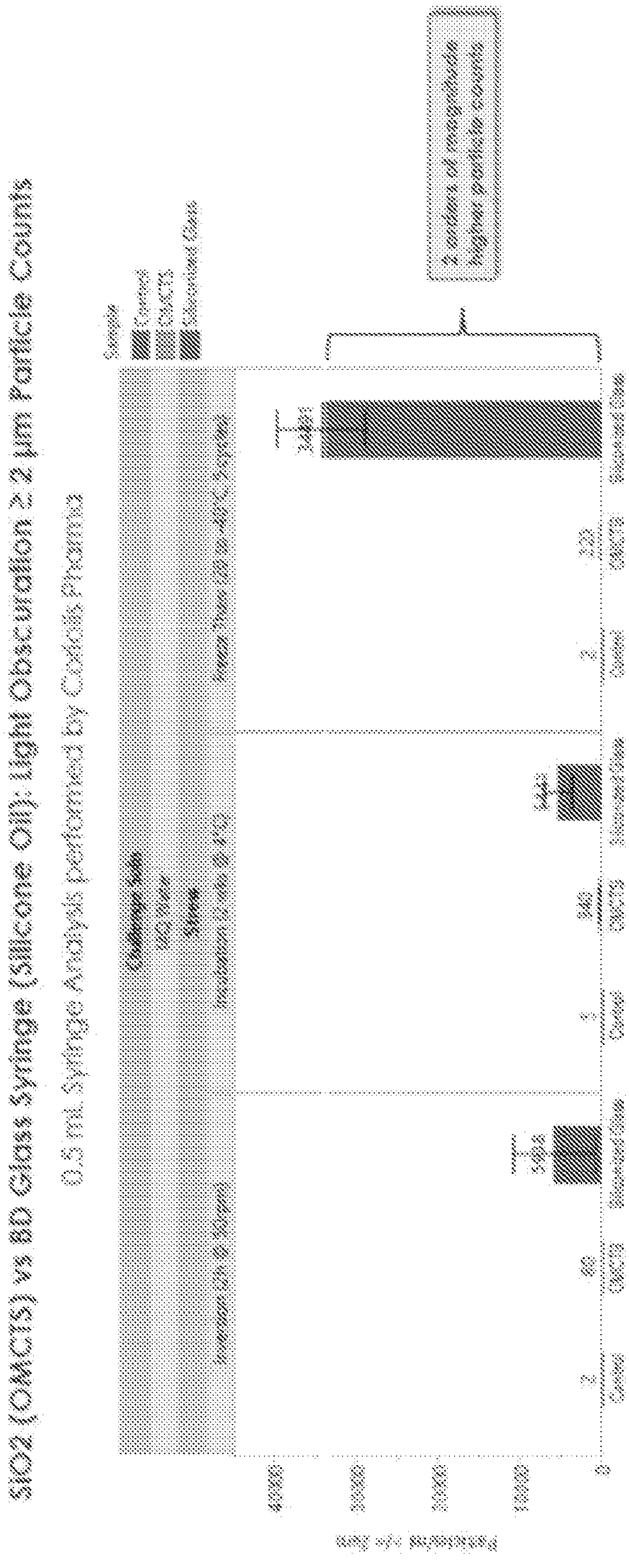
SiO2 (OMCTS) vs BD Glass Syringe (Silicone Oil): Light Obscuration ≥ 2 µm Particle Counts

Figure 50

EO Residual Panel of Terminally Sterilized 0.5 ml Syringes:
Siumlated-Use Extraction with MQ Water @25°C performed by WuXi AppTec

SiO2 Coated Syringe Equivalent to Glass in Terms of Blocking Ethylene Oxide (EO) Ingress

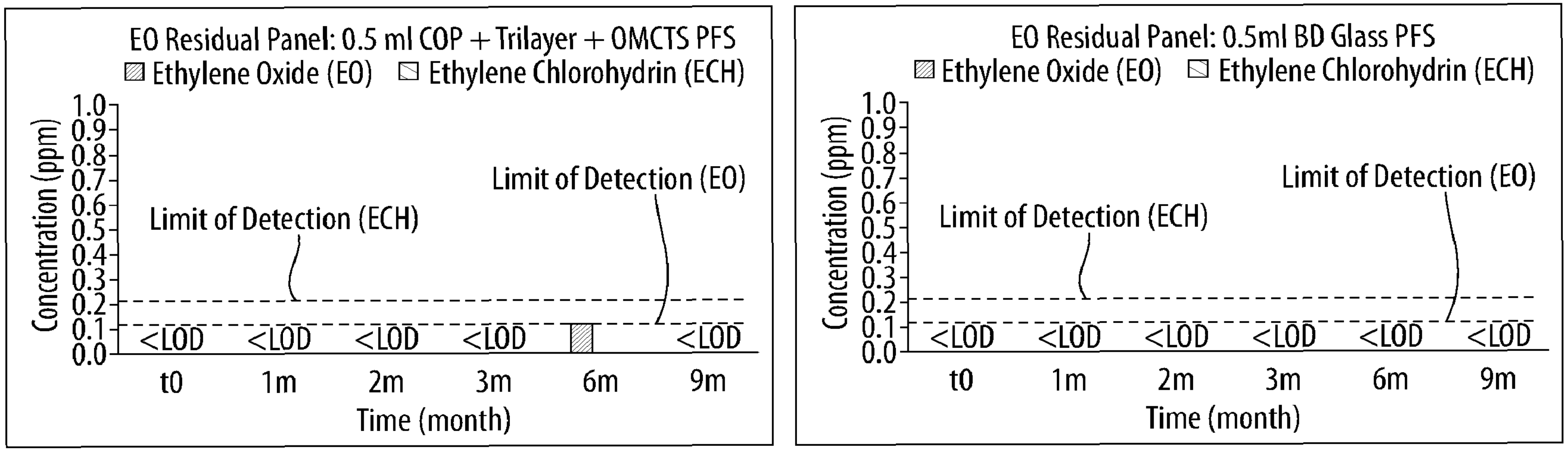


FIG. 51

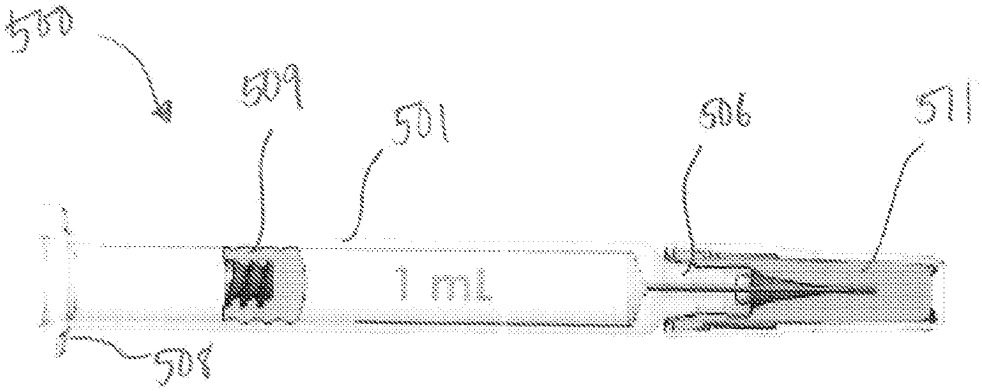


Figure 52

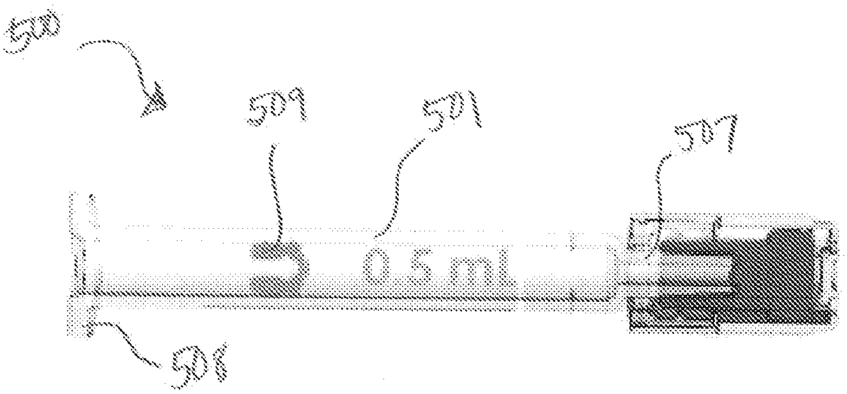


Figure 53

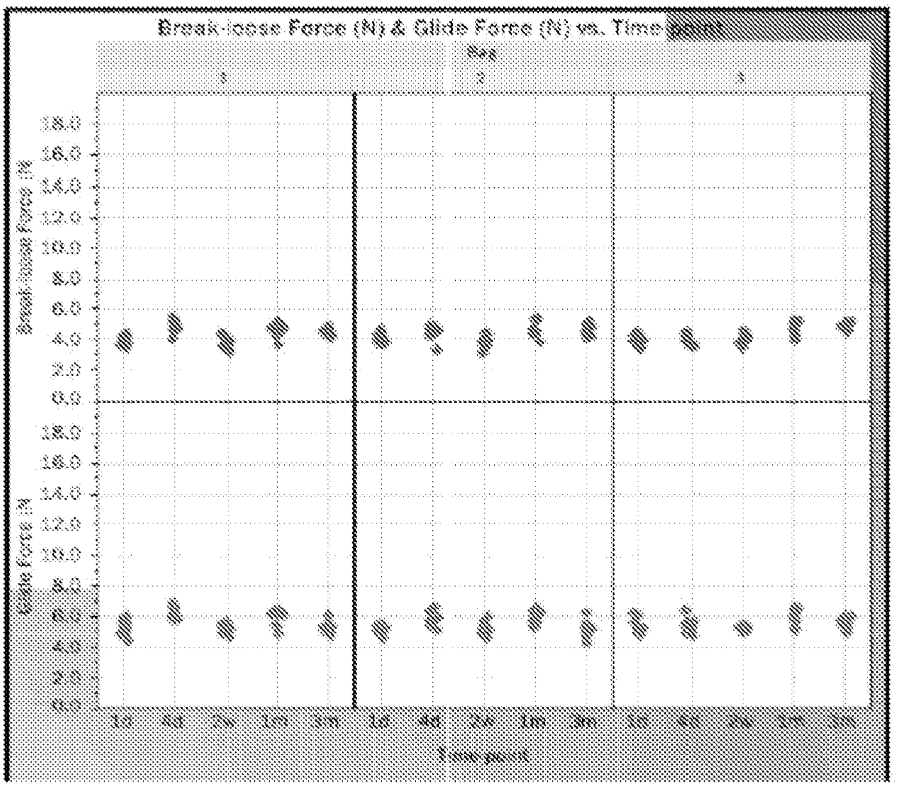


Figure 54

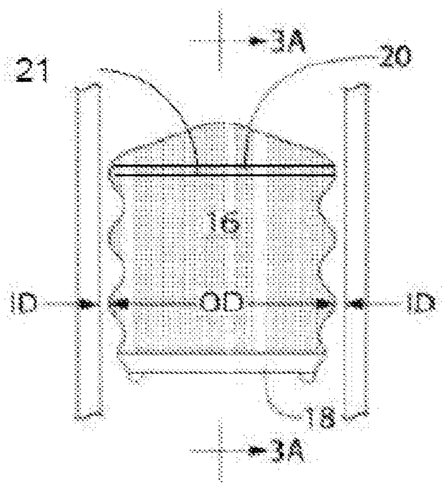


Figure 55

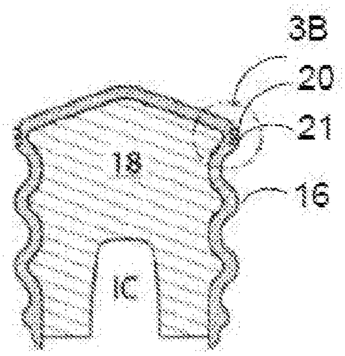


Figure 56

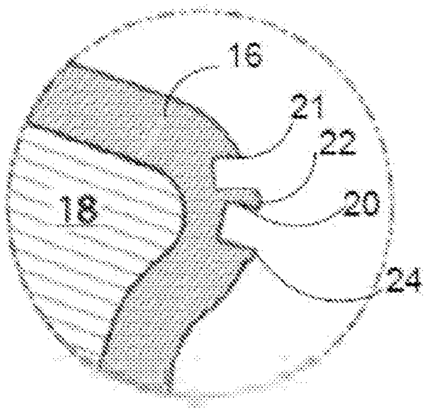


Figure 57

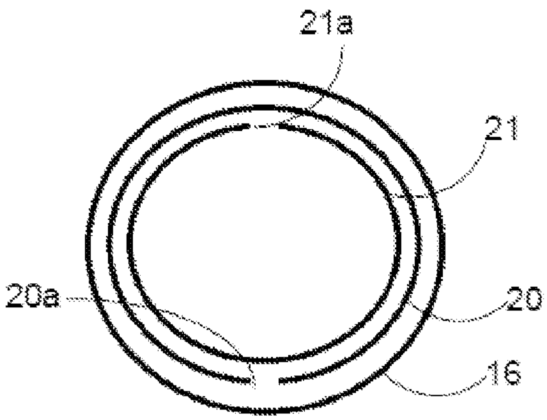


Figure 58

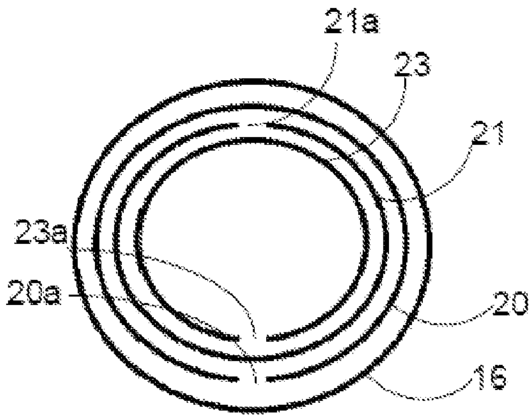


Figure 59

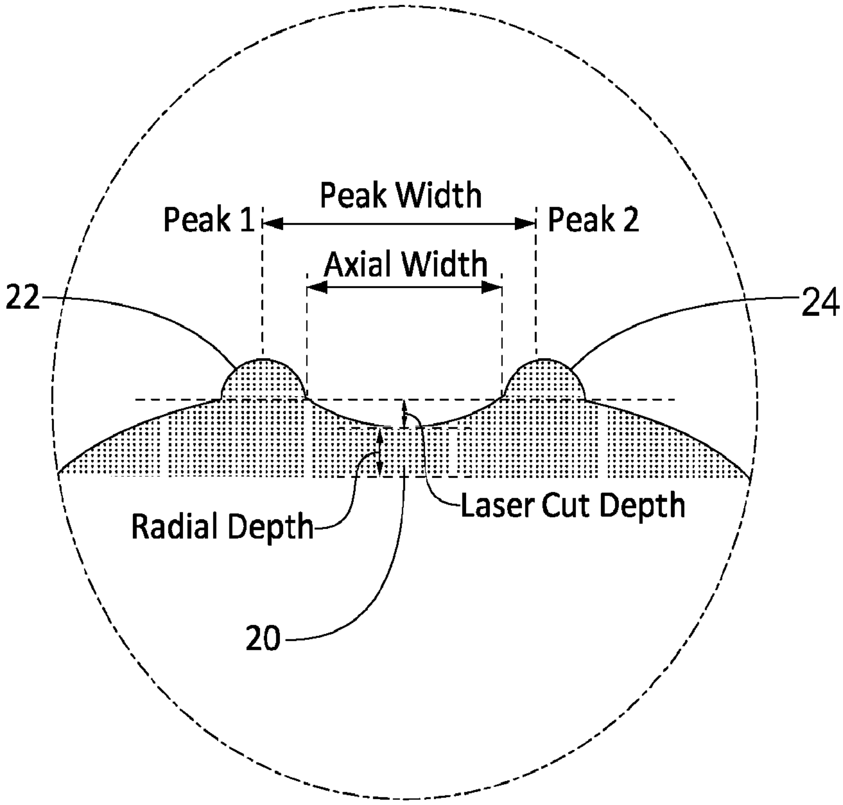
Peak Width
Peak 1
Peak 2
Axial Width
22
24
Radial Depth
Laser Cut Depth
20

FIG. 60

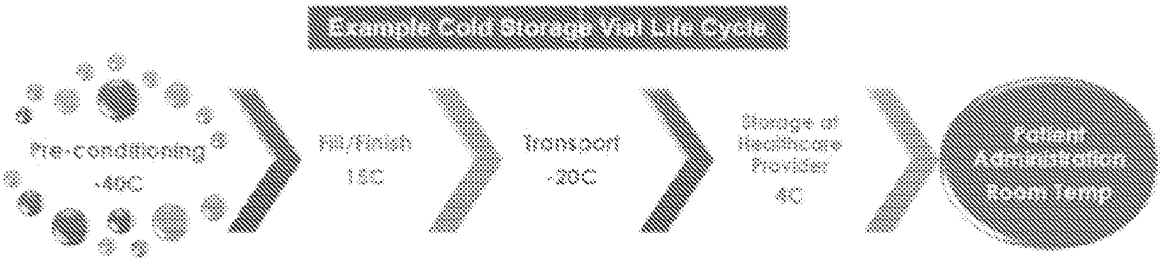


Figure 61

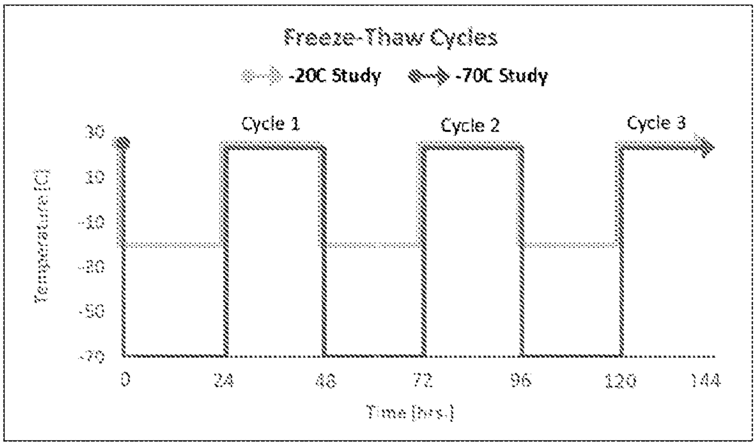


Figure 62

| Vial Geometry | Material | Low Temp. (C) | Fill Volume (mL) | n | # of Failures | | | |
|---|---|---|---|---|---|---|---|---|
| | | | | | Cycle 1 | Cycle 2 | Cycle 3 | Total |
| 2 mL | COP-690R | -20 | 1.0 | 50 | 0 | 0 | 0 | 0 |
| | COP-690R | -20 | 2.0 | 50 | 0 | 0 | 0 | 0 |
| | COP-690R | -70 | 1.0 | 50 | 0 | 0 | 0 | 0 |
| | COP-690R | -70 | 2.0 | 50 | 0 | 0 | 0 | 0 |
| 10 mL | COP-690R | -20 | 6.5 | 50 | 0 | 0 | 0 | 0 |
| | COC-504 | -20 | 6.5 | 50 | 0 | 0 | 0 | 0 |
| | COP-690R | -70 | 6.5 | 50 | 0 | 0 | 0 | 0 |
| | COC-504 | -70 | 6.5 | 50 | 0 | 0 | 0 | 0 |

*No observed defects or cracks after 3 cycles at -20C or -70C*

Figure 63

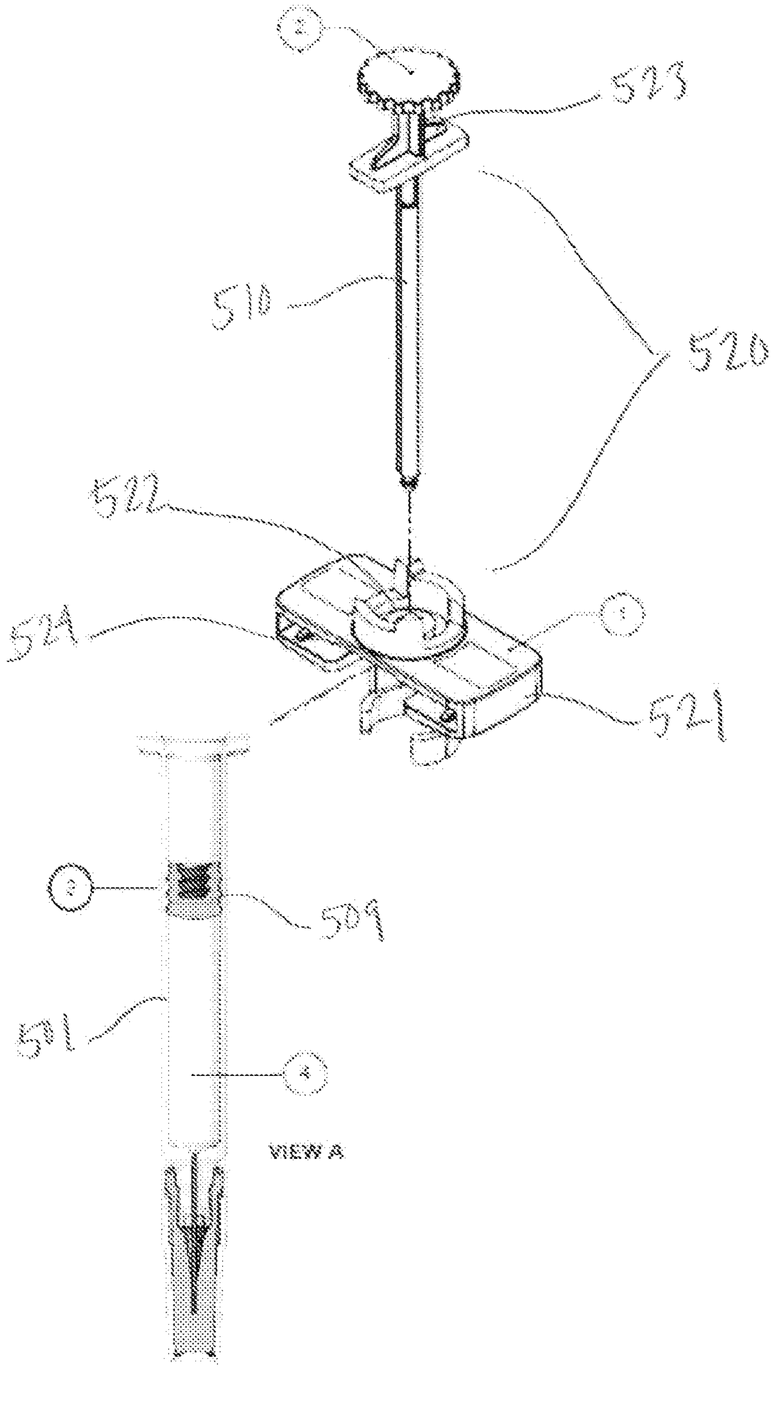


Figure 64

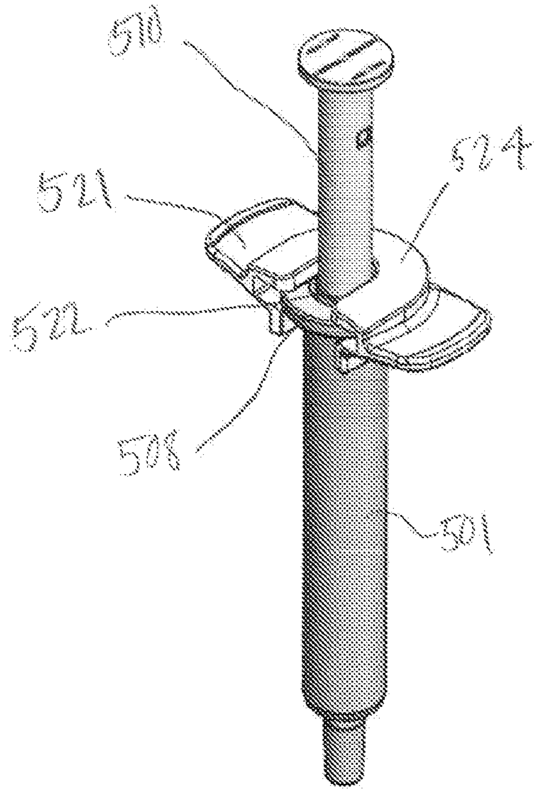


Figure 65

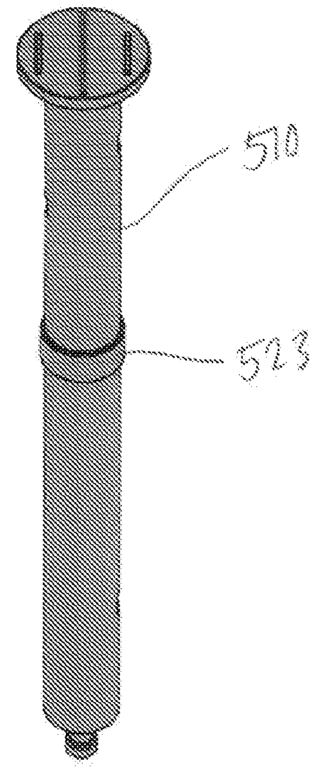


Figure 66

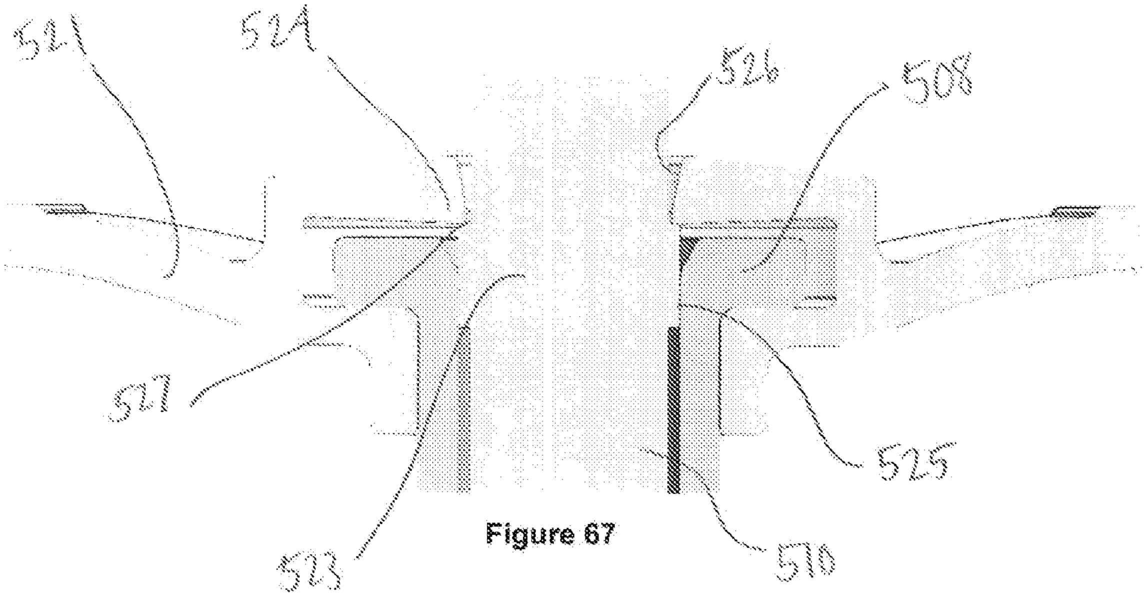


Figure 67

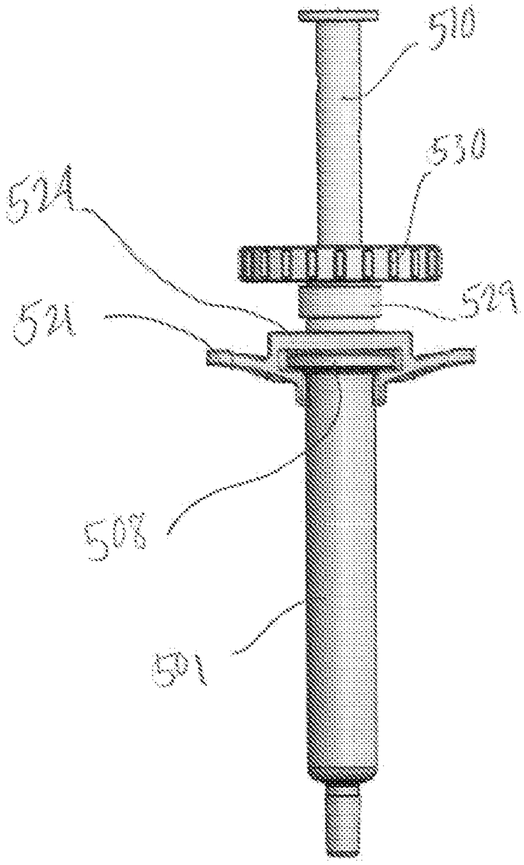


Figure 68

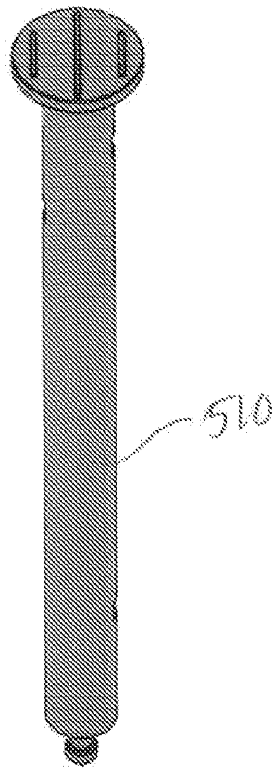


Figure 69

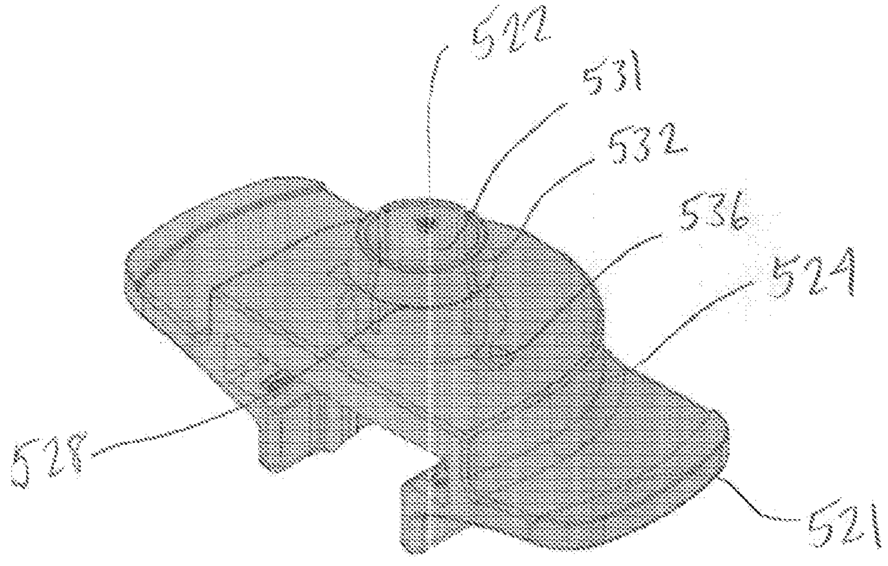


Figure 70

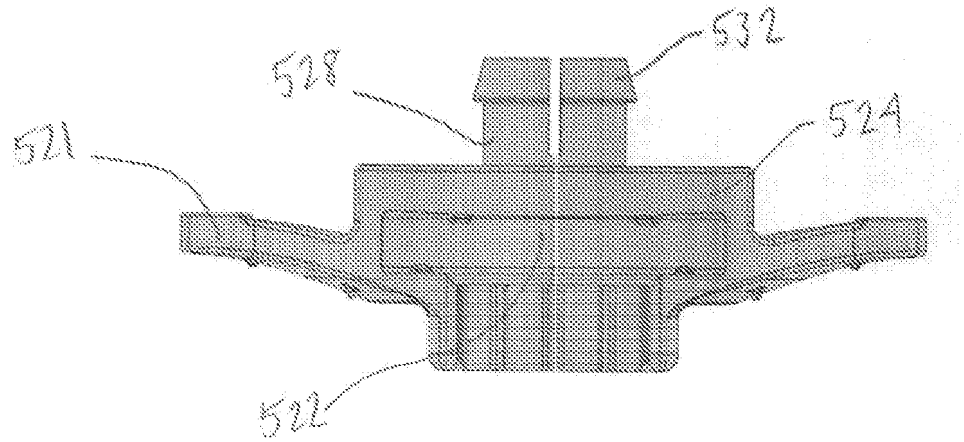


Figure 71

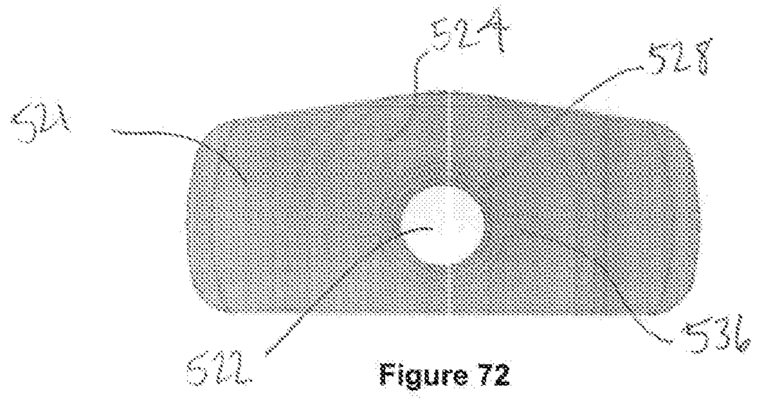


Figure 72

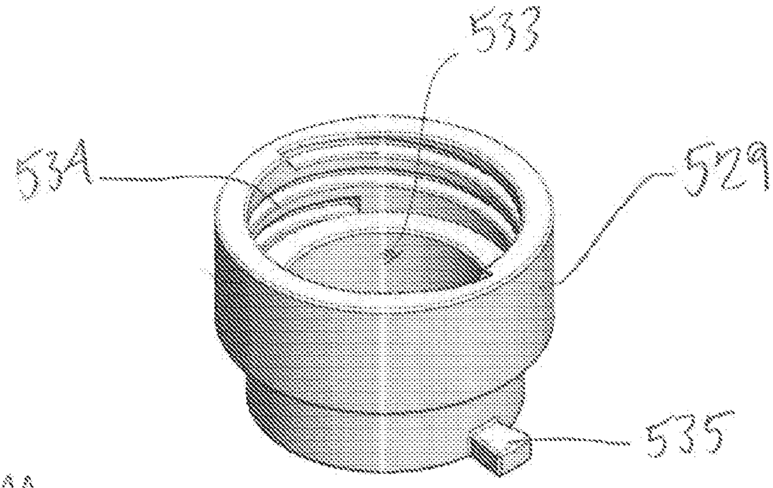


Figure 73

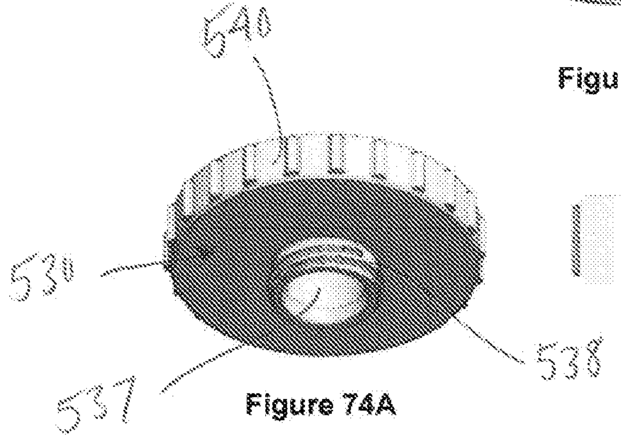


Figure 74A

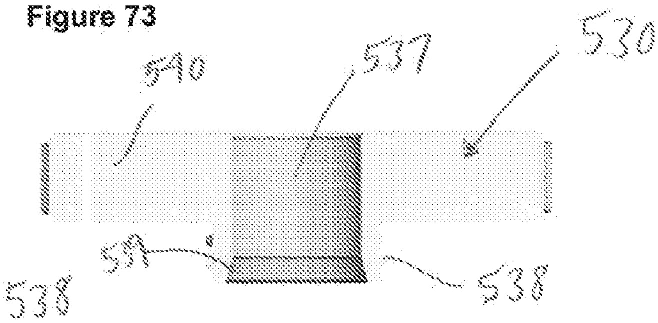


Figure 74B

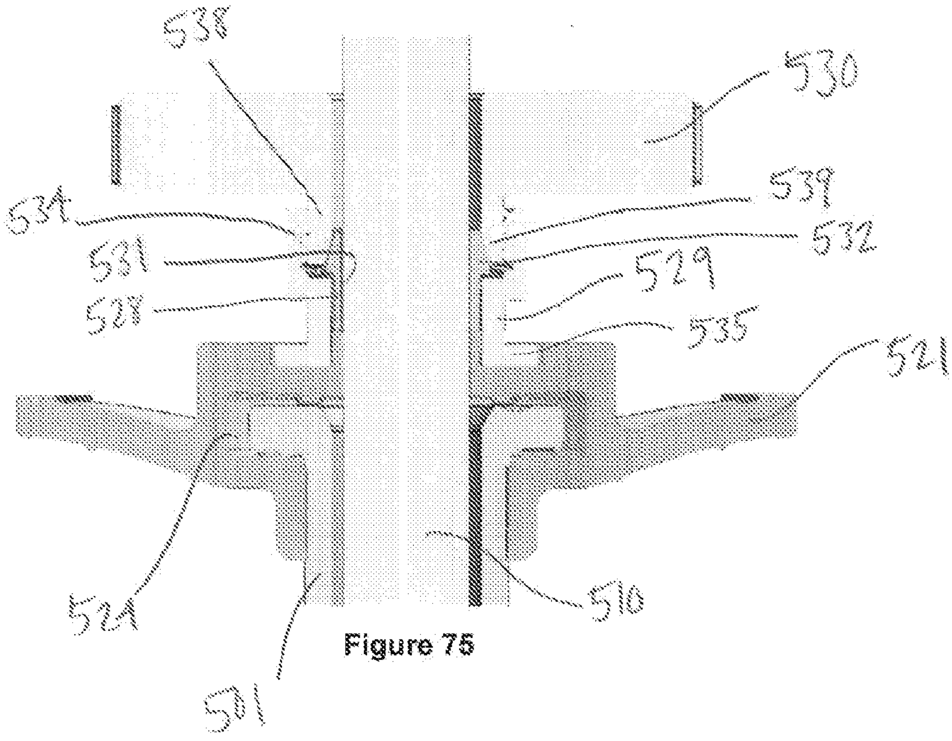


Figure 75

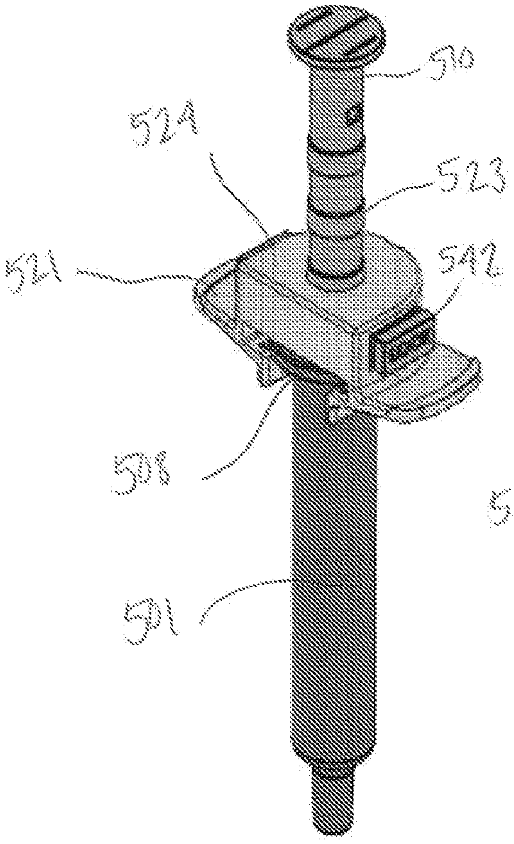

Figure 76

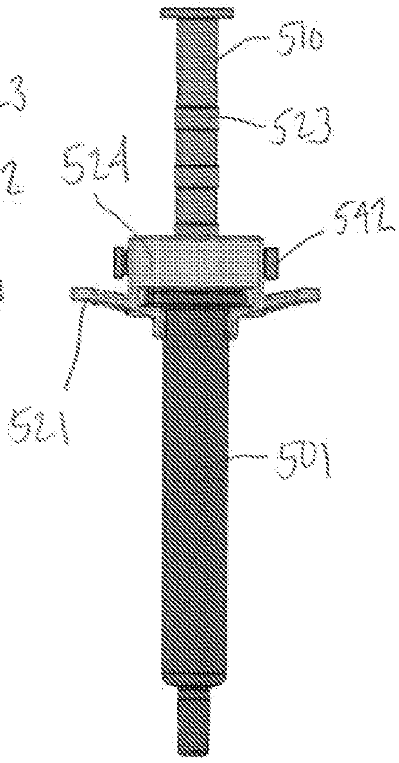

Figure 77

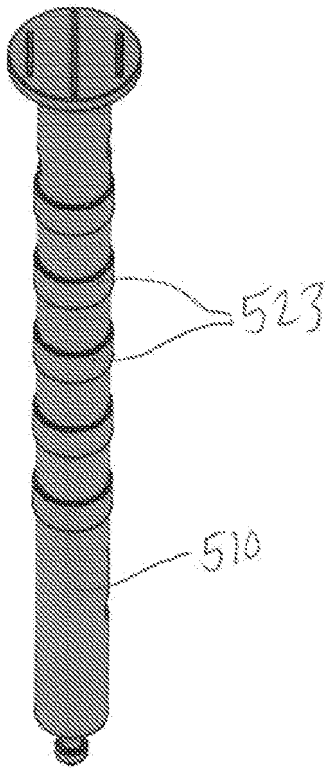

Figure 78

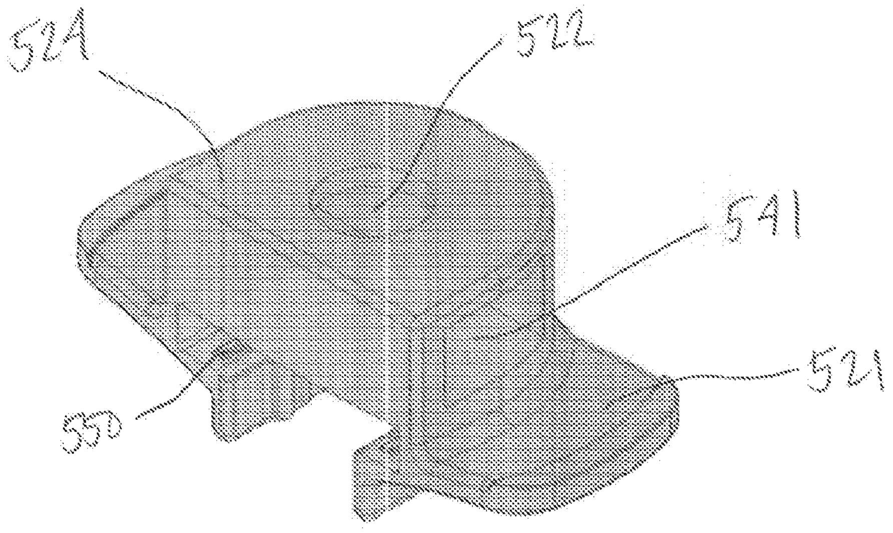


Figure 79

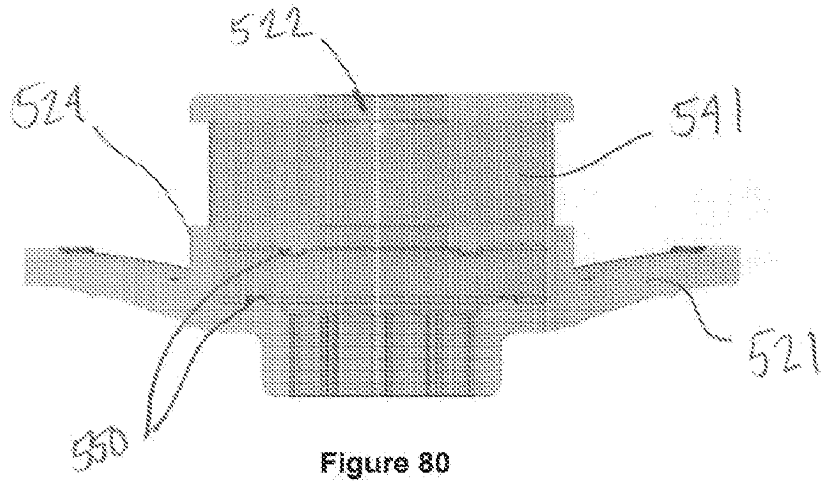


Figure 80

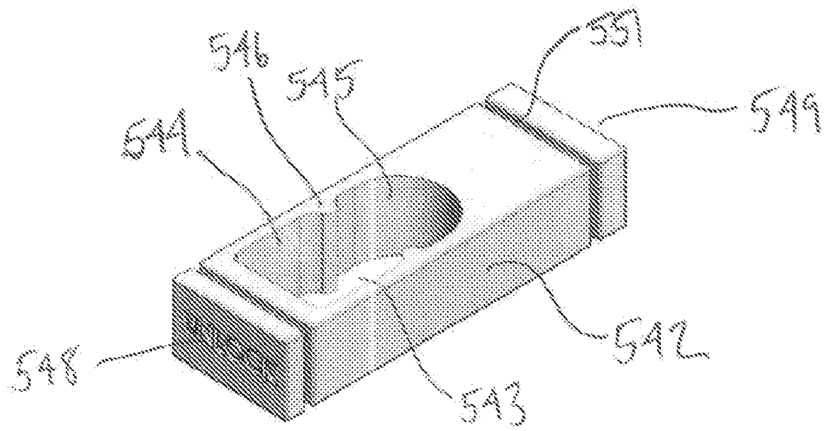


Figure 81

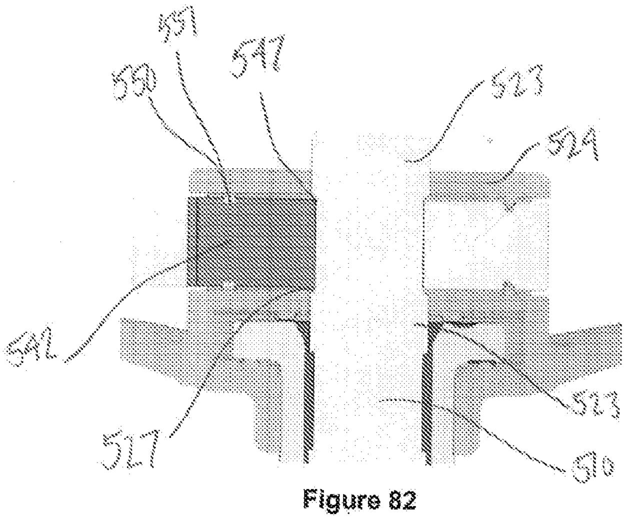


Figure 82

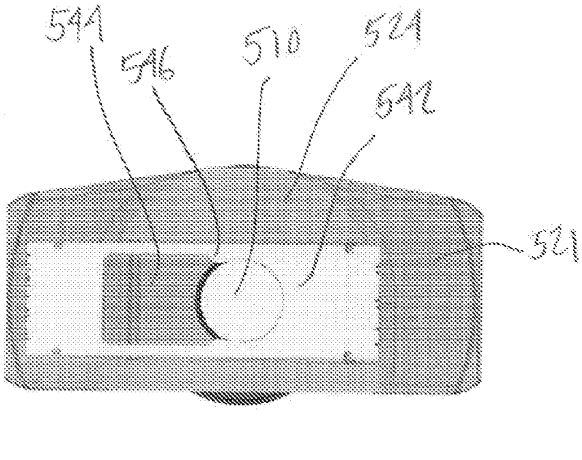


Figure 83

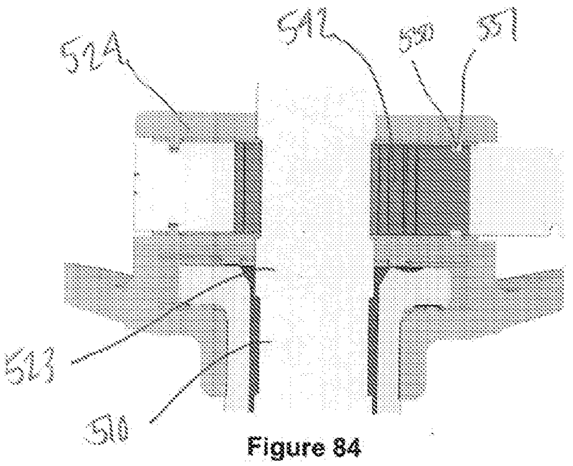


Figure 84

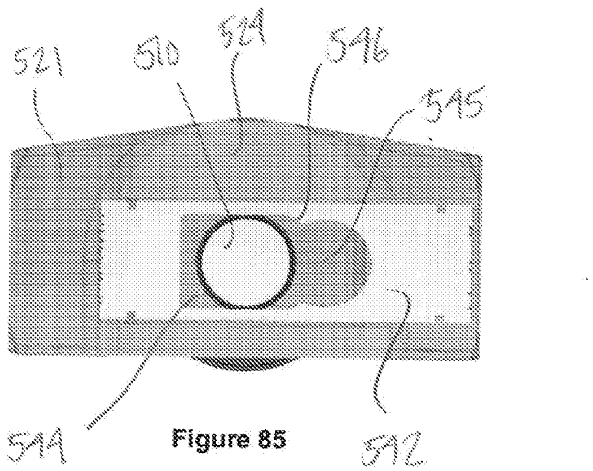


Figure 85

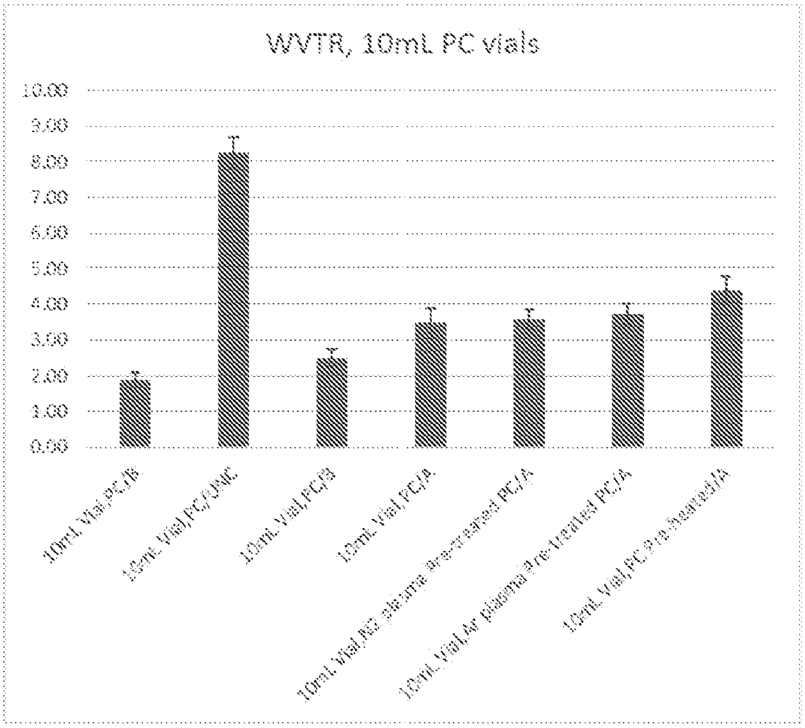


Figure 86

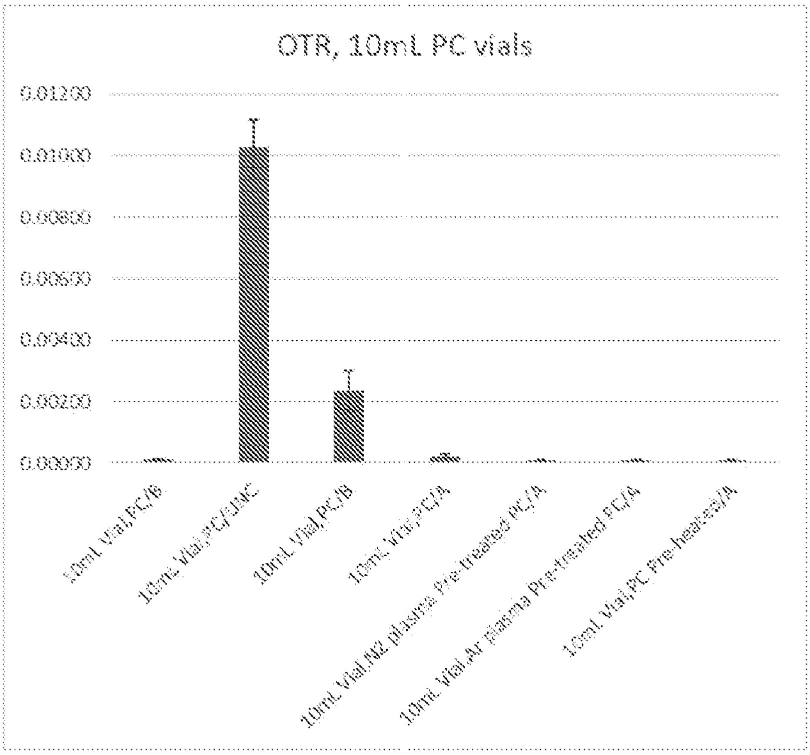


Figure 87

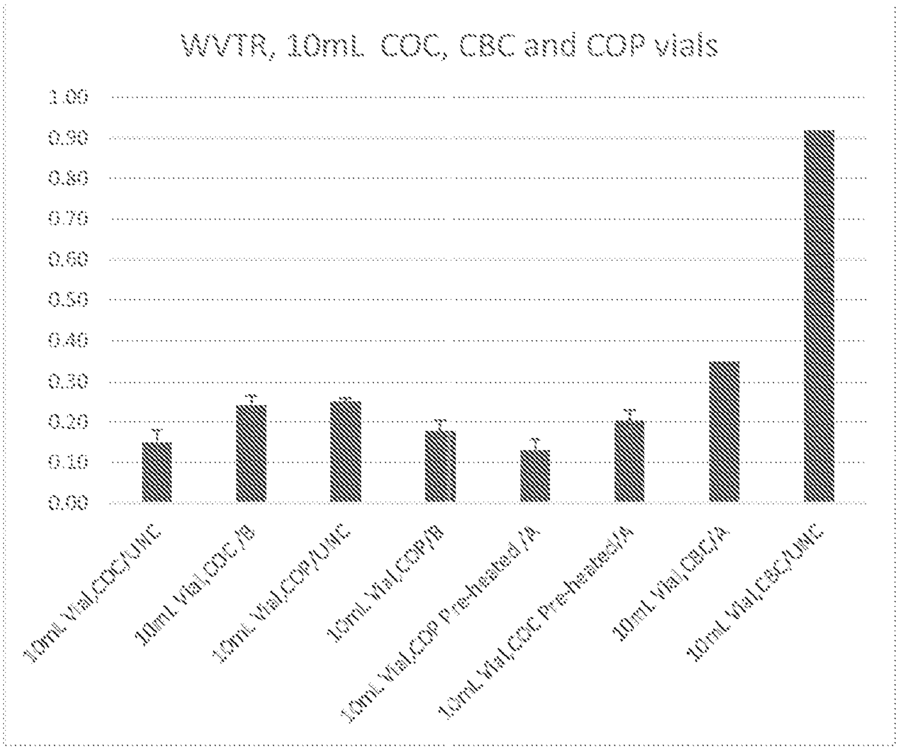


Figure 88

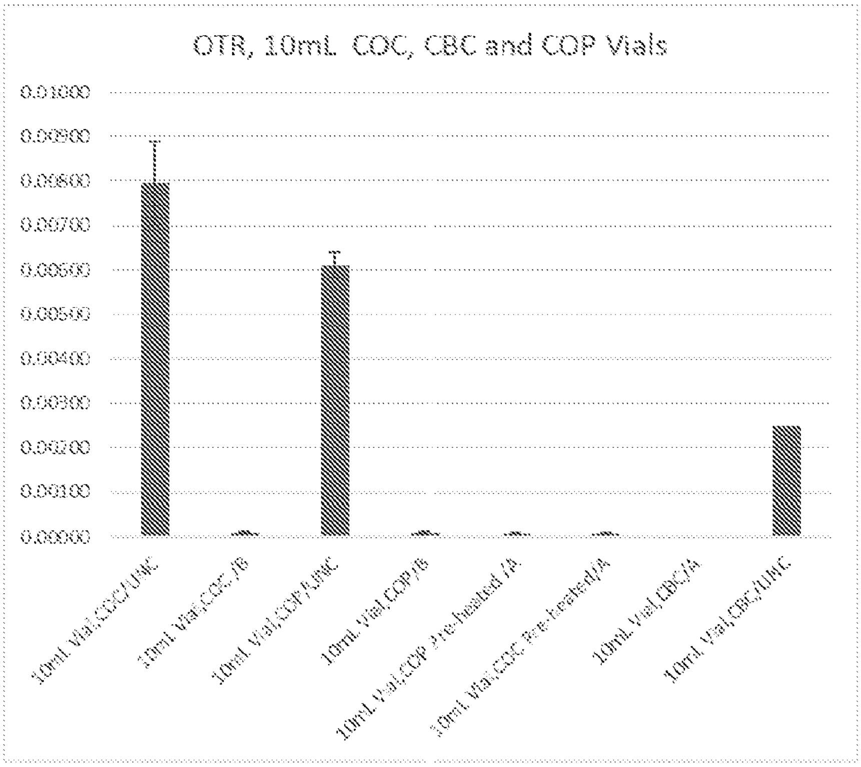


Figure 89

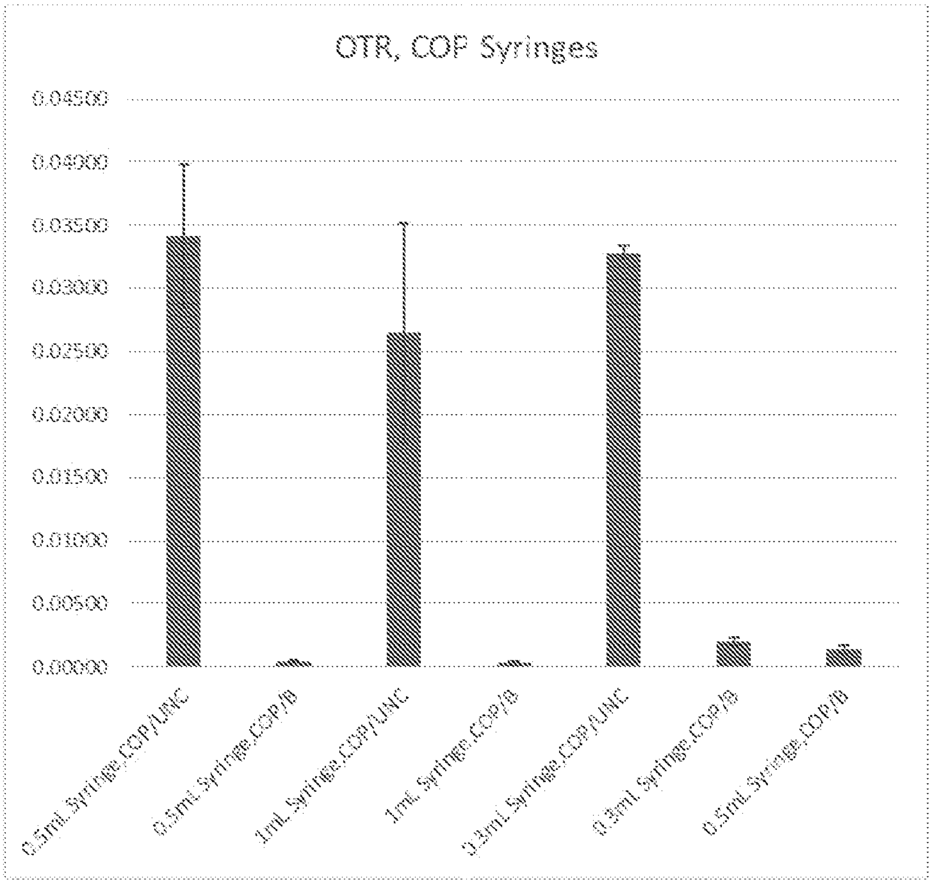
OTR, COP Syringes
0.04500
0.04000
0.03500
0.03000
0.02500
0.02000
0.01500
0.01000
0.00500
0.00000
0.5mL Syringe.COP/UNC
0.5mL Syringe.COP/B
1mL Syringe.COP/UNC
1mL Syringe.COP/B
0.3mL Syringe.COP/UNC
0.3mL Syringe.COP/B
0.5mL Syringe.COP/B

Figure 90

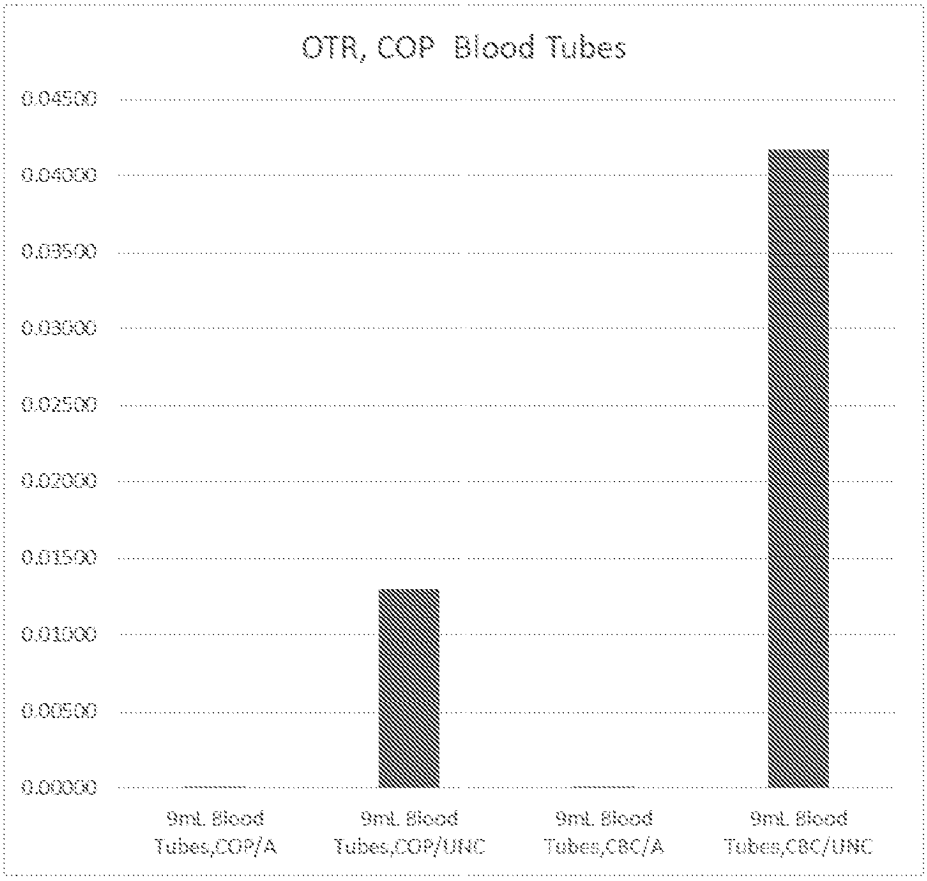


**Figure 91**

# Blood Tubes WVTR

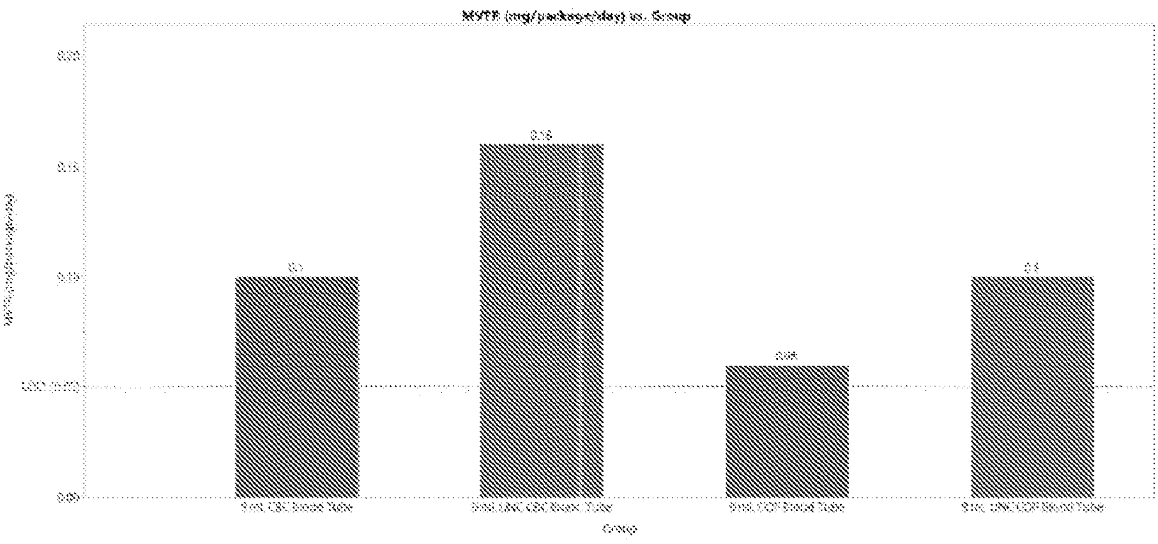


**Figure 92**

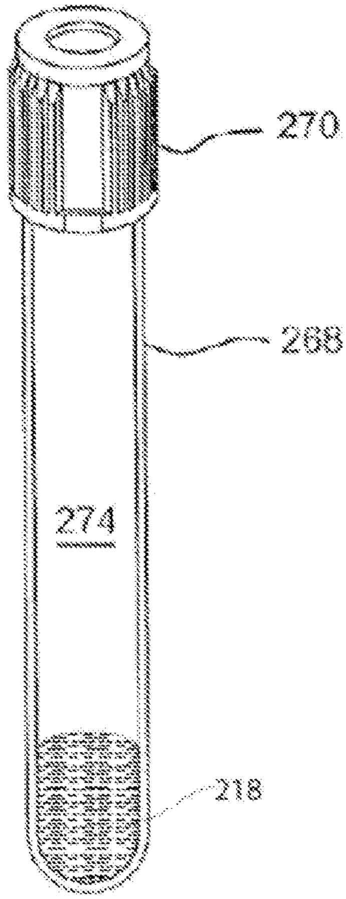


Figure 93

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2015290080 A1
- US 2014251859 A1
- US 2015335823 A1
- EP 3437743 A1
- US 8067070 B
- EP 2251671 A2
- US 7985188 B
- WO 2014130349 A

### Non-patent literature cited in the description


- **VICTOR KG** ; **LEVAC L** ; **TIMMINS M** ; **VEALE, J.** Method Development for Container Closure Integrity Evaluation via Headspace Gas Ingress by Using Frequency Modulation Spectroscopy. *J Pharm. Sci. Technol.*, November 2017, vol. 71 (6), 429-453